(19)

Europäisches
Patentamt
European
Patent Office
Office européen
des brevets

(11)     **EP 4 075 142 A1**

(12)     **EUROPEAN PATENT APPLICATION**

(43) Date of publication:
**19.10.2022 Bulletin 2022/42**

(21) Application number: **21168225.7**

(22) Date of filing: **13.04.2021**

(51) International Patent Classification (IPC):
***G01N 33/68*** *(2006.01)*

(52) Cooperative Patent Classification (CPC):
**G01N 33/6893;** G01N 2333/70503; G01N 2800/26

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Universitätsklinikum Jena
07747 Jena (DE)**

(72) Inventors:
- **Slevogt, Hortense
  07743 Jena (DE)**
- **Müller, Mario
  07743 Jena (DE)**

(74) Representative: **König Szynka Tilmann von Renesse
Patentanwälte Partnerschaft mbB Düsseldorf
Mönchenwerther Straße 11
40545 Düsseldorf (DE)**

(54)     **BIOMARKER FOR IN VITRO DIAGNOSIS AND/OR PROGNOSIS OF A SYSTEMIC INFLAMMATION**

(57)     The present invention relates to the field of *in vitro* diagnosis of a systemic inflammation or prognosis of a risk of mortality of a subject with a systemic inflammation. In another aspect, the invention relates to the field of monitoring a systemic inflammation. The invention further relates to the use of a biomarker for *in vitro* diagnosing a systemic inflammation in a subject or prognosing a risk of mortality of a subject with a systemic inflam- mation. In particular, the biomarker is soluble V-set and immunoglobulin domain-containing protein 4 (sVSIG4). Preferably, the systemic inflammation is caused by an infectious agent, more preferably is a sepsis.

EP 4 075 142 A1

SEQUENCE LISTING

<110>    Universitätsklinikum Jena

<120>    BIOMARKER FOR IN VITRO DIAGNOSIS AND/OR PROGNOSIS OF A SYSTEMIC
         INFLAMMATION

<130>    62 060 K

<160>    18

<170>    PatentIn version 3.5

<210>    1
<211>    399
<212>    PRT
<213>    Homo sapiens

<400>    1

Met Gly Ile Leu Leu Gly Leu Leu Leu Leu Gly His Leu Thr Val Asp
1               5                   10                  15


Thr Tyr Gly Arg Pro Ile Leu Glu Val Pro Glu Ser Val Thr Gly Pro
            20                  25                  30


Trp Lys Gly Asp Val Asn Leu Pro Cys Thr Tyr Asp Pro Leu Gln Gly
        35                  40                  45


Tyr Thr Gln Val Leu Val Lys Trp Leu Val Gln Arg Gly Ser Asp Pro
        50                  55                  60


Val Thr Ile Phe Leu Arg Asp Ser Ser Gly Asp His Ile Gln Gln Ala
65                  70                  75                  80


Lys Tyr Gln Gly Arg Leu His Val Ser His Lys Val Pro Gly Asp Val
                85                  90                  95


Ser Leu Gln Leu Ser Thr Leu Glu Met Asp Asp Arg Ser His Tyr Thr
                100                 105                 110


Cys Glu Val Thr Trp Gln Thr Pro Asp Gly Asn Gln Val Val Arg Asp
            115                 120                 125


Lys Ile Thr Glu Leu Arg Val Gln Lys Leu Ser Val Ser Lys Pro Thr
        130                 135                 140


Val Thr Thr Gly Ser Gly Tyr Gly Phe Thr Val Pro Gln Gly Met Arg
145                 150                 155                 160


Ile Ser Leu Gln Cys Gln Ala Arg Gly Ser Pro Pro Ile Ser Tyr Ile
                165                 170                 175

```
Trp Tyr Lys Gln Gln Thr Asn Asn Gln Glu Pro Ile Lys Val Ala Thr
        180             185             190

Leu Ser Thr Leu Leu Phe Lys Pro Ala Val Ile Ala Asp Ser Gly Ser
        195             200             205

Tyr Phe Cys Thr Ala Lys Gly Gln Val Gly Ser Glu Gln His Ser Asp
        210             215             220

Ile Val Lys Phe Val Val Lys Asp Ser Ser Lys Leu Leu Lys Thr Lys
225             230             235             240

Thr Glu Ala Pro Thr Thr Met Thr Tyr Pro Leu Lys Ala Thr Ser Thr
            245             250             255

Val Lys Gln Ser Trp Asp Trp Thr Thr Asp Met Asp Gly Tyr Leu Gly
        260             265             270

Glu Thr Ser Ala Gly Pro Gly Lys Ser Leu Pro Val Phe Ala Ile Ile
        275             280             285

Leu Ile Ile Ser Leu Cys Cys Met Val Val Phe Thr Met Ala Tyr Ile
        290             295             300

Met Leu Cys Arg Lys Thr Ser Gln Gln Glu His Val Tyr Glu Ala Ala
305             310             315             320

Arg Ala His Ala Arg Glu Ala Asn Asp Ser Gly Glu Thr Met Arg Val
            325             330             335

Ala Ile Phe Ala Ser Gly Cys Ser Ser Asp Glu Pro Thr Ser Gln Asn
            340             345             350

Leu Gly Asn Asn Tyr Ser Asp Glu Pro Cys Ile Gly Gln Glu Tyr Gln
        355             360             365

Ile Ile Ala Gln Ile Asn Gly Asn Tyr Ala Arg Leu Leu Asp Thr Val
        370             375             380

Pro Leu Asp Tyr Glu Phe Leu Ala Thr Glu Gly Lys Ser Val Cys
385             390             395
```

```
<210>  2
<211>  321
<212>  PRT
<213>  Homo sapiens

<400>  2
```

```
Met Gly Ile Leu Leu Gly Leu Leu Leu Leu Gly His Leu Thr Val Asp
1               5               10              15

Thr Tyr Gly Arg Pro Ile Leu Glu Val Pro Glu Ser Val Thr Gly Pro
            20              25              30

Trp Lys Gly Asp Val Asn Leu Pro Cys Thr Tyr Asp Pro Leu Gln Gly
            35              40              45

Tyr Thr Gln Val Leu Val Lys Trp Leu Val Gln Arg Gly Ser Asp Pro
        50              55              60

Val Thr Ile Phe Leu Arg Asp Ser Ser Gly Asp His Ile Gln Gln Ala
65              70              75              80

Lys Tyr Gln Gly Arg Leu His Val Ser His Lys Val Pro Gly Asp Val
            85              90              95

Ser Leu Gln Leu Ser Thr Leu Glu Met Asp Asp Arg Ser His Tyr Thr
            100             105             110

Cys Glu Val Thr Trp Gln Thr Pro Asp Gly Asn Gln Val Val Arg Asp
            115             120             125

Lys Ile Thr Glu Leu Arg Val Gln Lys Leu Ser Val Ser Lys Pro Thr
    130             135             140

Val Thr Thr Gly Ser Gly Tyr Gly Phe Thr Val Pro Gln Gly Met Arg
145             150             155             160

Ile Ser Leu Gln Cys Gln Ala Arg Gly Ser Pro Pro Ile Ser Tyr Ile
            165             170             175

Trp Tyr Lys Gln Gln Thr Asn Asn Gln Glu Pro Ile Lys Val Ala Thr
            180             185             190

Leu Ser Thr Leu Leu Phe Lys Pro Ala Val Ile Ala Asp Ser Gly Ser
    195             200             205

Tyr Phe Cys Thr Ala Lys Gly Gln Val Gly Ser Glu Gln His Ser Asp
    210             215             220

Ile Val Lys Phe Val Val Lys Asp Ser Ser Lys Leu Leu Lys Thr Lys
225             230             235             240

Thr Glu Ala Pro Thr Thr Met Thr Tyr Pro Leu Lys Ala Thr Ser Thr
            245             250             255
```

```
Val Lys Gln Ser Trp Asp Trp Thr Thr Asp Met Asp Gly Tyr Leu Gly
            260             265             270

Glu Thr Ser Ala Gly Pro Gly Lys Ser Leu Pro Val Phe Ala Ile Ile
            275             280             285

Leu Ile Ile Ser Leu Cys Cys Met Val Val Phe Thr Met Ala Tyr Ile
            290             295             300

Met Leu Cys Arg Lys Thr Ser Gln Gln Glu His Val Tyr Glu Ala Ala
305             310             315             320

Arg
```

<210> 3
<211> 305
<212> PRT
<213> Homo sapiens

<400> 3

```
Met Gly Ile Leu Leu Gly Leu Leu Leu Leu Gly His Leu Thr Val Asp
1               5               10              15

Thr Tyr Gly Arg Pro Ile Leu Glu Val Pro Glu Ser Val Thr Gly Pro
            20              25              30

Trp Lys Gly Asp Val Asn Leu Pro Cys Thr Tyr Asp Pro Leu Gln Gly
            35              40              45

Tyr Thr Gln Val Leu Val Lys Trp Leu Val Gln Arg Gly Ser Asp Pro
            50              55              60

Val Thr Ile Phe Leu Arg Asp Ser Ser Gly Asp His Ile Gln Gln Ala
65              70              75              80

Lys Tyr Gln Gly Arg Leu His Val Ser His Lys Val Pro Gly Asp Val
            85              90              95

Ser Leu Gln Leu Ser Thr Leu Glu Met Asp Asp Arg Ser His Tyr Thr
            100             105             110

Cys Glu Val Thr Trp Gln Thr Pro Asp Gly Asn Gln Val Val Arg Asp
            115             120             125

Lys Ile Thr Glu Leu Arg Val Gln Lys His Ser Ser Lys Leu Leu Lys
            130             135             140
```

Thr Lys Thr Glu Ala Pro Thr Thr Met Thr Tyr Pro Leu Lys Ala Thr
145             150             155             160

Ser Thr Val Lys Gln Ser Trp Asp Trp Thr Thr Asp Met Asp Gly Tyr
                165             170             175

Leu Gly Glu Thr Ser Ala Gly Pro Gly Lys Ser Leu Pro Val Phe Ala
            180             185             190

Ile Ile Leu Ile Ile Ser Leu Cys Cys Met Val Val Phe Thr Met Ala
            195             200             205

Tyr Ile Met Leu Cys Arg Lys Thr Ser Gln Gln Glu His Val Tyr Glu
    210             215             220

Ala Ala Arg Ala His Ala Arg Glu Ala Asn Asp Ser Gly Glu Thr Met
225             230             235             240

Arg Val Ala Ile Phe Ala Ser Gly Cys Ser Ser Asp Glu Pro Thr Ser
            245             250             255

Gln Asn Leu Gly Asn Asn Tyr Ser Asp Glu Pro Cys Ile Gly Gln Glu
            260             265             270

Tyr Gln Ile Ile Ala Gln Ile Asn Gly Asn Tyr Ala Arg Leu Leu Asp
            275             280             285

Thr Val Pro Leu Asp Tyr Glu Phe Leu Ala Thr Glu Gly Lys Ser Val
    290             295             300

Cys
305

<210> 4
<211> 111
<212> PRT
<213> Homo sapiens

<400> 4

Pro Ile Leu Glu Val Pro Glu Ser Val Thr Gly Pro Trp Lys Gly Asp
1               5               10              15

Val Asn Leu Pro Cys Thr Tyr Asp Pro Leu Gln Gly Tyr Thr Gln Val
            20              25              30

Leu Val Lys Trp Leu Val Gln Arg Gly Ser Asp Pro Val Thr Ile Phe
            35              40              45

```
Leu Arg Asp Ser Ser Gly Asp His Ile Gln Gln Ala Lys Tyr Gln Gly
    50                  55              60

Arg Leu His Val Ser His Lys Val Pro Gly Asp Val Ser Leu Gln Leu
65                  70              75                  80

Ser Thr Leu Glu Met Asp Asp Arg Ser His Tyr Thr Cys Glu Val Thr
                85                  90                  95

Trp Gln Thr Pro Asp Gly Asn Gln Val Val Arg Asp Lys Ile Thr
            100             105             110
```

```
<210>   5
<211>   84
<212>   PRT
<213>   Homo sapiens

<400>   5
```

```
Pro Thr Val Thr Thr Gly Ser Gly Tyr Gly Phe Thr Val Pro Gln Gly
1               5               10              15

Met Arg Ile Ser Leu Gln Cys Gln Ala Arg Gly Ser Pro Pro Ile Ser
            20              25              30

Tyr Ile Trp Tyr Lys Gln Gln Thr Asn Asn Gln Glu Pro Ile Lys Val
            35              40              45

Ala Thr Leu Ser Thr Leu Leu Phe Lys Pro Ala Val Ile Ala Asp Ser
    50                  55                  60

Gly Ser Tyr Phe Cys Thr Ala Lys Gly Gln Val Gly Ser Glu Gln His
65                  70                  75                  80

Ser Asp Ile Val
```

```
<210>   6
<211>   264
<212>   PRT
<213>   Homo sapiens

<400>   6
```

```
Arg Pro Ile Leu Glu Val Pro Glu Ser Val Thr Gly Pro Trp Lys Gly
1               5               10              15

Asp Val Asn Leu Pro Cys Thr Tyr Asp Pro Leu Gln Gly Tyr Thr Gln
            20              25              30
```

```
Val Leu Val Lys Trp Leu Val Gln Arg Gly Ser Asp Pro Val Thr Ile
        35                  40                  45

Phe Leu Arg Asp Ser Ser Gly Asp His Ile Gln Gln Ala Lys Tyr Gln
        50                  55                  60

Gly Arg Leu His Val Ser His Lys Val Pro Gly Asp Val Ser Leu Gln
65                  70                  75                  80

Leu Ser Thr Leu Glu Met Asp Asp Arg Ser His Tyr Thr Cys Glu Val
                85                  90                  95

Thr Trp Gln Thr Pro Asp Gly Asn Gln Val Val Arg Asp Lys Ile Thr
                100                 105                 110

Glu Leu Arg Val Gln Lys Leu Ser Val Ser Lys Pro Thr Val Thr Thr
        115                 120                 125

Gly Ser Gly Tyr Gly Phe Thr Val Pro Gln Gly Met Arg Ile Ser Leu
    130                 135                 140

Gln Cys Gln Ala Arg Gly Ser Pro Pro Ile Ser Tyr Ile Trp Tyr Lys
145                 150                 155                 160

Gln Gln Thr Asn Asn Gln Glu Pro Ile Lys Val Ala Thr Leu Ser Thr
                165                 170                 175

Leu Leu Phe Lys Pro Ala Val Ile Ala Asp Ser Gly Ser Tyr Phe Cys
                180                 185                 190

Thr Ala Lys Gly Gln Val Gly Ser Glu Gln His Ser Asp Ile Val Lys
                195                 200                 205

Phe Val Val Lys Asp Ser Ser Lys Leu Leu Lys Thr Lys Thr Glu Ala
        210                 215                 220

Pro Thr Thr Met Thr Tyr Pro Leu Lys Ala Thr Ser Thr Val Lys Gln
225                 230                 235                 240

Ser Trp Asp Trp Thr Thr Asp Met Asp Gly Tyr Leu Gly Glu Thr Ser
                245                 250                 255

Ala Gly Pro Gly Lys Ser Leu Pro
                260
```

<210> 7
<211> 21

195

<212>    PRT
<213>    Homo sapiens

<400>    7

Val Phe Ala Ile Ile Leu Ile Ile Ser Leu Cys Cys Met Val Val Phe
1               5                   10                  15

Thr Met Ala Tyr Ile
                20

<210>    8
<211>    95
<212>    PRT
<213>    Homo sapiens

<400>    8

Met Leu Cys Arg Lys Thr Ser Gln Gln Glu His Val Tyr Glu Ala Ala
1               5                   10                  15

Arg Ala His Ala Arg Glu Ala Asn Asp Ser Gly Glu Thr Met Arg Val
            20                  25                  30

Ala Ile Phe Ala Ser Gly Cys Ser Ser Asp Glu Pro Thr Ser Gln Asn
            35                  40                  45

Leu Gly Asn Asn Tyr Ser Asp Glu Pro Cys Ile Gly Gln Glu Tyr Gln
        50                  55                  60

Ile Ile Ala Gln Ile Asn Gly Asn Tyr Ala Arg Leu Leu Asp Thr Val
65                  70                  75                  80

Pro Leu Asp Tyr Glu Phe Leu Ala Thr Glu Gly Lys Ser Val Cys
                85                  90                  95

<210>    9
<211>    21
<212>    PRT
<213>    Homo sapiens

<400>    9

Gly Asp Val Asn Leu Pro Cys Thr Tyr Asp Pro Leu Gln Gly Tyr Thr
1               5                   10                  15

Gln Val Leu Val Lys
                20

<210>    10
<211>    10
<212>    PRT
<213>    Homo sapiens

<400> 10

Gly Ser Asp Pro Val Thr Ile Phe Leu Arg
1               5               10

<210> 11
<211> 17
<212> PRT
<213> Homo sapiens

<400> 11

Val Pro Gly Asp Val Ser Leu Gln Leu Ser Thr Leu Glu Met Asp Asp
1               5               10                  15

Arg

<210> 12
<211> 19
<212> PRT
<213> Homo sapiens

<400> 12

Ser His Tyr Thr Cys Glu Val Thr Trp Gln Thr Pro Asp Gly Asn Gln
1               5               10                  15

Val Val Arg

<210> 13
<211> 23
<212> PRT
<213> Homo sapiens

<400> 13

Leu Ser Val Ser Lys Pro Thr Val Thr Thr Gly Ser Gly Tyr Gly Phe
1               5               10                  15

Thr Val Pro Gln Gly Met Arg
                20

<210> 14
<211> 18
<212> PRT
<213> Homo sapiens

<400> 14

Pro Thr Val Thr Thr Gly Ser Gly Tyr Gly Phe Thr Val Pro Gln Gly
1               5               10                  15

```
Met Arg


<210>  15
<211>  8
<212>  PRT
<213>  Homo sapiens

<400>  15

Ile Ser Leu Gln Cys Gln Ala Arg
1               5


<210>  16
<211>  11
<212>  PRT
<213>  Homo sapiens

<400>  16

Gly Ser Pro Pro Ile Ser Tyr Ile Trp Tyr Lys
1               5               10


<210>  17
<211>  10
<212>  PRT
<213>  Homo sapiens

<400>  17

Val Ala Thr Leu Ser Thr Leu Leu Phe Lys
1               5               10


<210>  18
<211>  15
<212>  PRT
<213>  Homo sapiens

<400>  18

Pro Ala Val Ile Ala Asp Ser Gly Ser Tyr Phe Cys Thr Ala Lys
1               5               10              15
```

**Description**

**FIELD OF THE INVENTION**

The present invention relates to the field of *in vitro* diagnosis of a systemic inflammation or prognosis of a risk of mortality of a subject with a systemic inflammation. In another aspect, the invention relates to the field of monitoring a systemic inflammation. The invention further relates to the use of a biomarker for *in vitro* diagnosing a systemic inflammation in a subject or prognosing a risk of mortality of a subject with a systemic inflammation. Preferably, the systemic inflammation is caused by an infectious agent, more preferably is a sepsis.

The methods according to the invention also relate to the field of decision making processes regarding therapeutic interventions in subjects, in particular human subjects, suffering from a systemic inflammation, in particular from sepsis.

**BACKGROUND OF THE INVENTION**

Systemic inflammations, in particular systemic inflammations caused by an infectious agent such as sepsis, represent a significant cause of mortality throughout the world. Specifically, sepsis is the third most common cause of death in Germany and other developed countries.

Sepsis typically occurs when pathogens, or the toxins they produce, spread from a localized site of inflammation throughout the body via the circulation, reaching distant organs and triggering a systemic inflammation. Systemic inflammation can lead to the failure of individual or several organs as well as multi-organ failure and, in the case of an additional severe drop in blood pressure, to septic shock. Hence, sepsis, severe sepsis or septic shock are typically caused by an infectious agent.

Septic shock is associated with a high lethality. An early diagnosis of a systemic inflammation, in particular of sepsis, followed by adequate therapeutic intervention, thus is critical for the therapeutic success and disease outcome.

However, systemic inflammation, in particular sepsis, is difficult to diagnose; an effective monitoring is challenging. Bloodstream infections, in particular bacteremia, and systemic infections are also a great challenge in diagnosis and therapy.

Microbiological methods, such as culturing of patient blood and subsequent identification of pathogens, are very time-consuming and not reliable due to a high incidence of false-negative results. Moreover, the interpretation of microbiological findings in critically ill patients is often problematic, because microorganisms may be detected that may merely represent colonizers, but are not necessarily indicative for a systemic inflammation caused by an infectious agent.

Biophysical detection methods, such as mass spectrometry, in particular matrix-assisted laser desorption ionization-time of flight mass spectrometry (MALDI-TOF MS), can also be used to detect a pathogen in a blood sample based on protein profiles characteristic for each pathogen. However, this method is usually done with blood samples that are pre-cultured at least for a short time. Moreover, this method is typically employed on positive blood cultures, i. e. requiring an additional method step before MALDI-TOF MS (Clin. Microbiol. Infect. 2020; 26:142-150).

By introducing molecular biological methods, such as PCR diagnostics, pathogens can be identified in biological samples in a targeted and partially parallelized manner. Pathogen detection in patient blood samples by PCR analysis is highly sensitive and specific, but sample preparation and measurement is time-consuming and in some cases can only be performed by highly experienced staff. Multiplex PCR can be used to perform multiple pathogen detections in parallel, but the number of measurements that can be performed in parallel is limited. Therefore, negative PCR results cannot exclude a present infection with a non-tested pathogen.

Markers known and used in clinical diagnostics that correlate with the strength of the systemic inflammation or with the severity of the infection are mainly procalcitonin (PCT), C-reactive protein (CRP) and interleukin-6 (IL-6) (Crit. Care 2020; 24(1):287; Anaesthesiol. Intensive Ther. 2019; 51:299-305) among others. PCT is detectable in the plasma of healthy individuals at low concentrations (below 0.1 ng/ml); under conditions of severe sepsis caused by bacteria, PCT concentrations can increase 5,000 to 10,000 fold. The increase in plasma PCT concentration is time-dependent and occurs quite rapidly after infection. PCT is detectable before an increase in CRP concentration, but appears more slowly than the upregulation of cytokines. However, major surgery, polytrauma, and cardiogenic shock may also result in markedly elevated plasma PCT concentrations due to a systemic inflammatory response, reducing the general applicability of PCT as a sepsis marker. In sum, current markers alone and even the combination of PCT and CRP are nonsatisfying for the diagnosis of systemic inflammations, in particular of sepsis, because their positive and negative predictive values are too low.

Other sepsis biomarkers have been described in the literature (Crit. Care 2020; 24(1):287), but most of them have not been fully verified or their validity is severely limited due to cohort studies with small case numbers, individual case studies, or lack of comparisons with PCT and CRP.

In summary, none of the current biomarkers allows for clear and unambiguous conclusions regarding a diagnosis of systemic inflammations, in particular of sepsis.

## SUMMARY OF THE INVENTION

Hence, it is an objective of the present invention to provide a biomarker for diagnosing a systemic inflammation or prognosing a risk of mortality of a subject with a systemic inflammation, in particular a systemic inflammation caused by an infectious agent, in particular a sepsis. It is furthermore an objective of the present invention to provide an improved biomarker or combination of biomarkers for such purposes.

In a particular aspect of the invention, it is an objective to provide a more reliable biomarker than the presently used biomarkers, such as PCT and CRP, specifically in view of the differentiation of systemic inflammation caused by an infectious agent, such as sepsis, and a systemic inflammation which is not caused by an infectious agent, such as SIRS.

The present invention as defined in the claims solves at least one of these objectives. In particular, at least one of these objects is solved by a method of in vitro diagnosing a systemic inflammation or prognosing a risk of mortality of a subject with a systemic inflammation, wherein the method comprises

a) determining the level of soluble V-set and immunoglobulin domain-containing protein 4 (sVSIG4) in a biological sample, and
b) drawing a conclusion as to the diagnosis of a systemic inflammation or the prognosis of a risk of mortality of a subject with a systemic inflammation from the presence and/or level of sVSIG4.

The inventors surprisingly identified that soluble V-set and immunoglobulin-containing protein 4 (sVSIG4), or combinations of sVSIG4 and other proteins and peptides in the biological sample, e.g. whole blood, plasma, and serum, can be used as a sole or supportive diagnostic criterion for a systemic inflammation. In particular, the sVSIG4 or combinations of sVSIG4 and other proteins and peptides can be used for in vitro diagnosis of a systemic inflammation caused by an infectious agent, such as sepsis, systemic infection or bloodstream infection, or a systemic inflammation not caused by an infectious agent, such as SIRS.

Hence, with the method according to the present invention, it is possible to reliably diagnose a systemic inflammation, in particular sepsis, by determining the level of sVSIG4 without the need of any other biomarker or diagnostic tool. The method is sensitive and specific. The method is fast and does not require culturing of a biological sample. It is an unbiased method in that no pre-diagnosis is necessary. It is also possible to combine the diagnosis with other biomarkers or diagnostic tools.

The method is performed in vitro in that it makes use of a biological sample which has previously been taken from the subject, for example a human patient.

The conclusion as to the diagnosis of a systemic inflammation or the prognosis of a risk of mortality from the presence and/or level of sVSIG4 can be drawn on basis of a cut-off level (a threshold) to indicate a systemic inflammation, in particular SIRS, bloodstream infection or sepsis. The conclusion can also be drawn on basis of differential expression between the biological samples of two subjects, for example one healthy subject and one subject with a suspected systemic inflammation, in particular sepsis.

The method allows identifying a subject with a systemic inflammation, in particular sepsis, at an early stage of the disease. This is particularly useful for the therapy decision. In addition, the method allows adapting the therapy in the course of infection if above steps a) and b) are repeated at least one time (monitoring). For example, if a high level of sVSIG4 is detected in a patient that has already been diagnosed with a sepsis, it is likely that a more aggressive therapy, for example in case of a bacterial pathogen as infectious agent a more aggressive antibiotic therapy or a different antibiotic therapy, is necessary.

In addition, the method allows prognosing a risk of mortality of a subject with a systemic inflammation. Thereby, individual patients can be subjected to targeted enhanced monitoring in order to detect complications in the course of the disease at an early stage. The determination of the concentration of sVSIG4 alone, or in combination with one or more other biomarkers, over time is also suitable for monitoring the effect of antimicrobial therapy through an increase or decrease in the level of the biomarkers.

The inventors have also found that biological samples from a subject, such as human plasma, can contain proteins at altered concentrations in the biological sample from patients with severe sepsis or septic shock and patients with systemic inflammatory response syndrome (SIRS) with or without organ dysfunction (collected according to sepsis-2 definition) with significantly different abundance or concentration in the two groups.

Thus, in a further aspect of the invention, a method of distinguishing between SIRS and sepsis in a subject is provided, wherein the method comprises:

a) determining the level of sVSIG4 in a biological sample of said subject, and
b) comparing the level of sVSIG4 in the biological sample with a reference level of sVSIG4 in a biological sample of a subject suffering from SIRS,

wherein an increased level in the biological sample of step a) compared with the reference level of step b) indicates a sepsis in the subject of step a).

In a further aspect, a method of distinguishing between a systemic inflammation not caused by an infectious agent (i. e. a sterile systemic inflammation) and bacteremia in a subject is provided, wherein the method comprises:

a) determining the level of sVSIG4 in a biological sample of said subject, and
b) comparing the level of sVSIG4 in the biological sample with a reference level of sVSIG4 in a biological sample of a subject suffering from sterile systemic inflammation,

wherein an increased level in the biological sample of step a) compared with the reference level of step b) indicates bacteremia in the subject of step a).

According to another aspect of the invention, the invention relates to an antibiotic agent for use in a method of treating an infection in a subject or treating a subject with a suspected infection, wherein the infection is part of a bloodstream infection, systemic infection or sepsis and wherein the bloodstream infection, systemic infection or sepsis is diagnosed or monitored by the level of sVSIG4 in a biological sample. Thereby, therapy can be individualized and adapted according to the subject's need.

According to a further aspect of the invention, sVSIG4 is used as a biomarker for *in vitro* diagnosing a systemic inflammation in a subject or prognosing a risk of mortality of a subject with a systemic inflammation. sVSIG4 can be used as a sole biomarker or as a biomarker in combination with other biomarkers or diagnostic tools.

According to yet a further aspect of the invention, a kit is provided comprising a binding molecule to sVSIG4 and a binding molecule to at least one further biomarker for the quantitative detection of sVSIG4 and the at least one further biomarker.

Other objects, features, advantages and aspects of the present application will become apparent to those skilled in the art from the following description and appended claims. It should be understood, however, that the following description, appended claims, and specific examples, while indicating preferred embodiments of the application, are given by way of illustration only.

**BRIEF DESCRIPTION OF THE FIGURES**

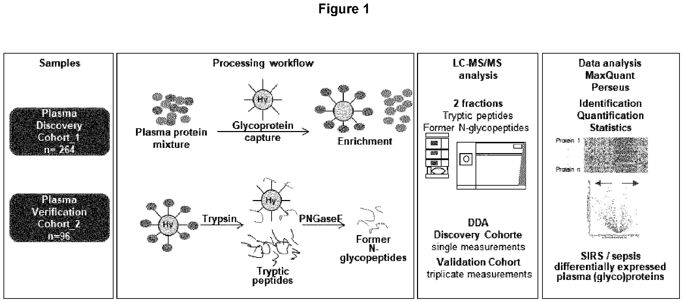

**Fig. 1:** Experimental design. Proteomic workflow for the analysis of plasma samples of SIRS and sepsis patients. Glycoproteins were captured on sepharose beads by hydrazide chemistry, on bead digested with trypsin, and N-glycopeptides were released with PNGaseF. Tryptic peptides and former N-glycopeptides were analyzed individually by LC-MS/MS. Peptide identification and protein inference was done in MaxQuant and statistical analysis was done with Perseus.

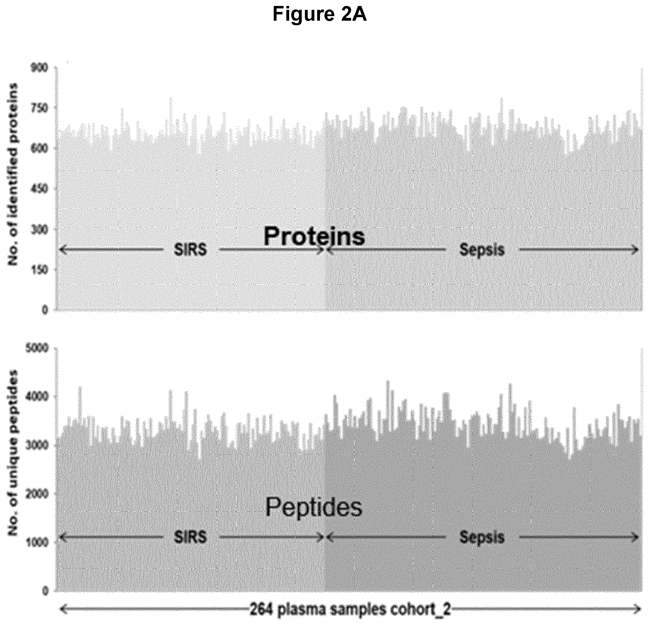

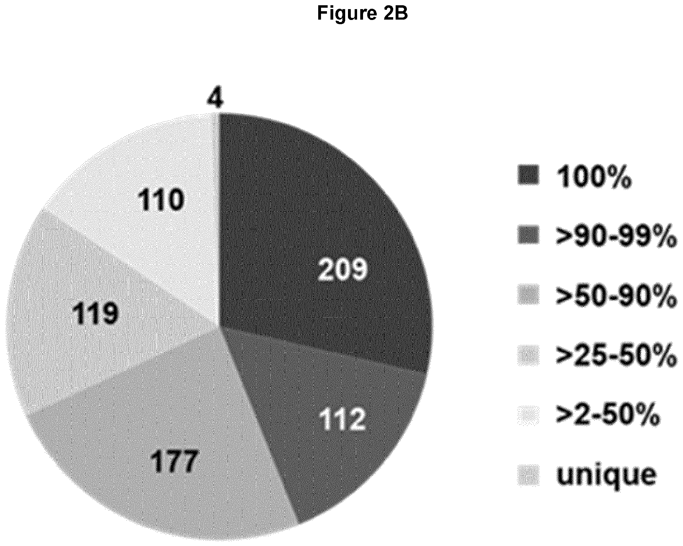

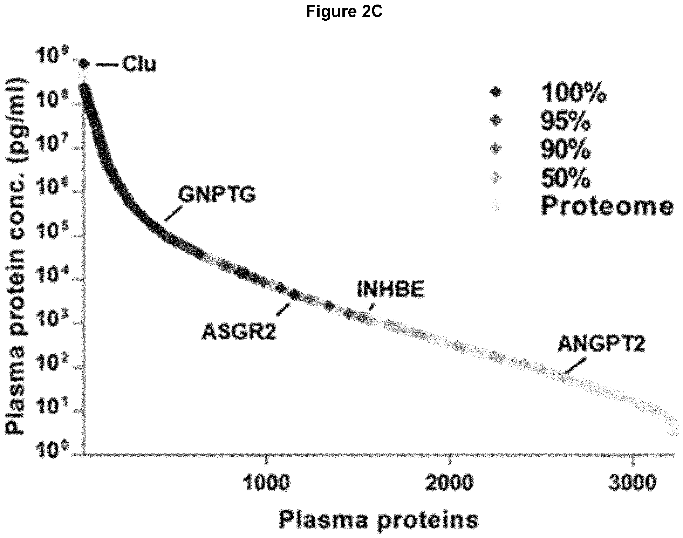

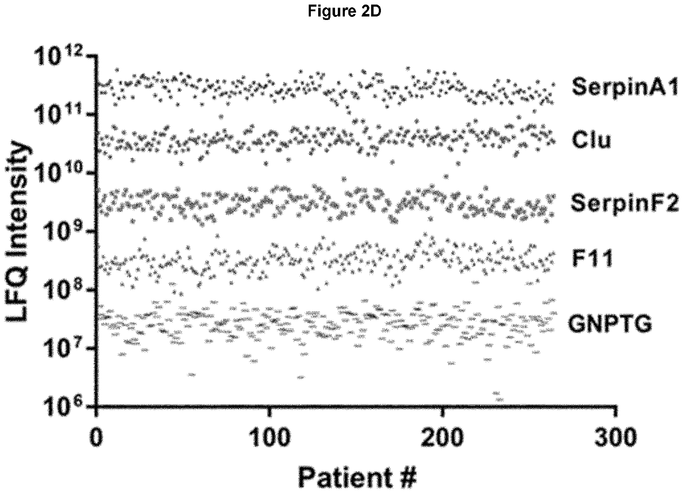

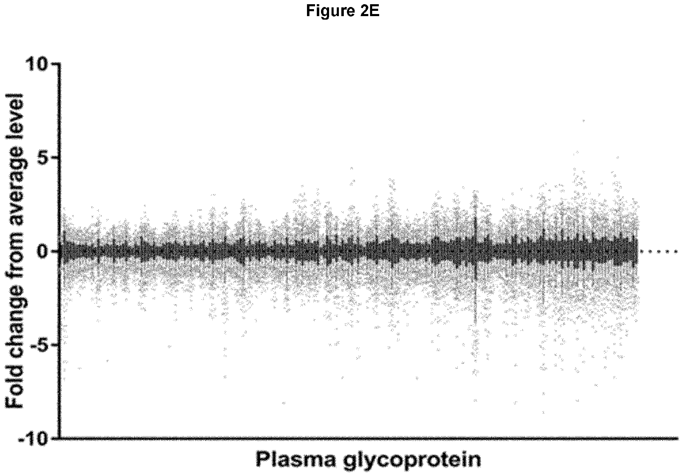

**Fig. 2:** Proteomic identification and reproducible quantification among the patient samples from the discovery cohort. A: Number of peptides and proteins identified in 264 individual plasma samples of patients. B: Frequency of glycoprotein identifications in plasma from SIRS and sepsis patients. C: Plasma protein concentration range in the data set. Plasma proteins identified with 100% (black), 95% (dark grey), 90% (medium grey) and 50% (light grey) reproducibility in the proteomic data set compared to plasma protein concentration data compiled at The Human Protein Atlas (light grey). D: Reproducibility of the LFQ intensities of five proteins detected in 100% of all samples covering nearly five orders of magnitude. Plotted are values of Alpha-1-antitrypsin (A1AT, gene name SERPINA1), Clusterin (CLUS, gene name CLU), Alpha-2-antiplasmin (A2AP, gene name SERPINF2), Coagulation factor XI (FA11, gene name F11), and N-acetylglucosamine-1-phosphotransferase subunit gamma (GNPTG, gene name GNPTG). E: Distribution of the fold changes of glycoprotein LFQ intensities from their average abundance levels across the cohort is shown in box plots and outliers. Shown are glycoproteins identified with 100% reproducibility and sorted according to their average intensity level (decreasing from left to right).

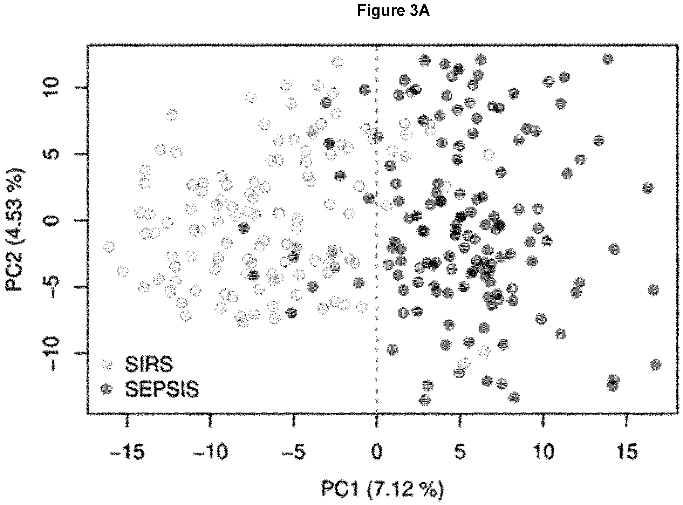

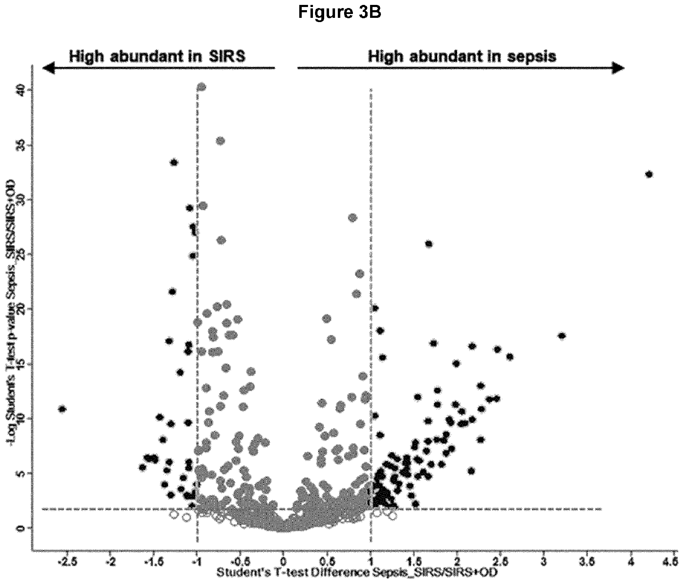

**Fig. 3:** Differential expression of plasma proteins in SIRS and sepsis patients of the discovery cohort. A: Principle component analysis of the proteomic data set. The first component explains 7.12% of the variation in the data set. In light grey: SIRS patients and in dark grey: sepsis patients. B: Volcano plot of differentially abundant proteins in SIRS and sepsis patients. The t-test p-value is plotted against the abundance fold change of all identified proteins with identifications in minimum 10 patients. Data points of the lower center area of the plot (grey, open circles) indicate proteins with unchanged or with no significant fold change, whereas data points in the upper left and upper right quadrants indicate proteins (black and grey filled circles) with significant (FDR less than 0.05 (Benjamini-Hochberg adjusted) lesser abundance in the sepsis group (left) or significantly higher abundance in the sepsis group (right). Black circles display plasma proteins with fold changes ≥2 and grey circles display significant differentially abundant proteins with FC<2.

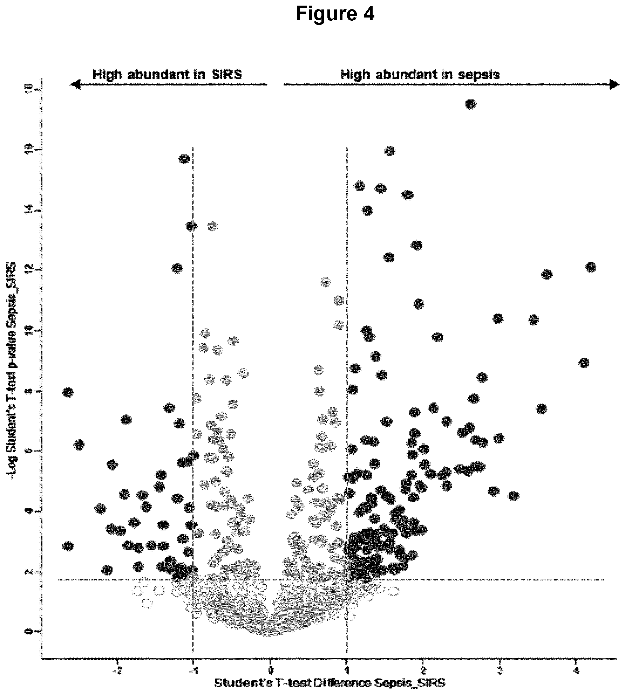

**Fig. 4:** Differential abundance of plasma proteins in SIRS and sepsis patients of the validation cohort. Volcano plot of differentially abundant proteins in SIRS and sepsis patients. The t-test p-value is plotted against the abundance fold change of all identified proteins with identifications in minimum 10 patients. Data points of the lower center area of the plot (grey, open circles) indicate proteins with unchanged or with no significant fold change, whereas data points in the upper left and upper right quadrants indicate proteins (light grey and dark grey filled circles) with significant (FDR less than 0.05 (Benjamini-Hochberg adjusted) lesser abundance in the sepsis group (left) or significant higher abundance in the sepsis group (right). Red circles display plasma proteins with fold changes ≥2 and grey circles display significant differentially abundant proteins with FC<2.

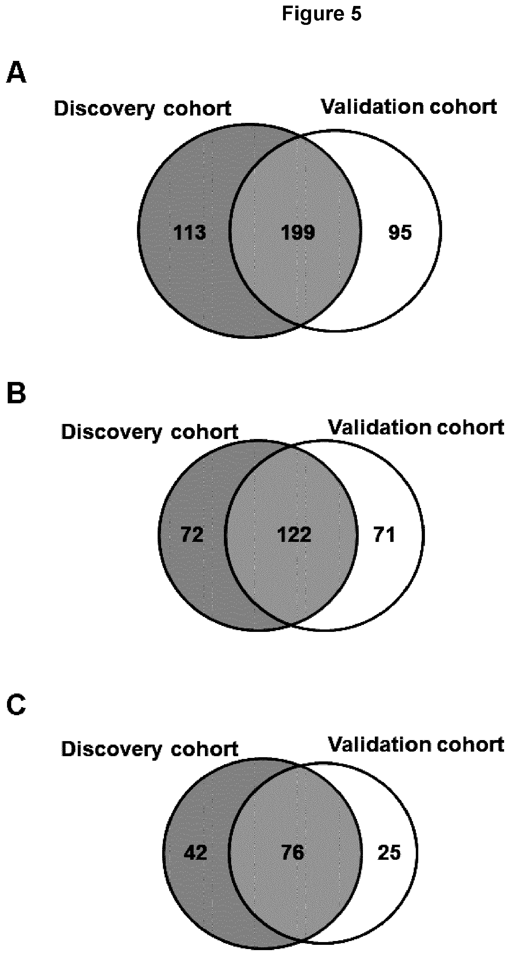

**Fig. 5:** Overlap between significant differentially abundant plasma proteins in SIRS and sepsis patients identified in the discovery cohort (left) and in the validation cohort (right) depicted as Venn diagrams. A: Overlap between all significant plasma proteins identified in both cohorts. B+C: Overlap between plasma proteins with significant higher (B) or lower (C) plasma levels in sepsis patients.

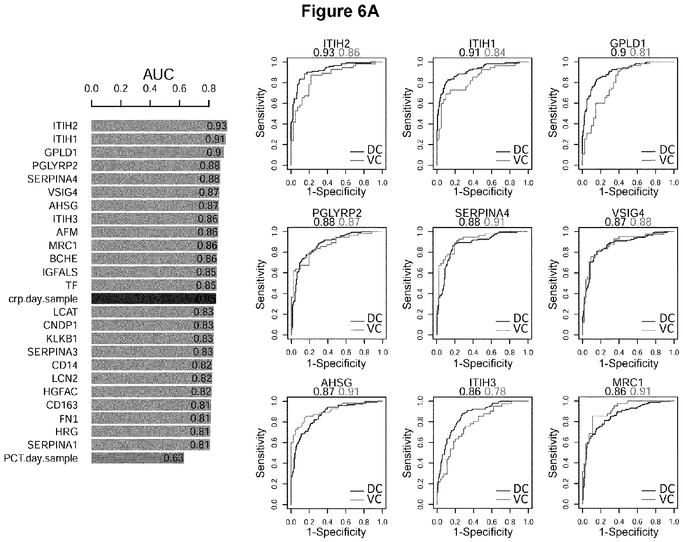

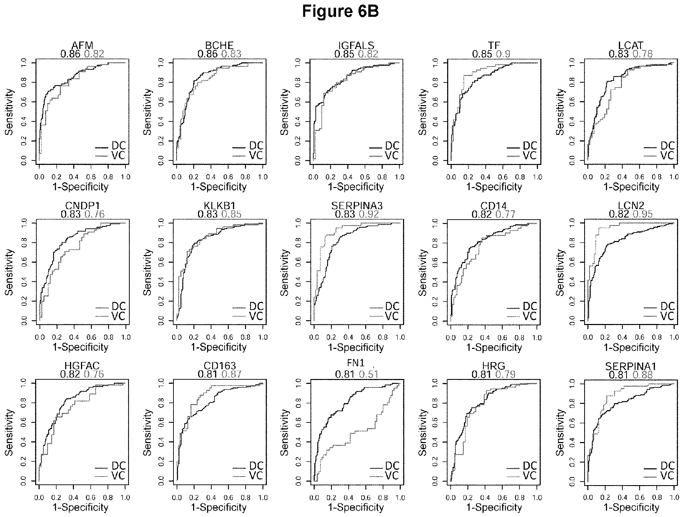

**Fig. 6:** A+B: Receiver operating characteristics curves (ROC) of the 24 best performing plasma proteins in the discovery cohort data set for the discrimination between SIRS and sepsis. A left: bar diagram depicting area under the curve (AUC) values for plasma proteins identified in the proteomic data set (discovery cohort) and PCT and CRP (dark grey) values from the clinic. Plots depict ROC curves for the given proteins and their characteristics in the discovery cohort (DC, black) and validation cohort (VC, grey). For simplicity gene names are given ITHIH1 = Inter-alpha-trypsin inhibitor heavy chain H1, ITIH2 = Inter-alpha-trypsin inhibitor heavy chain H2, GPLD1 = Phosphatidylinositol-glycan-specific phospholipase D, PGLYRP2 = N-acetylmuramoyl-L-alanine amidase, SERPINA4 = Kallistatin, VSIG4 = soluble V-set and immunoglobulin domain-containing protein 4, AHSG = Alpha-2-HS-glycoprotein, ITIH3 = Inter-alpha-trypsin inhibitor heavy chain H3, AFM = Afamin, MRC1 = Macrophage mannose receptor 1, BCHE = Cholinesterase, IGFALS = Insulin-like growth factor-binding protein complex acid labile subunit, TF = Serotransferrin, crp.day.sample = CRP measured in the clinics at sampling day, LCAT = Phosphatidylcholine-sterol acyltransferase, CNDP1 = Beta-Ala-His dipeptidase, KLKB1 = Plasma kallikrein, SERPINA3 = Alpha-1-antichymotrypsin, CD14 = Monocyte differentiation antigen CD14, LCN2 = Neutrophil gelatinase-associated lipocalin, HGFAC = Hepatocyte growth factor activator, CD163 = Scavenger receptor cysteine-rich type 1 protein M130, FN1.15 = Fibronectin, isoform 15, HRG = Heme transporter HRG1, SERPINA1 = Alpha-1-antitrypsin, PCT.day.sample = PCT measured in the clinics at sampling day

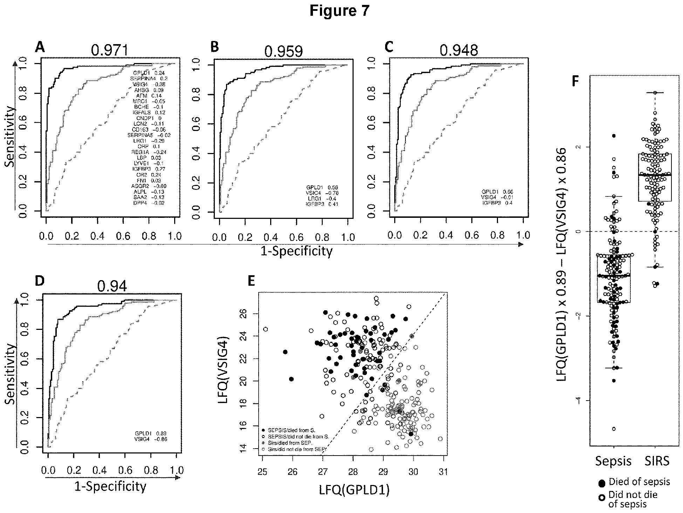

**Fig. 7:** Receiver operating characteristics curves for the combination of the 24 best performing plasma proteins (A) discriminating between SIRS and sepsis patients demonstrating an FC of at least two which were selected for a linear discrimination analysis. In each step of a backward elimination process the protein with the least contribution to the LDA's resulting predictor was eliminated from the list until the list contained only four (B), three (C), or two (D) proteins: Phosphatidylinositol-glycan-specific phospholipase D (GPLD1) and soluble V-set and immunoglobulin domain-containing protein 4 (VSIG4). The linear combination of these two proteins (LDA) was selected as a new predictor. (E): x-y plot of the linear predictor derived from GPLD1 and sVSIG4 (black open circles: sepsis patients, black filled circles: sepsis patients who died from sepsis; grey open circles SIRS patients, filled grey circles: SIRS patients who died from sepsis), and (F) scatter plot of the distribution of SIRS and sepsis patients with the linear predictor derived from proteomic results for GPLD1 and sVSIG4. For simplicity gene names are given: GPLD1 = Phosphatidylinositol-glycan-specific phospholipase D, SERPINA4 = Kallistatin, VSIG4 = soluble V-set and immunoglobulin domain-containing protein 4, AHSG = Alpha-2-HS-glycoprotein, AFM = Afamin, MRC1 = Macrophage mannose receptor 1, BCHE = Cholinesterase, IGFALS = Insulin-like growth factor-binding protein complex acid labile subunit, CNDP1 = Beta-Ala-His dipeptidase, LCN2 = Neutrophil gelatinase-associated lipocalin, CD163 = Scavenger receptor cysteine-rich type 1 protein M130, SERPINA5 = Plasma serine protease inhibitor, LRG1 = Leucine-rich alpha-2-glycoprotein, CRP = C-reactive protein, REG1A = Lithostathin-1-alpha, LBP = Lipopolysaccharide-binding protein, LYVE1 = Lymphatic vessel endothelial hyaluronic acid receptor 1, IGFBP3 = Insulin-like growth factor-binding protein 3, CR2 = Complement receptor type 2, FN1 = Fibronectin, ASGR2 = Asialoglycoprotein receptor 2, ALPL = Alkaline phosphatase, tissue-nonspecific isozyme, SAA2 = Serum amyloid A-2 protein, DPP4 = Dipeptidyl peptidase 4.

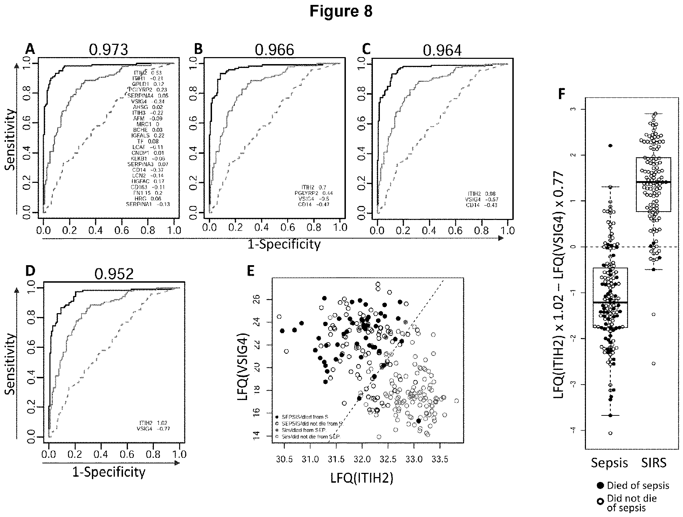

**Fig. 8:** Receiver operating characteristics curves for the combination of the 24 best performing plasma proteins (A) discriminating between SIRS and sepsis patients (no FC criterion) that were selected for a linear discrimination analysis. In each step of a backward elimination process the protein with the least contribution to the LDA's resulting predictor was eliminated from the list until the list contained only four (B), three (C), or two proteins (D): Inter-alpha-trypsin inhibitor heavy chain H1 (ITIH2) and soluble V-set and immunoglobulin domain-containing protein 4 (VSIG4). The linear combination of these two proteins (LDA) was selected as a new predictor. (E) x-y plot of the linear predictor derived from ITIH2 and sVSIG4 (black open circles: sepsis patients, black filled circles: sepsis patients who died from sepsis; grey open circles SIRS patients, filled grey circles: SIRS patients who died from sepsis), and (F) scatter plot of the distribution of SIRS and sepsis patients with the linear predictor derived from proteomic results for ITIH2 and sVSIG4. For simplicity gene names are given: ITHIH2 = Inter-alpha-trypsin inhibitor heavy chain H2, ITIH1 = Inter-alpha-trypsin inhibitor heavy chain H1, GPLD1 = Phosphatidylinositol-glycan-specific phospholipase D, PGLYRP2 = N-acetylmuramoyl-L-alanine amidase, SERPINA4 = Kallistatin, VSIG4 = soluble V-set and immunoglobulin domain-containing protein 4, AHSG = Alpha-2-HS-glycoprotein, ITIH3 = Inter-alpha-trypsin inhibitor heavy chain H3, AFM = Afamin, MRC1 = Macrophage mannose receptor 1, BCHE = Cholinesterase, IGFALS = Insulin-like growth factor-binding protein complex acid labile subunit, TF = Serotransferrin, LCAT = Phosphatidylcholine-sterol acyltransferase, CNDP1 = Beta-Ala-His dipeptidase, KLKB1 = Plasma kallikrein, SERPINA3 = Alpha-1-antichymotrypsin, CD14 = Monocyte differentiation antigen CD14, LCN2 = Neutrophil gelatinase-associated lipocalin, HGFAC = Hepatocyte growth factor activator, CD163 = Scavenger receptor cysteine-rich type 1 protein M130, FN1.15 = Fibronectin, isoform 15, HRG = Heme transporter HRG1, SERPINA1 = Alpha-1-antitrypsin.

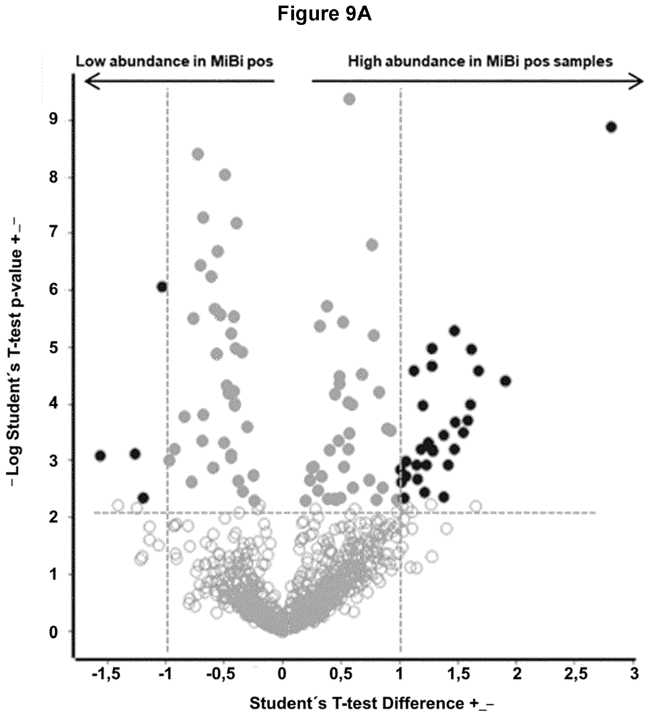

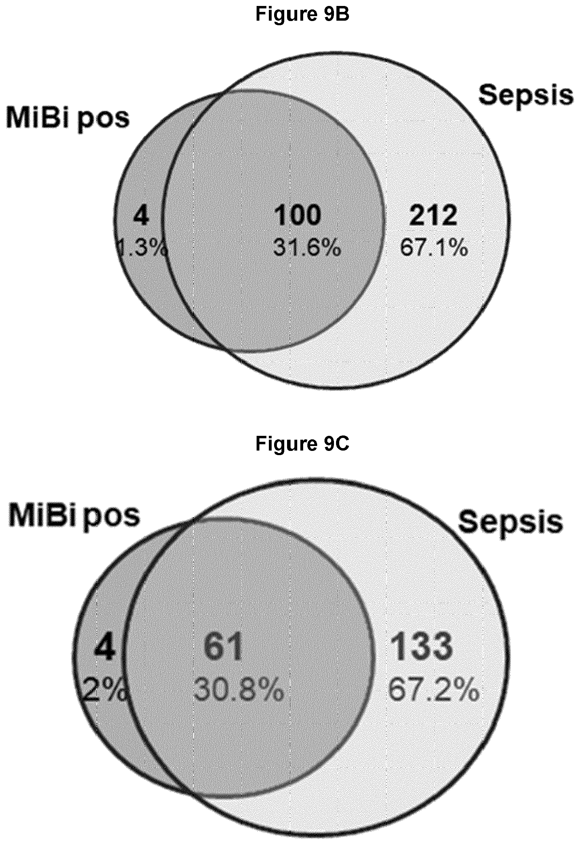

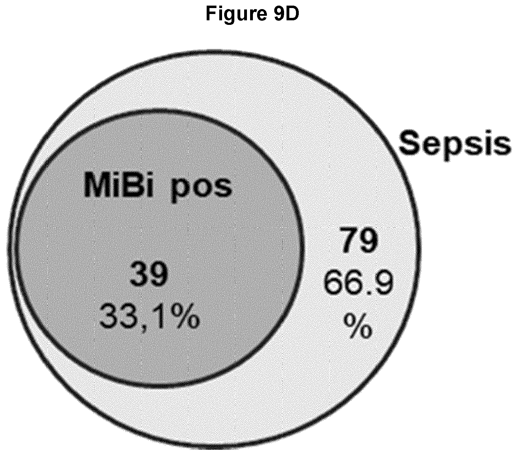

**Fig. 9**: Differential abundance of plasma proteins in patients with microbiological culture proven positive or culture negative results of the discovery cohort in comparison to plasma proteins detected with differential abundance in the sepsis patient group. A: Volcano plot of differentially abundant proteins in patients with positive or negative (SIRS and sepsis) culture results. The t-test p-value is plotted against the abundance fold change of all identified proteins with identifications in minimum 10 patients. Data points of the lower center area of the plot (grey, open circles) indicate proteins with unchanged or with no significant fold change, whereas data points in the upper left and upper right quadrants indicate proteins (black and grey filled circles) with significant (FDR less than 0.05 (Benjamini-Hochberg adjusted) lesser abundance (left) or significant higher abundance in the culture positive patient group (right). Black filled circles display plasma proteins with fold changes ≥2 and grey filled circles display significant differentially abundant proteins with FC<2. B: Overlap of significant differential abundant plasma proteins of the comparisons SIRS versus sepsis patients (right) and microbiological positive versus negative (left) results depicted in a Venn diagram. C+D: Overlap between plasma proteins with higher (C) levels in microbiological culture positive (left) and higher levels in sepsis patients and (D) plasma proteins detected at lesser abundance in patients with microbiological culture positive results and lesser abundance in the sepsis patient group.

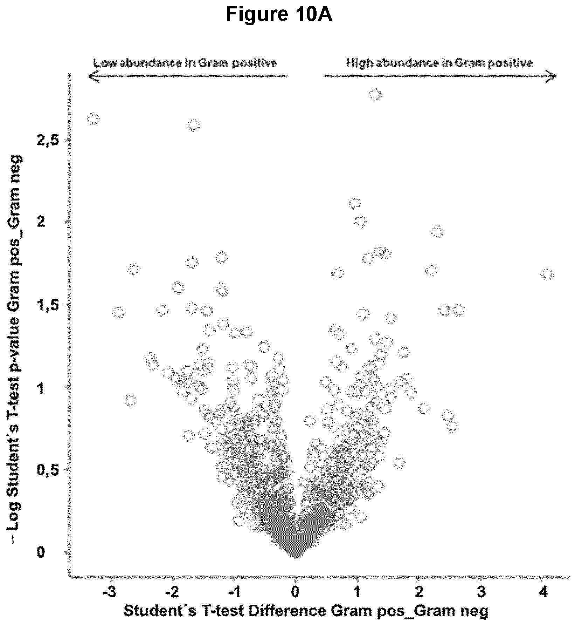

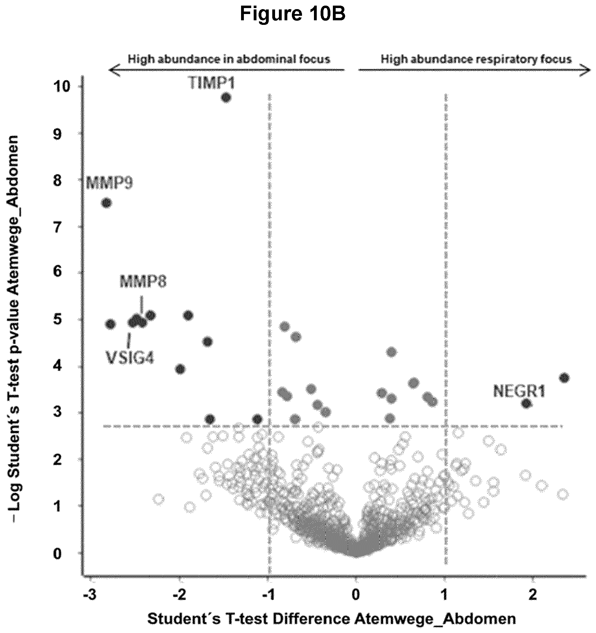

**Fig. 10**: Plasma protein abundance in septic patients with gram-negative or gram-positive bacteremia detected by culture methods (A) and with primary infection focus in the respiratory tract or abdominal (B). A+B: Volcano plots of differentially abundant proteins. The t-test p-value is plotted against the abundance fold change of all identified proteins with identifications in minimum 10 patients. Data points of the lower center area of the plot (grey, open circles) indicate proteins with unchanged or with no significant fold change, whereas data points in the upper left and upper right quadrants indicate proteins (black and grey filled circles) with significant (FDR less than 0.05 (Benjamini-Hochberg adjusted) lesser abundance (left) or significant higher abundance in the comparisons gram-positive (right) versus gram-negative (left) group or proteins higher abundant in the patient group with abdominal focus (left) and respiratory tract focus (right), respectively. Black filled circles display plasma proteins with fold changes ≥2 and filled grey circles display significant differentially abundant proteins with FC<2. Note the absence of significant proteins in the gram-negative versus gram-positive contrast. Highlighted are proteins TIMP1, MMP9, MMP8 and sVSIG4 with high abundance in patients with abdominal focus and NEGR1 with high abundance in patients with respiratory focus.

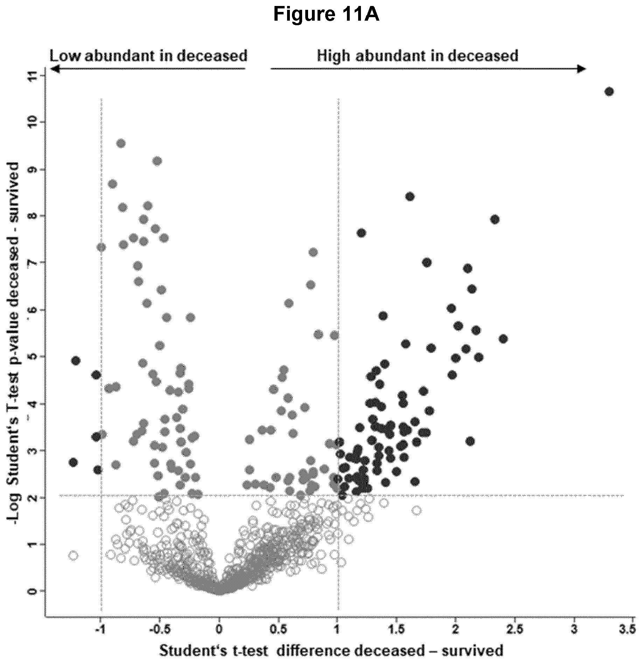

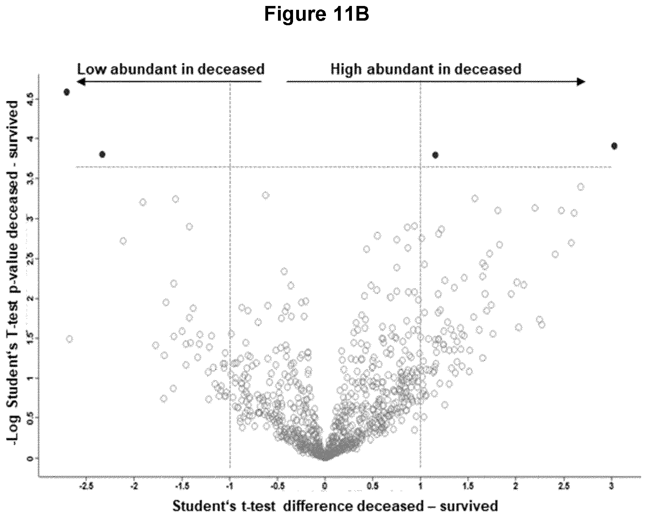

**Fig. 11:** Differential plasma protein abundance at day 1 and day 2 in patients who survive or patients to die of sepsis. A+B: Volcano plots of differentially abundant plasma proteins in the (A) discovery cohort and (B) validation cohort. The t-test p-value is plotted against the abundance fold change of all identified proteins with identifications in minimum 10 patients. Data points of the lower center area of the plot (grey, open circles) indicate proteins with unchanged or with no significant fold change, whereas data points in the upper left and upper right quadrants indicate proteins (black and grey filled circles) with significant (FDR less than 0.05 (Benjamini-Hochberg adjusted) lesser abundance (left) or significant higher abundance in patients to die of sepsis (deceased). Black filled circles display plasma proteins with significant fold changes ≥2 and filled grey circles display significant differentially abundant proteins with FC<2.

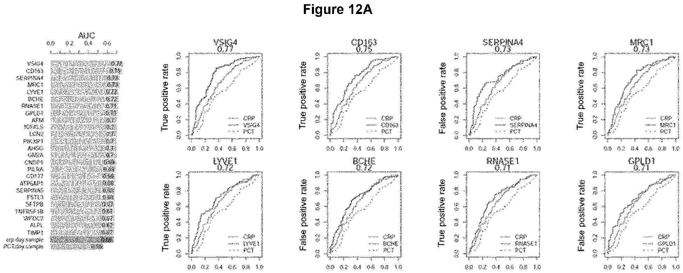

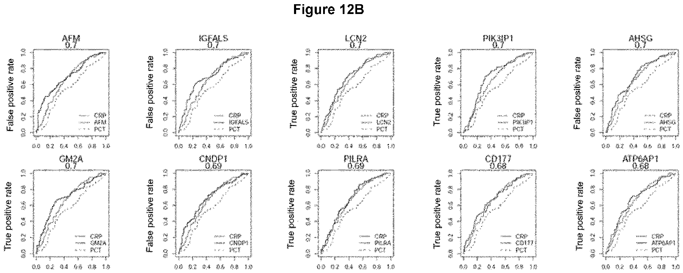

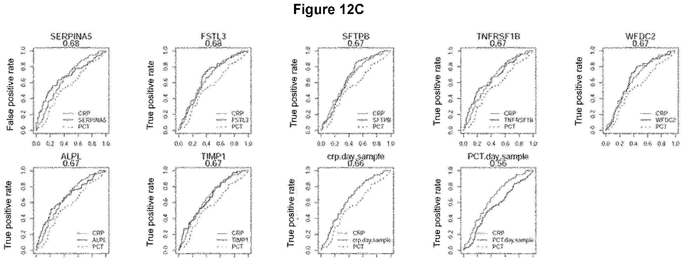

**Fig. 12**: A-C: Receiver operating characteristics (ROC) curves of 25 best performing plasma proteins in the data set of the discovery cohort for discriminating survivors and patients to die of sepsis. A left: bar diagram depicting area under the curve (AUC) values for plasma proteins identified in the proteomic data set and PCT and CRP (dark grey) values from the clinic. Plots depict ROC curves for the given proteins and their characteristics in the discovery cohort (black) and CRP (grey line) or PCT (dotted grey line). For simplicity gene names are given: VSIG4 = soluble V-set and immunoglobulin domain-containing protein 4, CD163 = Scavenger receptor cysteine-rich type 1 protein M130, SERPINA4 = Kallistatin, MRC1 = Macrophage mannose receptor LYVE1 = Lymphatic vessel endothelial hyaluronic acid receptor 1, BCHE = Cholinesterase, RNASE1 = Ribonuclease pancreatic, GPLD1 = Phosphatidylinositol-glycan-specific phospholipase D, AFM = Afamin, IGFALS = Insulin-like growth factor-binding protein complex acid labile subunit, LCN2 = Neutrophil gelatinase-associated lipocalin, PIK3IP1 = Phosphoinositide-3-kinase-interacting protein 1, AHSG = Alpha-2-HS-glycoprotein, GM2A = Ganglioside GM2 activator, CNDP1 = Beta-Ala-His dipeptidase, PILRA = Paired immunoglobulin-like type 2 receptor alpha, CD177 = CD177 antigen, ATP6AP1 = V-type proton ATPase subunit S1, SERPINA5 = Plasma serine protease inhibitor, FSTL3 = Follistatin-related protein 3, SFTPB = Pulmonary surfactant-associated protein B, TNFRSF1B = Tumor necrosis factor receptor superfamily member 1B, WFDC2 = WAP four-disulfide core domain protein 2, ALPL = Alkaline phosphatase, tissue-nonspecific isozyme, TIMP1 = Metalloproteinase inhibitor 1, crp.day.sample and PCT.day.sample = CRP and PCT measured in the clinics at sampling day.

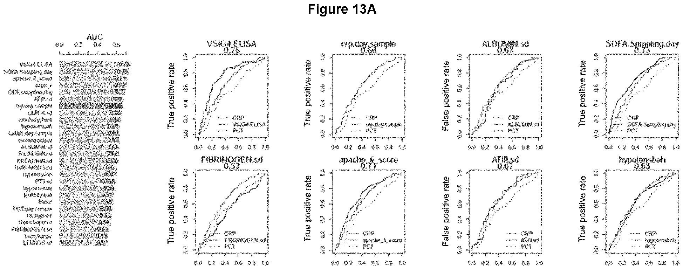

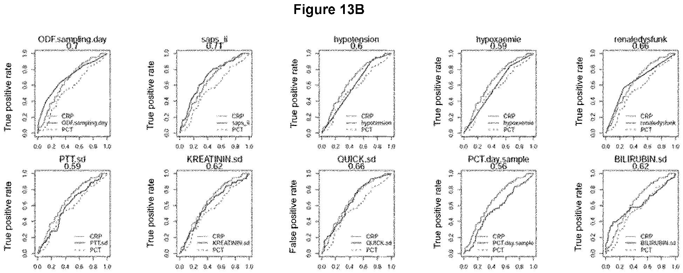

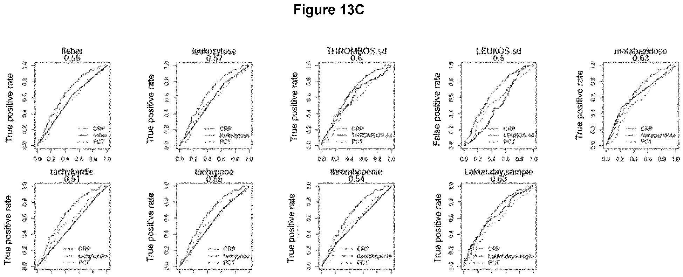

**Fig. 13**: A-C: Receiver operating characteristics curves (ROC) of sVSIG4 measured by ELISA and clinical parameters of patients of the discovery cohort. A left: bar diagram depicting area under the curve (AUC) values for plasma proteins identified in the proteomic data set and PCT and CRP (dark grey) values from the clinic. Plots depict ROC curves for the given proteins and their characteristics in the discovery cohort (black) and CRP (grey line) or PCT (dotted grey line).

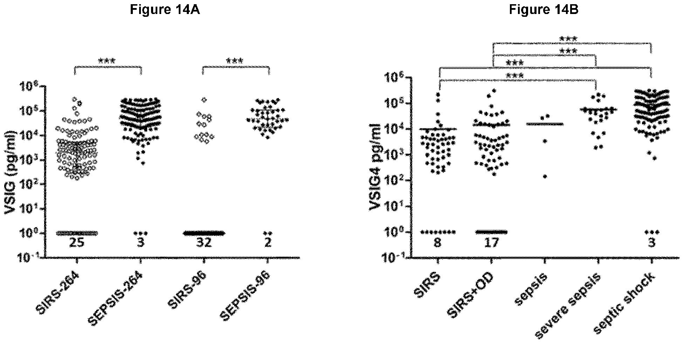

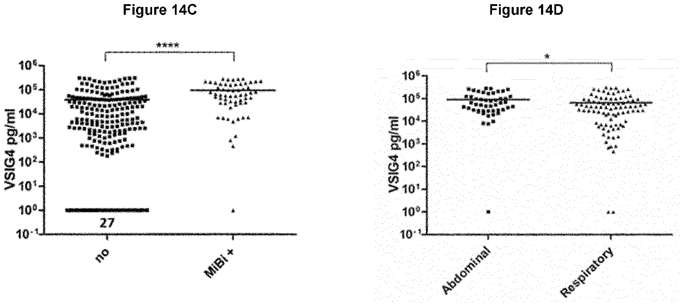

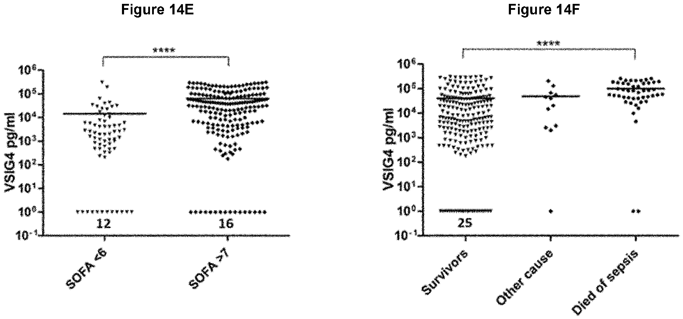

**Fig. 14**: Soluble VSIG4 detection in plasma samples with ELISA. Plasma samples from SIRS and sepsis patients of the discovery and validation cohort were diluted and detected with a sandwich ELISA against human VSIG4 protein. A: Detection of sVSIG4 in SIRS and sepsis patient samples of the discovery cohort (264) and validation cohort (96) (*** = p<0.0001). B: Plasma level of sVSIG4 in the subgroups SIRS, SIRS+Organdysfunction (OD), sepsis, severe sepsis and septic shock of both cohorts (***=p<0.001). C: Plasma levels of sVSIG4 in microbiological positive (MiBi+) and negative plasma samples (no). (****= p<0.001). D: sVSIG4 plasma levels in sepsis patients with abdominal focus or focus in the respiratory tract (*=p<0.0139). E: sVSIG4 levels in patients (SIRS and sepsis) with SOFA-score ≤6 or SOFA-score ≥7 (****= p<0.0001). F: sVSIG4 level in plasma at day 1 or day 2 after diagnosis in patients, who survived, died of sepsis or died of other reasons (****=p<0.0001).

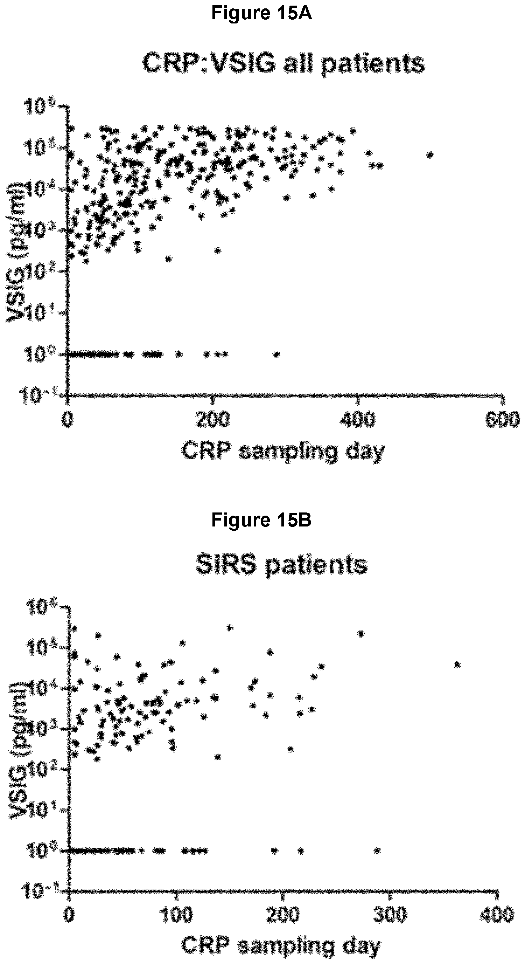

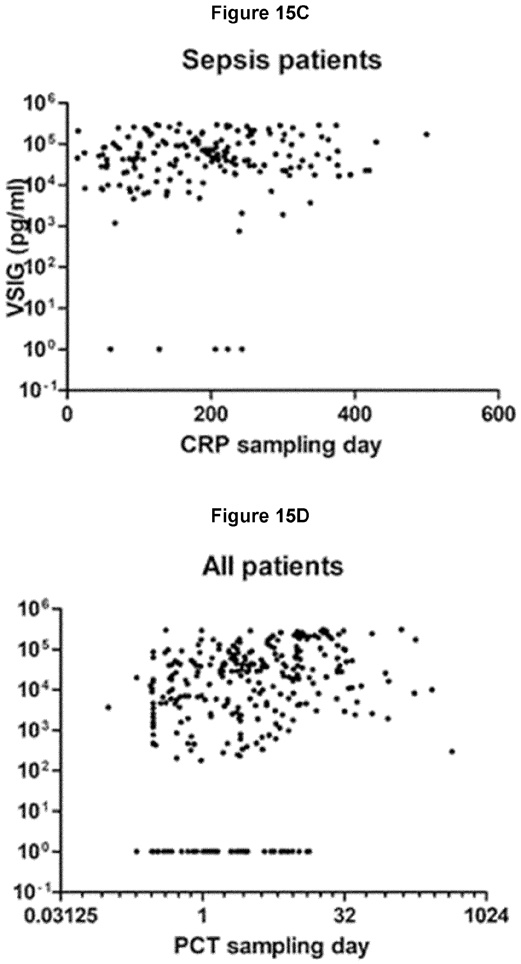

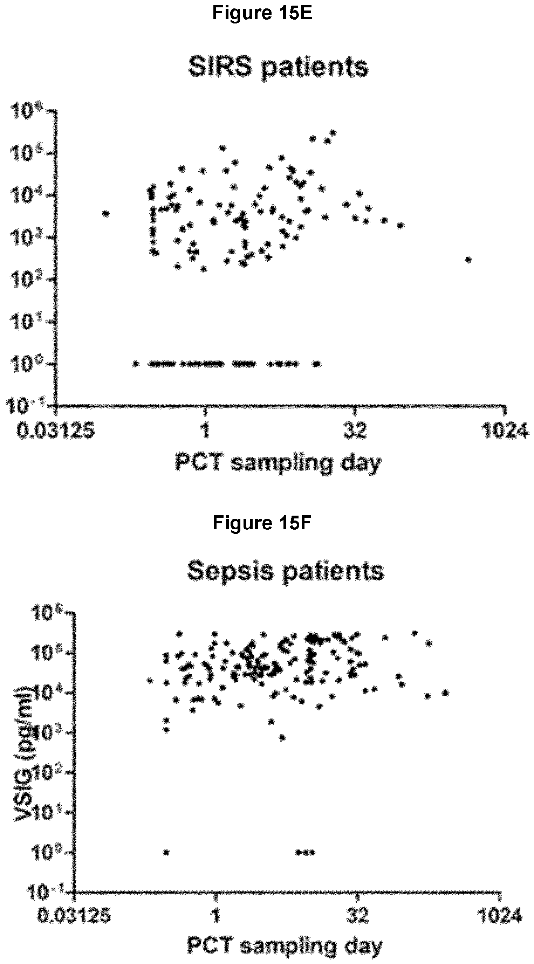

**Fig. 15**: Correlation of sVSIG4 (ELISA) with CRP and PCT at sampling day. x-y plots of sVSIG4 and CRP (A-C) or PCT (D-E) concentrations. A and D: sVSIG4 correlations in all patients of the discovery cohort, B and E: sVSIG4 correlation in SIRS patients and C and F: correlation of sVSIG in sepsis patients.

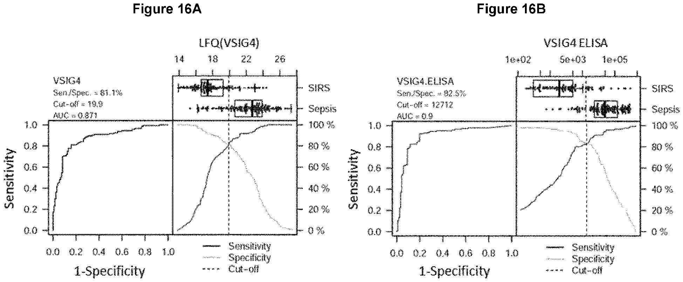

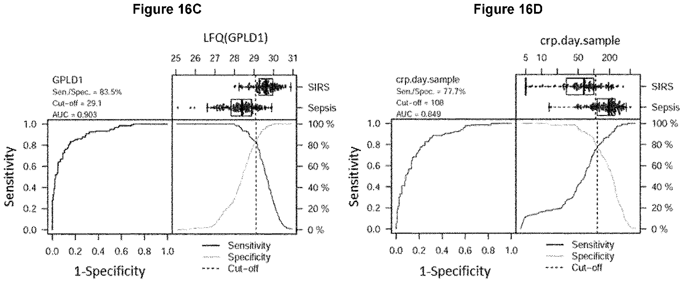

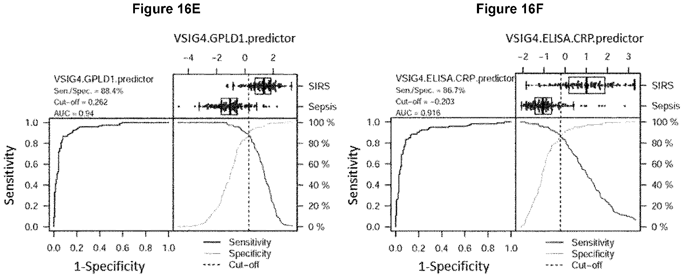

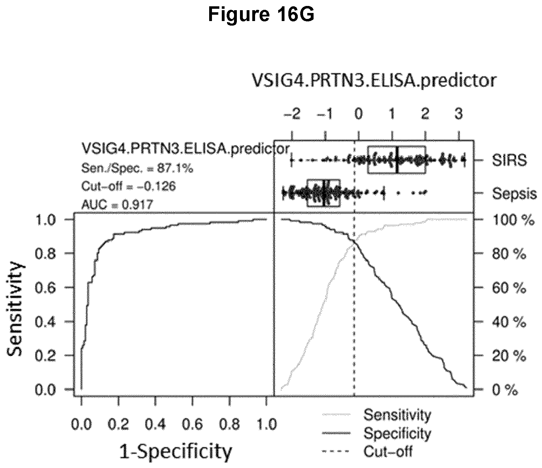

**Fig. 16**: Sepsis prediction with soluble V-set and immunoglobulin domain-containing protein 4 (sVSIG4), Phosphatidylinositol-glycan-specific phospholipase D (GPLD1), C-reactive protein (CRP) and sVSIG4- linear combinations with GPLD1, CRP and Myeloblastin (PRTN3). Sensitivity, specificity, cut-off values (sensitivity = specificity), and AUC of biomarkers for sepsis diagnosis. A: Proteomic data for sVSIG4 alone. B: sVSIG4 ELISA data alone. C: Phosphatidylinositol-glycan-specific phospholipase D (GPLD1) proteomic data alone. D: CRP-values measured in the clinic alone. E: linear combination of sVSIG4 and GPLD1 proteomic data. F: linear combination of sVSIG4 (ELISA data) and CRP clinical data. G: Linear combination of sVSIG4 (ELISA data) and PRTN3.

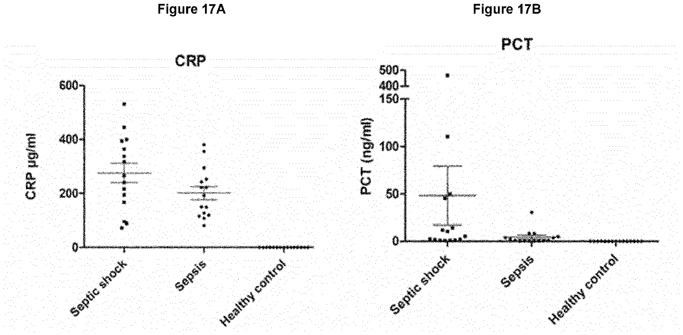

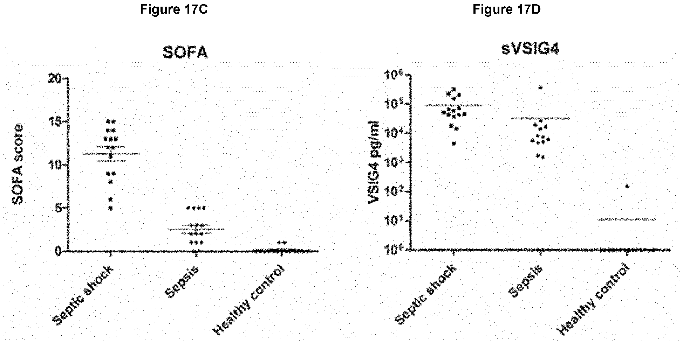

**Fig. 17**: Soluble VSIG4 detection in plasma samples with ELISA. CRP (A), PCT (B), SOFA-score (C) and sVSIG4 (D) levels in sepsis patients, septic shock patients and healthy controls in a cohort collected and grouped according to Sepsis-3 definition.

## DETAILED DESCRIPTION OF THE INVENTION

There is currently no parameter that alone can lead to the diagnosis of a systemic inflammation, in particular of SIRS, a bloodstream infection, a systemic infection or sepsis. A combination of laboratory values, hemodynamic data, and organ function, as well as (historically) other vital signs, are included to make the diagnosis. Even the prerequisite of a sepsis diagnosis, documented or suspected infection, cannot currently be clearly described by a single parameter (laboratory value or vital sign) alone. Furthermore, critically ill patients may also have organ dysfunction that need not be causally related to infection. Therefore, there is a continuous need for reliable methods for early diagnosis of systemic inflammation, in particular for diagnosing or prognosing sepsis.

The present invention provides a method of *in vitro* diagnosing a systemic inflammation or prognosing a risk of mortality of a subject with a systemic inflammation, in particular a systemic inflammation caused by an infectious agent, in particular sepsis. In particular, the protein-based biomarker soluble V-set and immunoglobulin domain-containing protein 4 (sVSIG4) allows for early detection of a systemically spreading infection. The protein-based biomarker is advantageously readily available in biological samples, such as body fluids, and can be directly measured, as the biomarker is soluble in said biological sample.

In a first aspect of the invention, a method of *in vitro* diagnosing a systemic inflammation or prognosing a risk of mortality of a subject with a systemic inflammation is provided, wherein the method comprises

a) determining the level of soluble V-set and immunoglobulin domain-containing protein 4 (sVSIG4) in a biological sample, and

b) drawing a conclusion as to the diagnosis of a systemic inflammation or the prognosis of a risk of mortality of a subject with a systemic inflammation from the presence and/or level of sVSIG4.

*"In vitro* Diagnosis" according to the present invention refers to a diagnosis that does not require the presence of the subject to be diagnosed because the biological sample has previously been obtained from the subject. Subsequently, the biological sample can be analyzed, i. e. the level of sVSIG4 can be determined, in the absence of the subject.

VSIG4 (V-set and immunoglobulin domain containing 4) is also known as complement receptor of the immunoglobulin superfamily (CRIg) and Z39Ig. VSIG4 is a type I transmembrane glycoprotein, O-glycosylated on extracellular threonin-264. It is a B7 family-related protein and an Ig superfamily member. It is involved in phagocytic processes on macrophages and is a strong negative regulator of T-cell proliferation. VSIG4 is a potent inhibitor of the alternative complement pathway convertases. VSIG4 contains an extracellular domain which comprises Ig-like domain 1 (SEQ ID NO: 4), or Ig-like domain 1 (SEQ ID NO: 4) and Ig-like domain 2 (SEQ ID NO: 5), and further a transmembrane domain and a cytoplasmic domain.

VSIG4 is particularly selected from the group consisting of VSIG4 isoform 1 (UniProt Identifier Q9Y279-1), VSIG4 isoform 2 (UniProt Identifier Q9Y279-2) and VSIG4 isoform 3 (UniProt Identifier Q9Y279-3) or has an amino acid sequence which is at least 90% identical to the amino acid sequence of VSIG4 isoform 1 (UniProt Identifier Q9Y279-1), VSIG4 isoform 2 (UniProt Identifier Q9Y279-2) or VSIG4 isoform 3 (UniProt Identifier Q9Y279-3).

Table 1. UniProt Identifiers and sequences of VSIG4 isoforms. Sequences were retrieved from the UniProt Database with release number 2021_01.

| Name | UniProt Identifier | Sequence | SEQ ID NO: |
|------|--------------------|----------|------------|
| VSIG4 isoform 1 | Q9Y279-1 | MGILLGLLLLGHLTVDTYGRPILEVPESVTGPWKGDV NLPCTYDPLQGYTQVLVKWLVQRGSDPVTIFLRDSS GDHIQQAKYQGRLHVSHKVPGDVSLQLSTLEMDDRS HYTCEVTWQTPDGNQVVRDKITELRVQKLSVSKPTV TTGSGYGFTVPQGMRISLQCQARGSPPISYIWYKQQ TNNQEPIKVATLSTLLFKPAVIADSGSYFCTAKGQVGS EQHSDIVKFVVKDSSKLLKTKTEAPTTMTYPLKATSTV KQSWDWTTDMDGYLGETSAGPGKSLPVFAIILIISLCC MVVFTMAYIMLCRKTSQQEHVYEAARAHAREANDSG ETMRVAIFASGCSSDEPTSQNLGNNYSDEPCIGQEY QIIAQINGNYARLLDTVPLDYEFLATEGKSVC | 1 |

(continued)

| Name | UniProt Identifier | Sequence | SEQ ID NO: |
|---|---|---|---|
| VSIG4 isoform 2 | Q9Y279-2 | MGILLGLLLLGHLTVDTYGRPILEVPESVTGPWKGDV NLPCTYDPLQGYTQVLVKWLVQRGSDPVTIFLRDSS GDHIQQAKYQGRLHVSHKVPGDVSLQLSTLEMDDRS HYTCEVTWQTPDGNQVVRDKITELRVQKLSVSKPTV TTGSGYGFTVPQGMRISLQCQARGSPPISYIWYKQQ TNNQEPIKVATLSTLLFKPAVIADSGSYFCTAKGQVGS EQHSDIVKFVVKDSSKLLKTKTEAPTTMTYPLKATSTV KQSWDWTTDMDGYLGETSAGPGKSLPVFAIILIISLCC MVVFTMAYIMLCRKTSQQEHVYEAAR | 2 |
| VSIG4 isoform 3 | Q9Y279-3 | MGILLGLLLLGHLTVDTYGRPILEVPESVTGPWKGDV NLPCTYDPLQGYTQVLVKWLVQRGSDPVTIFLRDSS GDHIQQAKYQGRLHVSHKVPGDVSLQLSTLEMDDRS HYTCEVTWQTPDGNQVVRDKITELRVQKHSSKLLKT KTEAPTTMTYPLKATSTVKQSWDWTTDMDGYLGETS AGPGKSLPVFAIILIISLCCMVVFTMAYIMLCRKTSQQE HVYEAARAHAREANDSGETMRVAIFASGCSSDEPTS QNLGNNYSDEPCIGQEYQIIAQINGNYARLLDTVPLDY EFLATEGKSVC | 3 |

Isoform 2 misses amino acids 322 to 399 of isoform 1. In Isoform 3 amino acids 138 to 232 of isoform 1 are replaced by histidine (H). The extracellular Ig-like domain 1 (SEQ ID NO: 4) encompasses amino acid 21 to 131 of either isoform. The extracellular Ig-like domain 2 (SEQ ID NO: 5) encompasses amino acids 143-226 of isoform 1 and isoform 2 and is missing in isoform 3.

Table 2. Sequences of Ig-like domain 1, Ig-like domain 2, extracellular domain, transmembrane domain and cytoplasmic domain of VSIG4.

| Name | Sequence | No. of amino acids of VSIG4 isoform 1 | SEQ ID NO: |
|---|---|---|---|
| Ig-like domain 1 | PILEVPESVTGPWKGDVNLPCTYDPLQGYTQV LVKWLVQRGSDPVTIFLRDSSGDHIQQAKYQG RLHVSHKVPGDVSLQLSTLEMDDRSHYTCEV TWQTPDGNQVVRDKIT | 21-131 | 4 |
| Ig-like domain 2 | PTVTTGSGYGFTVPQGMRISLQCQARGSPPIS YIWYKQQTNNQEPIKVATLSTLLFKPAVIADSG SYFCTAKGQVGSEQHSDIV | 143-226 | 5 |

(continued)

| Name | Sequence | No. of amino acids of VSIG4 isoform 1 | SEQ ID NO: |
|---|---|---|---|
| Extracellular domain | RPILEVPESVTGPWKGDVNLPCTYDPLQGYTQ VLVKWLVQRGSDPVTIFLRDSSGDHIQQAKYQ GRLHVSHKVPGDVSLQLSTLEMDDRSHYTCE VTWQTPDGNQVVRDKITELRVQKLSVSKPTVT TGSGYGFTVPQGMRISLQCQARGSPPISYIWY KQQTNNQEPIKVATLSTLLFKPAVIADSGSYFC TAKGQVGSEQHSDIVKFVVKDSSKLLKTKTEA PTTMTYPLKATSTVKQSWDWTTDMDGYLGET SAGPGKSLP | 20-283 | 6 |
| Transmembrane domain | VFAIILIISLCCMVVFTMAYI | 284-304 | 7 |
| Cytoplasmic domain | MLCRKTSQQEHVYEAARAHAREANDSGETM RVAIFASGCSSDEPTSQNLGNNYSDEPCIGQE YQIIAQINGNYARLLDTVPLDYEFLATEGKSVC | 305-399 | 8 |

The protein soluble VSIG4 (sVSIG4) preferably comprises the extracellular domain or a fragment of the extracellular domain of VSIG4. In another preferred embodiment, sVSIG4 does not comprise the transmembrane domain and cytoplasmic domain of VSIG4. In a particular embodiment, sVSIG4 comprises the extracellular domain or a fragment of the extracellular domain of VSIG4 and does not comprise the transmembrane domain and cytoplasmic domain of VSIG4.

sVSIG4 is a soluble protein, i. e. sVSIG4 is a free protein which is preferably not bound to a cell or integrated into a membrane, such as a cell membrane. sVSIG4 is preferably soluble in plasma, in particular in human plasma. In a preferred embodiment, sVSIG4 is dissolved in the biological sample, in particular in the non-cellular fraction of the biological sample. Thus, sVSIG4 can be detected in a cell-free or cell-depleted biological sample.

Preferably, the extracellular domain comprises Ig-like domain 1 (SEQ ID NO: 4), or Ig-like domain 1 (SEQ ID NO: 4) and Ig-like domain-2 (SEQ ID NO: 5). In another preferred embodiment, the extracellular domain comprises the sequence as defined in SEQ ID NO: 6 which comprises Ig-like domain 1 (SEQ ID NO: 4) and Ig-like domain-2 (SEQ ID NO: 5) and a first linker region between Ig-like domain 1 (SEQ ID NO: 4) and Ig-like domain-2 (SEQ ID NO: 5) and a second linker region after Ig-like domain 2 (SEQ ID NO: 5).

Determining a "level" of sVSIG4 or any other biomarker is synonymous with determining the concentration of sVSIG4 or such other biomarker in the biological sample. The level can be determined with various methods as further explained *infra*.

"Systemic Inflammation" according to the present invention refers to a systemic response of a subject, preferably a human subject, to a harmful stimulus. The harmful stimulus is for example an infectious agent, i. e. a pathogen. The harmful stimulus may also be a trauma, polytrauma or severe burns. In that case the systemic inflammation is not caused by an infectious agent. The response typically involves a strong reaction of the subject's immune system including the release of cytokines from immune cells, to result in a systemic reaction (as opposed to a local reaction) of the subject.

"Infection" refers to the invasion of a subject by an infectious agent, i. e. pathogens. Hence, a systemic inflammation may be caused by an infectious agent. Infectious agents, i. e. pathogens, may be bacteria, viruses or fungi, in particular bacteria or viruses. It is also possible that more than one type of pathogen infects the subject, for example an infection by bacteria and fungi.

The systemic inflammation caused by an infectious agent is preferably a sepsis, a systemic infection, or a bloodstream infection, more preferably a sepsis.

The systemic inflammation may also be not caused by an infectious agent. This is typically referred to as a sterile systemic inflammation and means that no infectious agent was detectable. Preferably, the systemic inflammation not caused by an infectious agent is a medical condition called systemic inflammatory reaction syndrome (SIRS). SIRS is characterized by a systemic reaction of a subject.

"Systemic infection" is an infection which has started locally but subsequently has spread across the organs of the subject. Hence, systemic infections in a human typically involve different parts of the body or more than one body system at the same time.

"Bloodstream infection" refers to an infection present in the blood. Bloodstream infections typically occur when a pathogen enters the bloodstream during surgery or due to foreign bodies, such as catheters or cardiac valves. Bloodstream infections include bacteremias, viremias and fungemias, depending on the pathogen (bacteria, viruses or fungi, respectively). Bacteremias are most common.

"Sepsis" can be defined in several ways. According to the "sepsis-2 definition" (Int. Arch. Allergy Appl. Immunol. 1984; 73:97-103), an excessive inflammatory response including a so-called cytokine storm is held responsible for the pathogenesis of sepsis. A SIRS with suspected or proven infection is referred to as sepsis. The German Sepsis Society lists four SIRS criteria in its sepsis definition in addition to the requirement of documented or suspected infection: (1) fever ($\geq$38.0°C) or hypothermia ($\leq$36.0°C), (2) tachycardia (heart rate $\geq$90/min), (3) tachypnea (respiratory rate $\geq$20/min) or hyperventilation (confirmed by taking an arterial blood gas analysis with $PaCO2 \leq 4.3$ kPa or 33 mmHg), (4) leukocytosis ($\geq$ 12,000/mm$^3$) or leukopenia ($\leq$ 4,000/mm$^3$), or more than 10% immature neutrophils on differential blood count.

"Severe sepsis" according to the sepsis-2 definition is referred to a medical condition wherein one or more organs fail. If this event also results in an extreme drop in blood pressure, in which the heart can no longer pump the blood through severely dilated blood vessels despite sufficient administration of fluids, and vasopressors are needed this is referred to as "septic shock".

In the sense of the present invention, the term "sepsis" includes "severe sepsis" and "septic shock" unless specified separately.

The "sepsis-3 definition" (JAMA 2016; 315:801-10; JAMA 2016; 315:775-87) replaces the SIRS criteria with an empirical, data-based, and evidence-driven definition of sepsis. Accordingly, sepsis according to the sepsis-3 definition is defined as life-threatening organ dysfunction caused by a dysregulated host response to infection. Because organ failure is already a component of sepsis by definition, the classification severe sepsis is not used in this definition. According to this definition, sepsis is present if (A) there is a diagnosed or suspected infection and (B) the Sequential Organ Failure Assessment (SOFA; see Intensive Care Med. 1996; 22:707-10) score increases by $\geq$ 2 points. According to this definition, septic shock is a subset of sepsis in which underlying circulatory and cellular/metabolic abnormalities are profound enough to substantially increase mortality. For this reason, in addition to severe hypotension lactate concentration is measured for the diagnosis of septic shock, and values $\geq$ 2 mmol/L after volume substitution are used as a criterion for septic shock (JAMA 2016; 315:775-87). Outside of intensive care management of patients, it is advised to use the so-called Quick Sequential Organ Failure Assessment-(qSOFA; see Jama 2016; 315:762-74) score, an abbreviated procedure to detect organ failure, to intensively monitor patients with suspected systemic infection. Regular collection of the qSOFA score outside of ITS departments has gained acceptance as a predictive value for detecting vital threats to high-risk patients, but it can only be performed by trained staff and requires time.

Additionally, microbiological and molecular biological methods are currently used to detect an infection or a suspected infection, in particular a systemic infection or a suspected systemic infection, which is part of a sepsis, severe sepsis or septic shock (according to sepsis-2 and sepsis-3).

Current diagnostics are disadvantageous because typically samples are taken from all body localizations that appear as a possible focus of the infection, for example infectious foci, blood, liquor, bronchoalveolar lavage or urine. The decision from which sites samples should be taken requires careful consideration on the part of the treatment team. If bloodstream infection is suspected, e.g. due to a catheter-induced infection, a culture from the catheter is also recommended.

According to the present invention, this decision-making process with respect to the localization of the infection focus is not necessary. The invention merely requires one biological sample, typically whole blood or plasma isolated from whole blood. It is not necessary to make a decision whether an infection is suspected or not because the method allows discriminating between a systemic inflammation caused by an infectious agent and a systemic inflammation not caused by an infectious agent, in particular between sepsis and SIRS, based on the level of sVSIG4 in the biological sample. The determination of the level of sVSIG4 in the biological sample is fast and reliable. Fast determination is especially advantageous because the earlier the diagnosis, the earlier the onset of therapy which goes along with a decrease of mortality. For example, if a bacterial infection is suspected and diagnosed by the use of a specific biomarker, in particular causing a sepsis, therapy with broad-spectrum antibiotics can be readily initiated. Fast onset of therapy is especially crucial for sepsis management. Therapy with a specific antibiotic can be initiated after analysis of a blood culture but this is not time-critical as long as treatment with broad-spectrum antibiotics have been initiated. Hence, the present invention helps by enabling fast onset of treatment of systemic inflammations, in particular caused by an infectious agent.

Sepsis is most frequently caused by bacterial pathogens, although viruses, protozoa and fungi can also rarely trigger a sepsis. Early targeted use of antibiotics can contain the inflammatory response caused by bacterial pathogens, reduce severe courses, and decrease mortality rates. Conversely, delay in diagnosis can increase the risk of serious outcomes such as organ failure, chronic pain, amputation, or death. Despite the recognized importance of early diagnosis, diagnosis remains difficult due to the complexity of the disease with diverse physiological and biochemical changes and manifestations.

In terms of sepsis therapy, a "bundle" of therapies has been established, which produces greater benefit in terms of outcome than the individual therapeutic interventions. The current sepsis bundle includes determination of lactate, obtaining blood cultures and administering antibiotics.

The risk of mortality of a subject with systemic inflammation, in particular with sepsis, relates to the risk to die of the systemic inflammation, in particular to die of sepsis. The present invention allows determining the risk of mortality due to a systemic inflammation, in particular due to sepsis based on the presence and/or level of sVSIG4.

Typically, the risk of mortality is defined for a certain time frame, for example the risk of mortality can refer to the risk to die within at least 7 days, at least 10 days, at least 15 days, at least 20 days, at least 25 days or at least 30 days, preferably within 28 days, after diagnosis of the systemic inflammation, particularly of sepsis.

The risk can be expressed as probability in percentage terms. For example, a risk of mortality of a subject with a systemic inflammation of 20 % within 28 days means that there is a probability of 20 % that the subject dies of the systemic inflammation within 28 days.

In case a subject is at a higher risk to die of the systemic inflammation, i. e. the subject with systemic inflammation has a high risk of mortality, such as 60 %, 70 %, 80 % or 90 %, the subject can be subjected to targeted enhanced monitoring in order to detect complications at an early stage. The subject could for example be transferred to an intermediate care unit or transferred to an intensive care unit. Hence, it is very beneficial in the clinical practice to know about the risk of mortality.

The risk of mortality can preferably be detected very early in disease progression, typically on day 1 or 2 after diagnosis. A subject typically has a higher risk to die of the systemic inflammation caused by an infectious agent, typically within 28 days, if the level of sVSIG4 in a biological sample, preferably in human plasma, is high, e. g. at least 4500 pg/ml, at least 9000 pg/ml, at least 15000 pg/ml, at least 40000 pg/ml, or at least 50000 pg/ml.

Hence, in a preferred embodiment of the present invention, a level of sVSIG4 in the biological sample of at least 4500 pg/ml, at least 9000 pg/ml, at least 15000 pg/ml, at least 40000 pg/ml, or at least 50000 pg/ml indicates a risk of mortality of a subject with a systemic inflammation caused by an infectious agent of at least 50 % within 28 days. The specificity is preferably about 82 %. In a preferred embodiment, the subject is a human subject. Preferably, the biological sample is plasma.

In a particular embodiment of the present invention, a level of sVSIG4 in the biological sample of at least 4500 pg/ml, preferably at least 9000 pg/ml, more preferably at least 15000 pg/ml, more preferably at least 40000 pg/ml, more preferably at least 50000 pg/ml, indicates a risk of mortality of a subject with a systemic inflammation caused by an infectious agent of at least 50 % within 28 days. The specificity is preferably about 82 %. In a preferred embodiment, the subject is a human subject. Preferably, the biological sample is plasma.

In another preferred embodiment of the present invention, a level of sVSIG4 in the biological sample of at least 70000 pg/ml, at least 75000 pg/ml, at least 80000 pg/ml, or at least 85000 pg/ml indicates a risk of mortality of a subject with a systemic inflammation caused by an infectious agent of at least 30 % within 28 days. The specificity is preferably about 85 %. In a preferred embodiment, the subject is a human subject. Preferably, the biological sample is plasma.

In a particular embodiment of the present invention, a level of sVSIG4 in the biological sample of at least 70000 pg/ml, preferably at least 75000 pg/ml, more preferably at least 80000 pg/ml, more preferably at least 85000 pg/ml, indicates a risk of mortality of a subject with a systemic inflammation caused by an infectious agent of at least 30 % within 28 days. The specificity is preferably about 85 %. In a preferred embodiment, the subject is a human subject. Preferably, the biological sample is plasma.

The level of sVSIG4 in the biological sample also has an impact on the therapy management. If the level of sVSIG4 in the biological sample indicates a systemic inflammation caused by an infectious agent, such as a bloodstream infection or a sepsis, the subject can be treated accordingly. The therapist thereby learns when to take therapeutic measures. It is advantageous that the method of the present invention allows a very fast therapy decision. For example, a therapy with antibiotics can be initiated as soon as the level of sVSIG4 is known.

If necessary, differential diagnosis can follow. For example, in case a bloodstream infection is suspected because the subject has for example a catheter, a culture from the catheter may be started and reveal the potential pathogen. In case a sepsis is suspected, a blood culture may reveal the responsible pathogen. Further diagnostic measures can also serve the purpose of distinguishing between sepsis, severe sepsis and septic shock. Nevertheless, the method according to the present invention gives a fast indication whether the subject suffers from a systemic inflammation caused or not caused by an infectious agent and, in case of a systemic inflammation caused by an infectious agent, whether the subject suffers from a bloodstream infection or sepsis.

The method may also include further parameters for diagnosing a systemic inflammation, preferably a systemic inflammation caused by an infectious agent, the further parameters being in particular Sequential Organ Failure Assessment (SOFA) score, Quick Sequential Organ Failure Assessment (qSOFA) score, Acute Physiology and Chronic Healthy Evaluation II (APACHE-II) score and/or Simplified Acute Physiology Score II (SAPS-II).

The "Acute Physiology and Chronic Health Evaluation II" (APACHE-II) score is an ICU severity-of-disease classification system. It is applied within 24h after ICU admission and an integer score from 0 to 71 is computed based on several measurements. A higher score corresponds to more severe disease and a higher risk of death. The point score is calculated from the following variables: blood-gas tension (PaO2) or alveolar-arterial gradient (AaDO2), body temperature, mean arterial pressure, blood pH, heart rate, respiratory rate, serum sodium, serum potassium, creatinine, hematocrit, white blood cell count, Glasgow Coma Scale.

The Simplified Acute Physiology Score II (SAPS-II) is also a disease severity classification scoring system in the ICU. The point score is calculated from the parameters: age, heart rate, systolic blood pressure, temperature, Glasgow Coma Scale, mechanical ventilation or continuous positive airway pressure (CPAP), blood gas tension (PaO2), fraction of inspired oxygen (FiO2), urine output, blood urea nitrogen, sodium, potassium, bicarbonate, bilirubin, white blood cell count, chronic diseases, type of admisson.

In a preferred embodiment, a level of sVSIG4 in the biological sample of at least 20 pg/ml, at least 50 pg/ml, at least 100 pg/ml, at least 150 pg/ml, at least 200 pg/ml, or at least 250 pg/ml indicates the systemic inflammation. In a preferred embodiment, the subject is a human subject. Preferably, the biological sample is plasma.

In a particular embodiment, a level of sVSIG4 in the biological sample of at least 20 pg/ml, preferably at least 50 pg/ml, more preferably at least 100 pg/ml, more preferably at least 150 pg/ml, more preferably at least 200 pg/ml more preferably at least 250 pg/ml indicates the systemic inflammation. In a preferred embodiment, the subject is a human subject. Preferably, the biological sample is plasma.

Hence, a level of sVSIG4 of at least 20 pg/ml, at least 50 pg/ml, at least 100 pg/ml, at least 150 pg/ml, at least 200 pg/ml, or at least 250 pg/ml indicates that the subject is not healthy and in particular has a systemic inflammation. The systemic inflammation can be any type of systemic inflammation including SIRS and sepsis.

In a preferred embodiment, a level of sVSIG4 in the biological sample of at least 20 pg/ml, at least 50 pg/ml, at least 100 pg/ml, at least 150 pg/ml, at least 200 pg/ml, or at least 250 pg/ml and less than 100000 pg/ml, less than 50000 pg/ml, less than 12500 pg/ml, less than 10000 pg/ml, or less than 6000 pg/ml indicates the SIRS. In a preferred embodiment, the subject is a human subject. Preferably, the biological sample is plasma.

In particular embodiment, a level of sVSIG4 in the biological sample of at least 20 pg/ml, preferably at least 50 pg/ml, more preferably at least 100 pg/ml, more preferably at least 150 pg/ml, more preferably at least 200 pg/ml, more preferably at least 250 pg/ml and less than 100000 pg/ml, preferably less than 50000 pg/ml, more preferably less than 12500 pg/ml, more preferably less than 10000 pg/ml, more preferably less than 6000 pg/ml indicates the SIRS. In a preferred embodiment, the subject is a human subject. Preferably, the biological sample is plasma.

In another preferred embodiment, a level of sVSIG4 in the biological sample of at least 200 pg/ml, at least 250 pg/ml, at least 300 pg/ml, at least 350 pg/ml, or at least 400 pg/ml indicates the bloodstream infection. For example, when an infection is suspected or proven the indicated levels point to a bloodstream infection. In a preferred embodiment, the subject is a human subject. Preferably, the biological sample is plasma.

In a particular embodiment, a level of sVSIG4 in the biological sample of at least 200 pg/ml, preferably at least 250 pg/ml, more preferably at least 300 pg/ml, more preferably at least 350 pg/ml, more preferably at least 400 pg/ml indicates the bloodstream infection. For example, when an infection is suspected or proven the indicated levels point to a bloodstream infection. In a preferred embodiment, the subject is a human subject. Preferably, the biological sample is plasma.

In another preferred embodiment, a level of sVSIG4 in the biological sample of at least 500 pg/ml, at least 550 pg/ml, at least 600 pg/ml, at least 650 pg/ml, at least 700 pg/ml, at least 750 pg/ml, at least 1000 pg/ml, at least 1500 pg/ml, at least 2500 pg/ml, at least 3500 pg/ml, at least 5000 pg/ml, at least 7500 pg/ml, at least 10000 pg/ml, or at least 12500 pg/ml indicates the sepsis. In a preferred embodiment, the subject is a human subject. Preferably, the biological sample is plasma.

In a particular embodiment, a level of sVSIG4 in the biological sample of at least 500 pg/ml, preferably at least 550 pg/ml, more preferably at least 600 pg/ml, more preferably at least 650 pg/ml, more preferably at least 700 pg/ml, more preferably at least 750 pg/ml, more preferably at least 1000 pg/ml, more preferably at least 1500 pg/ml, more preferably at least 2500 pg/ml, more preferably at least 3500 pg/ml, more preferably at least 5000 pg/ml, more preferably at least 7500 pg/ml, more preferably at least 10000 pg/ml, more preferably at least 12500 pg/ml indicates the sepsis. In a preferred embodiment, the subject is a human subject. Preferably, the biological sample is plasma.

In particular, a cut-off value of at least 1500 pg/ml, at least 5000 pg/ml, at least 10000 pg/ml, or at least 12500 pg/ml allows identifying a subject having sepsis and not having SIRS. In a preferred embodiment, the subject is a human subject. Preferably, the biological sample is plasma.

Hence, in one embodiment, a level of sVSIG4 in the biological sample of at least 1500 pg/ml, at least 5000 pg/ml, at least 10000 pg/ml, or at least 12500 pg/ml indicates the sepsis and discriminates between SIRS and sepsis. In a preferred embodiment, the subject is a human subject. Preferably, the biological sample is plasma.

In a particular embodiment, a level of sVSIG4 in the biological sample of at least 1500 pg/ml, preferably at least 5000 pg/ml, more preferably at least 10000 pg/ml, more preferably at least 12500 pg/ml indicates the sepsis and discriminates between SIRS and sepsis. In a preferred embodiment, the subject is a human subject. Preferably, the biological sample is plasma.

The protein sVSIG4 may also be termed a biomarker in the context of the present invention. A "biomarker" in the sense of the present invention refers for example to a biological compound, such as a peptide, a polypeptide, or a protein, preferably a polypeptide or a protein. Hence, the biomarker may be protein-based, i. e. the biomarker comprises a polypeptide or a protein, preferably is a polypeptide or a protein.

A biomarker in the sense of the present invention is preferably a human polypeptide or protein. The names of the biomarkers are usually presented herein as full names and their abbreviation in parentheses, or only the abbreviation is presented. All biomarkers and their sequences and abbreviations are retrieved from the UniProt database. The abbreviations of the biomarkers (also known as entry names) can alternatively have the suffix "_HUMAN". With and without the suffix the same polypeptide or protein is meant.

In one aspect, a biomarker which level in a biological sample from a subject exceeds a reference level (cut-off value) indicates a systemic inflammation per se. The same or another reference level, preferably a higher reference level, indicates a sepsis. A level exceeds a reference level if the numerical value of the level is higher than the numerical value of the reference level. The method of determining the level can be any suitable method and will be further described *infra.*

"Cut-off value" as used herein refers to an assay cut-off value (threshold or reference level) that is used to assess diagnostic, prognostic, or therapeutic efficacy results by comparing the assay results against the predetermined cut-off value / sVSIG4 level, where the predetermined cut-off value / sVSIG4 level has already been linked or associated with various clinical parameters (e.g., presence of disease, stage of disease, severity of disease, progression, non-progression, or improvement of disease, etc.). The disclosure provides exemplary predetermined levels. However, it is well-known that cut-off values may vary depending on the nature of the immunoassay (e.g., antibodies employed, reaction conditions, sample purity, etc.). It is further well within the ordinary skill of one in the art to adapt the disclosure herein for other immunoassays to obtain immunoassay-specific cut-off values for those other immunoassays based on the description provided by this disclosure. Whereas the precise value of the predetermined cut-off/ sVSIG4 level may vary between assays, the correlations as described herein are generally applicable.

Cut-off values / reference levels can be used herein to determine whether an individual is suffering from a systemic inflammation caused by an infectious agent, or from a systemic inflammation not caused by an infectious agent, or is a healthy subject with no systemic inflammation. The reference levels of sVSIG4 or sVSIG4 in combination with other markers for infection can be used to determine whether a subject is suffering from a systemic inflammation caused by an infectious agent, or from a systemic inflammation not caused by an infectious agent, or is a healthy subject that is free of a systemic inflammation. The reference level of sVSIG4 alone, or used in particular combinations, may be a predetermined cut-off value, or a level determined from a control subject, wherein that control subject is known to be a healthy subject without a systemic inflammation, a subject with a systemic inflammation not caused by an infectious agent (e. g. SIRS) or a subject with a systemic inflammation caused by an infectious agent.

Cut-off values (or predetermined cut-off values) may be determined by a receiver operating curve (ROC) analysis from biological samples of a patient group. ROC analysis, as generally known in the biological arts, is a determination of the ability of a test to discriminate one condition from another, e.g. to determine the performance of markers in identifying subjects with a systemic inflammation caused or not caused by an infectious agent, in particular sepsis, severe sepsis or septic shock. Alternatively, cutoff values can be determined by a quartile analysis of biological samples of a patient group. A cut-off value can also be determined by selecting a value that corresponds to any value in the 25th-75th percentile range, preferably a value that corresponds to the 25th percentile, the 50th percentile or the 75th percentile, preferably the 75th percentile.

A cut-off value can for example be determined experimentally as shown in Figure 16. For example, a cut-off value can be the level at which the sensitivity and specificity curves cross. However, a lower cut-off value can also be chosen which will be concomitant with an increase in sensitivity at the cost of lower specificity. A higher cut-off value is also possible which will be concomitant with lower sensitivity and increased specificity. A high sensitivity typically means that every positive subject is identified, yet at the expense of false-positives. A high specificity means that the subjects not affected are reliably excluded but at the expense of false-negatives. Depending on one's need, an optimal cut-off value can be chosen.

In another aspect, the biomarker is differentially abundant in a biological sample from a subject having a systemic inflammation, in particular a sepsis, as compared to a comparable biological sample from a control subject. The control subject may be a healthy subject or a subject with no diagnosed or suspected infection (a non-infected subject) or a subject with a negative diagnosis of sepsis. For example, the control subject is a subject suffering from SIRS.

The terms "peptide", "polypeptide" and "protein" can be used synonymously and refer to a polymer of amino acids and are not limited to a minimum or maximum length. Both full-length proteins and fragments thereof are encompassed by the definition.

Posttranslational modifications of the peptide, the polypeptide, or the protein are also encompassed, for example, glycosylation, acetylation, hydroxylation, phosphorylation, and/or oxidation, in particular glycosylation.

The term "differentially abundant" or "differentially expressed" relates to a difference in the quantity, i. e. the determined level, of a biomarker in a biological sample taken from a subject having, for example, systemic inflammation, in particular a sepsis, as compared to a control subject. The level of the biomarker may thus be increased or decreased as compared to a control subject.

A biomarker is differentially abundant or differentially expressed between two biological samples if the level of the biomarker in one biological sample is different from the level of the biomarker in the other sample. For example, a biomarker is differentially abundant or differentially expressed in two biological samples if the level of the biomarker is increased by at least about 20%, at least about 30%, at least about 50%, at least about 80%, at least about 100%, at least about 200%, at least about 400% compared with the other biological sample, or if it is detectable in one biological sample and not detectable in the other biological sample.

The systemic inflammation may be caused by an infectious agent or not be caused by an infectious agent. In case the systemic inflammation is caused by an infectious agent, it may be a sepsis, systemic infection or bloodstream infection. In case the systemic inflammation is not caused by an infectious agent, for example no infectious agent is detectable, the systemic inflammation may be SIRS.

Typically, the infectious agent is a bacterium, a fungus or a virus. In one embodiment, the bacterium is a gram positive or a gram negative bacterium.

The virus may be influenza A virus, influenza B virus, respiratory syncytial virus (RSV), rhinovirus, and/or coronavirus, in particular severe acute respiratory syndrome coronavirus type 2 (SARS-CoV2).

In a very preferred embodiment, the systemic inflammation is caused by an infectious agent and is a sepsis, a severe sepsis or a septic shock according to the sepsis-2 definition.

In one embodiment, the method further comprises:

- determining the level of one or more additional biomarkers in the biological sample; and
- in step b) drawing a conclusion as to the diagnosis of a systemic inflammation or the prognosis of a risk of mortality of a subject with systemic inflammation from the presence and/or level of sVSIG4 in combination with the presence and/or level of the one or more additional biomarkers.

The one or more additional biomarkers may serve to confirm or undermine the diagnosis or prognosis. The one or more additional biomarkers are preferably detectable in the same biological sample as sVSIG4 and are preferably also soluble in that they are not bound to a cell or membrane. Although the method allows diagnosis and prognosis based on solely sVSIG4 as a biomarker, one or more further biomarkers may thus be helpful.

The one or more additional biomarkers can be revealed experimentally, e. g. in that differentially abundant proteins in sepsis and SIRS patients are analyzed or in that levels of biomarkers of patients, which have subsequently died of the systemic inflammation, are compared.

The one or more additional biomarkers are preferably selected from the group consisting of IgGFc-binding protein (FCGBP), GDNF family receptor alpha-2 (GFRA2), Carboxypeptidase D (CBPD), Asialoglycoprotein receptor 1 (ASGR1), Hypoxia upregulated protein 1 (HYOU1), Tenascin (TENA), Polymeric immunoglobulin receptor (PIGR), Sushi, von Willebrand factor type A, EGF and Pentraxin domain-containing protein 1 (SVEP1), Interleukin-1 receptor type 2 (IL1R2), Laminin subunit beta-1 (LAMB1), Endoplasmic reticulum chaperone BiP (BIP), Peroxidasin homolog (PXDN), Follistatin-related protein 1 (FSTL1), Leucine-rich alpha-2 glycoprotein (A2GL), Metalloproteinase inhibitor 1 (TIMP1), NPC intracellular cholesterol transporter 2 (NPC2), Peptidoglycan recognition protein 1 (PGRP1), Lactotransferrin (TRFL), Dystonin (DYST), Lipopolysaccharide-binding protein (LBP), Haptoglobin (HPT), Asialoglycoprotein receptor 2 (ASGR2), Non-secretory ribonuclease (RNAS2), Azurocidin (CAP7), Chitinase-3-like protein 1 (CH3L1), Tumor necrosis factor receptor superfamily member 1B (TNR1B), HLA class II histocompatibility antigen gamma chain (HG2A), Olfactomedin-4 (OLFM4), Leukocyte immunoglobulin-like receptor subfamily A member 3 (LIRA3), Insulin-like growth factor-binding protein 2 (IBP2), Growth/differentiation factor 15 (GDF15), Laminin subunit alpha-2 (LAMA2), Tyrosine-protein phosphatase non-receptor type substrate 1 (SHPS1), Basigin (BASI), Fibroleukin (FGL2), Cathepsin Z (CATZ), E-selectin (LYAM2), Lymphatic vessel endothelial hyaluronic acid receptor 1 (LYVE1), Disintegrin and metalloproteinase domain-containing protein 9 (ADAM9), Desmocollin-2 (DSC2), Interleukin-1 receptor-like 1 (ILRL1), Matrix metalloproteinase-9 (MMP9), Cystatin-C (CYTC), Interleukin-18-binding protein (I18BP), Paired immunoglobulin-like type 2 receptor alpha (PILRA), Macrophage mannose receptor 1 (MRC1), Osteopontin (OSTP), Scavenger receptor cysteine-rich type 1 protein M130 (C163A), Integral membrane protein 2B (ITM2B), Fibrinogen-like protein 1 (FGL1), Serum amyloid A-1 protein (SAA1), Interleukin-1 receptor antagonist protein (IL1RA), Nucleobindin-1 (NUCB1), Golgi membrane protein 1 (GOLM1), Dystroglycan (DAG1), CD177 antigen (CD177), Alkaline phosphatase, tissue-nonspecific isozyme (PPBT), Neutrophil collagenase (MMP8), Myeloblastin (PRTN3), Neutrophil elastase (ELNE), Beta-1,4-galactosyltransferase 1 (B4GT1), C-reactive protein (CRP), WAP four-disulfide core domain protein 2 (WFDC2), Follistatin-related protein 3 (FSTL3), Ribonuclease pancreatic (RNAS1), Neutrophil gelatinase-associated lipocalin (NGAL), Serum amyloid A-2 protein (SAA2), Lithostathine-1-alpha (REG1A), Plasma kallikrein (KLKB1), Phosphatidylcholine-sterol acyltransferase (LCAT), Serotransferrin (TRFE), Procalcitonin (PCT)), Complement receptor type 2 (CR2), Voltage-dependent calcium channel subunit alpha-2/delta-1 (CA2D1) Indian hedgehog protein (IHH), Serum paraoxonase/lactonase 3 (PON3), Fibronectin (FINC), Beta-Ala-His-dipeptidase (CNDP1), Insulin-like growth factor-binding protein complex acid labile subunit (ALS), Phosphatidylinositol-glycan-specific phospholipase D (PHLD), Insulin-like growth factor-binding protein 3 (IBP3), Anthrax toxin receptor 1 (ANTR1), Neuronal growth regulator 1 (NEGR1), Plasma serine protease inhibitor (IPSP), Intelectin-1 (ITLN1), Kallistatin (KAIN), Fibroblast growth factor receptor 1 (FGFR1), Alpha-2-HS-glycoprotein (FETUA), Cholinesterase (CHLE), Afamin (AFAM), Cathepsin F (CATF), Cholesteryl ester transfer protein (CETP), Cadherin-related family member 5 (CDHR5), Inter-alpha-trypsin inhibitor heavy chain H2 (ITIH2), N-acetylmuramoyl-L-alanine amidase (PGRP2), Contactin-1 (CNTN1), Apolipoprotein(a) (APOA), Cell growth regulator with EF hand domain protein 1 (CGRE1), Coagulation factor VIII (FA8), Phospholipid transfer protein (PLTP), Protein Z-dependent protease inhibitor (ZPI), Alpha-1-antichymotrypsin (AACT), Vitamin K-dependent protein S (PROS), Coagulation factor XIII B chain (F13B), Prenylcysteine oxidase 1 (PCYOX), Serum amyloid A-4 protein (SAA4) Apolipoprotein C-I (APOC1), Prolyl endopeptidase FAP (SEPR), Dipeptidylpeptidase 4 (DPP4), Angiotensin-converting enzyme 2 (ACE2), Interleukin-1 receptor accessory protein (IL1AP), Di-N-acetylchitobiase (DIAC), Hepatocyte growth factor activator (HGFA), Selenoprotein P (SEPP1), A disintegrin and metalloproteinase with thrombospondin motifs 13 (ATS13), Monocyte differentiation antigen CD14 (CD14), Complement factor H-related protein 1 (FHR1), von Willebrand factor (VWF), Laminin subunit gamma-1 (LAMC1), Scavenger receptor class A member 5 (SCAR5), ADAMTS-like protein 4 (ATL4), Cullin-1 (CUL1), Pulmonary surfactant-associated protein B (PSPB), Neuroblastoma suppressor of tumorigenicity (NBL1), Ganglioside GM2 activator (SAP3), Protein disulfide isomerase CRELD1 (CREL1), Cadherin-related family member 2 (CDHR2), Chymotrypsin-like elastase family member 3B (CEL3B), Phosphoinositide-3-kinase-interacting protein 1 (P3IP1), Lithostathine-1-beta (REG1B), Kininogen-1 (KNG1), Versican core protein (CSPG2), EMILIN-2 (EMIL2), Carcinoembryonic antigen-related cell adhesion molecule 6 (CEAM6), Fibromodulin (FMOD), Beta-galactoside alpha-2,6-sialyltransferase 1 (SIAT1), Leukocyte immunoglobulin-like receptor subfamily B member 5 (LIRB5), Latent-transforming growth factor beta-binding protein 2 (LTBP2), Chromogranin-A (CMGA) and Adseverin (ADSV), Histidine-rich glycoprotein (HRG), Inter-alpha-trypsin inhibitor heavy chain H1 (ITIH1), and Inter-alpha-trypsin inhibitor heavy chain H3 (ITIH3), wherein in step b) a conclusion is drawn as to the diagnosis of a systemic inflammation. Preferably, the systemic inflammation is sepsis.

In a particular embodiment, the one or more additional biomarkers in the biological sample are selected from one or more of the following:

(i) Inter-alpha-trypsin inhibitor heavy chain H2 (ITIH2), Inter-alpha-trypsin inhibitor heavy chain H1 (ITIH1), Phosphatidylinositol-glycan-specific phospholipase D (PHLD), N-acetylmuramoyl-L-alanine amidase (PGRP2), Kallistatin (KAIN), Alpha-2-HS-glycoprotein (FETUA), Inter-alpha-trypsin inhibitor heavy chain H3 (ITIH3), Afamin (AFAM), Macrophage mannose receptor 1 (MRC1), Cholinesterase (CHLE), Insulin-like growth factor-binding protein complex acid labile subunit (ALS), Serotransferrin (TRFE), Phosphatidylcholine-sterol acyltransferase (LCAT), Beta-Ala-His dipeptidase (CNDP1), Plasma kallikrein (KLKB1), Alpha-1-antichymotrypsin (AACT), Monocyte differentiation antigen CD14 (CD14), Neutrophil gelatinase-associated lipocalin (NGAL), Hepatocyte growth factor activator (HGFA), Scavenger receptor cysteine-rich type 1 protein M130 (C163A), Fibronectin (FINC), Histidine-rich glycoprotein (HRG), and Alpha-1-antitrypsin (A1AT);

(ii) Phosphatidylinositol-glycan-specific phospholipase D (PHLD), Kallistatin (KAIN), Alpha-2-HS-glycoprotein (FETUA), Afamin (AFAM), Macrophage mannose receptor 1 (MRC1), Cholinesterase (CHLE), Insulin-like growth factor-binding protein complex acid labile subunit (ALS), Beta-Ala-His dipeptidase (CNDP1), Neutrophil gelatinase-associated lipocalin (NGAL), Scavenger receptor cysteine-rich type 1 protein M130 (C163A), Plasma serine protease inhibitor (IPSP), Leucine-rich alpha-2-glycoprotein (A2GL), Lithostathine-1-alpha (REG1A), Lipopolysaccharide-binding protein (LBP), Lymphatic vessel endothelial hyaluronic acid receptor 1 (LYVE1), Insulin-like growth factor-binding protein 3 (IBP3), Complement receptor type 2 (CR2), Fibronectin (FINC), Asialoglycoprotein receptor 2 (ASGR2), Alkaline phosphatase, tissue-nonspecific isozyme (PPBT), Serum amyloid A-2 protein (SAA2), and Dipeptidylpeptidase 4 (DPP4);

(iii) Inter-alpha-trypsin inhibitor heavy chain H2 (ITIH2), N-acetylmuramoyl-L-alanine amidase (PGRP2), and Monocyte differentiation antigen CD14 (CD14);

(iv) Inter-alpha-trypsin inhibitor heavy chain H2 (ITIH2);

(v) Phosphatidylinositol-glycan-specific phospholipase D (PHLD), Leucine-rich alpha-2-glycoprotein (A2GL), and Insulin-like growth factor-binding protein 3 (IBP3);

(vi) Phosphatidylinositol-glycan-specific phospholipase D (PHLD);

(vii) C-reactive protein (CRP);

(viii) Procalcitonin (PCT);

(ix) Lithostathine-1-alpha (REG1A);

(x) Myeloblastin (PRTN3);

(xi) CRP and PCT,

wherein in step b) a conclusion is drawn as to the diagnosis of a systemic inflammation. Preferably, the systemic inflammation is sepsis.

In the sense of the present invention, sVSIG4 as a biomarker can be combined with one or more of the one or more additional biomarkers as recited *supra*. In particular, sVSIG4 as a biomarker can be combined with one or more of groups (i) to (xi). Hence, in one embodiment, in step a) in addition to determining the level of sVSIG4, the level of one or more further soluble proteins in the biological sample is determined which can be used as biomarkers. Although it is sufficient to only determine the level of sVSIG4, it may be beneficial for the diagnosis to determine the levels of further soluble proteins.

In another preferred embodiment, the one or more additional biomarkers are preferably selected from the group consisting of Kininogen-1 (KNG1), Tenascin (TENA), Versican core protein (CSPG2), Cadherin-related family member 2 (CDHR2), EMILIN-2 (EMIL2), Osteopontin (OSTP), Tyrosine-protein phosphatase non-receptor type substrate 1 (SHPS1), Lithostathine-1-beta (REG1B), Carcinoembryonic antigen-related cell adhesion molecule 6 (CEAM6), Paired immunoglobulin-like type 2 receptor alpha (PILRA), HLA class II histocompatibility antigen gamma chain (HG2A), Scavenger receptor cysteine-rich type 1 protein M130 (C163A), Fibroleukin (FGL2), Follistatin-related protein 3 (FSTL3), Fibromodulin (FMOD), Beta-galactoside alpha-2,6-sialyltransferase 1 (SIAT1), Myeloblastin (PRTN3), Leukocyte immunoglobulin-like receptor subfamily B member 5 (LIRB5), N-acetylmuramoyl-L-alanine amidase (PGRP2), Interleukin-1 receptor-like 1 (ILRL1), Neutrophil gelatinase-associated lipocalin (NGAL), Latent-transforming growth factor beta-binding protein 2 (LTBP2), Interleukin-1 receptor antagonist protein (IL1RA), Chromogranin-A (CMGA), Phosphoinositide-3-kinase-interacting protein 1 (P3IP1), Ribonuclease pancreatic (RNAS1), Ganglioside GM2 activator (SAP3), Neutrophil elastase (ELNE), Adseverin (ADSV), Disintegrin and metalloproteinase domain-containing protein 9 (ADAM9), Lithostathine-1-alpha (REG1A), Nucleobindin-1 (NUCB1), and WAP four-disulfide core domain protein 2 (WFDC2), wherein in step b) a conclusion is drawn as to the prognosis of a risk of mortality of a subject with a systemic inflammation. Preferably, the systemic inflammation is sepsis.

In a particular embodiment, the one or more additional biomarkers in the biological sample are selected from one or more of the following:

(i) Kininogen-1 (KNG1) and Tenascin (TENA);

(ii) Versican core protein (CSPG2), Cadherin-related family member 2 (CDHR2), EMILIN-2 (EMIL2), Osteopontin (OSTP), Tyrosine-protein phosphatase non-receptor type substrate 1 (SHPS1), Lithostathine-1-beta (REG1B), Carcinoembryonic antigen-related cell adhesion molecule 6 (CEAM6), Paired immunoglobulin-like type 2 receptor alpha (PILRA), HLA class II histocompatibility antigen gamma chain (HG2A), Scavenger receptor cysteine-rich type 1 protein M130 (C163A), Fibroleukin (FGL2), Follistatin-related protein 3 (FSTL3), Fibromodulin (FMOD), Beta-galactoside alpha-2,6-sialyltransferase 1 (SIAT1), Myeloblastin (PRTN3), Leukocyte immunoglobulin-like receptor subfamily B member 5 (LIRB5), N-acetylmuramoyl-L-alanine amidase (PGRP2), Interleukin-1 receptor-like 1 (ILRL1), Neutrophil gelatinase-associated lipocalin (NGAL), Latent-transforming growth factor beta-binding protein 2 (LTBP2), Interleukin-1 receptor antagonist protein (IL1RA), Chromogranin-A (CMGA), Phosphoinositide-3-kinase-interacting protein 1 (P3IP1), Ribonuclease pancreatic (RNAS1), Ganglioside GM2 activator (SAP3), Neutrophil elastase (ELNE), Adseverin (ADSV), Disintegrin and metalloproteinase domain-containing protein 9 (ADAM9), Lithostathine-1-alpha (REG1A), Nucleobindin-1 (NUCB1), and WAP four-disulfide core domain protein 2 (WFDC2);

(iii) Scavenger receptor cysteine-rich type 1 protein M130 (C163A), Kallistatin (KAIN), Macrophage mannose receptor 1 (MRC1), Lymphatic vessel endothelial hyaluronic acid receptor 1 (LYVE1), Cholinesterase (CHLE), Ribonuclease pancreatic (RNAS1), Phospahtidylinositol-glycan-specific phospholipase D (PHLD), Afamin (AFAM), Insulin-like growth factor-binding protein complex acid labile subunit (ALS), Neutrophil gelatinase-associated lipocalin (NGAL), Phosphoinositide-3-kinase-interacting protein 1 (P3IP1), Alpha-2-HS-glycoprotein (FETUA), Ganglioside GM2 activator (SAP3), Beta-Ala-His-dipeptidase (CNDP1), Paired immunoglobulin-like type 2 receptor alpha (PILRA), CD177 antigen (CD177), V-type proton ATPase subunit S1 (VAS1), Plasma serine protease inhibitor (IPSP), Follistatin-related protein 3 (FSTL3), Pulmonary surfactant-associated protein B (PSPB), Tumor necrosis factor receptor superfamily member 1B (TNR1B), WAP four-disulfide core domain protein 2 (WFDC2), Alkaline phosphatase, tissue-nonspecific isozyme (PPBT), and Metalloproteinase inhibitor 1 (TIMP1);

(iv) C-reactive protein (CRP);

(v) Procalcitonin (PCT);

(vi) Lithostathine-1-alpha;

(vii) CRP and PCT,

wherein in step b) a conclusion is drawn as to the prognosis of a risk of mortality of a subject with a systemic inflammation. Preferably, the systemic inflammation is sepsis.

In the sense of the present invention, sVSIG4 as a biomarker can be combined with one or more of the one or more additional biomarkers as recited *supra.* In particular, sVSIG4 as a biomarker can be combined with one or more of groups (i) to (vii). Hence, in one embodiment, in step a) in addition to determining the level of sVSIG4, the level of one or more further soluble proteins in the biological sample is determined which can be used as biomarkers. Although it is sufficient to only determine the level of sVSIG4, it may be beneficial for the prognosis of mortality to determine the levels of further soluble proteins.

In a very preferred embodiment, sVSIG4 can be used in combination with CRP, Myeloblastin (PRTN3), Phosphatidylinositol-glycan-specific phospholipase D (PHLD) and/or PCT for diagnosis of a systemic inflammation, preferably caused by an infectious agent, preferably sepsis. Hence, sVSIG4 can be used in combination with CRP in the diagnosis of sepsis. In another embodiment, sVSIG4 is used in combination with myeloblastin (PRTN3) in the diagnosis of sepsis. In another embodiment, sVSIG4 is used in combination with Phosphatidylinositol-glycan-specific phospholipase D (PHLD) in the diagnosis of sepsis. In another embodiment, sVSIG4 is used in combination with PCT in the diagnosis of sepsis. In yet another embodiment, sVSIG4 is used in combination with CRP and PCT in the diagnosis of sepsis.

The same combinations are also beneficial for determining the risk of mortality of a subject with a systemic inflammation, preferably sepsis.

A predictor can be constructed as a linear combination of two variables. The respective weights for the two variables can be calculated using a linear discriminant analysis (LDA) for the classification of SIRS vs sepsis patients. Prior to LDA the data is preferably log-transformed, centered (to mean) and scaled (by standard deviation). Sensitivity and specificity are preferably plotted for each predictor.

For example, with the linear combination of sVSIG4 and CRP, the following calculation can be used to calculate the predictor (cf. Figure 16F):

$$\text{predictor} = -0.4268478 \times \ln(\text{sVSIG4}) - 0.4500494 \times \ln(\text{CRP}) + 5.927552$$

A predictor calculated by this equation less than 3, preferably less than 0.5, preferably less than 0, more preferably less than -0.2 may indicate a sepsis based on the determination of the levels of sVSIG4 and CRP in the biological sample.

Hence, in a preferred embodiment, a method is provided, wherein a predictor based on the determination of the levels of sVSIG4 and CRP in the biological sample and calculated by - 0.4268478 x ln(sVSIG4) - 0.4500494 x ln(CRP) + 5.927552 less than 3, preferably less than 0.5, preferably less than 0, more preferably less than -0.2 indicates the sepsis. In a preferred embodiment, the subject is a human subject. Preferably, the biological sample is plasma.

In another preferred embodiment, a method is provided, wherein a predictor based on the determination of the levels of sVSIG4 and CRP in the biological sample and calculated by - 0.4268478 x ln(sVSIG4) - 0.4500494 x ln(CRP) + 5.927552 less than 1, preferably less than 0, more preferably less than -1 indicates a risk of mortality of a subject with a systemic inflammation, preferably a sepsis, of at least 50 % within 28 days. In a preferred embodiment, the subject is a human subject. Preferably, the biological sample is plasma.

With the linear combination of sVSIG4 and myeloblastin (PRTN3), the following calculation can be used to calculate the predictor (cf. Figure 16G):

$$\text{predictor} = -0.4331127 \times \ln(\text{sVSIG4}) - 0.4901329 \times \ln(\text{PRTN3}) + 9.942252$$

Hence, in a preferred embodiment, a predictor based on the determination of the levels of sVSIG4 and myeloblastin (PRTN3) in the biological sample and calculated by -0.4331127 x ln(sVSIG4) -0.4901329 x ln(PRTN3) +9.942252 less than 2, preferably less than 1, more preferably less than -0.1 indicates the sepsis. In a preferred embodiment, the subject is a human subject. Preferably, the biological sample is plasma.

In another preferred embodiment, a predictor based on the determination of the levels of sVSIG4 and myeloblastin (PRTN3) in the biological sample and calculated by -0.4331127 x ln(sVSIG4) -0.4901329 x ln(PRTN3) +9.942252 less than 0, preferably less than -1 indicates a risk of mortality of a subject with a systemic inflammation, preferably a sepsis, of at least 50 % within 28 days. In a preferred embodiment, the subject is a human subject. Preferably, the biological sample is plasma.

In one embodiment, sVSIG4 is a glycoprotein, i. e. a protein which contains oligosaccharide chains (glycans) covalently attached to amino acid side-chains. The glycosylation in particular comprises N-glycosylation and/or O-glycosylation, preferably O-glycosylation.

The glycosylation can for example be analyzed by chemical oxidation of glycan structures linked to sVSIG4 by hydrazide chemistry resulting in aldehydes and modification with either biotin-labelled crosslinkers or hydrazide-activated agarose beads forming a stable conjugate via hydrazone bonds. Biotinylation of sVSIG4 can be detected by immunoprecipitation of labelled sVSIG4 and Western Blot analysis using avidin- or streptavidin conjugated with Horseradish peroxidase.

Hence, the method may comprise enriching glycosylated proteins in the biological sample. Thereby, the sample is more concentrated with respect to the glycosylated proteins and the determination of sVSIG4 would be improved.

The enriching of glycosylated proteins in the biological sample is preferably performed prior to step a). In particular, enriching glycosylated proteins in the biological sample includes performing one or more separation steps with the biological sample, in particular prior to step a).

In one embodiment, the one or more separation steps include chromatographic separation, preferably affinity chromatographic separation. The person skilled in the art is familiar with chromatographic methods.

In a preferred embodiment, the affinity chromatographic separation is selected from the group consisting of lectin affinity chromatography, separation by hydrazide chemistry, hydrophilic interaction chromatography and immunoaffinity chromatography.

In a further embodiment proteins of the biological sample are enzymatically degraded in the one or more separation steps for further analysis, in particular quantitative analysis. Enzymatic degradation can also be referred to as proteolytic cleavage and allows analysis such as mass spectrometry.

The determination step a) is preferably performed by one or more methods selected from the group consisting of chromatography, spectrometry, electrophoresis, spectroscopy, biochemical assay and immunoassay, preferably spectrometry, in particular mass spectrometry, and/or immunoassay.

Preferably, the mass spectrometry is a liquid chromatography-mass spectrometry (LC-MS), LC-MS/MS or matrix-assisted laser desorption ionization-time of flight mass spectrometry (MALDI-TOF MS).

Proteomic analyses of serum and plasma samples by mass spectrometry are often difficult because 95% of the total plasma protein amount is accounted for by 20 highly abundant proteins, which means that proteins present in significantly lower amounts are often not detectable or not reproducible. To avoid this problem, it is possible to not analyze the entirety of plasma proteins, but a selection of plasma proteins which are first enriched and subsequently analyzed. The focus is for example on glycosylated proteins, which are enzymatically posttranslationally modified with sugar groups after their synthesis at the ribosome. Yet, mass spectrometry is useful in the search for unknown proteins.

In another preferred embodiment, the immunoassay is selected from one or more of the group consisting of enzyme-linked immunoassay (ELISA), immunoscreening, lateral flow immunochromatographic assay, magnetic immunoassay and radio immunoassay, preferably ELISA. In a very preferred embodiment, ELISA is performed for specifically and accurately determining the level of sVSIG4 in the biological sample. The ELISA is preferably performed with a monoclonal antibody specific for human VSIG4, especially specific for the extracellular domain of human VSIG4. ELISA is advantageous because it is not necessary to process the biological sample, such as whole blood, plasma or serum, before use. The person skilled in the art is familiar with the ELISA technique. The ELISA may also include a further antibody directed against the extracellular domain of VSIG4 to perform a sandwich ELISA.

The ELISA may also include a further antibody directed against another biomarker so that the level of sVSIG4 and another biomarker can be determined.

It is also within the sense of the present invention that step a) comprises performing LC-MS, LC-MS/MS or MALDI-TOF MS and a subsequent step of analyzing the obtained data for determining the level of sVSIG4 in the biological sample. This combination may further enhance accuracy.

In a particularly preferred embodiment, the method comprises:

- performing a depletion step and/or a fractionation step and/or enriching glycosylated proteins in the biological sample prior to step a); and
- performing mass spectrometry in step a) for determining the level of sVSIG4 in the biological sample.

The "depletion step" typically involves separating and discarding cells or cell debris from the biological sample, for example by centrifugation. "Fractionation" in the sense of the present invention refers to separating the biological sample into its component parts, for example by centrifugation. The component parts are typically blood plasma, which can also be fractionated into its components, leukocytes and platelets, and erythrocytes.

Fractionation of plasma typically involves changing the conditions of the plasma (e.g., the temperature, the acidity or the hydration of proteins) so that proteins that are normally dissolved in the plasma fluid become insoluble, forming large clumps, called precipitate. Precipitation can for example be achieved with trichloroacetic acid, acetone, methanol-chloroform or ammonium sulfate. The insoluble protein can be collected by centrifugation.

The depletion step and/or a fractionation step and/or the enriching of glycosylated proteins in the biological sample prior to step a) typically serves the purpose of increasing the concentration of the protein to be detected, for example sVSIG4. In case the level of sVSIG4 is high enough for detection, these steps may be omitted.

The method may also comprise,

- enriching glycosylated proteins comprising performing an affinity chromatographic separation step, in particular lectin affinity chromatography, separation by hydrazide chemistry or immunoaffinity chromatography;
- optionally, enzymatically degrading proteins of the biological sample in the affinity chromatographic separation step.

In case the optional degradation step is performed, the level of sVSIG4 is determined on basis of the enzymatically degraded proteins, i. e. peptides. In case the optional degradation step is not performed, the level of sVSIG4 may be determined on basis of the soluble protein on the whole.

Possible peptides of the degradation step may be produced by proteolytic degradation or chemical cleavage methods. Examples for the use of trypsin protease for the proteolytic digestion of plasma-associated sVSIG4 are the ones depicted in Table 3. Other enzymes for the proteolytic digestion of plasma-associated sVSIG4 may also be used.

Table 3. Tryptic peptide sequences identified by mass spectrometry.

| Peptide sequence | No. of amino acids of VSIG4 isoform 1 | SEQ ID NO: |
|---|---|---|
| GDVNLPCTYDPLQGYTQVLVK | 35-55 | 9 |
| GSDPVTIFLR | 61-70 | 10 |
| VPGDVSLQLSTLEMDDR | 92-108 | 11 |
| SHYTCEVTWQTPDGNQVVR | 108-127 | 12 |
| LSVSKPTVTTGSGYGFTVPQGMR | 138-160 | 13 |
| PTVTTGSGYGFTVPQGMR | 143-160 | 14 |
| ISLQCQAR | 161-168 | 15 |
| GSPPISYIWYK | 169-179 | 16 |
| VATLSTLLFK | 190-199 | 17 |
| PAVIADSGSYFCTAK | 200-214 | 18 |

Preferably, the biological sample is a body fluid sample. Typically, a biological sample has a cellular and a non-cellular fraction. In a particularly preferred embodiment, the biological sample is the non-cellular fraction of a biological sample.

The body fluid sample is preferably selected from one or more of the group consisting of whole blood, plasma, serum, synovial fluid, pleural effusion, lymphatic fluid, urine, liquor, cerebrospinal fluid, ascites, and bronchial lavage, and samples derived from the foregoing, in particular cell-free or cell-depleted samples derived from the foregoing samples by removing cells.

In a particularly preferred embodiment, the biological sample is selected from the group consisting of whole blood, plasma and serum. Plasma or blood plasma refers to the liquid portion of blood, i. e. it is essentially cell-depleted, preferably cell-free. Serum typically refers to blood plasma without fibrinogens. Whole blood, plasma and serum can be easily retrieved from a subject and processed making the inventive method convenient and easy to perform.

The biological sample is prepared according to the usual standards. For example, in case blood is drawn for isolation of plasma, typically anticoagulants are added to the blood sample, such as heparin, EDTA and/or citrate. Other supplements are also possible.

In a preferred embodiment, the biological sample is a cell-free or cell-depleted sample. This is particularly useful because sVSIG4 is a soluble protein.

In a preferred method of the present invention, the biological sample is processed prior to step a), in particular by obtaining the non-cellular fraction of the biological sample.

In a particularly preferred embodiment, the biological sample is a body fluid, in particular whole blood, plasma or serum, and the biological sample is processed prior to step a).

Processing in the sense of the present invention comprises cell separation or depletion and/or chromatography.

In case of an infection, the focus of infection may be important to combat the causing pathogen. Thus, it is advantageous to identify the region of infection. Hence, in a preferred embodiment, the systemic inflammation is caused by an infectious agent and a conclusion is drawn as to the region of origin of the infection caused by the infectious agent by the method according to the invention.

The region of origin of the infection caused by the infectious agent may be the abdomen, the respiratory system, or the urinary tract.

One or more regional biomarkers can assist in identifying the region of infection. Hence, the method may further comprise:

- determining the level of one or more regional biomarkers in the biological sample; and
- determining the region of origin of infection on basis of the level of the one or more regional biomarkers in the biological sample.

The one or more regional biomarkers are preferably selected from one or more of:

(i) the group consisting of Cell growth regulator with EF hand domain protein 1 (CGRE1), Coagulation factor VIII (FA8), Phospholipid transfer protein (PLTP), Protein Z-dependent protease inhibitor (ZPI), Alpha-1-antichymotrypsin (AACT), Matrix metalloproteinase-9 (MMP9), Interleukin-1 receptor antagonist protein (IL1RA), Neutrophil collagenase (MMP8), Chitinase-3-like protein 1 (CH3L1), Interleukin-1 receptor-like 1 (ILRL1), CD177 antigen (CD177), Neutrophil gelatinase-associated lipocalin (NGAL), Lactotransferrin (TRFL), Interleukin-18-binding protein (I18BP), Metalloproteinase inhibitor 1 (TIMP1), Lipopolysaccharide-binding protein (LBP), Macrophage mannose receptor 1 (MRC1), IgGFc-binding protein (FCGBP), and Inter-alpha-inhibitor heavy chain H3 (ITIH3) for determining an infection originating from the abdomen; or

(ii) the group consisting of Neuronal growth regulator 1 (NEGR1), Apolipoprotein C-I (APOC1), Plasma serine protease inhibitor (IPSP), Serum amyloid A-4 protein (SAA4), Phosphatidylinositol-glycan-specific phospholipase D (PHLD), Prenylcysteine oxidase 1 (PCYOX), Coagulation factorXIII B chain (F13B), Inter-alpha-inhibitor heavy chain H1 (ITIH1), Inter-alpha-inhibitor heavy chain H2 (ITIH2), and Vitamin K-dependent protein S (PROS) for determining an infection originating from the respiratory system.

In a very preferred embodiment of the present invention, the subject is a human subject. The subject may be healthy or ill based on a suspected or confirmed diagnosis. A human subject may also be referred to as patient.

In a further aspect of the present invention, a method of monitoring a systemic inflammation of a subject is provided, wherein the method comprises:

i) performing the method of *in vitro* diagnosing a systemic inflammation or prognosing a risk of mortality of a subject with a systemic inflammation as stated *supra*; and
ii) repeating step i) at least one time.

Monitoring allows assessing the therapeutic success or therapeutic failure. This in turn allows individually adapting the therapy. Hence, the method may further comprise repeating step ii) until diagnosing the absence of the systemic inflammation, or for monitoring the therapeutic success or therapeutic failure.

In one embodiment, wherein repeating step ii) comprises performing step i) at least two times, such as at least three times, at least four times, at least five times, at least six times, at least seven times, at least eight times, at least nine times, at least 10 times, at least 12 times, at least 15 times, at least 20 times, at least 25 times, at least 30 times, or at least 35 times, preferably at least 25 times.

In another embodiment, the method comprises repeating step ii) within 12 hours, in particular within 24 hours, more particularly within 48 hours for therapy control (monitoring). Usually, the step is repeated every 48 hours. If a worsening of the symptoms is observed or the subject is at a higher risk, for example because the subject has acquired another infection, the step may be repeated every 24 hours or every 12 hours.

In another embodiment, monitoring the therapeutic success or therapeutic failure comprises repeating step i) at least one time after a treatment of the systemic inflammation has been initiated or completed, preferably repeating performing step i) until diagnosing the absence of the systemic inflammation.

Monitoring also includes monitoring a subject with a non-infectious systemic inflammation with respect to development of a sepsis and progression of disease.

In a further aspect of the invention, a method of treating a systemic inflammation is provided comprising:

i) performing the method as described above, and

ii) initiating a treatment against the systemic inflammation.

A sepsis may be diagnosed in step b) and treatment may be initiated in step ii) with an antibiotic agent. The antibiotic treatment may be a standard antibiotic treatment according to the prevailing national and international guidelines, for example a broad spectrum antibiotic. The treatment with an antibiotic agent combats an infection with bacteria.

In a further aspect of the present invention, an antibiotic agent for use in a method of treating an infection in a subject or treating a subject with a suspected infection is provided, wherein the infection is part of a bloodstream infection, systemic infection or sepsis and wherein the bloodstream infection, systemic infection or sepsis is diagnosed or monitored by the level of sVSIG4 in a biological sample. In a preferred embodiment, the bloodstream infection, systemic infection or sepsis, preferably the sepsis, is diagnosed or monitored by the method as defined above. In a further preferred embodiment, the subject has an increased level of sVSIG4. The level may be compared to a reference level of sVSIG4 in a non-infected control.

In a further aspect of the present invention, a method of distinguishing between SIRS and sepsis in a subject is provided, wherein the method comprises:

a) determining the level of sVSIG4 in a biological sample of said subject, and
b) comparing the level of sVSIG4 in the biological sample with a reference level of sVSIG4 in a biological sample of a subject suffering from SIRS,

wherein an increased level in the biological sample of step a) compared with the reference level of step b) indicates a sepsis in the subject of step a). The method is particularly performed as described herein.

SIRS in this context in particular includes SIRS with and without organ dysfunction.

In yet a further aspect of the invention, sVSIG4 is used as a biomarker for *in vitro* diagnosing a systemic inflammation in a subject or prognosing a risk of mortality of a subject with a systemic inflammation. sVSIG4 can be used as the sole biomarker or in combination with one or more further biomarkers. The method is particularly performed as described herein.

In yet a further aspect of the invention, a kit is provided comprising a binding molecule to sVSIG4 and a binding molecule to at least one further biomarker for the quantitative detection of sVSIG4 and the at least one further biomarker.

Preferably, the at least one further biomarker detected by means of the kit is CRP and/or PCT.

It is also preferred to combine the kit with the determination of the lactate level in the subject's biological sample.

In a preferred embodiment, the kit is used by means of whole blood, plasma or serum of a subject, preferably plasma.

The detection may be based on a chromogenic, fluorescent and/or luminescent reaction, and/or a chromatographic method.

The binding molecule in the kit is preferably selected from the group consisting of an antibody, an aptamer, and a nanobody.

Preferably, the kit is based on an ELISA or chromatography.

In a preferred embodiment, the kit is a quick test or POC (point-of-care) test. Thereby, a fast and reliable diagnosis and prognosis is possible.

## FURTHER EMBODIMENTS

Embodiments of the present invention are described again and in further detail in the following. The present invention in particular discloses and provides for the following embodiments:

1. A method of *in vitro* diagnosing a systemic inflammation or prognosing a risk of mortality of a subject with a systemic inflammation, wherein the method comprises

c) determining the level of soluble V-set and immunoglobulin domain-containing protein 4 (sVSIG4) in a biological sample, and

d) drawing a conclusion as to the diagnosis of a systemic inflammation or the prognosis of a risk of mortality of a subject with a systemic inflammation from the presence and/or level of sVSIG4.

2. The method according to embodiment 1, wherein the systemic inflammation is caused by an infectious agent.

3. The method according to embodiment 2, wherein the systemic inflammation caused by an infectious agent is a sepsis, a systemic infection, or a bloodstream infection, preferably a sepsis.

4. The method according to embodiment 2 or 3, wherein the infectious agent is a bacterium, a fungus or a virus.

5. The method according to embodiment 4, wherein the bacterium is a gram negative or a gram positive bacterium.

6. The method according to embodiment 4, wherein the virus is influenza A virus, influenza B virus, respiratory syncytial virus (RSV), rhinovirus, and/or coronavirus, in particular severe acute respiratory syndrome coronavirus type 2 (SARS-CoV2).

7. The method according to embodiment 1, wherein the systemic inflammation is not caused by an infectious agent.

8. The method according to embodiment 7, wherein the systemic inflammation not caused by an infectious agent is systemic inflammatory reaction syndrome (SIRS).

9. The method according to one or more of embodiments 1 to 8, wherein a level of sVSIG4 in the biological sample of at least 20 pg/ml, at least 50 pg/ml, at least 100 pg/ml, at least 150 pg/ml, at least 200 pg/ml, or at least 250 pg/ml indicates the systemic inflammation.

10. The method according to one or more of embodiments 1 to 8, wherein a level of sVSIG4 in the biological sample of at least 20 pg/ml, at least 50 pg/ml, at least 100 pg/ml, at least 150 pg/ml, at least 200 pg/ml, or at least 250 pg/ml and less than 100000 pg/ml, less than 50000 pg/ml, less than 12500 pg/ml, less than 10000 pg/ml, or less than 6000 pg/ml indicates the SIRS.

11. The method according to one or more of embodiments 3 to 6, wherein a level of sVSIG4 in the biological sample of at least 200 pg/ml, at least 250 pg/ml, at least 300 pg/ml, at least 350 pg/ml, or at least 400 pg/ml indicates the bloodstream infection.

12. The method according to one or more of embodiments 3 to 6, wherein a level of sVSIG4 in the biological sample of at least 500 pg/ml, at least 550 pg/ml, at least 600 pg/ml, at least 650 pg/ml, at least 700 pg/ml, at least 750 pg/ml, at least 1000 pg/ml, at least 1500 pg/ml, at least 2500 pg/ml, at least 3500 pg/ml, at least 5000 pg/ml, at least 7500 pg/ml, at least 10000 pg/ml, or at least 12500 pg/ml indicates the sepsis.

13. The method according to one or more of embodiments 3 to 6, wherein a level of sVSIG4 in the biological sample of at least 1500 pg/ml, at least 5000 pg/ml, at least 10000 pg/ml, or at least 12500 pg/ml indicates the sepsis and discriminates between SIRS and sepsis.

14. The method according to one or more of embodiments 1 to 8, wherein a level of sVSIG4 in the biological sample of at least 4500 pg/ml, at least 9000 pg/ml, at least 15000 pg/ml, at least 40000 pg/ml, or at least 50000 pg/ml indicates a risk of mortality of a subject with a systemic inflammation caused by an infectious agent of at least 50 % within 28 days.

15. The method according to one or more of embodiments 1 to 8, wherein a level of sVSIG4 in the biological sample of at least 70000 pg/ml, at least 75000 pg/ml, at least 80000 pg/ml, or at least 85000 pg/ml, indicates a risk of mortality of a subject with a systemic inflammation caused by an infectious agent of at least 30 % within 28 days.

16. The method according to one or more of embodiments 1 to 15, wherein sVSIG4 comprises the extracellular domain or a fragment of the extracellular domain of VSIG4.

17. The method according to one or more of embodiments 1 to 16, wherein sVSIG4 does not comprise the transmembrane domain and cytoplasmic domain of VSIG4.

18. The method according to embodiment 16 or 17, wherein VSIG4 is selected from the group consisting of VSIG4 isoform 1 (UniProt Identifier Q9Y279-1; SEQ ID NO: 1), VSIG4 isoform 2 (UniProt Identifier Q9Y279-2; SEQ ID NO: 2) and VSIG4 isoform 3 (UniProt Identifier Q9Y279-3; SEQ ID NO: 3) or has an amino acid sequence which is at least 90% identical to the amino acid sequence of VSIG4 isoform 1 (UniProt Identifier Q9Y279-1; SEQ ID NO: 1), VSIG4 isoform 2 (UniProt Identifier Q9Y279-2; SEQ ID NO: 2) or VSIG4 isoform 3 (UniProt Identifier Q9Y279-3; SEQ ID NO: 3).

19. The method according to one or more of embodiments 16 to 18, wherein the extracellular domain comprises Ig-like domain 1 (SEQ ID NO: 4), or Ig-like domain 1 (SEQ ID NO: 4) and Ig-like domain-2 (SEQ ID NO: 5), or the extracellular domain as defined in SEQ ID NO: 6.

20. The method according to one or more of embodiments 1 to 19, wherein sVSIG4 is dissolved in the biological sample, in particular in the non-cellular fraction of the biological sample.

21. The method according to one or more of embodiments 1 to 20, wherein the method further comprises:

- determining the level of one or more additional biomarkers in the biological sample; and
- in step b) drawing a conclusion as to the diagnosis of a systemic inflammation or the prognosis of a risk of mortality of a subject with a systemic inflammation from the presence and/or level of sVSIG4 in combination with the presence and/or level of the one or more additional biomarkers.

22. The method according to embodiment 21, wherein the one or more additional biomarkers are selected from the group consisting of IgGFc-binding protein (FCGBP), GDNF family receptor alpha-2 (GFRA2), Carboxypeptidase D (CBPD), Asialoglycoprotein receptor 1 (ASGR1), Hypoxia upregulated protein 1 (HYOU1), Tenascin (TENA), Polymeric immunoglobulin receptor (PIGR), Sushi, von Willebrand factor type A, EGF and Pentraxin domain-containing protein 1 (SVEP1), Interleukin-1 receptor type 2 (IL1R2), Laminin subunit beta-1 (LAMB1), Endoplasmic reticulum chaperone BiP (BIP), Peroxidasin homolog (PXDN), Follistatin-related protein 1 (FSTL1), Leucine-rich alpha-2 glycoprotein (A2GL), Metalloproteinase inhibitor 1 (TIMP1), NPC intracellular cholesterol transporter 2 (NPC2), Peptidoglycan recognition protein 1 (PGRP1), Lactotransferrin (TRFL), Dystonin (DYST), Lipopolysaccharide-binding protein (LBP), Haptoglobin (HPT), Asialoglycoprotein receptor 2 (ASGR2), Non-secretory ribonuclease (RNAS2), Azurocidin (CAP7), Chitinase-3-like protein 1 (CH3L1), Tumor necrosis factor receptor superfamily member 1B (TNR1B), HLA class II histocompatibility antigen gamma chain (HG2A), Olfactomedin-4 (OLFM4), Leukocyte immunoglobulin-like receptor subfamily A member 3 (LIRA3), Insulin-like growth factor-binding protein 2 (IBP2), Growth/differentiation factor 15 (GDF15), Laminin subunit alpha-2 (LAMA2), Tyrosine-protein phosphatase non-receptor type substrate 1 (SHPS1), Basigin (BASI), Fibroleukin (FGL2), Cathepsin Z (CATZ), E-selectin (LYAM2), Lymphatic vessel endothelial hyaluronic acid receptor 1 (LYVE1), Disintegrin and metalloproteinase domain-containing protein 9 (ADAM9), Desmocollin-2 (DSC2), Interleukin-1 receptor-like 1 (ILRL1), Matrix metalloproteinase-9 (MMP9), Cystatin-C (CYTC), Interleukin-18-binding protein (I18BP), Paired immunoglobulin-like type 2 receptor alpha (PILRA), Macrophage mannose receptor 1 (MRC1), Osteopontin (OSTP), Scavenger receptor cysteine-rich type 1 protein M130 (C163A), Integral membrane protein 2B (ITM2B), Fibrinogen-like protein 1 (FGL1), Serum amyloid A-1 protein (SAA1), Interleukin-1 receptor antagonist protein (IL1RA), Nucleobindin-1 (NUCB1), Golgi membrane protein 1 (GOLM1), Dystroglycan (DAG1), CD177 antigen (CD177), Alkaline phosphatase, tissue-nonspecific isozyme (PPBT), Neutrophil collagenase (MMP8), Myeloblastin (PRTN3), Neutrophil elastase (ELNE), Beta-1,4-galactosyltransferase 1 (B4GT1), C-reactive protein (CRP), WAP four-disulfide core domain protein 2 (WFDC2), Follistatin-related protein 3 (FSTL3), Ribonuclease pancreatic (RNAS1), Neutrophil gelatinase-associated lipocalin (NGAL), Serum amyloid A-2 protein (SAA2), Lithostathine-1-alpha (REG1A), Plasma kallikrein (KLKB1), Phosphatidylcholine-sterol acyltransferase (LCAT), Serotransferrin (TRFE), Procalcitonin (PCT)), Complement receptor type 2 (CR2), Voltage-dependent calcium channel subunit alpha-2/delta-1 (CA2D1) Indian hedgehog protein (IHH), Serum paraoxonase/lactonase 3 (PON3), Fibronectin (FINC), Beta-Ala-His-dipeptidase (CNDP1), Insulin-like growth factor-binding protein complex acid labile subunit (ALS), Phosphatidylinositol-glycan-specific phospholipase D (PHLD), Insulin-like growth factor-binding protein 3 (IBP3), Anthrax toxin receptor 1 (ANTR1), Neuronal growth regulator 1 (NEGR1), Plasma serine protease inhibitor (IPSP), Intelectin-1 (ITLN1), Kallistatin (KAIN), Fibroblast growth factor receptor 1 (FGFR1), Alpha-2-HS-glycoprotein (FETUA), Cholinesterase (CHLE), Afamin (AFAM), Cathepsin F (CATF), Cholesteryl ester transfer protein (CETP), Cadherin-related family member 5 (CDHR5), Inter-alpha-trypsin inhibitor heavy chain H2 (ITIH2), N-acetylmuramoyl-L-alanine amidase (PGRP2), Contactin-1 (CNTN1), Apolipoprotein(a) (APOA), Cell growth regulator with EF hand domain protein 1 (CGRE1), Coagulation factor VIII (FA8), Phospholipid transfer protein (PLTP), Protein Z-dependent protease inhibitor (ZPI), Alpha-1-antichymotrypsin (AACT), Vitamin K-dependent protein S (PROS), Coagulation factor XIII B chain (F13B), Prenylcysteine oxidase 1 (PCYOX), Serum amyloid A-4 protein (SAA4) Apolipoprotein C-I (APOC1), Prolyl endopeptidase FAP (SEPR), Dipeptidylpeptidase 4 (DPP4), Angiotensin-converting enzyme 2 (ACE2), Interleukin-1 receptor accessory protein (IL1AP), Di-N-acetylchitobiase (DIAC), Hepatocyte growth factor activator (HGFA), Selenoprotein P (SEPP1), A disintegrin and metalloproteinase with thrombospondin motifs 13 (ATS13), Monocyte differentiation antigen CD14 (CD14), Complement factor H-related protein 1 (FHR1), von Willebrand factor (VWF), Laminin subunit gamma-1 (LAMC1), Scavenger receptor class A member 5 (SCAR5), ADAMTS-like protein 4 (ATL4), Cullin-1 (CUL1), Pulmonary surfactant-associated protein B (PSPB), Neuroblastoma suppressor of tumorigenicity (NBL1), Ganglioside GM2 activator (SAP3), Protein disulfide isomerase CRELD1 (CREL1), Cadherin-related family member 2 (CDHR2), Chymotrypsin-like elastase family member 3B (CEL3B), Phosphoinositide-3-kinase-interacting protein 1 (P3IP1), Lithostathine-1-beta (REG1B), Kininogen-1 (KNG1), Versican core protein (CSPG2), EMILIN-2 (EMIL2), Carcinoembryonic antigen-related cell adhesion molecule 6 (CEAM6), Fibromodulin (FMOD), Beta-galactoside alpha-2,6-sialyltransferase 1 (SIAT1), Leukocyte immunoglobulin-like receptor subfamily B member 5 (LIRB5), Latent-transforming growth factor beta-binding protein 2 (LTBP2), Chromogranin-A (CMGA) and Adseverin (ADSV), Histidine-rich glycoprotein (HRG), Inter-alpha-trypsin inhibitor heavy chain H1 (ITIH1), and Inter-alpha-trypsin inhibitor heavy chain H3 (ITIH3), wherein in step b) a conclusion is drawn as to the diagnosis of a systemic inflammation.

23. The method according to embodiment 21 or 22, wherein the one or more additional biomarkers in the biological sample are selected from one or more of the following:

(i) Inter-alpha-trypsin inhibitor heavy chain H2 (ITIH2), Inter-alpha-trypsin inhibitor heavy chain H1 (ITIH1), Phosphatidylinositol-glycan-specific phospholipase D (PHLD), N-acetylmuramoyl-L-alanine amidase (PGRP2), Kallistatin (KAIN), Alpha-2-HS-glycoprotein (FETUA), Inter-alpha-trypsin inhibitor heavy chain H3 (ITIH3), Afamin (AFAM), Macrophage mannose receptor 1 (MRC1), Cholinesterase (CHLE), Insulin-like growth factor-binding protein complex acid labile subunit (ALS), Serotransferrin (TRFE), Phosphatidylcholine-sterol acyltransferase (LCAT), Beta-Ala-His dipeptidase (CNDP1), Plasma kallikrein (KLKB1), Alpha-1-antichymotrypsin (AACT), Monocyte differentiation antigen CD14 (CD14), Neutrophil gelatinase-associated lipocalin (NGAL), Hepatocyte growth factor activator (HGFA), Scavenger receptor cysteine-rich type 1 protein M130 (C163A), Fibronectin (FINC), Histidine-rich glycoprotein (HRG), and Alpha-1-antitrypsin (A1AT);

(ii) Phosphatidylinositol-glycan-specific phospholipase D (PHLD), Kallistatin (KAIN), Alpha-2-HS-glycoprotein (FETUA), Afamin (AFAM), Macrophage mannose receptor 1 (MRC1), Cholinesterase (CHLE), Insulin-like growth factor-binding protein complex acid labile subunit (ALS), Beta-Ala-His dipeptidase (CNDP1), Neutrophil gelatinase-associated lipocalin (NGAL), Scavenger receptor cysteine-rich type 1 protein M130 (C163A), Plasma serine protease inhibitor (IPSP), Leucine-rich alpha-2-glycoprotein (A2GL), Lithostathine-1-alpha (REG1A), Lipopolysaccharide-binding protein (LBP), Lymphatic vessel endothelial hyaluronic acid receptor 1 (LYVE1), Insulin-like growth factor-binding protein 3 (IBP3), Complement receptor type 2 (CR2), Fibronectin (FINC), Asialoglycoprotein receptor 2 (ASGR2), Alkaline phosphatase, tissue-nonspecific isozyme (PPBT), Serum amyloid A-2 protein (SAA2), and Dipeptidylpeptidase 4 (DPP4);

(iii) Inter-alpha-trypsin inhibitor heavy chain H2 (ITIH2), N-acetylmuramoyl-L-alanine amidase (PGRP2), and Monocyte differentiation antigen CD14 (CD14);

(iv) Inter-alpha-trypsin inhibitor heavy chain H2 (ITIH2);

(v) Phosphatidylinositol-glycan-specific phospholipase D (PHLD), Leucine-rich alpha-2-glycoprotein (A2GL), and Insulin-like growth factor-binding protein 3 (IBP3);

(vi) Phosphatidylinositol-glycan-specific phospholipase D (PHLD);

(vii) C-reactive protein (CRP);

(viii) Procalcitonin (PCT);

(ix) Lithostathine-1-alpha (REG1A);

(x) Myeloblastin (PRTN3);

(xi) CRP and PCT,

wherein in step b) a conclusion is drawn as to the diagnosis of a systemic inflammation.

24. The method according to embodiment 21, wherein the one or more additional biomarkers are selected from the group consisting of Kininogen-1 (KNG1), Tenascin (TENA), Versican core protein (CSPG2), Cadherin-related family member 2 (CDHR2), EMILIN-2 (EMIL2), Osteopontin (OSTP), Tyrosine-protein phosphatase non-receptor type substrate 1 (SHPS1), Lithostathine-1-beta (REG1B), Carcinoembryonic antigen-related cell adhesion molecule 6 (CEAM6), Paired immunoglobulin-like type 2 receptor alpha (PILRA), HLA class II histocompatibility antigen gamma chain (HG2A), Scavenger receptor cysteine-rich type 1 protein M130 (C163A), Fibroleukin (FGL2), Follistatin-related protein 3 (FSTL3), Fibromodulin (FMOD), Beta-galactoside alpha-2,6-sialyltransferase 1 (SIAT1), Myeloblastin (PRTN3), Leukocyte immunoglobulin-like receptor subfamily B member 5 (LIRB5), N-acetylmuramoyl-L-alanine amidase (PGRP2), Interleukin-1 receptor-like 1 (ILRL1), Neutrophil gelatinase-associated lipocalin (NGAL), Latent-transforming growth factor beta-binding protein 2 (LTBP2), Interleukin-1 receptor antagonist protein (IL1RA), Chromogranin-A (CMGA), Phosphoinositide-3-kinase-interacting protein 1 (P3IP1), Ribonuclease pancreatic (RNAS1), Ganglioside GM2 activator (SAP3), Neutrophil elastase (ELNE), Adseverin (ADSV), Disintegrin and metalloproteinase domain-containing protein 9 (ADAM9), Lithostathine-1-alpha (REG1A), Nucleobindin-1 (NUCB1), and WAP four-disulfide core domain protein 2 (WFDC2),

wherein in step b) a conclusion is drawn as to the prognosis of a risk of mortality of a subject with a systemic inflammation.

25. The method according to embodiment 21 or 22, wherein the one or more additional biomarkers in the biological sample are selected from one or more of the following:

(i) Kininogen-1 (KNG1) and Tenascin (TENA);

(ii) Versican core protein (CSPG2), Cadherin-related family member 2 (CDHR2), EMILIN-2 (EMIL2), Osteopontin (OSTP), Tyrosine-protein phosphatase non-receptor type substrate 1 (SHPS1), Lithostathine-1-beta (REG1B), Carcinoembryonic antigen-related cell adhesion molecule 6 (CEAM6), Paired immunoglobulin-like type 2 receptor alpha (PILRA), HLA class II histocompatibility antigen gamma chain (HG2A), Scavenger receptor cysteine-rich type 1 protein M130 (C163A), Fibroleukin (FGL2), Follistatin-related protein 3 (FSTL3), Fibromodulin (FMOD), Beta-galactoside alpha-2,6-sialyltransferase 1 (SIAT1), Myeloblastin (PRTN3), Leukocyte immunoglobulin-like receptor subfamily B member 5 (LIRB5), N-acetylmuramoyl-L-alanine amidase (PGRP2), Interleukin-1 receptor-like 1 (ILRL1), Neutrophil gelatinase-associated lipocalin (NGAL), Latent-transforming growth factor beta-binding protein 2 (LTBP2), Interleukin-1 receptor antagonist protein (IL1RA), Chromogranin-A (CMGA), Phosphoinositide-3-kinase-interacting protein 1 (P3IP1), Ribonuclease pancreatic (RNAS1), Ganglioside GM2 activator (SAP3), Neutrophil elastase (ELNE), Adseverin (ADSV), Disintegrin and metalloproteinase domain-containing protein 9 (ADAM9), Lithostathine-1-alpha (REG1A), Nucleobindin-1 (NUCB1), and WAP four-disulfide core domain protein 2 (WFDC2);

(iii) Scavenger receptor cysteine-rich type 1 protein M130 (C163A), Kallistatin (KAIN), Macrophage mannose receptor 1 (MRC1), Lymphatic vessel endothelial hyaluronic acid receptor 1 (LYVE1), Cholinesterase (CHLE), Ribonuclease pancreatic (RNAS1), Phospahtidylinositol-glycan-specific phospholipase D (PHLD), Afamin (AFAM), Insulin-like growth factor-binding protein complex acid labile subunit (ALS), Neutrophil gelatinase-associated lipocalin (NGAL), Phosphoinositide-3-kinase-interacting protein 1 (P3IP1), Alpha-2-HS-glycoprotein (FETUA), Ganglioside GM2 activator (SAP3), Beta-Ala-His-dipeptidase (CNDP1), Paired immunoglobulin-like type 2 receptor alpha (PILRA), CD177 antigen (CD177), V-type proton ATPase subunit S1 (VAS1), Plasma serine protease inhibitor (IPSP), Follistatin-related protein 3 (FSTL3), Pulmonary surfactant-associated protein B (PSPB), Tumor necrosis factor receptor superfamily member 1B (TNR1B), WAP four-disulfide core domain protein 2 (WFDC2), Alkaline phosphatase, tissue-nonspecific isozyme (PPBT), and Metalloproteinase inhibitor 1 (TIMP1);

(iv) C-reactive protein (CRP);

(v) Procalcitonin (PCT);

(vi) Lithostathine-1-alpha;

(vii) CRP and PCT,

wherein in step b) a conclusion is drawn as to the prognosis of a risk of mortality of a subject with a systemic inflammation.

26. The method according to one or more of embodiments 3 to 25, wherein a predictor based on the determination of the levels of sVSIG4 and CRP in the biological sample and calculated by -0.4268478 x ln(sVSIG4) - 0.4500494 x ln(CRP) + 5.927552 less than 3, preferably less than 0.5, preferably less than 0, more preferably less than -0.2 indicates the sepsis.

27. The method according to one or more of embodiments 1 to 25, wherein a predictor based on the determination of the levels of sVSIG4 and CRP in the biological sample and calculated by -0.4268478 x ln(sVSIG4) - 0.4500494 x ln(CRP) + 5.927552 less than 1, preferably less than 0, more preferably less than -1 indicates a risk of mortality of a subject with a systemic inflammation, preferably a sepsis, of at least 50 % within 28 days.

28. The method according to one or more of embodiments 3 to 25, wherein a predictor based on the determination of the levels of sVSIG4 and myeloblastin (PRTN3) in the biological sample and calculated by -0.4331127 x ln(sVSIG4) -0.4901329 x ln(PRTN3) +9.942252 less than 2, preferably less than 1, more preferably less than -0.1 indicates the sepsis.

29. The method according to one or more of embodiments 1 to 25, wherein a predictor based on the determination of the levels of sVSIG4 and myeloblastin (PRTN3) in the biological sample and calculated by -0.4331127 x ln(sVSIG4) -0.4901329 x ln(PRTN3) +9.942252 less than 0, preferably less than -1 indicates a risk of mortality of a subject with a systemic inflammation, preferably a sepsis, of at least 50 % within 28 days.

30. The method according to one or more of embodiments 1 to 25, wherein in step a) in addition to determining the level of sVSIG4, the level of one or more further soluble proteins in the biological sample is determined.

31. The method according to one or more of embodiments 1 to 30, wherein the method includes further parameters for diagnosing a systemic inflammation, the further parameters being in particular Sequential Organ Failure Assessment (SOFA) score, Quick Sequential Organ Failure Assessment (qSOFA) score, Acute Physiology and Chronic Healthy Evaluation II (APACHE-II) score and/or Simplified Acute Physiology Score II (SAPS-II).

32. The method according to one or more of embodiments 1 to 31, wherein the method comprises enriching glycosylated proteins in the biological sample.

33. The method according to embodiment 32, wherein the enriching glycosylated proteins in the biological sample is performed prior to step a).

34. The method according to one or more of embodiments 32 or 33, wherein enriching glycosylated proteins in the biological sample includes performing one or more separation steps with the biological sample, in particular prior to step a).

35. The method according to embodiment 34, wherein the one or more separation steps include chromatographic separation, preferably affinity chromatographic separation.

36. The method according to embodiment 35, wherein the affinity chromatographic separation is selected from the group consisting of lectin affinity chromatography, separation by hydrazide chemistry, hydrophilic interaction chromatography and immunoaffinity chromatography.

37. The method according to one or more of embodiments 34 to 36, wherein proteins of the biological sample are enzymatically degraded in the one or more separation steps.

38. The method according to one or more of embodiments 1 to 37, wherein step a) is performed by one or more methods selected from the group consisting of chromatography, spectrometry, electrophoresis, spectroscopy, biochemical assay and immunoassay, preferably spectrometry, in particular mass spectrometry, and/or immunoassay.

39. The method according to embodiment 38, wherein the mass spectrometry is a liquid chromatography-mass spectrometry (LC-MS), LC-MS/MS or matrix-assisted laser desorption ionization-time of flight mass spectrometry (MALDI-TOF MS).

40. The method according to embodiment 38 or 39, wherein the immunoassay is selected from one or more of the group consisting of enzyme-linked immunosorbent assay (ELISA), immunoscreening, lateral flow immunochromatographic assay, magnetic immunoassay and radio immunoassay, preferably ELISA.

41. The method according to one or more of embodiments 1 to 40, wherein step a) comprises performing LC-MS, LC-MS/MS or MALDI-TOF MS and a subsequent step of analyzing the obtained data for determining the level of sVSIG4 in the biological sample.

42. The method according to one or more of embodiments 1 to 41, wherein the method comprises:

- performing a depletion step and/or a fractionation step and/or enriching glycosylated proteins in the biological sample prior to step a); and
- performing mass spectrometry in step a) for determining the level of sVSIG4 in the biological sample.

43. The method according to embodiment 42, wherein

- enriching glycosylated proteins comprises performing an affinity chromatographic separation step, in particular lectin affinity chromatography, separation by hydrazide chemistry or immunoaffinity chromatography;
- optionally, proteins of the biological sample are enzymatically degraded in the affinity chromatographic separation step.

44. The method according to one or more of embodiments 1 to 43, wherein the biological sample is a body fluid sample.

45. The method according to one or more of embodiments 1 to 44, wherein the biological sample is the non-cellular fraction of a biological sample.

46. The method according to embodiment 44 or 45, wherein the body fluid sample is selected from one or more of the group consisting of whole blood, plasma, serum, synovial fluid, pleural effusion, lymphatic fluid, urine, liquor, cerebrospinal fluid, ascites, and bronchial lavage, and samples derived from the foregoing, in particular cell-free or cell-depleted samples derived from the foregoing samples by removing cells.

47. The method according to one or more of embodiments 1 to 46, wherein the biological sample is selected from the group consisting of whole blood, plasma and serum, preferably plasma.

48. The method according to one or more of embodiments 1 to 47, wherein the biological sample is a cell-free or cell-depleted sample.

49. The method according to one or more of embodiments 1 to 48, wherein the biological sample is processed prior to step a), in particular by obtaining the non-cellular fraction of the biological sample.

50. The method according to one or more of embodiments 1 to 49, wherein the biological sample is a body fluid, in particular whole blood, plasma or serum, and the biological sample is processed prior to step a).

51. The method according to embodiment 49 or 50, wherein processing comprises cell separation or depletion and/or chromatography.

52. The method according to one or more of embodiments 1 to 51, wherein additionally a conclusion is drawn as to the region of origin of the infection caused by the infectious agent.

53. The method according to embodiment 52, wherein the region of origin of the infection caused by the infectious agent is the abdomen, the respiratory system, or the urinary tract.

54. The method according to one or more of embodiments 52 or 43, wherein the method further comprises:

- determining the level of one or more regional biomarkers in the biological sample; and
- determining the region of origin of infection caused by the infectious agent on basis of the presence and/or level of the one or more regional biomarkers in the biological sample.

55. The method according to embodiment 54, wherein the one or more regional biomarkers are selected from one or more of:

(i) the group consisting of Cell growth regulator with EF hand domain protein 1 (CGRE1), Coagulation factor VIII (FA8), Phospholipid transfer protein (PLTP), Protein Z-dependent protease inhibitor (ZPI), Alpha-1-antichymotrypsin (AACT), Matrix metalloproteinase-9 (MMP9), Interleukin-1 receptor antagonist protein (IL1RA), Neutrophil collagenase (MMP8), Chitinase-3-like protein 1 (CH3L1), Interleukin-1 receptor-like 1 (ILRL1), CD177 antigen (CD177), Neutrophil gelatinase-associated lipocalin (NGAL), Lactotransferrin (TRFL), Interleukin-18-binding protein (I18BP), Metalloproteinase inhibitor 1 (TIMP1), Lipopolysaccharide-binding protein (LBP), Macrophage mannose receptor 1 (MRC1), IgGFc-binding protein (FCGBP), and Inter-alpha-inhibitor heavy chain H3 (ITIH3) for determining an infection originating from the abdomen; or
(ii) the group consisting of Neuronal growth regulator 1 (NEGR1), Apolipoprotein C-I (APOC1), Plasma serine protease inhibitor (IPSP), Serum amyloid A-4 protein (SAA4), Phosphatidylinositol-glycan-specific phospholipase D (PHLD), Prenylcysteine oxidase 1 (PCYOX), Coagulation factor XIII B chain (F13B), Inter-alpha-inhibitor heavy chain H1 (ITIH1), Inter-alpha-inhibitor heavy chain H2 (ITIH2), and Vitamin K-dependent protein S (PROS) for determining an infection originating from the respiratory system.

56. The method according to one or more of embodiments 1 to 55, wherein the subject is a human subject.

57. A method of monitoring a systemic inflammation of a subject, wherein the method comprises:

iii) performing the method of *in vitro* diagnosing a systemic inflammation or prognosing a risk of mortality of a subject with a systemic inflammation according to one or more of embodiments 1 to 56; and

iv) repeating step i) at least one time.

58. The method according to embodiment 57, wherein the method comprises repeating step ii) until diagnosing the absence of the systemic inflammation, or for monitoring the therapeutic success or therapeutic failure.

59. The method according to embodiment 57 or 58, wherein repeating step ii) comprises performing step i) at least two times, such as at least three times, at least four times, at least five times, at least six times, at least seven times, at least eight times, at least nine times, at least 10 times, at least 12 times, at least 15 times, at least 20 times, at least 25 times, at least 30 times, or at least 35 times, preferably at least 25 times.

60. The method according to one or more of embodiments 57 to 59, wherein the method comprises repeating step ii) within 12 hours, in particular within 24 hours, more particularly within 48 hours.

61. The method according to embodiment 58, wherein monitoring the therapeutic success or therapeutic failure comprises repeating step i) at least one time after a treatment of the systemic inflammation has been initiated or completed, preferably repeating performing step i) until diagnosing the absence of the systemic inflammation.

62. The method according to one or more of embodiments 57 to 61, wherein a subject with a systemic inflammation not caused by an infectious agent is monitored with respect to development of a sepsis.

63. A method of treating a systemic inflammation comprising:

    i) performing the method according to one or more of embodiments 1 to 62, and

    ii) initiating a treatment against the systemic inflammation.

64. The method according to embodiment 63, wherein a sepsis is diagnosed in step b) and treatment is initiated in step ii) of embodiment 63 with an antibiotic agent.

65. An antibiotic agent for use in a method of treating an infection in a subject or treating a subject with a suspected infection, wherein the infection is part of a bloodstream infection, systemic infection or sepsis and wherein the bloodstream infection, systemic infection or sepsis is diagnosed or monitored by the level of sVSIG4 in a biological sample.

66. The antibiotic agent for use in a method according to embodiment 65, wherein the subject has an increased level of sVSIG4 compared to a non-infected control.

67. The antibiotic agent for use in a method according to one or more of embodiments 65 or 66, wherein the bloodstream infection, systemic infection or sepsis is diagnosed or monitored by the method as defined according to one or more of embodiments 1 to 62.

68. A method of distinguishing between SIRS and sepsis in a subject, wherein the method comprises:

    a) determining the level of sVSIG4 in a biological sample of said subject, and
    b) comparing the level of sVSIG4 in the biological sample with a reference level of sVSIG4 in a biological sample of a subject suffering from SIRS,

wherein an increased level in the biological sample of step a) compared with the reference level of step b) indicates a sepsis in the subject of step a).

69. The method according to embodiment 68, wherein the method has the features as defined in one or more of embodiments 1 to 62.

70. Use of sVSIG4 as a biomarker for *in vitro* diagnosing a systemic inflammation in a subject or prognosing a risk of mortality of a subject with a systemic inflammation.

71. The use of embodiment 70, wherein the sVSIG4 is used in a method as defined according to one or more of embodiments 1 to 62.

72. A kit comprising a binding molecule to sVSIG4 and a binding molecule to at least one further biomarker for the quantitative detection of sVSIG4 and the at least one further biomarker.

73. The kit according to embodiment 72, wherein the detection is based on a chromogenic, fluorescent and/or luminescent reaction, and/or a chromatographic method.

74. The kit according to embodiment 72 or 73, wherein the binding molecule is selected from the group consisting of an antibody, an aptamer, and a nanobody.

75. The kit according to one or more of embodiments 72 to 74, wherein the kit is a quick test or POC (point-of-care) test.

This invention is not limited by the exemplary methods and materials disclosed herein, and any methods and materials similar or equivalent to those described herein can be used in the practice or testing of embodiments of this invention. Numeric ranges are inclusive of the numbers defining the range. The headings provided herein are not limitations of the various aspects or embodiments of this invention which can be read by reference to the specification as a whole.

As used in the subject specification, items and claims, the singular forms "a", "an" and "the" include plural aspects unless the context clearly dictates otherwise. The terms "include," "have," "comprise" and their variants are used synonymously and are to be construed as non-limiting. Further components and steps may be present. Throughout the specification, where compositions are described as comprising components or materials, it is additionally contemplated that the compositions can in embodiments also consist essentially of, or consist of, any combination of the recited components or materials, unless described otherwise. Reference to "the disclosure" and "the invention" and the like includes single or multiple aspects taught herein; and so forth. Aspects taught herein are encompassed by the term "invention".

It is preferred to select and combine preferred embodiments described herein and the specific subject-matter arising from a respective combination of preferred embodiments also belongs to the present disclosure.

## EXAMPLES

It should be understood that the following examples are for illustrative purpose only and are not to be construed as limiting this invention in any manner. The below examples demonstrate that sVSIG4 is suitable for use in a method of *in vitro* diagnosis of a systemic inflammation, in particular sepsis. Specifically, the examples show that sVSIG4 is a suitable biomarker for distinguishing a systemic inflammation caused by an infectious agent, such as a sepsis, from a systemic inflammation not caused by an infectious agent, such as SIRS. The examples further demonstrate that sVSIG4 is a suitable biomarker for use in a method of monitoring a systemic inflammation of a subject, in particular sepsis.

### Identification of sepsis-associated biomarkers

For the purpose of identifying sepsis-associated biomarkers in patient plasma, plasma samples available from the biobank of Jena University Hospital were used, which were collected according to the sepsis-2 definition at Jena University Hospital, Department of Anesthesiology and Critical Care Medicine (sepsis criteria according to American College of Chest Physicians/Society of Critical Care Medicine consensus conference; see Chest 1992; 101(6):1644-55.). Patients in the intensive care unit with positive SIRS criteria who were neither suspected nor proven to have an underlying infection served as the control group. Patients were recruited by dividing them into four groups: Group 1 - patients with SIRS, Group 2 - patients with SIRS and organ dysfunction, Group 3 - patients with severe sepsis, and Group 4 - patients with septic shock. Samples from these patients were finally combined into 2 groups for proteomic analyses: a SIRS group with SIRS and SIRS+organ dysfunction subgroups, and a sepsis group with severe sepsis and septic shock subgroups. Patients with lower grade "sepsis" according to sepsis-2 definition were not analyzed.

For the analyses, citrated plasma samples from the earliest possible time (day 1 or 2) after sepsis diagnosis by the treating physician were selected to identify early markers of systemic infection and onset of severe sepsis or septic shock. In a first patient cohort, 264 patients, 143 with severe sepsis (n=26) or septic shock (n=117) and 121 patients with SIRS (n=53) or SIRS+organ dysfunction (n=68) were studied (see clinical parameters in Table 4). The two groups of patients showed no differences in age or body mass index (BMI). Slightly more men than women were recruited in both groups, but the groups did not differ statistically significantly. The critical care scoring systems SOFA-score, APACHE-score, and SAPS-score were significantly increased in the sepsis group. Fever, leukocytosis, hypoxemia, renal dysfunction, metabolic acidosis, and hypotension also occurred significantly more frequently in the sepsis group. PCT and CRP were significantly elevated in the sepsis group. Microbiological verification of infection was successful in 39.2% of all cases in the sepsis group, with gram-positive bacteria detected in 34.3%, gram-negative bacteria in 16.1%, and fungal infections in 7.7% of all positive cases. 53.8% of all sepsis patients had an infection focus in the respiratory tract, 35.0% in the abdomen, and 4.9% in the genitourinary tract. The mortality rates for in-hospital mortality and intensive care unit mortality were 44.8% and 33.5%, respectively, in the sepsis group and 14% and 9.0%, respectively, in the SIRS group.

Table 4. Clinical parameters of patients with sepsis or SIRS in the discovery cohort (cohort 1) for the First Available Sample (day 1 or 2) for each patient. Data are median and interquartile range (IQR) or counts, No. (%). Definition of abbreviations: APACHE = Acute Physiology and Chronic Healthy Evaluation II, SOFA = Sequential Organ Failure Assessment, SAPS = Simplified Acute Physiology Score II, PCT = Procalcitonin (ng/ml), CRP = C-reactive protein (mg/l), WBC: white blood count, ICU = intensive care unit. Statistical analysis was done by Mann-Whitney-U-Test or

§Chi-square test.

| Parameters | SIRS group (n=121) SIRS n=53, SIRS+OD n=68 | Sepsis group (n=143) Severe sepsis n=26, Septic shock n=117 | p-value |
|---|---|---|---|
| **Gender,** female/male | 39/82 (32%) | 51/92 (36%) | 0.648§ |
| **Age,** median, (IQR range) | 66 (58-73) | 67(54-74) | 0.598 |
| **SOFA** median (IQR) | 7.0 (5-9) | 10.0 (8-11) | <0.001 |
| **APACHE** median (IQR) | 14 (10-18) | 20 (15-24) | <0.001 |
| **SAPS** median (IQR) | 33 (23-42) | 40 (33-48) | <0.001 |
| **WBC** cells/mm$^3$median (IQR) | 14.3 (10.8-20.3) | 18.0 (10.7-27.3) | <0.008 |
| **PCT conc.** median (IQR) | 2.0 (0.5-6.0) | 3.3 (1.5-10.4) | <0.001 |
| **CRP** conc. median (IQR) | 64.5 (29.8-98.4) | 194 (116-253) | <0.001 |
| **Hospital mortality** No. (%) | 17 (14.0) | 64 (44.8) | <0.001§ |
| **ICU mortality** No., (%) | 11 (9.0) | 48 (33.5) | <0.001§ |
| **Died** of **sepsis** No., (%) | 4 (3.3) | 43 (30.0) | <0.001§ |
| **Fever** No., (%) | 51 (42.1) | 95 (66.4) | <0.001§ |
| **Tachycardia** No., (%) | 102 (84.3) | 12990.2) | 0.207§ |
| **Tachypnea** No., (%) | 75 (62.0) | 96 (67.1) | 0.457§ |
| **Leukocytosis** No., (%) | 70 (57.8) | 110 (76.9) | 0.001§ |
| **Thrombocytopenia** No., (%) | 32 (26.4) | 32 (22.4) | 0.532§ |
| **Hypoxemia** No., (%) | 65 (53.7) | 124 (86.7) | <0.001§ |
| **Hypotension** No., (%) | 74 (61.2) | 135 (94.4) | <0.001§ |
| **Vasoactive drug treatment** No., (%) | 35 (28.9) | 108 (75.5) | <0.001§ |
| **Renal dysfunction** No., (%) | 22 (18.2) | 71 (49.7) | <0.001§ |
| **Metabolic acidosis** No., (%) | 26 (21.5) | 54 (37.8) | 0.006§ |
| **Bilirubin** sample day >20 μM | 34 (28.1) | 64 (44.8) | |
| **Bilirubin conc.** (median (IQR) | 13.9 (10.1-20.3) | 18 (12.05-31) | <0.001 |
| **Lactate** sample day>2mmol litre$^{-1}$ | 74 (61.2) | 84 (58.7) | |
| **Lactat conc.** median (IQR) | 2.4 (1.4-3.7) | 2.2 (1.5-3.8) | 0.709 |
| **Microbiology** (No. positive) | | 56 (39.2) | |
| Gram positive No., (%) | | 49 (34.3) | |
| Gram negative No., (%) | | 23 (16.1) | |
| Fungi No., (%) | | 11 (7.7) | |
| **Focus** | | | |
| Respiratory | | 77 (53.8) | |
| Abdominal | | 50 (35.0) | |

(continued)

| Parameters | SIRS group (n=121) SIRS n=53, SIRS+OD n=68 | Sepsis group (n=143) Severe sepsis n=26, Septic shock n=117 | p-value |
|---|---|---|---|
| Urinary | | 7 (4.9) | |
| Other | | 20(14.0) | |

In order to also detect proteins which are present in lower amounts, glycosylated proteins in the plasma samples are enriched. Taking advantage of sugar residues covalently linked to the protein scaffold, the glycosylated proteins are coupled to a solid phase (Sepharose) via so-called hydrazide chemistry after mild oxidation. Non-glycosylated proteins are washed away and the enriched preparation of plasma glycoproteins can be processed for proteomic studies and mass spectrometry. The glycoproteins covalently linked to sepharose are reduced at cysteine residues, these are stabilized in their oxidation state by alkylation, and after extensive washing the glycoproteins are cut into peptides at the solid phase by a protease (trypsin), which can now be freely removed in the supernatant of the sepharose. A few peptides remain on the sepharose, which still carry sugar residues that ensure linkage to the sepharose. By adding another enzyme, PNGaseF, sugar residues of the most common type of glycosylation, known as N-glycosylation, can be cleaved directly from the peptide portion of the protein, allowing even these last peptides of the glycoproteins to enter solution and become accessible to MS analysis. At the previously used N-glycosylation site released by PNGases, an asparagine is converted by deamination to an aspartate, which can be detected by the mass change and subsequent mass spectrometric analysis. The resulting two peptide fractions of a plasma sample (trypsin and PNGaseF peptides) are separated by reversed-phase liquid chromatography and analyzed by mass spectrometry (LC-MS/MS), providing good sensitivity for glycoproteins present in low concentrations due to the low complexity of the samples (especially the PNGaseF peptide fraction). The results of both fractions are again combined in one sample in the subsequent software-assisted calculation of the raw data (Figure 1). The result is the glycoprotein profile of the plasma sample, with a few non-glycosylated proteins that were not completely removed as contaminants. Modern mass spectrometry, in addition to identifying the peptides and proteins present in the sample, also provides quantitative values for the identified peptides. These can be transferred into quantitative values for the individual proteins contained in the sample via the software packages used to analyze the samples. The analysis software used, MaxQuant (version 1.5.5.1), calculates the relative concentration of proteins and gives the abundance as a so-called label-free-quantity (LFQ) value. In the final result, result lists of the identified proteins with their relative quantitative values for the individual proteins in the sample are obtained, which can be further used and compared for statistical considerations.

After analysis of the 264 plasma samples from the Discovery cohort, a total of 997 different proteins in the samples in the entire data set were identified, including 731 glycoproteins (Table 6 below).

On average, $685 \pm 40$ plasma proteins were identified in the plasma samples of the sepsis group and $663 \pm 35$ in the SIRS group (Figure 2A). 209 glycoproteins could be identified and quantified in 100% of all patient samples, and 498 glycoproteins could be found in $\geq 50\%$ of all patient samples (Figure 2B). A comparison with quantitative values for plasma proteins of the Human Plasma Protein Consortium shows that the plasma proteins identified with 50% reproducibility are in the concentration range of 0.1-1 ng/ml (Figure 2C), which corresponds to a very good sensitivity of the method.

Comparison of quantitative values for five selected glycoproteins with 100% detection reproducibility covering a concentration range of 5 orders of magnitude (LFQ values) showed that label-free-quantity (LFQ) values for these proteins in samples from individual critically ill patients showed comparable variance (Figure 2D). That is, reproducibility over the measurement range was high, with standard deviations (SD) ranging from 33.4-57.1 %, with a slight tendency toward higher standard deviations at lower LFQ intensities (Figure 2E).

A principal component analysis (PCA) showed that there are criteria in the data set that allow differentiating the SIRS group from the sepsis group on the basis of variance. The proportion of the first principal component is 7.12%. The following statistical analysis of the patient plasma samples showed that a surprising number of significantly different abundant proteins were detectable in the plasma samples of the SIRS and sepsis patient groups. A total of 312 proteins were significantly unequally expressed (t-test, $p_{adjusted} < 0.05$) in the two groups, but many of these were expressed with only a minor fold change (FC) between the two groups, with values less than a doubling or a halving (FC$\leq$2). 194 plasma proteins showed increased and 118 showed decreased abundance in the sepsis group compared with the SIRS group. 127 proteins showed a significant difference in intensity with FCs of >2. The significantly different abundant plasma proteins identified in the samples from sepsis patients and SIRS patients represent the plasma protein signature of sepsis detected and identified by this untargeted proteomic approach in the Discovery cohort.

**Identification of sVSIG4 in the validation cohort**

Results from the 264-patient discovery cohort were then compared with results from a second, 96-patient cohort for verification (validation cohort, SIRS group: n=55, including 30 with SIRS and 26 with SIRS+organ dysfunction or sepsis group: n= 41, including 7 with severe sepsis and 34 with septic shock, see also Table 5).

Table 5. Clinical parameters of patients with sepsis or SIRS in the validation cohort for the first available sample for each patient.

Data are median and interquartile range (IQR) or counts, No. (%). Definition of abbrevations: APACHE = Acute Physiology and Chronic Healthy Evaluation II, SOFA = Sequential Organ Failure Assessment, SAPS = Simplified Acute Physiology Score II, PCT = Procalcitonin (ng/ml), CRP = C-reactive protein (mg/l), WBC: white blood count, ICU = intensive care unit. Statistical analysis was done by Mann-Whitney-U-Test unless otherwise specified. §Chisquare test.

| Parameters | SIRS group (n=55) SIRS n=53, SIRS+OD=68 | Sepsis group (n=41) Severe Sepsis n=7 Septic shock n=34 | p-value |
|---|---|---|---|
| Gender, female/male | 17/38 (31%) | 7/34 (17%) | 0.190§ |
| Age, median, (IQR range) | 67 (59-73) | 67 (59-74) | 0.882 |
| SOFA median (IQR) | 8 (6-9) | 10 (8-13) | 0.001 |
| APACHE median (IQR) | 14 (12-19) | 19 (16-22) | <0.001 |
| SAPS median (IQR) | 35 (31-45) | 40 (32-50) | 0.172 |
| WBC cells/mm°median (IQR) | 14.4 (12.7-17.8) | 11.1 (0.6-14.8) | 0.024 |
| PCT conc. median (IQR) | 0.7 (0.3-3.0) | 7.4 (3.3-20.7) | <0.001 |
| CRP conc. median (IQR) | 26 (9-51) | 183 (84-249) | <0.001 |
| Hospital mortality No | 14 (25.4) | 18 (43.9) | 0.093§ |
| ICU mortality No, (%) | 13 (23.6) | 17 (41) | 0.064§ |
| Died of sepsis No, (%) | 3 (5.4) | 16 (39.0) | <0.001§ |

Also in this cohort, SOFA-score, APACHE ii-score, CRP- and PCT-levels were significantly higher in the sepsis group than in the SIRS group, whereas mortality was only slightly higher in the sepsis group than in the SIRS group. The plasma samples in the Validation cohort were processed as the plasma samples in the Discovery cohort. In contrast to the analyses of the Discovery cohort, the LC-MS/MS analyses of the Validation cohort were performed by triplicate measurements (technical triplicates) to increase the sensitivity and reproducibility of peptide and protein identification in the samples of the Validation cohort.

Of the 987 proteins, including 695 glycoproteins, identified in the validation cohort (Table 6), 294 plasma proteins differed significantly when compared between the sepsis and SIRS groups, including 193 with higher concentration or abundance and 101 with lower concentration or abundance (Figure 4).

Table 6. Peptide and Protein Identifications in Discovery and Validation Cohorts

| | Discovery Cohort (Cohort-1, n=264) | Validation Cohort (Cohort-2, n=96) |
|---|---|---|
| Total protein IDs | 997 | 987 |
| Total peptide IDs | 11870 | 11749 |
| Total glycoprotein IDs | 731 | 695 |
| Protein IDs in SIRS group | 663±35 | 769±35 |
| Protein IDs in sepsis group | 685±40 | 787±30 |
| Peptide IDs in SIRS group | 4644±335 | 5907±419 |
| Peptide IDs in sepsis group | 4783±436 | 5746±787 |

Comparing the two cohorts, a total of 199 plasma proteins showed significantly different concentration or abundance in both cohorts (Figure 5A) and a total of 122 plasma proteins showed concurrent higher and 76 plasma proteins showed concurrent lower abundance or concentration in the sepsis group (Figure 5B and 5C). One plasma protein showed different trends. 183 of the 199 plasma proteins with significantly different abundance in the SIRS and sepsis groups are annotated as glycoproteins in the UniProt database; for the remaining 16 proteins (8%) with different expression in the SIRS and sepsis groups, no glycosylation is known to date. Among these 16 plasma proteins were C-reactive protein (CRP), detected at higher levels in the sepsis group, and the acute-phase proteins serum amyloid protein-1 (SAA1) and serum amyloid protein-2 (SAA2). Surprisingly, the soluble protein V-set and immunoglobulin domain-containing protein 4 (sVSIG4), a protein annotated as non-glycosylated, showed the greatest fold change between the sepsis and SIRS groups of all detected plasma proteins in both the Discovery cohort and the Validation cohort. In both cohorts, the protein was detectable at significantly elevated levels in the sepsis patient samples (Table 7).

Table 7. Protein accession numbers (protein ID), entry name, p-value, fold changes (FC), and peptide numbers of significantly differentially abundant proteins in SIRS and sepsis patients identified in the discovery cohort and in the Validation cohort. The protein IDs and entry names were retrieved from the UniProt database with release number 2016_04. The entry names can alternatively have the suffix "_HUMAN"; with and without the suffix the same proteins are meant.

| | | | Discovery cohort | | | Validation cohort | | |
|---|---|---|---|---|---|---|---|---|
| Protein ID | Entry name | Glycoprotein | -Log (p-value) | (log2) FC | Peptides | -Log (p-value) | (log2) FC | Peptides |
| O95954 | FTCD | no | n.s. | n.s. | n.s. | 2.85228 | -2.63627 | 5 |
| P20023 | CR2 | yes | 10.85869 | -2.54925 | 7 | 2.82939 | -1.40195 | 7 |
| P13533 | MYH6 | no | n.s. | n.s. | n.s. | 2.03800 | -2.13309 | 4 |
| Q460N5 | PAR14 | no | n.s. | n.s. | n.s. | 3.41223 | -2.08325 | 6 |
| Q96BZ4 | PLD4 | yes | n.s. | n.s. | n.s. | 4.57912 | -1.91356 | 5 |
| Q9BUN1 | MENT | no | n.s. | n.s. | n.s. | 7.05859 | -1.87653 | 3 |
| P01815 | HV270 | no | n.s. | n.s. | n.s. | 2.87848 | -1.85459 | 3 |
| P48668 | K2C6C | no | n.s. | n.s. | n.s. | 2.17547 | -1.73105 | 24 |
| Q58EX2 | SDK2 | yes | n.s. | n.s. | n.s. | 2.77593 | -1.72234 | 13 |
| Q12884 | SEPR | yes | 5.54315 | -1.62576 | 8 | n.s. | n.s. | n.s. |
| P54289 | CA2D1 | yes | 6.44915 | -1.57349 | 7 | 3.34510 | -1.95818 | 9 |
| Q86WI1 | PKHL1 | yes | n.s. | n.s. | n.s. | 2.85831 | -1.54880 | 14 |
| Q14623 | IHH | yes | 6.26138 | -1.48455 | 4 | 6.23053 | -2.49334 | 3 |
| Q15166 | PON3 | yes | 10.15695 | -1.43075 | 9 | 5.79752 | -0.81345 | 11 |
| P80748 | LV321 | no | n.s. | n.s. | n.s. | 2.16690 | -1.40744 | 4 |
| Q16620 | NTRK2 | yes | 8.07698 | -1.39460 | 5 | n.s. | n.s. | n.s. |
| P02751 | FINC | yes | 3.98355 | -1.36884 | 125 | 3.53520 | -1.39887 | 132 |
| Q9H4A9 | DPEP2 | yes | 5.31958 | -1.34774 | 9 | n.s. | n.s. | n.s. |
| Q96KN2 | CNDP1 | yes | 17.06681 | -1.32284 | 23 | 5.77563 | -0.74380 | 26 |
| O15394 | NCAM2 | yes | n.s. | n.s. | n.s. | 2.34320 | -1.31108 | 6 |
| P06733 | ENOA | no | n.s. | n.s. | n.s. | 2.06702 | -1.30352 | 3 |
| P00488 | F13A | yes | 9.53807 | -1.30220 | 16 | n.s. | n.s. | n.s. |
| P35858 | ALS | yes | 21.60502 | -1.27939 | 24 | 5.61257 | -1.14802 | 25 |
| P80108 | PHLD | yes | 33.38612 | -1.26073 | 31 | 7.75516 | -0.96839 | 34 |
| Q08722 | CD47 | yes | n.s. | n.s. | n.s. | 2.11478 | -1.21427 | 2 |

(continued)

| | | | Discovery cohort | | | Validation cohort | | |
|---|---|---|---|---|---|---|---|---|
| Protein ID | Entry name | Glycoprotein | -Log (p-value) | (log2) FC | Peptides | -Log (p-value) | (log2) FC | Peptides |
| P17301 | ITA2 | yes | n.s. | n.s. | n.s. | 4.41517 | -1.21321 | 7 |
| P17936 | IBP3 | yes | 14.25121 | -1.19441 | 10 | 5.01541 | -0.72078 | 9 |
| Q9H6X2 | ANTR1 | yes | 3.55618 | -1.18447 | 5 | 4.07624 | -2.21770 | 5 |
| P02724 | GLPA | yes | n.s. | n.s. | n.s. | 3.09220 | -1.13304 | 1 |
| Q7Z3B1 | NEGR1 | yes | 3.05653 | -1.11955 | 3 | 1.90726 | -1.17813 | 3 |
| P06727 | APOA4 | yes | 2.87506 | -1.11356 | 20 | n.s. | n.s. | n.s. |
| P27487 | DPP4 | yes | 9.67789 | -1.09836 | 17 | 4.19121 | -0.58980 | 16 |
| P05154 | IPSP | yes | 16.13163 | -1.09771 | 18 | 12.07452 | -1.21809 | 17 |
| Q8VWVA 0 | ITLN1 | yes | 5.52307 | -1.09392 | 5 | 4.15089 | -1.61798 | 5 |
| P29622 | KAIN | yes | 29.25833 | -1.08233 | 27 | 15.69186 | -1.11925 | 28 |
| Q9Y6Z7 | COL10 | yes | 2.93052 | -1.06047 | 5 | n.s. | n.s. | n.s. |
| P11362 | FGFR1 | yes | 1.98596 | -1.05969 | 6 | 3.52916 | -1.03325 | 7 |
| P02765 | FETUA | yes | 27.51895 | -1.04856 | 21 | 13.47822 | -1.02510 | 24 |
| P06276 | CHLE | yes | 24.88774 | -1.04756 | 24 | 8.39287 | -0.80198 | 25 |
| Q5T749 | KPRP | no | n.s. | n.s. | n.s. | 2.01231 | -1.03945 | 10 |
| P05556 | ITB1 | yes | 3.31770 | -1.02023 | 9 | n.s. | n.s. | n.s. |
| Q9P121 | NTRI | yes | n.s. | n.s. | n.s. | 2.05303 | -1.01654 | 3 |
| P43652 | AFAM | yes | 27.00885 | -1.01600 | 32 | 8.37722 | -0.79478 | 43 |
| Q9UBX1 | CATF | yes | 3.97709 | -1.00228 | 3 | 5.21468 | -1.42816 | 5 |
| P11597 | CETP | yes | 7.08541 | -0.98447 | 20 | 6.91503 | -1.18542 | 21 |
| Q15113 | PCOC1 | yes | 5.22984 | -0.97550 | 14 | 2.62138 | -0.62662 | 13 |
| P12821 | ACE | yes | 5.35372 | -0.97334 | 11 | n.s. | n.s. | n.s. |
| Q9HBB8 | CDHR5 | yes | 7.12981 | -0.96951 | 8 | 3.25375 | -0.93980 | 7 |
| Q76LX8 | ATS13 | yes | 16.14614 | -0.94897 | 22 | 4.24615 | -0.49812 | 25 |

| Protein ID | Entry name | Glycoprotein | Discovery cohort | | | Validation cohort | | |
|---|---|---|---|---|---|---|---|---|
| | | | -Log (p-value) | (log2) FC | Peptides | -Log (p-value) | (log2) FC | Peptides |
| P07711 | CATL1 | yes | 1.79108 | -0.94650 | 4 | n.s. | n.s. | n.s. |
| P19823 | ITIH2 | yes | 40.33277 | -0.94269 | 34 | 9.43589 | -0.87866 | 42 |
| Q16853 | AOC3 | yes | 2.71186 | -0.94258 | 11 | 2.73369 | -0.56871 | 10 |
| P00533 | EGFR | yes | 5.40367 | -0.93467 | 8 | n.s. | n.s. | n.s. |
| Q96PD5 | PGRP2 | yes | 29.47285 | -0.93098 | 24 | 9.89650 | -0.84622 | 27 |
| Q12860 | CNTN1 | yes | 4.95790 | -0.90772 | 14 | 5.84927 | -1.00132 | 13 |
| Q9Y274 | SIA10 | yes | 2.35444 | -0.90247 | 3 | n.s. | n.s. | n.s. |
| P08519 | APOA | yes | 1.90884 | -0.90012 | 34 | 3.62501 | -1.77782 | 39 |
| P13647 | K2C5 | yes | 2.35589 | -0.89434 | 27 | 1.87966 | -1.15309 | 18 |
| Q7Z7M0 | MEGF8 | yes | 7.33149 | -0.89376 | 14 | 1.88272 | -0.30747 | 12 |
| P02766 | TTHY | yes | 12.81991 | -0.89057 | 11 | 2.88836 | -0.54172 | 13 |
| P27918 | PROP | yes | 7.81230 | -0.89035 | 11 | 6.68418 | -0.69617 | 10 |
| Q04756 | HGFA | yes | 19.63287 | -0.88573 | 17 | 5.34487 | -0.56985 | 18 |
| P01880 | IGHD | yes | 2.34898 | -0.87326 | 12 | n.s. | n.s. | n.s. |
| P06396 | GELS | yes | 9.66758 | -0.86256 | 22 | n.s. | n.s. | n.s. |
| P13591 | NCAM1 | yes | 10.65726 | -0.85821 | 14 | 4.07688 | -0.38527 | 14 |
| Q8IWV2 | CNTN4 | yes | 4.86353 | -0.82460 | 7 | n.s. | n.s. | n.s. |
| Q9UGM5 | FETUB | yes | 16.09321 | -0.82153 | 15 | 6.87482 | -0.76816 | 13 |
| P27169 | PON1 | yes | 18.00233 | -0.81690 | 23 | 6.34383 | -0.66667 | 27 |
| O95445 | APOM | yes | 17.44955 | -0.81240 | 13 | 4.39319 | -0.51614 | 13 |
| Q8NI99 | ANGL6 | yes | 2.62265 | -0.80884 | 5 | 1.81205 | -0.99913 | 2 |
| Q6P179 | ERAP2 | yes | 2.26940 | -0.80323 | 8 | n.s. | n.s. | n.s. |
| P01775 | HV323 | yes | 1.99527 | -0.79815 | 3 | n.s. | n.s. | n.s. |
| P02647 | APOA1 | yes | 8.48704 | -0.79694 | 17 | n.s. | n.s. | n.s. |

EP 4 075 142 A1

| | | | Discovery cohort | | | Validation cohort | | |
|---|---|---|---|---|---|---|---|---|
| Protein ID | Entry name | Glycoprotein | -Log (p-value) | (log2) FC | Peptides | -Log (p-value) | (log2) FC | Peptides |
| Q8TDL5 | BPIB1 | yes | 1.94158 | -0.79356 | 12 | 5.54757 | -2.06991 | 9 |
| Q5SRE5 | NU188 | yes | 1.83948 | -0.78328 | 4 | n.s. | n.s. | n.s. |
| P04180 | LCAT | yes | 20.23253 | -0.76640 | 16 | 6.06947 | -0.63050 | 18 |
| P49908 | SEPP1 | yes | 16.10852 | -0.75953 | 12 | 6.41766 | -0.73873 | 15 |
| P11279 | LAMP1 | yes | n.s. | n.s. | n.s. | 2.29629 | -0.73910 | 6 |
| P01619 | KV320 | no | n.s. | n.s. | n.s. | 1.78039 | -0.73889 | 3 |
| P35579 | MYH9 | yes | 4.35518 | -0.73187 | 23 | n.s. | n.s. | n.s. |
| P19827 | ITIH1 | yes | 35.36591 | -0.73096 | 45 | 8.36351 | -0.57664 | 50 |
| Q9UHG3 | PCYOX | yes | 11.16822 | -0.72818 | 18 | 5.29774 | -0.55815 | 19 |
| P02787 | TRFE | yes | 26.31718 | -0.72390 | 95 | 13.47821 | -0.76254 | 95 |
| Q9NZK5 | ADA2 | yes | 2.12870 | -0.71341 | 10 | n.s. | n.s. | n.s. |
| Q9Y646 | CBPQ | yes | 3.63157 | -0.71127 | 4 | n.s. | n.s. | n.s. |
| P05452 | TETN | yes | 12.08175 | -0.68835 | 10 | 3.29132 | -0.44206 | 11 |
| Q9NPH3 | IL1AP | yes | 5.34132 | -0.68182 | 18 | 3.62798 | -0.48825 | 12 |
| P04070 | PROC | yes | 14.65624 | -0.66887 | 15 | 4.66803 | -0.60382 | 13 |
| P04196 | HRG | yes | 18.75544 | -0.66042 | 29 | 7.17119 | -0.63990 | 30 |
| P03952 | KLKB1 | yes | 20.47325 | -0.65868 | 38 | 9.36433 | -0.69100 | 40 |
| P09172 | DOPO | yes | 2.52992 | -0.65210 | 21 | n.s. | n.s. | n.s. |
| P55056 | APOC4 | yes | 5.70976 | -0.63621 | 6 | 2.11000 | -0.49342 | 6 |
| P05546 | HEP2 | yes | 17.64180 | -0.62925 | 33 | 5.83125 | -0.54271 | 34 |
| Q92496 | FHR4 | yes | n.s. | n.s. | n.s. | 1.78953 | -0.61723 | 16 |
| P08887 | IL6RA | yes | n.s. | n.s. | n.s. | 1.84871 | -0.61502 | 8 |
| Q6YHK3 | CD109 | yes | 4.14834 | -0.61078 | 19 | 2.73926 | -0.42499 | 21 |
| P23467 | PTPRB | yes | 1.72869 | -0.59501 | 6 | 1.87443 | -0.35949 | 10 |

| Protein ID | Entry name | Glycoprotein | Discovery cohort | | | Validation cohort | | |
|---|---|---|---|---|---|---|---|---|
| | | | -Log (p-value) | (log2) FC | Peptides | -Log (p-value) | (log2) FC | Peptides |
| P05160 | F13B | yes | 17.63693 | -0.58714 | 31 | 6.55158 | -0.51882 | 32 |
| 014791 | APOL1 | yes | n.s. | n.s. | n.s. | 3.02746 | -0.55905 | 9 |
| P05090 | APOD | yes | 7.89774 | -0.55061 | 15 | n.s. | n.s. | n.s. |
| Q9HDC9 | APMAP | yes | 6.16854 | -0.54676 | 13 | n.s. | n.s. | n.s. |
| O75882 | ATRN | yes | 19.06558 | -0.53353 | 50 | 7.56905 | -0.48680 | 52 |
| P02656 | APOC3 | yes | 3.04817 | -0.52581 | 10 | 2.44700 | -0.70887 | 10 |
| P02654 | APOC1 | no | 3.21785 | -0.52463 | 5 | 4.89368 | -0.86199 | 5 |
| O00533 | NCHL1 | yes | 7.28459 | -0.52236 | 25 | n.s. | n.s. | n.s. |
| P55290 | CAD13 | yes | 3.14546 | -0.51940 | 15 | n.s. | n.s. | n.s. |
| P00748 | FA12 | yes | 8.45979 | -0.50957 | 27 | 3.84583 | -0.37897 | 27 |
| Q9NQ79 | CRAC1 | yes | 2.38547 | -0.48997 | 12 | 4.14075 | -0.70276 | 14 |
| Q9NY97 | B3GN2 | yes | 2.41644 | -0.48187 | 8 | 2.09263 | -0.59074 | 7 |
| P43251 | BTD | yes | 11.07057 | -0.46239 | 17 | 9.66522 | -0.48062 | 18 |
| P00747 | PLMN | yes | 12.61206 | -0.45914 | 52 | 3.72903 | -0.26781 | 58 |
| P00739 | HPTR | no | 5.28272 | -0.45765 | 34 | 2.18900 | -0.41788 | 33 |
| P22105 | TENX | yes | 3.59435 | -0.45302 | 37 | n.s. | n.s. | n.s. |
| Q01459 | DIAC | yes | 2.43474 | -0.45216 | 10 | 4.21704 | -0.76352 | 10 |
| P01625 | KV401 | yes | 3.78905 | -0.45189 | 4 | n.s. | n.s. | n.s. |
| P04114 | APOB | yes | 4.70135 | -0.42850 | 327 | 3.63876 | -0.44486 | 358 |
| P08709 | FA7 | yes | 2.76795 | -0.41424 | 11 | 3.24669 | -0.71626 | 10 |
| P02768 | ALBU | yes | 3.26136 | -0.39802 | 92 | n.s. | n.s. | n.s. |
| P43121 | MUC18 | yes | 2.44156 | -0.39630 | 17 | n.s. | n.s. | n.s. |
| Q12913 | PTPRJ | yes | 3.68260 | -0.39553 | 17 | n.s. | n.s. | n.s. |
| P01008 | ANT3 | yes | 12.91435 | -0.38193 | 49 | 4.41999 | -0.28158 | 53 |

| Protein ID | Entry name | Glycoprotein | Discovery cohort | | | Validation cohort | | |
|---|---|---|---|---|---|---|---|---|
| | | | -Log (p-value) | (log2) FC | Peptides | -Log (p-value) | (log2) FC | Peptides |
| P01042 | KNG1 | yes | 14.29681 | -0.37394 | 38 | 7.96298 | -2.64147 | 37 |
| Q16610 | ECM1 | yes | 7.27676 | -0.35521 | 25 | 2.12493 | -0.24314 | 21 |
| 075144 | ICOSL | yes | 2.06043 | -0.35451 | 8 | 1.89668 | -0.41756 | 8 |
| P01023 | A2MG | yes | 6.98337 | -0.34708 | 128 | n.s. | n.s. | n.s. |
| P01613 | KVD33 | yes | 2.20305 | -0.34058 | 2 | n.s. | n.s. | n.s. |
| P10909 | CLUS | yes | 7.75577 | -0.32917 | 31 | 2.18959 | -0.21784 | 39 |
| P00734 | THRB | yes | 8.24632 | -0.29782 | 50 | 3.34416 | -0.32133 | 50 |
| P13473 | LAMP2 | yes | n.s. | n.s. | n.s. | 1.88010 | -0.29219 | 13 |
| P01860 | IGHG3 | yes | 1.97363 | -0.29194 | 27 | n.s. | n.s. | n.s. |
| P02749 | APOH | yes | 3.84464 | -0.28255 | 31 | 2.27566 | -0.29154 | 32 |
| P14151 | LYAM1 | yes | 2.66556 | -0.24187 | 10 | n.s. | n.s. | n.s. |
| P33151 | CADH5 | yes | 2.67279 | -0.23705 | 20 | 1.88633 | -0.22821 | 19 |
| Q96IY4 | CBPB2 | yes | n.s. | n.s. | n.s. | 1.94741 | -0.23490 | 20 |
| P13671 | CO6 | yes | 4.17551 | -0.23128 | 40 | 1.87498 | -0.20101 | 39 |
| P01857 | IGHG1 | yes | 2.02166 | -0.21750 | 38 | n.s. | n.s. | n.s. |
| P08603 | CFAH | yes | 7.83921 | -0.21367 | 94 | n.s. | n.s. | n.s. |
| P48740 | MASP1 | yes | 3.53649 | -0.20698 | 26 | n.s. | n.s. | n.s. |
| P07225 | PROS | yes | 4.35855 | -0.19474 | 29 | n.s. | n.s. | n.s. |
| P04217 | A1BG | yes | n.s. | n.s. | n.s. | 2.17355 | -0.19042 | 26 |
| P51884 | LUM | yes | 1.78747 | -0.18885 | 20 | n.s. | n.s. | n.s. |
| P01024 | CO3 | yes | 1.81376 | -0.14519 | 145 | n.s. | n.s. | n.s. |
| P07357 | CO8A | yes | 2.14317 | -0.12358 | 26 | n.s. | n.s. | n.s. |
| P00751 | CFAB | yes | 1.78967 | 0.14738 | 60 | n.s. | n.s. | n.s. |
| P09871 | C1S | yes | 2.14784 | 0.16084 | 34 | 4.15587 | 0.38069 | 41 |

| Protein ID | Entry name | Glycoprotein | Discovery cohort | | | Validation cohort | | |
|---|---|---|---|---|---|---|---|---|
| | | | -Log (p-value) | (log2) FC | Peptides | -Log (p-value) | (log2) FC | Peptides |
| P06681 | CO2 | yes | 3.34179 | 0.19394 | 43 | n.s. | n.s. | n.s. |
| P22792 | CPN2 | yes | 3.43057 | 0.20781 | 27 | 3.89646 | 0.28186 | 29 |
| Q9NZP8 | C1RL | yes | 2.79373 | 0.21098 | 18 | n.s. | n.s. | n.s. |
| P08697 | A2AP | yes | 3.24390 | 0.21171 | 32 | n.s. | n.s. | n.s. |
| P36955 | PEDF | yes | n.s. | n.s. | n.s. | 2.28753 | 0.22102 | 21 |
| P11717 | MPRI | yes | n.s. | n.s. | n.s. | 1.97633 | 0.23118 | 19 |
| P00736 | C1R | yes | 4.27817 | 0.25902 | 33 | 3.17033 | 0.31239 | 35 |
| Q9UJJ9 | GNPTG | yes | 1.86275 | 0.25905 | 8 | 1.96651 | 0.31172 | 6 |
| Q14624 | ITIH4 | yes | 6.55387 | 0.27648 | 69 | 3.02181 | 0.28661 | 70 |
| Q08380 | LG3BP | yes | 2.74751 | 0.28765 | 28 | 2.61308 | 0.41218 | 31 |
| P08195 | 4F2 | yes | n.s. | n.s. | n.s. | 2.22032 | 0.29107 | 7 |
| POCOL4 | CO4A | yes | 2.09240 | 0.29293 | 117 | n.s. | n.s. | n.s. |
| P19652 | A1AG2 | yes | 3.77280 | 0.31673 | 25 | 4.36650 | 0.33035 | 21 |
| P04004 | VTNC | yes | n.s. | n.s. | n.s. | 3.13936 | 0.32583 | 36 |
| P41222 | PTGDS | yes | 2.40867 | 0.33763 | 5 | n.s. | n.s. | n.s. |
| P08174 | DAF | yes | 2.10415 | 0.34032 | 6 | 1.89649 | 0.37660 | 5 |
| Q07954 | LRP1 | yes | n.s. | n.s. | n.s. | 2.56843 | 0.34108 | 50 |
| P02649 | APOE | yes | 2.07301 | 0.34376 | 17 | 6.19240 | 0.79243 | 21 |
| P16070 | CD44 | yes | 3.35073 | 0.34621 | 5 | n.s. | n.s. | n.s. |
| P02679 | FIBG | yes | n.s. | n.s. | n.s. | 3.08736 | 0.34909 | 55 |
| P15151 | PVR | yes | 2.22784 | 0.35489 | 8 | 8.04490 | 1.07755 | 8 |
| P01591 | IGJ | yes | 2.99050 | 0.35726 | 11 | n.s. | n.s. | n.s. |
| Q92820 | GGH | yes | 2.47904 | 0.39841 | 12 | n.s. | n.s. | n.s. |
| P14625 | ENPL | yes | 2.33464 | 0.40999 | 18 | 10.18847 | 0.89841 | 17 |

| Protein ID | Entry name | Glycoprotein | Discovery cohort | | | Validation cohort | | |
|---|---|---|---|---|---|---|---|---|
| | | | -Log (p-value) | (log2) FC | Peptides | -Log (p-value) | (log2) FC | Peptides |
| Q8WZ75 | ROBO4 | yes | 2.18040 | 0.41293 | 13 | 4.40879 | 0.91956 | 10 |
| P02748 | CO9 | yes | 9.25697 | 0.41636 | 42 | n.s. | n.s. | n.s. |
| P01743 | HV146 | yes | 1.93680 | 0.41650 | 4 | 1.77375 | 0.55683 | 5 |
| P11047 | LAMC1 | yes | 1.84449 | 0.41884 | 19 | 6.32680 | 1.35593 | 15 |
| P13688 | CEAM1 | yes | 1.96616 | 0.41907 | 12 | 2.34897 | 0.45621 | 10 |
| 000391 | QSOX1 | yes | 6.30502 | 0.42759 | 27 | 2.87624 | 0.33374 | 26 |
| 000187 | MASP2 | no | n.s. | n.s. | n.s. | 2.87478 | 0.42770 | 15 |
| P55058 | PLTP | yes | 5.93225 | 0.43030 | 26 | 3.08348 | 1.34638 | 22 |
| P05155 | IC1 | yes | 11.45488 | 0.43699 | 36 | 4.95575 | 0.34019 | 35 |
| Q9UK55 | ZPI | yes | 7.01465 | 0.44968 | 23 | 8.68775 | 0.63152 | 21 |
| P19022 | CADH2 | yes | 2.02229 | 0.45222 | 15 | n.s. | n.s. | n.s. |
| Q6EMK4 | VASN | yes | 3.99504 | 0.45609 | 12 | n.s. | n.s. | n.s. |
| Q6UX71 | PXDC2 | yes | 2.01944 | 0.46597 | 9 | n.s. | n.s. | n.s. |
| Q9ULI3 | HEG1 | yes | n.s. | n.s. | n.s. | 2.12552 | 0.47089 | 12 |
| P01019 | ANGT | yes | 8.39295 | 0.47874 | 28 | 2.73631 | 0.37665 | 29 |
| P08637 | FCG3A | yes | 1.96554 | 0.49104 | 5 | 3.81553 | 0.72141 | 3 |
| P36980 | FHR2 | yes | 2.91122 | 0.49649 | 11 | n.s. | n.s. | n.s. |
| P01009 | A1AT | yes | 19.12260 | 0.49655 | 64 | 11.60368 | 0.72798 | 67 |
| P12111 | CO6A3 | yes | n.s. | n.s. | n.s. | 4.71125 | 0.50348 | 36 |
| Q03591 | FHR1 | yes | 3.64756 | 0.50506 | 19 | n.s. | n.s. | n.s. |
| Q7Z4W1 | DCXR | yes | 2.31083 | 0.52864 | 3 | n.s. | n.s. | n.s. |
| P27797 | CALR | yes | 1.93096 | 0.53667 | 5 | n.s. | n.s. | n.s. |
| P39060 | COIA1 | yes | n.s. | n.s. | n.s. | 2.02603 | 0.55146 | 11 |
| P02763 | A1AG1 | yes | 17.20090 | 0.55274 | 29 | 7.99656 | 0.64445 | 27 |

| Protein ID | Entry name | Glycoprotein | Discovery cohort | | | Validation cohort | | |
|---|---|---|---|---|---|---|---|---|
| | | | -Log (p-value) | (log2) FC | Peptides | -Log (p-value) | (log2) FC | Peptides |
| P02671 | FIBA | yes | n.s. | n.s. | n.s. | 5.13772 | 0.57283 | 62 |
| P19320 | VCAM1 | yes | 8.70539 | 0.57388 | 35 | 11.00188 | 0.89252 | 31 |
| Q92520 | FAM3C | yes | 2.33976 | 0.57658 | 2 | n.s. | n.s. | n.s. |
| Q13822 | ENPP2 | yes | n.s. | n.s. | n.s. | 3.03884 | 0.58376 | 21 |
| P33908 | MA1A1 | yes | n.s. | n.s. | n.s. | 4.30950 | 0.58716 | 15 |
| P34810 | CD68 | yes | 1.72329 | 0.58926 | 1 | n.s. | n.s. | n.s. |
| Q9Y5C1 | ANGL3 | yes | 2.34424 | 0.59605 | 12 | 8.52487 | 1.46071 | 13 |
| Q16706 | MA2A1 | yes | n.s. | n.s. | n.s. | 3.60331 | 0.59907 | 14 |
| O95393 | BMP10 | yes | 1.95169 | 0.59984 | 3 | n.s. | n.s. | n.s. |
| P07333 | CSF1R | yes | 7.05523 | 0.60575 | 14 | n.s. | n.s. | n.s. |
| P01594 | KV133 | no | n.s. | n.s. | n.s. | 1.78874 | 0.61693 | 4 |
| Q8NBP7 | PCSK9 | yes | 2.10827 | 0.62056 | 10 | 5.08546 | 1.08238 | 8 |
| P04275 | VWF | yes | 6.22664 | 0.63104 | 113 | 4.15533 | 0.80714 | 113 |
| Q13201 | MMRN1 | yes | 7.27270 | 0.63527 | 29 | 6.13114 | 0.64867 | 26 |
| P35555 | FBN1 | yes | n.s. | n.s. | n.s. | 5.26994 | 0.64030 | 12 |
| P04066 | FUCO | yes | 2.21719 | 0.64061 | 4 | n.s. | n.s. | n.s. |
| Q12805 | FBLN3 | yes | 10.95891 | 0.65213 | 19 | 6.48808 | 0.66785 | 17 |
| P20774 | MIME | yes | n.s. | n.s. | n.s. | 1.79509 | 0.65679 | 9 |
| Q5JRA6 | TGO1 | yes | n.s. | n.s. | n.s. | 2.15836 | 0.66023 | 9 |
| P13796 | PLSL | no | 2.25744 | 0.66067 | 12 | 2.74873 | 1.56760 | 10 |
| Q9BXR6 | FHR5 | yes | 11.22872 | 0.66241 | 25 | 4.76568 | 0.68321 | 23 |
| Q15833 | STXB2 | yes | 2.11406 | 0.66493 | 2 | n.s. | n.s. | n.s. |
| P14314 | GLU2B | yes | 2.66135 | 0.66743 | 8 | n.s. | n.s. | n.s. |
| P07339 | CATD | yes | n.s. | n.s. | n.s. | 2.95390 | 0.66977 | 13 |

(continued)

| Protein ID | Entry name | Glycoprotein | Discovery cohort | | | Validation cohort | | |
|---|---|---|---|---|---|---|---|---|
| | | | -Log (p-value) | (log2) FC | Peptides | -Log (p-value) | (log2) FC | Peptides |
| P15169 | CBPN | yes | n.s. | n.s. | n.s. | 3.06806 | 0.67386 | 9 |
| P98172 | EFNB1 | yes | 3.09476 | 0.69092 | 2 | 4.26700 | 1.32357 | 3 |
| P05164 | PERM | yes | 3.24403 | 0.69448 | 20 | 6.59249 | 1.89462 | 20 |
| P08294 | SODE | yes | n.s. | n.s. | n.s. | 1.81109 | 0.70665 | 9 |
| P02776 | PLF4 | yes | 2.97951 | 0.71000 | 1 | n.s. | n.s. | n.s. |
| P13987 | CD59 | yes | 1.83607 | 0.71193 | 2 | 3.28436 | 1.30340 | 2 |
| Q5ZPR3 | CD276 | yes | 2.28981 | 0.72646 | 10 | n.s. | n.s. | n.s. |
| P07996 | TSP1 | yes | n.s. | n.s. | n.s. | 4.30152 | 0.73345 | 41 |
| P98095 | FBLN2 | yes | 2.19871 | 0.73618 | 5 | 1.95991 | 0.98118 | 1 |
| Q9Y251 | HPSE | yes | 1.81668 | 0.74633 | 6 | 2.04520 | 1.37433 | 2 |
| P00451 | FA8 | yes | 6.55954 | 0.74681 | 35 | 6.37689 | 1.24272 | 20 |
| Q08ET2 | SIG14 | yes | 1.74085 | 0.74724 | 7 | 2.30092 | 0.98539 | 9 |
| Q06278 | AOXA | yes | 1.89397 | 0.74943 | 17 | n.s. | n.s. | n.s. |
| Q9UNN8 | EPCR | yes | 1.85404 | 0.74981 | 7 | 2.11536 | 1.05361 | 5 |
| Q86TH1 | ATL2 | yes | 3.28358 | 0.76832 | 14 | 4.59807 | 1.04264 | 12 |
| Q13093 | PAFA | yes | 2.06889 | 0.77194 | 14 | n.s. | n.s. | n.s. |
| P05362 | ICAM1 | yes | 11.98438 | 0.78407 | 19 | 13.98151 | 1.27649 | 19 |
| Q06033 | ITIH3 | yes | 28.34559 | 0.79306 | 40 | 6.08913 | 0.68843 | 46 |
| P53634 | CATC | yes | 2.60671 | 0.79368 | 5 | 5.26862 | 1.14544 | 6 |
| Q14767 | LTBP2 | yes | 1.95809 | 0.79406 | 4 | 1.77388 | 1.24459 | 5 |
| Q9NPR2 | SEM4B | yes | n.s. | n.s. | n.s. | 4.04264 | 0.79450 | 12 |
| Q6UXG3 | CLM9 | yes | 2.48356 | 0.79741 | 1 | n.s. | n.s. | n.s. |
| P21810 | PGS1 | yes | n.s. | n.s. | n.s. | 2.79464 | 0.80063 | 5 |
| Q99650 | OSMR | yes | 6.75640 | 0.80077 | 16 | 5.59257 | 1.37147 | 13 |

| Protein ID | Entry name | Glycoprotein | Discovery cohort | | | Validation cohort | | |
|---|---|---|---|---|---|---|---|---|
| | | | -Log (p-value) | (log2) FC | Peptides | -Log (p-value) | (log2) FC | Peptides |
| 014786 | NRP1 | yes | 7.33141 | 0.80166 | 20 | 7.28029 | 0.81945 | 18 |
| Q29960 | NELL1 | yes | 2.56837 | 0.82297 | 8 | n.s. | n.s. | n.s. |
| P08253 | HLAC | yes | n.s. | n.s. | n.s. | 1.88695 | 0.83117 | 14 |
| P50895 | MMP2 | yes | 1.88330 | 0.83295 | 4 | n.s. | n.s. | n.s. |
| P18850 | BCAM | yes | 1.78307 | 0.83459 | 3 | n.s. | n.s. | n.s. |
| P08571 | ATF6A | yes | 21.36105 | 0.84117 | 15 | 5.59383 | 0.57117 | 16 |
| Q96J42 | CD14 | yes | 1.95157 | 0.84750 | 3 | n.s. | n.s. | n.s. |
| P20908 | TXD15 | yes | 2.27201 | 0.85500 | 8 | n.s. | n.s. | n.s. |
| P19440 | CO5A1 | yes | n.s. | n.s. | n.s. | 2.45476 | 0.85700 | 6 |
| P78509 | GGT1 | yes | n.s. | n.s. | n.s. | 3.50111 | 0.86371 | 13 |
| P04438 | RELN | yes | 2.03730 | 0.87653 | 6 | n.s. | n.s. | n.s. |
| P01011 | AACT | yes | 23.21244 | 0.87704 | 52 | 14.83037 | 1.17358 | 52 |
| Q86UD1 | OAF | no | 9.50745 | 0.88302 | 8 | 3.73680 | 0.56235 | 5 |
| P23083 | HV102 | no | n.s. | n.s. | n.s. | 4.45111 | 0.88346 | 5 |
| Q12907 | LMAN2 | yes | 2.41435 | 0.88474 | 6 | n.s. | n.s. | n.s. |
| P48357 | OBRG | yes | 2.85685 | 0.88628 | 14 | 1.86827 | 0.83411 | 11 |
| Q16270 | LEPR | yes | 2.82047 | 0.88766 | 5 | 3.14515 | 1.10567 | 5 |
| P14543 | IBP7 | yes | n.s. | n.s. | n.s. | 2.95560 | 0.88805 | 23 |
| Q9Y2G1 | NID1 | yes | 1.80110 | 0.89738 | 1 | n.s. | n.s. | n.s. |
| P61626 | MYRF | yes | 2.67164 | 0.89784 | 4 | n.s. | n.s. | n.s. |
| Q9Y6R7 | LYSC | yes | 13.89568 | 0.90786 | 114 | 10.01542 | 1.26079 | 110 |
| 000451 | FCGBP | yes | 3.21938 | 0.90956 | 1 | 4.83859 | 2.30780 | 2 |
| 043505 | GFRA2 | yes | 2.61540 | 0.91243 | 5 | n.s. | n.s. | n.s. |
| O75976 | B4GA1 | yes | 2.26536 | 0.91465 | 6 | 4.11640 | 1.26819 | 4 |

| | | | Discovery cohort | | | Validation cohort | | |
|---|---|---|---|---|---|---|---|---|
| Protein ID | Entry name | Glycoprotein | -Log (p-value) | (log2) FC | Peptides | -Log (p-value) | (log2) FC | Peptides |
| P02792 | CBPD | yes | 2.44719 | 0.91685 | 4 | n.s. | n.s. | n.s. |
| Q92673 | FRIL | yes | 2.49431 | 0.91958 | 3 | n.s. | n.s. | n.s. |
| Q6UVK1 | SORL | yes | 2.44412 | 0.92402 | 6 | n.s. | n.s. | n.s. |
| P28070 | CSPG4 | yes | n.s. | n.s. | n.s. | 1.89186 | 0.92766 | 2 |
| P05109 | S10A8 | yes | 2.25983 | 0.92767 | 3 | n.s. | n.s. | n.s. |
| Q8VWVZ8 | OIT3 | yes | 4.61429 | 0.92902 | 12 | 4.28539 | 0.70409 | 9 |
| P07306 | ASGR1 | yes | 3.28801 | 0.93003 | 2 | 2.90357 | 1.42982 | 2 |
| Q9Y4L1 | HYOU1 | yes | 7.12715 | 0.93283 | 19 | 6.94524 | 0.84972 | 19 |
| P24821 | TENA | yes | 11.76862 | 0.94162 | 59 | 12.43858 | 1.54338 | 65 |
| P01833 | PIGR | yes | 12.09855 | 0.94459 | 33 | 3.86892 | 0.69013 | 29 |
| Q4LDE5 | SVEP1 | yes | 5.65240 | 0.94613 | 42 | 6.06358 | 1.06316 | 35 |
| O00300 | TR11B | yes | n.s. | n.s. | n.s. | 2.34616 | 0.95973 | 1 |
| P06331 | HV434 | yes | 2.38030 | 0.95999 | 2 | n.s. | n.s. | n.s. |
| Q9BRK5 | CAB45 | yes | n.s. | n.s. | n.s. | 1.84412 | 0.96299 | 4 |
| P27930 | IL1R2 | yes | 2.90251 | 0.96653 | 7 | 3.19242 | 1.21265 | 8 |
| P06737 | PYGL | yes | 2.26086 | 0.97076 | 14 | n.s. | n.s. | n.s. |
| Q9NPY3 | C1QR1 | yes | 2.99425 | 0.97817 | 12 | n.s. | n.s. | n.s. |
| P01824 | HV439 | yes | 3.82144 | 0.98068 | 4 | n.s. | n.s. | n.s. |
| P07942 | LAMB1 | yes | 2.83592 | 0.98096 | 17 | 4.70588 | 1.44631 | 18 |
| P08861 | CEL3B | yes | 2.26067 | 1.00020 | 5 | n.s. | n.s. | n.s. |
| P11021 | BIP | no | 2.33538 | 1.00040 | 13 | 3.07873 | 1.23926 | 13 |
| Q92626 | PXDN | yes | 3.31117 | 1.00521 | 9 | 4.39913 | 1.57583 | 10 |
| P02461 | CO3A1 | yes | 3.47437 | 1.00535 | 6 | n.s. | n.s. | n.s. |
| Q12841 | FSTL1 | yes | 3.95482 | 1.02864 | 7 | 6.26944 | 1.84547 | 6 |

(continued)

| Protein ID | Entry name | Glycoprotein | Discovery cohort | | | Validation cohort | | |
|---|---|---|---|---|---|---|---|---|
| | | | -Log (p-value) | (log2) FC | Peptides | -Log (p-value) | (log2) FC | Peptides |
| O95479 | G6PE | yes | n.s. | n.s. | n.s. | 5.12759 | 1.02333 | 13 |
| P48230 | T4S4 | yes | 2.60842 | 1.03073 | 1 | n.s. | n.s. | n.s. |
| Q13308 | PTK7 | yes | n.s. | n.s. | n.s. | 2.15758 | 1.04785 | 3 |
| P02750 | A2GL | yes | 20.10996 | 1.04941 | 24 | 8.75279 | 1.11714 | 24 |
| P01033 | TIMP1 | yes | 10.24891 | 1.05199 | 10 | 12.82187 | 1.91460 | 10 |
| Q5T5C0 | STXB5 | yes | 2.16351 | 1.05207 | 1 | n.s. | n.s. | n.s. |
| P10646 | TFPI1 | yes | n.s. | n.s. | n.s. | 2.90962 | 1.05276 | 4 |
| Q9Y5X9 | LIPE | yes | n.s. | n.s. | n.s. | 2.52241 | 1.06098 | 5 |
| P61916 | NPC2 | yes | 3.85486 | 1.06098 | 2 | 2.72719 | 1.69669 | 4 |
| P08581 | MET | yes | 2.65839 | 1.06412 | 7 | n.s. | n.s. | n.s. |
| O75594 | PGRP1 | yes | 4.55305 | 1.07944 | 5 | 7.43912 | 2.13348 | 4 |
| P02788 | TRFL | yes | 4.90960 | 1.09547 | 32 | 3.39243 | 1.49353 | 27 |
| Q03001 | DYST | no | 2.44655 | 1.09862 | 13 | 5.54377 | 2.02006 | 11 |
| P18428 | LBP | yes | 18.06551 | 1.11194 | 28 | 14.50218 | 1.80481 | 24 |
| Q9P2J2 | TUTLA | yes | n.s. | n.s. | n.s. | 2.18154 | 1.11467 | 3 |
| P00738 | HPT | yes | 8.50821 | 1.11557 | 52 | 6.98303 | 1.51806 | 51 |
| P35442 | TSP2 | yes | n.s. | n.s. | n.s. | 2.81164 | 1.11964 | 8 |
| P07307 | ASGR2 | yes | 15.59944 | 1.13462 | 8 | 9.79379 | 1.30585 | 8 |
| Q12866 | MERTK | yes | 3.56520 | 1.13501 | 8 | n.s. | n.s. | n.s. |
| P10645 | CMGA | yes | 2.91733 | 1.14212 | 14 | n.s. | n.s. | n.s. |
| P10153 | RNAS2 | yes | 4.71532 | 1.14295 | 5 | 4.45896 | 1.33039 | 5 |
| O75173 | ATS4 | yes | n.s. | n.s. | n.s. | 2.38950 | 1.15574 | 3 |
| P20160 | CAP7 | yes | 2.88239 | 1.15890 | 3 | 3.53067 | 1.72341 | 3 |
| P36222 | CH3L1 | yes | 2.32078 | 1.16093 | 15 | 4.52215 | 3.19040 | 17 |

| | | | Discovery cohort | | | Validation cohort | | |
|---|---|---|---|---|---|---|---|---|
| Protein ID | Entry name | Glycoprotein | -Log (p-value) | (log2) FC | Peptides | -Log (p-value) | (log2) FC | Peptides |
| Q9Y6U3 | ADSV | yes | 3.34410 | 1.16820 | 3 | n.s. | n.s. | n.s. |
| P98173 | FAM3A | no | n.s. | n.s. | n.s. | 3.95444 | 1.16992 | 1 |
| P24387 | CRHBP | yes | n.s. | n.s. | n.s. | 1.95579 | 1.17591 | 2 |
| P20333 | TNR1B | yes | 5.84146 | 1.18465 | 2 | 5.24321 | 2.10336 | 3 |
| P04233 | HG2A | yes | 3.22684 | 1.19070 | 4 | 2.70230 | 1.68550 | 3 |
| Q9H3S1 | SEM4A | yes | n.s. | n.s. | n.s. | 2.33564 | 1.22482 | 2 |
| P18827 | SDC1 | yes | 5.67505 | 1.22587 | 2 | n.s. | n.s. | n.s. |
| Q16394 | EXT1 | yes | n.s. | n.s. | n.s. | 2.20963 | 1.23404 | 6 |
| Q504Y2 | PKDCC | yes | 2.66762 | 1.23605 | 2 | n.s. | n.s. | n.s. |
| Q96HD1 | CREL1 | yes | 6.62576 | 1.24392 | 7 | n.s. | n.s. | n.s. |
| P10124 | SRGN | yes | n.s. | n.s. | n.s. | 3.25780 | 1.24444 | 1 |
| P08123 | CO1A2 | yes | n.s. | n.s. | n.s. | 5.20402 | 1.25903 | 4 |
| Q8IYS5 | OSCAR | yes | 4.47272 | 1.26514 | 8 | n.s. | n.s. | n.s. |
| Q6UX06 | OLFM4 | yes | 4.32069 | 1.27394 | 10 | 3.30363 | 1.43339 | 11 |
| Q15904 | VAS1 | yes | 4.73394 | 1.27963 | 4 | n.s. | n.s. | n.s. |
| Q8N6C8 | LIRA3 | yes | 5.50270 | 1.28561 | 15 | 15.98375 | 1.56223 | 15 |
| Q9UK05 | GDF2 | yes | n.s. | n.s. | n.s. | 2.85720 | 1.30635 | 2 |
| P18065 | IBP2 | no | 6.31672 | 1.31186 | 5 | 3.27487 | 1.53062 | 5 |
| 015123 | ANGP2 | yes | 3.41248 | 1.31223 | 7 | n.s. | n.s. | n.s. |
| P63104 | 1433Z | no | n.s. | n.s. | n.s. | 2.37127 | 1.32437 | 3 |
| Q99988 | GDF15 | yes | 4.07542 | 1.32756 | 3 | 2.97376 | 1.55999 | 3 |
| P12724 | ECP | yes | n.s. | n.s. | n.s. | 2.17843 | 1.33234 | 3 |
| P40199 | CEAM6 | yes | n.s. | n.s. | n.s. | 3.16738 | 1.37398 | 4 |
| P24043 | LAMA2 | yes | 5.08466 | 1.37859 | 16 | 9.80149 | 2.19251 | 15 |

| | | | Discovery cohort | | | Validation cohort | | |
|---|---|---|---|---|---|---|---|---|
| Protein ID | Entry name | Glycoprotein | -Log (p-value) | (log2) FC | Peptides | -Log (p-value) | (log2) FC | Peptides |
| P12109 | CO6A1 | yes | n.s. | n.s. | n.s. | 2.81664 | 1.38638 | 21 |
| O76061 | STC2 | yes | n.s. | n.s. | n.s. | 1.98913 | 1.38943 | 2 |
| P41271 | NBL1 | yes | 5.99206 | 1.40398 | 2 | n.s. | n.s. | n.s. |
| P37173 | TGFR2 | yes | n.s. | n.s. | n.s. | 2.42655 | 1.41020 | 4 |
| P78324 | SHPS1 | yes | 5.44485 | 1.41690 | 6 | 4.51775 | 1.52289 | 6 |
| P07988 | PSPB | yes | 6.46152 | 1.42139 | 17 | n.s. | n.s. | n.s. |
| Q9BXX0 | EMIL2 | yes | 5.94118 | 1.42886 | 4 | n.s. | n.s. | n.s. |
| P35613 | BASI | yes | 4.89261 | 1.43155 | 2 | 2.05940 | 1.46589 | 1 |
| P12110 | CO6A2 | yes | n.s. | n.s. | n.s. | 2.90103 | 1.43369 | 3 |
| P08311 | CATG | yes | n.s. | n.s. | n.s. | 2.79993 | 1.44286 | 8 |
| Q9NQS3 | NECT3 | yes | 3.87901 | 1.45379 | 5 | n.s. | n.s. | n.s. |
| Q14314 | FGL2 | yes | 2.98099 | 1.47175 | 4 | 5.18110 | 2.25781 | 3 |
| Q9UBR2 | CATZ | yes | 7.39283 | 1.51039 | 7 | 1.99250 | 0.81572 | 4 |
| O95450 | ATS2 | yes | 2.24300 | 1.51531 | 6 | n.s. | n.s. | n.s. |
| Q9UHI8 | ATS1 | yes | 7.80388 | 1.51812 | 4 | n.s. | n.s. | n.s. |
| P16581 | LYAM2 | yes | 6.28835 | 1.53822 | 10 | 10.87612 | 1.93995 | 7 |
| Q9Y5Y7 | LYVE1 | yes | 11.98494 | 1.54350 | 8 | 6.97538 | 2.31568 | 7 |
| Q13443 | ADAM9 | yes | 4.74585 | 1.54724 | 2 | 2.21000 | 1.73878 | 2 |
| Q02487 | DSC2 | yes | 6.16132 | 1.56943 | 11 | 2.54150 | 1.70165 | 9 |
| Q01638 | ILRL1 | yes | 5.15837 | 1.61164 | 15 | 6.76720 | 2.60672 | 15 |
| Q6Q788 | APOA5 | no | n.s. | n.s. | n.s. | 2.15047 | 1.61827 | 8 |
| Q9BU40 | CRDL1 | yes | n.s. | n.s. | n.s. | 2.04544 | 1.62970 | 3 |
| Q07000 | HLAC | yes | n.s. | n.s. | n.s. | 3.72208 | 1.63794 | 9 |
| P14780 | MMP9 | yes | 7.07030 | 1.64485 | 16 | 6.28824 | 2.78388 | 11 |

| Protein ID | Entry name | Glycoprotein | Discovery cohort | | | Validation cohort | | |
|---|---|---|---|---|---|---|---|---|
| | | | -Log (p-value) | (log2) FC | Peptides | -Log (p-value) | (log2) FC | Peptides |
| P01034 | CYTC | no | 4.75006 | 1.66041 | 5 | 1.81774 | 1.02492 | 6 |
| O95998 | 118BP | yes | 7.96544 | 1.66808 | 4 | 7.27421 | 1.89559 | 4 |
| Q9UKJ1 | PILRA | yes | 9.79282 | 1.66895 | 3 | 3.01008 | 1.16632 | 3 |
| P22897 | MRC1 | yes | 25.95184 | 1.67416 | 39 | 14.72995 | 1.44266 | 39 |
| P10451 | OSTP | yes | 5.86033 | 1.70067 | 8 | 8.45507 | 2.77160 | 2 |
| Q86VB7 | C163A | yes | 16.87486 | 1.72627 | 28 | 9.15459 | 1.38100 | 29 |
| P10253 | LYAG | yes | n.s. | n.s. | n.s. | 3.70770 | 1.74571 | 4 |
| P20061 | TCO1 | yes | n.s. | n.s. | n.s. | 3.44918 | 1.74930 | 6 |
| 043451 | MGA | yes | n.s. | n.s. | n.s. | 2.43692 | 1.75392 | 11 |
| Q96NZ9 | PRAP1 | no | n.s. | n.s. | n.s. | 3.41420 | 1.75584 | 2 |
| Q9Y287 | ITM2B | yes | 8.05416 | 1.76508 | 6 | 6.07105 | 2.01055 | 6 |
| Q08830 | FGL1 | no | 11.27936 | 1.76974 | 8 | 4.79382 | 1.98202 | 3 |
| P0DJI8 | SAA1 | no | 12.59947 | 1.77374 | 10 | 10.40360 | 2.98248 | 10 |
| Q15828 | CYTM | yes | n.s. | n.s. | n.s. | 3.26064 | 1.78565 | 2 |
| P15907 | SIAT1 | yes | 5.83542 | 1.82015 | 5 | n.s. | n.s. | n.s. |
| Q8WUA8 | TSK | yes | 8.03984 | 1.84549 | 8 | n.s. | n.s. | n.s. |
| P07858 | CATB | yes | n.s. | n.s. | n.s. | 5.90204 | 1.85876 | 12 |
| P42785 | PCP | yes | n.s. | n.s. | n.s. | 2.54688 | 1.86613 | 5 |
| P18510 | IL1RA | yes | 6.87476 | 1.87167 | 4 | 4.67441 | 2.92348 | 4 |
| Q02818 | NUCB1 | no | 8.55817 | 1.87732 | 12 | 6.42247 | 2.99106 | 8 |
| P61769 | B2MG | yes | n.s. | n.s. | n.s. | 3.63871 | 1.88157 | 1 |
| P00480 | OTC | no | n.s. | n.s. | n.s. | 3.35289 | 1.90336 | 4 |
| Q8NBJ4 | GOLM1 | yes | 9.90568 | 1.90706 | 11 | 5.32217 | 2.29165 | 7 |
| Q14118 | DAG1 | yes | 9.63426 | 1.93029 | 12 | 4.71907 | 1.77218 | 8 |

| Protein ID | Entry name | Glycoprotein | Discovery cohort | | | Validation cohort | | |
|---|---|---|---|---|---|---|---|---|
| | | | -Log (p-value) | (log2) FC | Peptides | -Log (p-value) | (log2) FC | Peptides |
| Q9BYE9 | CDHR2 | yes | 7.25270 | 1.94119 | 28 | n.s. | n.s. | n.s. |
| P13611 | CSPG2 | yes | n.s. | n.s. | n.s. | 4.85473 | 1.95988 | 11 |
| Q8N6Q3 | CD177 | yes | 11.29166 | 1.97986 | 3 | 5.50195 | 2.67147 | 2 |
| P11166 | GTR1 | yes | n.s. | n.s. | n.s. | 3.40269 | 1.98031 | 2 |
| P05186 | PPBT | yes | 15.05647 | 1.98826 | 7 | 4.13522 | 1.21779 | 6 |
| Q96FE7 | P3IP1 | yes | 9.51364 | 2.04260 | 3 | n.s. | n.s. | n.s. |
| P22894 | MMP8 | yes | 10.66964 | 2.05383 | 9 | 3.26299 | 1.60685 | 8 |
| P24158 | PRTN3 | yes | 9.59636 | 2.08745 | 9 | 5.40626 | 2.47814 | 8 |
| P08246 | ELNE | yes | 5.18490 | 2.16401 | 5 | 4.93041 | 1.79103 | 7 |
| P15291 | B4GT1 | yes | 9.90621 | 2.17112 | 7 | 2.45059 | 1.39832 | 7 |
| P02741 | CRP | no | 16.63801 | 2.17497 | 8 | 11.84687 | 3.62297 | 5 |
| P17900 | SAP3 | yes | 12.98102 | 2.26959 | 7 | n.s. | n.s. | n.s. |
| Q14508 | WFDC2 | yes | 8.05242 | 2.27279 | 4 | 7.41554 | 3.54897 | 2 |
| P48304 | REG1B | yes | 10.91310 | 2.28006 | 4 | n.s. | n.s. | n.s. |
| O95633 | FSTL3 | yes | 11.79504 | 2.36932 | 3 | 5.34808 | 2.58640 | 4 |
| P07998 | RNAS1 | yes | 11.83212 | 2.45625 | 3 | 3.41919 | 1.48639 | 4 |
| P80188 | NGAL | yes | 16.34685 | 2.46196 | 15 | 17.53393 | 2.61938 | 16 |
| P80511 | S10AC | no | n.s. | n.s. | n.s. | 6.62195 | 2.51935 | 1 |
| P0DJI9 | SAA2 | no | 15.65403 | 2.60907 | 11 | 8.92113 | 4.10664 | 12 |
| P01130 | LDLR | yes | n.s. | n.s. | n.s. | 7.75301 | 2.66205 | 3 |
| P00367 | DHE3 | no | n.s. | n.s. | n.s. | 6.38645 | 2.69080 | 16 |
| P26022 | PTX3 | yes | n.s. | n.s. | n.s. | 5.49726 | 2.74354 | 7 |
| P05451 | REG1A | yes | 17.60192 | 3.20631 | 6 | 10.36391 | 3.44469 | 6 |
| Q9Y279 | VSIG4 | yes | 32.32804 | 4.21055 | 10 | 12.10262 | 4.19468 | 9 |

Table 11 lists all identified peptides suitable for detection of plasma proteins with significantly different concentration in SIRS or sepsis patients (Table 7) (in both cohorts studied with significantly different concentration).

**Receiver-Operating-Characteristic (ROC) curve analyses**

To determine the diagnostic quality of the proteins detected with significantly altered concentration in the discovery cohort dataset, receiver operating characteristic (ROC) curve analyses were performed using the proteomically determined LFQ values, comparing the true-positive rate with the false-positive rate. The ROC curve of the clinically determined PCT value was taken as the baseline reference (Figure 6). The PCT value in the 264-patient discovery cohort dataset showed an AUC of only 0.63. In contrast, the CRP values detected in proteomics showed a significantly higher AUC of 0.81. The values for CRP were somewhat better when the values measured in the clinic were used; an AUC of 0.85 revealed a significantly improved sensitivity and specificity compared to the PCT value.

Thirteen of the proteins detected in the proteomic data set of the discovery cohort with significantly different plasma levels in the SIRS *vs.* the sepsis group showed an improved AUC compared with CRP (Figure 6): serotransferrin (TRFE, gene name:TF), AUC=0.85; insulin-like growth factor-binding protein complex acid labile subunit (ALS, gene name: IGFALS), AUC= 0. 85; cholinesterase (CHLE, gene name BCHE), AUC=0.86; macrophage mannose receptor 1, MMR (MRC1, gene name MRC1), AUC=0.86; afamin (AFAM, gene name AFM), AUC=0.86; inter-alpha-trypsin inhibitor heavy chain H3 (ITIH3, gene name ITIH3), AUC=0. 86; alpha-2-HS-glycoprotein (FETUA, gene name AHSG), AUC=0.87; soluble V-set and immunoglobulin domain-containing protein 4 (sVSIG4, gene nme VSIG4), AUC=0.87; calistatin (KAIN, gene name SERPINA4), AUC=0.88; N-acetylmuramoyl-L-alanine amidase (PGRP2, gene name PGLYRP2), AUC=0.88; phosphatidylinositol-glycan-specific phospholipase D (PHLD, gene name GPLD1), AUC=0.90; Inter-alpha-trypsin inhibitor heavy chain H1, (ITIH1, gene name ITHI1), AUC=0.91; inter-alpha-trypsin inhibitor heavy chain H2, (ITIH2, gene name ITIH2), AUC=0.93. However, of these 13 plasma proteins, TRFE, ITIH3, PGRP2, ITIH1, and ITIH2 showed only slightly different abundance levels in the SIRS vs. the sepsis group (FC ≤2). In the validation cohort, ALS (0.82), CHLE (0.83), AFAM (0.82), ITIH3 (0.78), PHLD (0.81), and ITIH1 (AUC=0.84) had a slightly lower AUC than CRP. In contrast, TRFE (AUC=0.90), MRC1 (AUC=0.91), FETUA (AUC=0.91), sVSIG4 (AUC=0.88), KAIN (AUC=0.91), and ITIH2 (AUC=0.86) also showed higher sensitivity and higher specificity than CRP in the validation cohort.

**Linear discrimination analysis (LDA)**

Using a linear discrimination analysis (LDA, backward elimination approach) with 24 plasma proteins (with fold-change exclusion criterion FC>2) that showed the highest AUC values in the Discovery cohort, it was then searched for a classifier that in combination yields an even better AUC than the individual markers (Figure 7). The AUC with all 24 marker candidates was 0.971, a combination of 4 markers (PHLD, IBP3, A2GL, and sVSIG4) showed an AUC of 0.959, and the combination of two plasma proteins, PHLD and sVSIG4, showed an AUC of 0.940.

The same approach was subsequently performed with plasma proteins without considering the fold-change criterion ≥2 between the SIRS and sepsis groups (Figure 8). The 24 proteins considered in the beginning achieved an AUC of 0.973. After reduction to four markers (ITIH2, PGRP2, sVSIG4, and CD14) an AUC of 0.966 could still be observed. Reduction to 2 markers (sVSIG4 and ITIH2) possessed an AUC=0.952, slightly improved over LDA with FC≥2 as an exclusion criterion.

The AUC of the combinations is superior to the use of CRP as the sole marker in both cases. Unexpectedly, sVSIG4 is included in the combinations with two markers in both approaches as a marker for the diagnosis of sepsis. Hence, sVSIG4 alone, or in combination with one (in the example with ITIH2 and PHLD) or more other plasma proteins is suitable for sepsis diagnosis and shows better AUC compared to the use of CRP and PCT.

**Microbiological comparison**

Statistical analysis of plasma proteins detected in patient samples with microbiologically positive results (59 patients) compared with plasma samples from patients without microbiologically positive results (205 patients, diagnosed with SIRS or sepsis; microbiological findings negative or not tested) in the Discovery cohort showed that 104 significantly differentially abundant plasma proteins were detectable in the data set, 34 of which had an FC≥2, differing in abundance between the two groups (Figure 9A). 100 of the significantly differentially detected proteins in the comparison of microbiological culture positive/negative (MiBi pos/neg) also showed significantly different abundance in the comparison sepsis to SIRS (Figure 9B). All 39 plasma proteins that showed decreased levels in the plasma of culture-positive patients compared with MiBi pos/neg were also detected with decreased levels in sepsis patients compared with SIRS patients (Figure 9D). Of the 65 plasma proteins detectable at elevated levels in the MiBi pos/neg comparison, 61 were also elevated in plasma from sepsis patients (Figure 9C). The results of the MiBi-pos/neg and SIRS/sepsis comparisons show a strong correlation, indicating that part of the results of the differently detectable plasma protein levels of the sepsis patients was actually caused by the systemic infection underlying the sepsis.

Among the plasma proteins that showed significantly higher plasma levels in the MiBi-positive patient samples, soluble V-set immunoglobulin domain-containing protein 4 (sVSIG4) was the protein that possessed the most pronounced positive fold change in this comparison (Table 8). This indicates that sVSIG4 is a suitable marker in the blood of patients for the diagnosis of systemic infections such as in critically ill patients with suspected sepsis.

Table 8. Protein accession numbers (protein ID), entry name, p-value and fold changes (FC) of significant differentially abundant proteins in patients with positive microbiological cultures and critically ill patients with negative microbiological results in the discovery cohort. The protein IDs and entry names are retrieved from the UniProt database with release number 2016_04. The entry names can alternatively have the suffix "_HUMAN"; with and without the suffix the same proteins are meant.

| Protein IDs | Entryname | -Log p-value | (log2) FC |
|---|---|---|---|
| P20023 | CR2 | 3.08137153 | -1.55758017 |
| P54289 | CA2D1 | 3.12552862 | -1.26334002 |
| Q12884 | SEPR | 2.34704837 | -1.19589657 |
| P27487 | DPP4 | 6.06088343 | -1.03259778 |
| Q16620 | NTRK2 | 3.00599068 | -0.97421245 |
| Q15166 | PON3 | 3.20248881 | -0.92370361 |
| P11597 | CETP | 3.7738715 | -0.83761395 |
| P12821 | ACE | 2.62878649 | -0.78018986 |
| P05154 | IPSP | 5.50140947 | -0.76697514 |
| P02765 | FETUA | 8.39837843 | -0.72460471 |
| P80108 | PHLD | 6.44183325 | -0.70373614 |
| Q96KN2 | CNDP1 | 3.35749662 | -0.68547451 |
| P29622 | KAIN | 7.27840681 | -0.67802198 |
| Q9NPH3 | IL1AP | 3.8148049 | -0.67775297 |
| P43652 | AFAM | 6.24263504 | -0.60666018 |
| Q01459 | DIAC | 2.8713128 | -0.59588869 |
| Q04756 | HGFA | 5.67731673 | -0.57603459 |
| P06276 | CHLE | 4.88015496 | -0.5669809 |
| Q96PD5 | PGRP2 | 6.70022277 | -0.5569823 |
| P49908 | SEPP1 | 5.57151637 | -0.53465789 |
| Q76LX8 | ATS13 | 3.31935801 | -0.50109036 |
| P02787 | TRFE | 8.03833789 | -0.49821518 |
| 095445 | APOM | 4.32890443 | -0.48065214 |
| P27169 | PON1 | 4.18947629 | -0.46799523 |
| Q9HDC9 | APMAP | 3.04928629 | -0.44306081 |
| Q9UHG3 | PCYOX | 3.10326861 | -0.44062759 |
| P19823 | ITIH2 | 5.25045691 | -0.44002637 |
| P04180 | LCAT | 4.21965451 | -0.41851358 |
| P03952 | KLKB1 | 5.5486052 | -0.41541664 |
| P00748 | FA12 | 4.0157504 | -0.40981195 |
| P04070 | PROC | 3.98458335 | -0.40952776 |

(continued)

| Protein IDs | Entryname | -Log p-value | (log2) FC |
|---|---|---|---|
| P05546 | HEP2 | 4.98254282 | -0.40011724 |
| O75882 | ATRN | 7.18587701 | -0.39795864 |
| Q9UGM5 | FETUB | 2.64638295 | -0.38100001 |
| P19827 | ITIH1 | 4.91348387 | -0.34536326 |
| O00533 | NCHL1 | 2.4620556 | -0.34233753 |
| P43251 | BTD | 3.59969811 | -0.30583442 |
| P01023 | A2MG | 2.74234988 | -0.24776139 |
| P05160 | F13B | 2.29771299 | -0.24015869 |
| P22792 | CPN2 | 2.30398461 | 0.19703667 |
| P00736 | C1R | 2.65821575 | 0.23619101 |
| P05155 | IC1 | 2.87306219 | 0.24978271 |
| P02748 | CO9 | 2.89014555 | 0.26532963 |
| O00391 | QSOX1 | 2.48362594 | 0.30376831 |
| P01009 | A1AT | 5.36818486 | 0.31705474 |
| P55058 | PLTP | 2.73005358 | 0.33381537 |
| P02763 | A1AG1 | 5.72549874 | 0.38314685 |
| P07333 | CSF1R | 2.3253627 | 0.39012844 |
| P19320 | VCAM1 | 3.19195476 | 0.39948027 |
| P02649 | APOE | 2.32270634 | 0.44033249 |
| P01011 | AACT | 4.17762073 | 0.44881445 |
| Q92820 | GGH | 2.31699889 | 0.45772388 |
| P05362 | ICAM1 | 3.35578132 | 0.47948711 |
| Q9BXR6 | FHR5 | 4.363787 | 0.48449098 |
| Q12805 | FBLN3 | 4.48591108 | 0.49007551 |
| P14625 | ENPL | 2.34461248 | 0.49162674 |
| P08571 | CD14 | 5.43558744 | 0.51505944 |
| Q03591 | FHR1 | 2.88487225 | 0.52813794 |
| P02750 | A2GL | 4.029204 | 0.56002402 |
| P24821 | TENA | 3.20414518 | 0.56576105 |
| Q06033 | ITIH3 | 9.3696118 | 0.57226284 |
| P18428 | LBP | 3.48128599 | 0.57324723 |
| P04275 | VWF | 3.99681955 | 0.59328509 |
| P11047 | LAMC1 | 2.52417405 | 0.60551887 |
| P01833 | PIGR | 4.51838493 | 0.67920961 |
| Q4LDE5 | SVEP1 | 2.65935867 | 0.7416346 |
| Q9Y6R7 | FCGBP | 6.80097966 | 0.76078455 |
| P07307 | ASGR2 | 5.20626719 | 0.78157479 |
| Q6ZMJ2 | SCAR5 | 2.3178046 | 0.79934157 |

(continued)

| Protein IDs | Entryname | -Log p-value | (log2) FC |
|---|---|---|---|
| P22897 | MRC1 | 4.20953887 | 0.82102808 |
| Q6UY14 | ATL4 | 2.52189546 | 0.85821879 |
| Q6GPI1 | CTRB2;CTRB1 | 3.56464265 | 0.89123633 |
| Q86VB7 | C163A | 3.52973563 | 0.92508253 |
| Q92626 | PXDN | 2.32180507 | 0.97703565 |
| Q08830 | FGL1 | 2.83913732 | 1.0122009 |
| Q13616 | CUL1 | 2.62937086 | 1.01842893 |
| Q8NBJ4 | GOLM1 | 2.34166677 | 1.03785103 |
| P07988 | PSPB | 2.72142863 | 1.05202117 |
| Q9UKJ1 | PILRA | 2.99553178 | 1.05576562 |
| Q9Y5Y7 | LYVE1 | 4.59415609 | 1.12516049 |
| Q8N6Q3 | CD177 | 2.91743126 | 1.14982574 |
| Q9Y287 | ITM2B | 2.67773266 | 1.15434336 |
| P41271 | NBL1 | 3.19919066 | 1.18768187 |
| P05186 | PPBT | 3.97790169 | 1.20102224 |
| P15291 | B4GT1 | 2.44505857 | 1.21233645 |
| P17900 | SAP3 | 2.92682836 | 1.23464415 |
| 095998 | I18BP | 3.32410112 | 1.24340534 |
| P0DJI8 | SAA1 | 4.66809734 | 1.27333635 |
| Q96HD1 | CREL1 | 4.98182273 | 1.27780229 |
| Q14118 | DAG1 | 3.19699188 | 1.27810841 |
| P22894 | MMP8 | 3.16218551 | 1.28475604 |
| Q14508 | WFDC2 | 2.35561119 | 1.37792124 |
| Q02818 | NUCB1 | 3.45178582 | 1.37840438 |
| Q9BYE9 | CDHR2 | 2.93015846 | 1.41221548 |
| P08861 | CEL3B | 3.19539348 | 1.46569493 |
| P02741 | CRP | 5.28935851 | 1.46695224 |
| Q96FE7 | P3IP1 | 3.67998074 | 1.4743947 |
| P07998 | RNAS1 | 3.49258377 | 1.54616251 |
| P10451 | OSTP | 3.70797353 | 1.58316367 |
| P48304 | REG1B | 3.99281268 | 1.60971291 |
| P80188 | NGAL | 4.95817658 | 1.61642568 |
| PODJI9 | SAA2 | 4.58758699 | 1.67493973 |
| P05451 | REG1A | 4.4104115 | 1.9091026 |
| Q9Y279 | VSIG4 | 8.87904988 | 2.80756706 |

**Comparison of sepsis caused by gram-positive and gram-negative bacteria and of sepsis with abdominal and respiratory origin**

sVSIG4 is equally elevated in systemic infections (microbiologically verified sepsis) caused by gram-positive (n=49) and gram-negative (n=23) bacteria in plasma (FC=1.002) (Figure 10A) and is thus useful for detecting systemic infections caused by either group of bacteria. However, there were differences when comparing different sites of origin of infection of the original site of inflammation in patient plasma from the Discovery cohort. For example, when comparing "focus abdominal" (n=50) versus "focus respiratory system" (n=77), there was a significant difference in plasma levels of 30 proteins, 13 of which had an FC≥2 (Figure 10B). Whereas particularly metalloproteinase-9 (MMP9), interleukin-1 receptors antagonist protein (IL1RN), soluble V-set and immunoglobulin domain-containing protein 4 (sVSIG4), metalloproteinase 8 (MMP8), Chitinase-3-like protein 1 (CHI3L1), Interleukin receptor-like 1 (IL1RA), CD177 antigen (CD177), Neutrophil gelatinase-associated lipocalin (NGAL), Lactotransferrin (TRFL), Interleukin-18-binding protein (IL18BP), metalloproteinase inhibitor 1 (TIMP1), and Cell growth regulator with EF hand domain protein 1 (CGRE1) showed a significantly increased plasma level (FC≥2) at abdominal focus, Neuronal growth regulator 1 (NEGR1) was increased in the data set (FC≥2) at respiratory focus (Table 9). Therefore, sVSIG4 is a suitable biomarker in plasma for the detection of systemic infections and sepsis with abdominal focus, but high sVSIG4 levels are also detectable in plasma samples of patients with systemic infections or sepsis with respiratory focus. sVSIG4 is thus also suitable as a biomarker for diagnostic purposes of systemic infections or sepsis with respiratory focus.

Table 9. Protein accession numbers (protein ID), entry name, p-value and fold changes (FC) of significant differentially abundant proteins in patient plasma with abdominal focus or focus in the respiratory system of the discovery cohort. Negative FC indicates higher abundance in patients with abdominal focus. The protein IDs and entry names are retrieved from the UniProt database with release number 2016_04. The entry names can alternatively have the suffix "HUMAN"; with and without the suffix the same proteins are meant.

| Protein ID | Entry name | -Log (pvalue) | (log2) FC |
|---|---|---|---|
| P14780 | MMP9 | 7.512205654 | -2.82460037 |
| P18510 | IL1RA | 4.909896103 | -2.78520465 |
| Q9Y279 | VSIG4 | 4.941267379 | -2.53294196 |
| P22894 | MMP8 | 5.013845754 | -2.48376327 |
| P36222 | CH3L1 | 4.943745264 | -2.41468359 |
| Q01638 | ILRL1 | 5.095015795 | -2.3319485 |
| Q8N6Q3 | CD177 | 3.939610912 | -1.99312876 |
| P80188 | NGAL | 5.093130034 | -1.90039267 |
| P02788 | TRFL | 4.529346784 | -1.68405437 |
| O95998 | I18BP | 2.864002746 | -1.65571646 |
| P01033 | TIMP1 | 9.767724806 | -1.46938083 |
| Q99674 | CGRE1 | 2.8599008 | -1.1166551 |
| P00451 | FA8 | 3.439292347 | -0.83886837 |
| P18428 | LBP | 4.855640214 | -0.81086941 |
| P22897 | MRC1 | 3.348461951 | -0.78439345 |
| Q9Y6R7 | FCGBP | 2.857496983 | -0.68810898 |
| P55058 | PLTP | 4.626845124 | -0.67781356 |
| Q9UK55 | ZPI | 3.515840958 | -0.50491835 |
| P01011 | AACT | 3.162276496 | -0.43595465 |
| Q06033 | ITIH3 | 3.019430156 | -0.34699502 |
| P07225 | PROS | 3.423984395 | 0.29631643 |
| P19823 | ITIH2 | 2.874333118 | 0.38155042 |

(continued)

| Protein ID | Entry name | -Log (pvalue) | (log2) FC |
|---|---|---|---|
| P19827 | ITIH1 | 4.298794097 | 0.40014553 |
| P05160 | F13B | 3.313673111 | 0.40502489 |
| Q9UHG3 | PCYOX | 3.624438242 | 0.65116467 |
| P80108 | PHLD | 3.655545207 | 0.65915758 |
| P35542 | SAA4 | 3.339607166 | 0.80727516 |
| P05154 | IPSP | 3.334887841 | 0.81164102 |
| P02654 | APOC1 | 3.242626938 | 0.86247809 |
| Q7Z3B1 | NEGR1 | 3.21064905 | 1.9347828 |

**Prognosis of mortality**

Further analysis of the discovery cohort dataset showed that plasma proteins were detected in patient samples already at the beginning of diagnosis (on day 1 or day 2) that correlated with an increased risk of mortality from sepsis. Statistical analysis of proteomic quantitative data from patients who died of sepsis compared with data from all other critically ill but surviving patients revealed a total of 179 significantly altered plasma proteins, 125 with a FC between the two groups of ≥2 (Figure 11A). In contrast, in the smaller, 96-patient Validation cohort, only 4 plasma proteins were significantly differentially abundant (detected when comparing the groups of sepsis decedents vs. survivors), three of which (soluble V-Set and immunoglobulin domain-containing protein 4, sVSIG4; tenascin, TENA; kininogen-1, KNG1) were also already identified as significantly altered in the Discovery cohort (Figure 11B). Table 10 lists significant differentially abundant proteins in patients who died of sepsis and critically ill patients who survived (discovery cohort and validation cohort).

Table 10. Protein accession numbers (protein ID), entry name, p-value, fold changes (FC), and peptide numbers of significant differentially abundant proteins in patients who died of sepsis and critically ill patients who survived identified in the discovery cohort and in the validation cohort. The protein IDs and entry names are retrieved from the UniProt database with release number 2016_04. The entry names can alternatively have the suffix "_HUMAN"; with and without the suffix the same proteins are meant.

| | | Discovery cohort | | | Validation cohort | |
|---|---|---|---|---|---|---|
| Protein ID | Entry name | Log(p-value) | (log2) FC | Peptides | Log(p-value) | (log2) FC |
| Q14623 | IHH | 2.75009255 | -1.22793992 | 4 | n.s. | n.s. |
| P02751 | FINC | 4.91551789 | -1.21008938 | 122 | n.s. | n.s. |
| Q15166 | PON3 | 3.29263755 | -1.03903157 | 9 | n.s. | n.s. |
| P11597 | CETP | 4.61095434 | -1.0364968 | 20 | n.s. | n.s. |
| Q8NI99 | ANGL6 | 2.58403424 | -1.02525044 | 5 | n.s. | n.s. |
| P35858 | ALS | 7.33942868 | -0.9951301 | 24 | n.s. | n.s. |
| Q86U17 | SPA11 | 3.35492013 | -0.98991567 | 5 | n.s. | n.s. |
| P35579 | MYH9 | 4.32298818 | -0.93019323 | 23 | n.s. | n.s. |
| Q76LX8 | ATS13 | 8.69260053 | -0.90362652 | 22 | n.s. | n.s. |
| P09172 | DOPO | 2.69374945 | -0.87277774 | 21 | n.s. | n.s. |
| Q96KN2 | CNDP1 | 4.35885187 | -0.87189195 | 23 | n.s. | n.s. |
| P29622 | KAIN | 9.55110161 | -0.82740154 | 27 | n.s. | n.s. |
| P06276 | CHLE | 8.18336877 | -0.81540846 | 24 | n.s. | n.s. |
| P80108 | PHLD | 7.38747309 | -0.8056155 | 31 | n.s. | n.s. |

(continued)

| Protein ID | Entry name | Discovery cohort | | | Validation cohort | |
| --- | --- | --- | --- | --- | --- | --- |
| | | Log(p-value) | (log2) FC | Peptides | Log(p-value) | (log2) FC |
| P17936 | IBP3 | 3.20297716 | -0.72007939 | 10 | n.s. | n.s. |
| Q9UGM5 | FETUB | 7.52848881 | -0.71937977 | 15 | n.s. | n.s. |
| Q9NPH3 | IL1AP | 3.3551727 | -0.68969538 | 18 | n.s. | n.s. |
| P43652 | AFAM | 6.94433621 | -0.68780317 | 32 | n.s. | n.s. |
| P02765 | FETUA | 6.6100095 | -0.67709399 | 21 | n.s. | n.s. |
| P05154 | IPSP | 3.42416887 | -0.64776231 | 18 | n.s. | n.s. |
| P02751 | FINC | 4.86877064 | -0.6438581 | 127 | n.s. | n.s. |
| P04180 | LCAT | 7.92029676 | -0.63452812 | 16 | n.s. | n.s. |
| P02751 | FINC | 3.57920121 | -0.63406886 | 126 | n.s. | n.s. |
| Q96PD5 | PGRP2 | 7.46326766 | -0.63271291 | 24 | n.s. | n.s. |
| P27169 | PON1 | 6.14271149 | -0.60990953 | 23 | n.s. | n.s. |
| P19823 | ITIH2 | 8.22376193 | -0.60126865 | 34 | n.s. | n.s. |
| Q04756 | HGFA | 4.62885728 | -0.55663892 | 17 | n.s. | n.s. |
| P02766 | TTHY | 3.1052683 | -0.54295455 | 11 | n.s. | n.s. |
| P08709 | FA7 | 2.73931127 | -0.53911339 | 11 | n.s. | n.s. |
| P05546 | HEP2 | 7.72302378 | -0.53885674 | 33 | n.s. | n.s. |
| O95445 | APOM | 4.45679548 | -0.53031005 | 13 | n.s. | n.s. |
| P19827 | ITIH1 | 9.17338167 | -0.52397387 | 45 | n.s. | n.s. |
| P55056 | APOC4 | 2.45733335 | -0.51495354 | 6 | n.s. | n.s. |
| P55290 | CAD13 | 2.01680047 | -0.50544904 | 15 | n.s. | n.s. |
| P04070 | PROC | 5.2469028 | -0.49946507 | 15 | n.s. | n.s. |
| P03952 | KLKB1 | 6.4297617 | -0.48471807 | 38 | n.s. | n.s. |
| Q9HDC9 | APMAP | 3.07069391 | -0.47981277 | 13 | n.s. | n.s. |
| P02647 | APOA1 | 2.08560823 | -0.4664324 | 17 | n.s. | n.s. |
| P00747 | PLMN | 7.53558393 | -0.46384894 | 52 | n.s. | n.s. |
| P05452 | TETN | 3.40620389 | -0.45658416 | 10 | n.s. | n.s. |
| P49908 | SEPP1 | 3.67126232 | -0.45608873 | 12 | n.s. | n.s. |
| P05160 | F13B | 5.83726652 | -0.44312265 | 31 | n.s. | n.s. |
| P04196 | HRG | 4.28649201 | -0.41554185 | 29 | n.s. | n.s. |
| P00739 | HPTR | 2.70748894 | -0.40882986 | 34 | n.s. | n.s. |
| O75636 | FCN3 | 2.63130452 | -0.40268162 | 15 | n.s. | n.s. |
| P02787 | TRFE | 3.71243361 | -0.35717211 | 95 | n.s. | n.s. |
| P00748 | FA12 | 2.57368797 | -0.34326586 | 27 | n.s. | n.s. |
| P02790 | HEMO | 4.25643834 | -0.3428616 | 43 | n.s. | n.s. |
| P02743 | SAMP | 2.26862339 | -0.32843156 | 16 | n.s. | n.s. |
| P07358 | CO8B | 4.64519174 | -0.32653007 | 31 | n.s. | n.s. |
| P43251 | BTD | 3.4778152 | -0.32542919 | 17 | n.s. | n.s. |

(continued)

| Protein ID | Entry name | Discovery cohort | | | Validation cohort | |
|---|---|---|---|---|---|---|
| | | Log(p-value) | (log2) FC | Peptides | Log(p-value) | (log2) FC |
| P02749 | APOH | 3.18916741 | -0.32404277 | 31 | n.s. | n.s. |
| P13671 | CO6 | 4.75454476 | -0.3200028 | 40 | n.s. | n.s. |
| O75882 | ATRN | 3.89379928 | -0.30778968 | 50 | n.s. | n.s. |
| P26927 | HGFL | 2.4266216 | -0.28362935 | 28 | n.s. | n.s. |
| P12259 | FA5 | 2.96944846 | -0.27780085 | 70 | n.s. | n.s. |
| P01042 | KNG1 | 4.32412728 | -0.25896531 | 38 | 3.80627996 | -2.33227374 |
| P07225 | PROS | 4.41665401 | -0.25521628 | 29 | n.s. | n.s. |
| P10909 | CLUS | 2.71484883 | -0.24050487 | 31 | n.s. | n.s. |
| P08603 | CFAH | 5.84472925 | -0.23896085 | 94 | n.s. | n.s. |
| P01008 | ANT3 | 3.27319786 | -0.22934429 | 49 | n.s. | n.s. |
| Q16610 | ECM1 | 2.07481733 | -0.22645308 | 25 | n.s. | n.s. |
| P07357 | CO8A | 3.31781978 | -0.20685927 | 26 | n.s. | n.s. |
| P00734 | THRB | 2.42620898 | -0.19821656 | 50 | n.s. | n.s. |
| P04003 | C4BPA | 2.06752377 | -0.17394013 | 48 | n.s. | n.s. |
| P05155 | IC1 | 2.26070556 | 0.23566718 | 36 | n.s. | n.s. |
| P02763 | A1AG1 | 2.57980572 | 0.2615262 | 29 | n.s. | n.s. |
| P01009 | A1AT | 3.23545409 | 0.26283845 | 64 | n.s. | n.s. |
| 000391 | QSOX1 | 2.27741204 | 0.30986962 | 27 | n.s. | n.s. |
| Q06033 | ITIH3 | 3.42824145 | 0.36611588 | 40 | n.s. | n.s. |
| Q9UJJ9 | GNPTG | 2.25622543 | 0.38413875 | 8 | n.s. | n.s. |
| P02750 | A2GL | 2.21345957 | 0.42897561 | 24 | n.s. | n.s. |
| P01011 | AACT | 3.4431659 | 0.43649899 | 52 | n.s. | n.s. |
| P55058 | PLTP | 4.30099889 | 0.45898558 | 26 | n.s. | n.s. |
| P08174 | DAF | 2.42567198 | 0.4835161 | 6 | n.s. | n.s. |
| Q13201 | MMRN1 | 3.8446122 | 0.52168493 | 29 | n.s. | n.s. |
| Q12805 | FBLN3 | 4.55966799 | 0.52862632 | 19 | n.s. | n.s. |
| P19320 | VCAM1 | 4.72006696 | 0.54606802 | 35 | n.s. | n.s. |
| P05362 | ICAM1 | 4.11635772 | 0.57973929 | 19 | n.s. | n.s. |
| P68871 | HBB | 2.35069877 | 0.58168575 | 12 | n.s. | n.s. |
| P08571 | CD14 | 6.14404913 | 0.59042272 | 15 | n.s. | n.s. |
| Q13822 | ENPP2 | 2.43418852 | 0.59562562 | 24 | n.s. | n.s. |
| Q15848 | ADIPO | 2.13105737 | 0.59952962 | 4 | n.s. | n.s. |
| P04275 | VWF | 3.76496674 | 0.62008591 | 113 | n.s. | n.s. |
| P01833 | PIGR | 3.36251133 | 0.62698675 | 33 | n.s. | n.s. |
| P07307 | ASGR2 | 3.92262965 | 0.72584464 | 8 | n.s. | n.s. |
| Q9H7U1 | CCSE2 | 2.41738284 | 0.74112212 | 2 | n.s. | n.s. |
| Q9NP78 | ABCB9 | 2.13012217 | 0.76736652 | 1 | n.s. | n.s. |

(continued)

| Protein ID | Entry name | Discovery cohort | | | Validation cohort | |
|---|---|---|---|---|---|---|
| | | Log(p-value) | (log2) FC | Peptides | Log(p-value) | (log2) FC |
| P11216 | PYGB | 2.78595616 | 0.7719977 | 7 | n.s. | n.s. |
| Q9Y6R7 | FCGBP | 6.54131491 | 0.77494308 | 114 | n.s. | n.s. |
| P07333 | CSF1R | 7.21925357 | 0.79524523 | 14 | n.s. | n.s. |
| Q4LDE5 | SVEP1 | 2.49760485 | 0.79730754 | 42 | n.s. | n.s. |
| Q01518 | CAP1 | 2.22871092 | 0.81266148 | 5 | n.s. | n.s. |
| P24821 | TENA | 5.47445981 | 0.83634118 | 59 | 3.79750258 | 1.15280480 |
| Q08830 | FGL1 | 2.28279479 | 0.96656894 | 8 | n.s. | n.s. |
| P18827 | SDC1 | 2.42098329 | 0.96791777 | 2 | n.s. | n.s. |
| P01033 | TIMP1 | 5.46284691 | 0.97815833 | 10 | n.s. | n.s. |
| P48357 | LEPR | 2.18444028 | 0.9942007 | 14 | n.s. | n.s. |
| O95393 | BMP10 | 3.19045568 | 1.01689158 | 3 | n.s. | n.s. |
| Q96HD1 | CREL1 | 2.92968044 | 1.0287374 | 7 | n.s. | n.s. |
| P02792 | FRIL | 2.03833259 | 1.04322237 | 4 | n.s. | n.s. |
| P53634 | CATC | 2.62817316 | 1.04728613 | 5 | n.s. | n.s. |
| P61626 | LYSC | 2.23475341 | 1.06531505 | 4 | n.s. | n.s. |
| P08648 | ITA5 | 2.63588105 | 1.06757974 | 5 | n.s. | n.s. |
| Q15063 | POSTN | 2.40882631 | 1.09820697 | 20 | n.s. | n.s. |
| Q12841 | FSTL1 | 2.86696374 | 1.1063594 | 7 | n.s. | n.s. |
| Q9UHI8 | ATS1 | 2.837539 | 1.13900535 | 4 | n.s. | n.s. |
| P50895 | BCAM | 2.12492591 | 1.16126645 | 4 | n.s. | n.s. |
| Q92626 | PXDN | 2.81842135 | 1.16274138 | 9 | n.s. | n.s. |
| Q6UX06 | OLFM4 | 2.40502576 | 1.16419793 | 10 | n.s. | n.s. |
| P16581 | LYAM2 | 2.44863746 | 1.1698984 | 10 | n.s. | n.s. |
| P02461 | CO3A1 | 2.97304197 | 1.17170634 | 6 | n.s. | n.s. |
| P61916 | NPC2 | 3.03754394 | 1.17995506 | 2 | n.s. | n.s. |
| Q9Y287 | ITM2B | 2.35256703 | 1.18367957 | 6 | n.s. | n.s. |
| P01824 | HV439 | 3.49889677 | 1.19026569 | 4 | n.s. | n.s. |
| P22897 | MRC1 | 7.63470692 | 1.20579442 | 39 | n.s. | n.s. |
| P04746 | AMYP | 2.1992487 | 1.21535845 | 7 | n.s. | n.s. |
| Q08ET2 | SIG14 | 2.58125269 | 1.21924185 | 7 | n.s. | n.s. |
| O75976 | CBPD | 2.41195721 | 1.22483024 | 6 | n.s. | n.s. |
| P48230 | T4S4 | 2.35063594 | 1.22764472 | 1 | n.s. | n.s. |
| Q15904 | VAS1 | 2.78093626 | 1.23382225 | 4 | n.s. | n.s. |
| P63261 | ACTB | 2.18867843 | 1.24745685 | 12 | n.s. | n.s. |
| P12318 | FCG2A | 2.19128689 | 1.25342436 | 5 | n.s. | n.s. |
| P02788 | TRFL | 4.0208494 | 1.27744359 | 32 | n.s. | n.s. |
| P13796 | PLSL | 4.56955514 | 1.28573772 | 12 | n.s. | n.s. |

(continued)

| Protein ID | Entry name | Discovery cohort | | | Validation cohort | |
|---|---|---|---|---|---|---|
| | | Log(p-value) | (log2) FC | Peptides | Log(p-value) | (log2) FC |
| Q8N6Q3 | CD177 | 3.2209174 | 1.28942313 | 3 | n.s. | n.s. |
| P02741 | CRP | 3.6686961 | 1.29764162 | 8 | n.s. | n.s. |
| Q16270 | IBP7 | 3.50274438 | 1.31878109 | 5 | n.s. | n.s. |
| P18065 | IBP2 | 4.03200458 | 1.32073883 | 5 | n.s. | n.s. |
| P20333 | TNR1B | 4.70666059 | 1.32356664 | 2 | n.s. | n.s. |
| P07988 | PSPB | 3.51518355 | 1.323627 | 17 | n.s. | n.s. |
| P35613 | BASI | 2.72565952 | 1.33657063 | 2 | n.s. | n.s. |
| P16284 | PECA1 | 2.57548699 | 1.33752794 | 6 | n.s. | n.s. |
| P22894 | MMP8 | 3.07380734 | 1.34840683 | 9 | n.s. | n.s. |
| Q02487 | DSC2 | 2.89625002 | 1.34905451 | 11 | n.s. | n.s. |
| P05186 | PPBT | 4.41785497 | 1.35794757 | 7 | n.s. | n.s. |
| Q8NBJ4 | GOLM1 | 3.47676142 | 1.37008564 | 11 | n.s. | n.s. |
| Q9UBR2 | CATZ | 3.94918595 | 1.37232991 | 7 | n.s. | n.s. |
| Q9Y5Y7 | LYVE1 | 5.87535694 | 1.38477406 | 8 | n.s. | n.s. |
| P37173 | TGFR2 | 2.32290507 | 1.40782312 | 4 | n.s. | n.s. |
| Q12907 | LMAN2 | 3.45460813 | 1.41497189 | 6 | n.s. | n.s. |
| P24043 | LAMA2 | 3.3513455 | 1.44112756 | 16 | n.s. | n.s. |
| P14780 | MMP9 | 3.55216517 | 1.44614447 | 16 | n.s. | n.s. |
| Q14118 | DAG1 | 3.52828922 | 1.45049075 | 12 | n.s. | n.s. |
| P15291 | B4GT1 | 2.84792128 | 1.45155582 | 7 | n.s. | n.s. |
| P13611 | CSPG2 | 2.54440028 | 1.50209026 | 15 | n.s. | n.s. |
| Q9BYE9 | CDHR2 | 3.08546336 | 1.53471998 | 28 | n.s. | n.s. |
| Q9BXX0 | EMIL2 | 4.16911948 | 1.54849993 | 4 | n.s. | n.s. |
| P04438 | HV270 | 3.50549052 | 1.55292515 | 6 | n.s. | n.s. |
| P10451 | OSTP | 3.12095055 | 1.5571218 | 8 | n.s. | n.s. |
| P78324 | SHPS1 | 4.01942869 | 1.5577667 | 6 | n.s. | n.s. |
| P48304 | REG1B | 3.40045026 | 1.56146932 | 4 | n.s. | n.s. |
| P40199 | CEAM6 | 2.85616904 | 1.56485437 | 8 | n.s. | n.s. |
| Q9UKJ1 | PILRA | 5.28245143 | 1.57886007 | 3 | n.s. | n.s. |
| P04233 | HG2A | 3.42800442 | 1.58906794 | 4 | n.s. | n.s. |
| Q86VB7 | C163A | 8.41315108 | 1.61353141 | 28 | n.s. | n.s. |
| Q14314 | FGL2 | 2.3298371 | 1.65406515 | 4 | n.s. | n.s. |
| O95633 | FSTL3 | 3.62466462 | 1.65765523 | 3 | n.s. | n.s. |
| Q06828 | FMOD | 3.19131517 | 1.66901188 | 2 | n.s. | n.s. |
| P15907 | SIAT1 | 3.38819447 | 1.72222858 | 5 | n.s. | n.s. |
| P24158 | PRTN3 | 4.26712302 | 1.72912265 | 9 | n.s. | n.s. |
| O75023 | LIRB5 | 3.38543795 | 1.74950522 | 7 | n.s. | n.s. |

(continued)

| Protein ID | Entry name | Discovery cohort | | Validation cohort | | |
|---|---|---|---|---|---|---|
| | | Log(p-value) | (log2) FC | Peptides | Log(p-value) | (log2) FC |
| O75594 | PGRP1 | 7.00488387 | 1.75491107 | 5 | n.s. | n.s. |
| Q01638 | ILRL1 | 3.84833514 | 1.7810369 | 15 | n.s. | n.s. |
| P80188 | NGAL | 5.1893638 | 1.78977183 | 15 | n.s. | n.s. |
| Q14767 | LTBP2 | 6.0346016 | 1.96640871 | 4 | n.s. | n.s. |
| P18510 | IL1RA | 4.61609587 | 1.9711799 | 4 | n.s. | n.s. |
| P10645 | CMGA | 4.96841668 | 1.99850054 | 14 | n.s. | n.s. |
| Q96FE7 | P3IP1 | 5.656079 | 2.02447772 | 3 | n.s. | n.s. |
| P07998 | RNAS1 | 5.17237657 | 2.08542297 | 3 | n.s. | n.s. |
| P17900 | SAP3 | 6.88676605 | 2.09765396 | 7 | n.s. | n.s. |
| P08246 | ELNE | 3.20231883 | 2.12209068 | 5 | n.s. | n.s. |
| Q9Y6U3 | ADSV | 6.44086105 | 2.13723126 | 3 | n.s. | n.s. |
| Q13443 | ADAM9 | 5.56537088 | 2.17278805 | 2 | n.s. | n.s. |
| P05451 | REG1A | 4.98736174 | 2.19621618 | 6 | n.s. | n.s. |
| Q02818 | NUCB1 | 7.9321741 | 2.32918641 | 12 | n.s. | n.s. |
| Q14508 | WFDC2 | 5.39001622 | 2.40279253 | 4 | n.s. | n.s. |
| Q9Y279 | VSIG4 | 10.6608539 | 3.29922395 | 10 | 3.91530032 | 3.02987284 |

In receiver-operating characteristic analyses of the Discovery cohort, sVSIG4 showed the highest AUC of all proteins identified in the data set that prognostically indicate sepsis with an increased risk of mortality (Figure 12). The AUC of sVSIG4 was 0.77, whereas the clinical data of CRP and PCT in this prognosis showed significantly worse AUC values of 0.66 and 0.56, respectively. The AUC of sVSIG4 for prognostic purposes to identify patients at increased risk of mortality was not only best in terms of the proteomic data set of detected plasma proteins but also performed better than the available clinical parameters, better than the SOFA-score, APACHE ii- and SAPS-score on the day of sample collection ($AUC_{SOFA}=0.73$, $AUC_{APACHEii}=0.71$, $AUC_{SAPS}=0.71$), which are measures of increase in organ dysfunction (Figure 13).

**Verification and quantitative analysis by ELISA**

Plasma levels of sVSIG4 were subsequently quantified by ELISA (enzyme-linked immunosorbent assay, quantitative) for verification. Using an appropriate antibody pair against the extracellular domain of VSIG4 (and thus binding to sVSIG4), plasma samples from the Discovery and Validation cohorts were assayed for sVSIG4. As a result, plasma samples from sepsis patients in the Discovery and Validation cohorts showed significantly elevated levels compared with the SIRS group: Concentration sVSIG4 (mean$\pm$SD) in plasma from sepsis patients was 83493$\pm$82876 pg/ml and 84391$\pm$83982 pg/ml in the Discovery and Validation cohorts, and 12572$\pm$40392 and 11532$\pm$41672 in plasma samples from SIRS patients in the Discovery and Validation cohorts, respectively (1-way ANOVA Dunn's multiple comparison test p<0. 0001, Figure 14A). With respect to the severity of the underlying disease, there was a significantly increased sVSIG4 level in plasma samples from patients with severe sepsis and septic shock compared to SIRS and SIRS+Organ dysfunction patients. Patients with septic shock showed the highest sVSIG4 plasma levels, but not significantly elevated compared to samples from patients with severe sepsis (Figure 14B). Significantly elevated levels of plasma-associated sVSIG4 (p<0.0001) were also detected for the patient groups with microbiologically verified positive results (compared to culture-negative patients, Figure 14C) and for patients with elevated SOFA-score ($\geq$7, compared to patients with SOFA$\leq$6, Figure 14E) in the discovery cohort. A slight but significant increase (p<0.05) in plasma sVSIG4 concentration was detected in patients with sepsis with abdominal focus vs. focus in the respiratory tract, consistent with the proteomic data (Figure 14D). Plasma samples from patients (day 1 or 2 after diagnosis) who died of sepsis during the course of the disease showed significantly increased early sVSIG4 plasma concentrations (Figure 14F) compared with surviving patients (SIRS and sepsis): sepsis (mean+SD) = 99617$\pm$76947 pg/mL and SIRS (mean+SD) = 40020$\pm$71920 pg/mL).

Comparison of sVSIG4 levels determined by ELISA with CRP and PCT levels measured in the clinic (discovery and verification cohorts, n= 360, Figure 15) showed that sVSIG4 levels in plasma of all patients correlate slightly with CRP levels, i.e. elevated CRP levels also result in elevated sVSIG4 levels in plasma of patients (Figure 15A). When samples from the SIRS group are examined alone, this correlation is lost (Figure 15B), with sVSIG4 levels ranging from 1000-10,000 pg/ml. In the patient group with sepsis, sVSIG4 levels are found to be high in all patients (in the range 10,000-100,000 pg/ml, Figure 15C) and a correlation to increasing CRP levels of patients on the same examination day is not apparent. Sepsis patients with low CRP values mostly already show high sVSIG4 values. The correlations with PCT are similar. While no correlation of plasma VSIG4 with PCT can be shown in the examinations of all patients or the patients of the SIRS group, it is evident, similarly to the comparison with CRP, that sVSIG4 appears in high concentrations on the day of examination in all patient groups (with 3 exceptions below the detection level), independent of the measured PCT value (Figure 15D and 15E). Particularly in the sepsis group, the sVSIG4 concentration is strongly elevated independent of the PCT value (Figure 15F).

In summary, the verification experiments by ELISA indicate that by using quantification methods to determine the sVSIG level in patient blood samples (e.g. in plasma) alone, or in combination with other biomarkers, it is possible to diagnostically differentiate patients with severe sepsis or septic shock from patients with SIRS or SIRS+organ dysfunction. Furthermore, high sVSIG4 levels already indicate an increased mortality risk of the patient at an early stage of the disease, so that this patient group can be identified and thus could get an individualized, more intensive monitoring in order to be able to therapeutically intervene at an early stage. sVSIG4 levels are particularly high in plasma in patients with microbiologically positive confirmations of a systemic infection and with high SOFA-score values.

The plasma concentration of sVSIG4 shows only a slight correlation to CRP (high CRP values correlate with high sVSIG4 values), but especially in the sepsis group, high sVSIG4 levels are already detectable when CRP values were still quite low on day 1 or 2 of diagnosis. sVSIG4 is therefore a marker for systemic infections, sepsis and septic shock that is independent of CRP as well as PCT.

As a predictor of sepsis with very good performance, sVSIG4 alone (Figure 16A+B) and sVSIG4 in combination with other biomarkers can be considered, e.g. PHLD (which also shows good performance alone, Figure 16C, mass spectrometry data), but also a combination with CRP (clinical data Figure 16D), which is already used in the clinic, is possible. The combination of sVSIG4 and PHLD (Figure 16E) has a sensitivity and specificity of 88.4% at a cut-off value of 0.262 (linear combination of the two markers) with an AUC of 0.94. Based on PHLD values only, quantitative determinations by ELISA may lead to even more accurate values and improved specificity/sensitivity. The combination of sVSIG4 and CRP performs slightly worse compared with the combination sVSIG4/PHLD but has a sensitivity and specificity of 86.7% at a cut-off value of -0.203 and an AUC of 0.916 (Figure 16F, ELISA data sVSIG4). The combination with myeloblastin (PRTN3) is also possible (Figure 16G) with a sensitivity and specificity of 87.1% at a cut-off value of -0.126 (linear combination of the two markers) with an AUC of 0.917.

## Verification of increased sVSIG4 abundance in plasma of sepsis patients (Sepsis-3 Definition)

In a cohort of sepsis patients (classified according to Sepsis-3 Definition) plasma levels of sVSIG4 were quantified by ELISA (enzyme-linked immunosorbent assay, quantitative) in EDTA-plasma from sepsis patients, patients with septic shock and a group of healthy volunteers (15 samples per group), using an appropriate antibody pair against the extracellular domain of VSIG4 (and thus binding to sVSIG4). Compared to the sepsis group, patients in the septic shock group showed higher CRP levels ($201\pm91.7$ mg/ml and $275.4\pm138.3$ mg/ml, (mean$\pm$SD) respectively, Fig. 17A), significantly higher PCT levels ($4.4\pm7.7$ ng/ml and $48.2\pm119.4$ ng/ml, (mean$\pm$SD) respectively, p=0.040 two-tailed t-test, Fig. 17B) and a significantly increased SOFA-score ($2.5\pm1.8$ and $11.3\pm3.2$, (mean$\pm$SD) respectively, p<0.0001, two-tailed t-test, Fig. 17C). In 14 out of 15 healthy volunteer samples, sVSIG4 in plasma was below the detection limit of the assay. Patients diagnosed with sepsis showed significantly elevated sVSIG4 levels in plasma, with a mean concentration of $31889\pm91603$ pg/ml (mean+SD), while two patients had sVSIG4 level below the detection level. Patients with septic shock showed further increased sVSIG4 levels in plasma samples with $90124\pm91684$ pg/ml (mean+SD), significantly increased compared to the sepsis group (p<0.0007, two-tailed t-test). The data indicate that sVSIG4 levels in sepsis patients (defined according to Sepsis-3 Definition) rise substantially with increased morbidity.

## Clinical study

The findings of the present invention can be further analyzed with the following clinical study which is described exemplarily.

1. Screening (n = 2000)

ICU admission:

Assessment for eligibility on 10 ICUs during a 15-month study recruitment period: Patients with severe cardiovascular diseases, patients diagnosed with sepsis or suspected sepsis, patients with septic shock

2. Assignment (n = 900) (Expected to be excluded (n = 1100))

Patient recruitment:

(i) Assessment of basic characteristics (age, gender, SOFA-score, APACHE-ii-score, SAPS-ii-score, CRP, PCT, hypotension treatment, lactate, organ dysfucntion, infection focus)
(ii) Sampling day 1 (max. 24h after ICU admission), EDTA blood sample for plasma preparation, storage at - 80°C

3. Follow-up

Follow-up visits:

(i) Assessment of basic characteristics (age, gender, SOFA-score, APACHE-ii-score, SAPS-ii-score, CRP, PCT, hypotension treatment, lactate, organ dysfunction, infection focus, microbiological result)
(ii) Patient sampling at day 1 and every other day (if applicable): EDTA blood sample for plasma preparation, storage at - 80°C

Cases: Infection group
(Urinary, respiratory, abdominal, wound, post-surgical)
Sepsis, n=300
Septic shock, n=300
Controls: Non-infection group, n=300
Severe cardiovascular diseases

4. Analysis

Cases to be analyzed (n = 900)
Marker_M1 plasma concentration
Controls to be analyzed (n = 300)
Marker_M1 plasma concentration

**Differentially abundant proteins in SIRS and sepsis patients**

The following Table 11 lists protein names and identified peptide sequences of differentially abundant proteins in SIRS and sepsis patients. In a preferred embodiment, the level of sVSIG4 can be combined with the level of any one or more of the identified proteins for the method as described herein.
Table 11. Protein accession numbers (Protein ID), gene names, protein names and identified peptide sequences of significantly differentially abundant proteins in the discovery and validation cohort of SIRS and sepsis patients. Sequences were retrieved from the UniProt Database with release number 2016_04 on May 11, 2016.

| Protein ID | Gene name | Protein name | Peptide sequence |
|---|---|---|---|
| Q13443 | ADAM9 | Disintegrin and metalloproteinase domain-containing protein 9 | GLLHLENASYGIEPLQNSSHFEHIIYR |
| | | | GYVEGVHNSSIALSDCFGLR |

(continued)

| Protein ID | Gene name | Protein name | Peptide sequence |
|---|---|---|---|
| Q76LX8 | ADAMTS13 | A disintegrin and metalloproteinase with thrombospondin motifs 13 | ACVGADLQAEMCNTQACEK |
| | | | AGAQQPAVALETCNPQPCPAR |
| | | | AHGEDDGEEILLDTQCQGLPRPEPQEA CSLEPCPPR |
| | | | CQVCGGDNSTCSPR |
| | | | DTCLGPQAQAPVPADFCQHLPK |
| | | | EHLDMCQALSCHTDPLDQSSCSR |
| | | | EYVTFLTVTPNLTSVYIANHR |
| | | | FDLELPDGNR |
| | | | GPGQADCAVAIGR |
| | | | IAIHALATNMGAGTEGANASYILIR |
| | | | IWGPLQEDADIQVYR |
| | | | LLPGPQENSVQSSACGR |
| | | | LLVPLLDGTECGVEK |
| | | | LPAPEPCVGMSCPPGWGHLDATSA GEK |
| | | | MSISPNTTYPSLLEDGR |
| | | | PLGEVVTLR |
| | | | PQPGSAGHPPDAQPGLYYSANEQCR |
| | | | QAVVVVVAAVR |
| | | | SLVELTPIAAVHGR |
| | | | TTAFHGQQVLYWESESSQAEMEFSEG FLK |
| | | | VLESSLNCSAGDMLLLWGR |
| | | | VPVQEELCGLASK |
| | | | WVNYSCLDQAR |
| | | | YGEEYGNLTRPDITFTYFQPK |
| | | | YGSQLAPETFYR |
| | | | YVLTNLNIGAELLR |
| Q86TH1 | ADAMTSL2 | ADAMTS-like protein 2 | CGICQGDGSSCTHVTGNYR |
| | | | DFTLN ETVNSIFAQGAPR |
| | | | GNAHLGYSLVTHIPAGAR |
| | | | GVCVSGKCEPIGCDGVLFSTHTLDK |
| | | | LFGHPGLDMELGPSQGQETNEVCEQA GGGACEGPPR |
| | | | MLSPGFDSSVYSDLCEAAEAVRPEER |
| | | | NCPAHWLAQDWER |
| | | | NFNIAGTVVK |

(continued)

| Protein ID | Gene name | Protein name | Peptide sequence |
|---|---|---|---|
| | | | NPACGPQWEMSEWSECTAK |
| | | | PMDVYETGIEYIVAQGPTNQGLNVMVW NQNGK |
| | | | PQPIYYGFSESAESQGLDGAGLMGFVP HNGSLYGQASSER |
| | | | SADVLALADEAGYYFFNGNYK |
| | | | SPSITFEYTLLQPPHESRPQPIYYGFSE SAESQGLDGAGL MGFVPHNGSLYGQASSER |
| | | | VANSSSEAPFPNVSTSLLTSAGNR |
| | | | YDGVEVDDSYCDALTRPEPVHEFC AGR |
| P43652 | AFM | Afamin | AESPEVCFNEESPK |
| | | | AIPVTQYLK |
| | | | CCKAESPEVCFNEESPK |
| | | | CMADKTLPECSKLPNNVLQEK |
| | | | DADPDTFFAK |
| | | | DIENFNSTQK |
| | | | DLLRNCCNTENPPGCYR |
| | | | ELISLVEDVSSNYDGCCEGDVVQCIR |
| | | | ESLLNHFLYEVAR |
| | | | FIEDNIEYITIIAFAQYVQEATFEEMEK |
| | | | FLVNLVK |
| | | | FTDSENVCQER |
| | | | FTFEYSR |
| | | | HELTDEELQSLFTNFANVVDK |
| | | | HFQNLGK |
| | | | HPDLSIPELLR |
| | | | IAPQLSTEELVSLGEK |
| | | | ICAMEGLPQK |
| | | | IVQIYKDLLR |
| | | | KSDVGFLPPFPTLDPEEK |
| | | | LCFFYNK |
| | | | LKHELTDEELQSLFTNFANVVDK |
| | | | LPNNVLQEK |
| | | | MVTAFTTCCTLSEEFACVDNLADLVFG ELCGVNENR |
| | | | NPFVFAPTLLTVAVHFEEVAK |
| | | | RHPDLSIPELLR |

(continued)

| Protein ID | Gene name | Protein name | Peptide sequence |
|---|---|---|---|
| | | | RNPFVFAPTLLTVAVHFEEVAK |
| | | | SCCEEQNK |
| | | | SDVGFLPPFPTLDPEEK |
| | | | TINPAVDHCCK |
| | | | TYVPPPFSQDLFTFHADMCQSQNEELQR |
| | | | VMNHICSKQDSISSK |
| | | | VVHFIYIAILSQK |
| | | | YAEDKFNETTEK |
| P01019 | AGT | Angiotensinogen | AAMVGMLANFLGFR |
| | | | ADSQAQLLLSTVVGVFTAPGLHLK |
| | | | ALQDQLVLVAAK |
| | | | ALQDQLVLVAAKLDTEDK |
| | | | ALQDQLVLVAAKLDTEDKLR |
| | | | ANAGKPKDPTFIPAPIQAK |
| | | | DPTFIPAPIQAK |
| | | | EPTESTQQLNKPEVLEVTLNR |
| | | | EPTESTQQLNKPEVLEVTLNRPFLFAVYDQSATALHFLGR |
| | | | FMQAVTGWK |
| | | | IDRFMQAVTGWK |
| | | | IYGMHSELWGVVHGATVLSPTAVFGTLASLYLGALDHTADR |
| | | | LDAHKVLSALQAVQGLLVAQGR |
| | | | LQAILGVPWK |
| | | | LQAILGVPWKDK |
| | | | LQAILGVPWKDKNCTSR |
| | | | PFLFAVYDQSATALHFLGR |
| | | | PKDPTFIPAPIQAK |
| | | | QPFVQGLALYTPVVLPR |
| | | | SLDFTELDVAAEK |
| | | | SLDFTELDVAAEKIDR |
| | | | SLDFTELDVAAEKIDRFMQAVTGVVK |
| | | | TGCSLMGASVDSTLAFNTYVHFQGK |
| | | | TIHLTMPQLVLQGSYDLQDLLAQAELPAILHTELNLQK |
| | | | VEGLTFQQNSLNWMK |
| | | | VGEVLNSIFFELEADER |

(continued)

| Protein ID | Gene name | Protein name | Peptide sequence |
|---|---|---|---|
| | | | VGEVLNSIFFELEADEREPTESTQQLNK PEVLEVTLNR |
| | | | VLSALQAVQGLLVAQGR |
| | | | VYIHPFHLVIHNESTCEQLAK |
| P02765 | AHSG | Alpha-2-HS-glycoprotein | AALAAFNAQNNGSNFQLEEISR |
| | | | AQLVPLPPSTYVEFTVSGTDCVAK |
| | | | AQLVPLPPSTYVEFTVSGTDCVAKEAT EAAK |
| | | | CNLLAEK |
| | | | CNLLAEKQYGFCK |
| | | | EATEAAKCNLLAEKQYGFCK |
| | | | EHAVEGDCDFQLLK |
| | | | EHAVEGDCDFQLLKLDGK |
| | | | FSVVYAK |
| | | | HTFMGVVSLGSPSGEVSHPR |
| | | | HTLNQIDEVK |
| | | | HTLNQIDEVKVWPQQPSGELFEIEIDTL ETTCHVLDPTPVAR |
| | | | KVCQDCPLLAPLNDTR |
| | | | KVCQDCPLLAPLNDTRVVHAAK |
| | | | QLKEHAVEGDCDFQLLK |
| | | | QLKEHAVEGDCDFQLLKLDGK |
| | | | QPNCDDPETEEAALVAIDYINQNLPW GYK |
| | | | TVVQPSVGAAAGPVVPPCPGR |
| | | | VCQDCPLLAPLNDTR |
| | | | VCQDCPLLAPLNDTRVVHAAK |
| | | | VVHAAKAALAAFNAQNNGSNFQLEE ISR |
| | | | VWPQQPSGELFEIEIDTLETTCHVLDPT PVAR |
| P05186 | ALPL | Alkaline phosphatase, tissue-nonspecific isozyme | AIGQAGSLTSSEDTLTVVTADHSHVFTF GGYTPR |
| | | | ANEGTVGVSAATER |
| | | | CNTTQGNEVTSILR |
| | | | ENVSMVDYAHNNYQAQSAVPLR |
| | | | LDGLDLVDTWK |
| | | | NNVTDPSLSEMVVVAIQILR |
| | | | TELLTLDPHNVDYLLGLFEPGDMQYE LNR |

(continued)

| Protein ID | Gene name | Protein name | Peptide sequence |
|---|---|---|---|
| | | | VVGGERENVSMVDYAHNNYQAQSAVPLR |
| Q9Y5C1 | ANGPTL3 | Angiopoietin-related protein 3 | DLLQTVEDQYK |
| | | | DLVFSTWDHK |
| | | | FAMLDDVK |
| | | | HDGIPAECTTIYNR |
| | | | IDGSQNFNETWENYK |
| | | | IDQDNSSFDSLSPEPK |
| | | | IELEDWK |
| | | | ILANGLLQLGHGLK |
| | | | LDGEFWLGLEK |
| | | | LNIFDQSFYDLSLQTSEIKEEEK |
| | | | MLIHPTDSESFE |
| | | | PSNSQVFHVYYCDVISGSPVVTLIQHR |
| | | | YLEEQLTNLIQNPETPEHPEVTSLK |
| | | | ATPEAANASELAALR |
| | | | FTGA VCWSGPASTR |
| | | | GDHELLVLLEDWGGR |
| | | | LCPGGAGGQQQVLPPPPLVPVVPVR |
| | | | YLEEQLTNLIQNPETPEHPEVTSLK |
| | | | YQDGVYWAEFR |
| Q8NI99 | ANGPTL6 | Angiopoietin-related protein 6 | ATPEAANASELAALR |
| | | | FTGAVCWSGPASTR |
| | | | GDHELLVLLEDWGGR |
| | | | LCPGGAGGQQQVLPPPPLVPVVPVR |
| | | | VLNASAEAQR |
| | | | YQDGVYWAEFR |
| Q9H6X2 | ANTXR1 | Anthrax toxin receptor 1 | DFNETQLAR |
| | | | DHVFPVNDGFQALQGIIHSILK |
| | | | INDSVTLNEKPFSVEDTYLLCPAPILK |
| | | | LDALWVLLR |
| | | | NLNNNMRR |
| | | | SGSVLHHWNEIYYFVEQLAHK |
| Q16853 | AOC3 | Membrane primary amine oxidase | DAFCVFEQNQGLPLR |
| | | | EALAIVFFGR |
| | | | ELPQASGLLHHCCFYK |
| | | | GDQDAGACEVNPLACLPQAAACAPDLPAFSHGGFSHN |

(continued)

| Protein ID | Gene name | Protein name | Peptide sequence |
|---|---|---|---|
| | | | GVDCPYLATYVDWHFLLESQAPK |
| | | | IQMLSFAGEPLPQNSSMAR |
| | | | KEEEPSSSSVFNQNDPWAPTVDFSDFINNETIAGK |
| | | | LGPGLVDAAQAR |
| | | | LLEMEEQAAFLVGSATPR |
| | | | LVYEISLQEALAIYGGNSPAAMTTR |
| | | | QPQPNVSELVVGPLPHPSYMR |
| | | | RPVLFQEYLDIDQMIFNR |
| | | | VDLDVAGLENWVWAEDMVFVPMAVPWSPEHQLQR |
| | | | YLYLASNHSNK |
| P04114 | APOB | Apolipoprotein B-100 | AALTELSLGSAYQAMILGVDSK |
| | | | AASGTTGTYQEWK |
| | | | ADSVVDLLSYNVQGSGETTYDHK |
| | | | ADYVETVLDSTCSSTVQFLEYELNVLGTHK |
| | | | AEPLAFTFSHDYK |
| | | | AHLDIAGSLEGHLR |
| | | | ALVDTLK |
| | | | ALVDTLKFVTQAEGAK |
| | | | ALVEQGFTVPEIK |
| | | | ALYWVNGQVPDGVSK |
| | | | AQIPILR |
| | | | AQNLYQELLTQEGQASFQGLK |
| | | | AQNLYQELLTQEGQASFQGLKDNVFDGLVR |
| | | | ARYHMKADSVVDLLSYNVQGSGETTYDHK |
| | | | ASGSLPYTQTLQDHLNSLK |
| | | | ATFQTPDFIVPLTDLR |
| | | | ATGVLYDYVNK |
| | | | ATGVLYDYVNKYHWEHTGLTLR |
| | | | ATLELSPWQMSALVQVHASQPSSFHDFPDLGQEVALNANTK |
| | | | ATLYALSHAVNNYHK |
| | | | ATVAVYLESLQDTK |
| | | | AVSMPSFSILGSDVR |
| | | | CSLLVLENELNAELGLSGASMK |
| | | | CVQSTKPSLMIQK |

(continued)

| Protein ID | Gene name | Protein name | Peptide sequence |
|------------|-----------|--------------|------------------|
| | | | DAVEKPQEFTIVAFVK |
| | | | DDKHEQDMVNGIMLSVEK |
| | | | DEPTYILNIK |
| | | | DEPTYILNIKR |
| | | | DFHSEYIVSASNFTSQLSSQVEQFLHR |
| | | | DFSAEYEEDGK |
| | | | DFSAEYEEDGKYEGLQEWEGK |
| | | | DFSLWEK |
| | | | DKAQNLYQELLTQEGQASFQGLK |
| | | | DKAQNLYQELLTQEGQASFQGLKDNVF DGLVR |
| | | | DKDQEVLLQTFLDDASPGDK |
| | | | DKDQEVLLQTFLDDASPGDKR |
| | | | DLKVEDIPLAR |
| | | | DNVFDGLVR |
| | | | DQEVLLQTFLDDASPGDK |
| | | | DQEVLLQTFLDDASPGDKR |
| | | | DSYDLHDLK |
| | | | EAQEVFK |
| | | | EELCTMFIR |
| | | | EEYFDPSIVGWTVK |
| | | | EFNLQNMGLPDFHIPENLFLK |
| | | | EFQVPTFTIPK |
| | | | EIFNMAR |
| | | | ELCTISHIFIPAMGNITYDFSFK |
| | | | ENFAGEATLQR |
| | | | ENLCLNLHK |
| | | | EQHLFLPFSYK |
| | | | ESQLPTVMDFR |
| | | | EVGTVLSQVYSK |
| | | | EVYGFNPEGK |
| | | | EYSGTIASEANTYLNSK |
| | | | FDHTNSLNIAGLSLDFSSK |
| | | | FEVDSPVYNATWSASLK |
| | | | FEVDSPVYNATWSASLKNK |
| | | | FFGEGTK |
| | | | FFSLLSGSLNSHGLELNADILGTDK |
| | | | FIIPGLK |

(continued)

| Protein ID | Gene name | Protein name | Peptide sequence |
|---|---|---|---|
| | | | FIIPSPK |
| | | | FLDMLIK |
| | | | FLDSNIK |
| | | | FNEFIQNELQEASQELQQIHQYIMALR |
| | | | FNEFIQNELQEASQELQQIHQYIMALRE EYFDPSIVGWTVK |
| | | | FNSSYLQGTNQITGR |
| | | | FPEVDVLTK |
| | | | FQFPGKPGIYTR |
| | | | FRETLEDTR |
| | | | FSDEGTHESQISFTIEGPLTSFGLSNK |
| | | | FSTPEFTILNTFHIPSFTIDFVEMK |
| | | | FSVPAGIVIPSFQALTAR |
| | | | FTYLINYIQDEINTIFSDYIPYVFK |
| | | | FVEGSHNSTVSLTTK |
| | | | FVEGSHNSTVSLTTKNMEVSVATTTK |
| | | | FVTQAEGAK |
| | | | GAVDHKLSLESLTSYFSIESSTK |
| | | | GAVDHKLSLESLTSYFSIESSTKGDVK |
| | | | GESKLEVLNFDFQANAQLSNPK |
| | | | GFEPTLEALFGK |
| | | | GIISALLVPPETEEAK |
| | | | GIISALLVPPETEEAKQVLFLDTVYGNC STHFTVK |
| | | | GISTSAASPAVGTVGMDMDEDDDFSK |
| | | | GISTSAASPAVGTVGMDMDEDDDFSK WNFYYSPQSSPDKK |
| | | | GLLIFDASSSWGPQMSASVHLDSK |
| | | | GLSDEAVTSLLPQLIEVSSPITLQALVQC GQPQCSTHILQWLK |
| | | | GMALFGEGK |
| | | | GMALFGEGKAEFTGR |
| | | | GMTRPLSTLISSSQSCQYTLDAK |
| | | | GNVATEISTER |
| | | | GTYGLSCQR |
| | | | GVISIPR |
| | | | GVISIPR HEODMVNGIMLSVEK |
| | | | HFVINLIGDFEVAEK |
| | | | HINIDQFVR |

(continued)

| Protein ID | Gene name | Protein name | Peptide sequence |
|---|---|---|---|
| | | | HIQNIDIQHLAGK |
| | | | HIYAISSAALSASY |
| | | | HLIDSLIDFLNFPR |
| | | | HRHSITNPLAVLCEFISQSIK |
| | | | HSITNPLAVLCEFISQSIK |
| | | | IADFELPTIIVPEQTIEIPSIK |
| | | | IAELSATAQEIIK |
| | | | IAIANIIDEIIEK |
| | | | IAIANIIDEIIEKLK |
| | | | IDDIWNLEVK |
| | | | IDFLNNYALFLSPSAQQASWQVSAR |
| | | | IEFEWNTGTNVDTK |
| | | | IEFEWNTGTNVDTKK |
| | | | IEGNLIFDPNNYLPK |
| | | | IEIPLPFGGK |
| | | | IGQDGISTSATTNLK |
| | | | IGVELTGR |
| | | | IHSGSFQSQVELSNDQEK |
| | | | IISDYHQQFR |
| | | | ILGEELGFASLHDLQLLGK |
| | | | INNQLTLDSNTK |
| | | | INPLALK |
| | | | INPLALKESVK |
| | | | IPSVQINFK |
| | | | IQSPLFTLDANADIGNGTTSANEAGIAA SITAK |
| | | | ITENDIQIALDDAK |
| | | | ITEVALMGHLSCDTK |
| | | | ITLIINWLQEALSSASLAHMK |
| | | | ITLPDFR |
| | | | IVQILPWEQNEQVK |
| | | | IYSLWEHSTK |
| | | | KGISTSAASPAVGTVGMDMDEDDDFSK |
| | | | KIISDYHQQFR |
| | | | KITEVALMGHLSCDTK |
| | | | KLTISEQNIQR |
| | | | KMTSNFPVDLSDYPK |
| | | | KYTYNYEAESSSGVPGTADSR |

(continued)

| Protein ID | Gene name | Protein name | Peptide sequence |
|---|---|---|---|
| | | | LAAYLMLMR |
| | | | LALWGEHTGQLYSK |
| | | | LAPGELTIIL |
| | | | LATALSLSNK |
| | | | LATALSLSNKFVEGSHNSTVSLTTK |
| | | | LDFSSQADLR |
| | | | LDFSSQADLRNEIK |
| | | | LDNIYSSDK |
| | | | LDNIYSSDKFYK |
| | | | LDVTTSIGR |
| | | | LEIQSQVDSQHVGHSVLTAK |
| | | | LELELRPTGEIEQYSVSATYELQR |
| | | | LEPLKLHVAGNLK |
| | | | LEVANMQAELVAK |
| | | | LEVANMQAELVAKPSVSVEFVTNMGIII PDFAR |
| | | | LEVLNFDFQANAQLSNPK |
| | | | LFLEETK |
| | | | LIDLSIQNYHTFLIYITELLK |
| | | | LIDLSIQNYHTFLIYITELLKK |
| | | | LIDVISMYR |
| | | | LIVAMSSWLQK |
| | | | LKTQFNNNEYSQDLDAYNTK |
| | | | LLLQMDSSATAYGSTVSK |
| | | | LLLQMDSSATAYGSTVSKR |
| | | | LLSGGNTLHL VSTTK |
| | | | LLSGGNTLHL VSTTKTEVIPPLIENR |
| | | | LNDLNSVLVMPTFHVPFTDLQVPSCK |
| | | | LNELSFK |
| | | | LNGEIQALELPQK |
| | | | LNGEIQALELPQKAEALK |
| | | | LNGESNLRFNSSYLQGTNQITGR |
| | | | LNIPKLDFSSQADLR |
| | | | LNTDIAGLASAIDMSTNYNSDSLHFSN VFR |
| | | | LPQQANDYLNSFNWER |
| | | | LPYTIITTPPLK |
| | | | LPYTIITTPPLKDFSLWEK |

(continued)

| Protein ID | Gene name | Protein name | Peptide sequence |
|---|---|---|---|
| | | | LQDFSDQLSDYYEK |
| | | | LQDFSDQLSDYYEKFIAESKR |
| | | | LQSTTVMNPYMK |
| | | | LSLESLTSYFSIESSTK |
| | | | LSLESLTSYFSIESSTKGDVK |
| | | | LSLPDFK |
| | | | LSLPDFKELCTISHIFIPAMGNITYDFSFK |
| | | | LSNDMMGSYAEMK |
| | | | LSNDMMGSYAEMKFDHTNSLNIAGLSLDFSSK |
| | | | LSNVLQQVK |
| | | | LSQLQTYMIQFDQYIK |
| | | | LSQLQTYMIQFDQYIKDSYDLHDLK |
| | | | LTISEQNIQR |
| | | | LTLDIQNK |
| | | | LVELAHQYK |
| | | | LVGFIDDAVK |
| | | | LVGFIDDAVKK |
| | | | LYQLQVPLLGVLDLSTNVYSNLYNWSASYSGGNTSTDHFSLR |
| | | | MDMTFSK |
| | | | MGLAFESTK |
| | | | MTSNFPVDLSDYPK |
| | | | MYQMDIQQELQR |
| | | | NDFFLHYIFMENAFELPTGAGLQLQISSSGVIAPGAK |
| | | | NFVASHIANILNSEELDIQDLK |
| | | | NFVASHIANILNSEELDIQDLKK |
| | | | NHLQLEGLFFTNGEHTSK |
| | | | NIFNFK |
| | | | NIILPVYDK |
| | | | NIQEYLSILTDPDGK |
| | | | NIQEYLSILTDPDGKGK |
| | | | NKADYVETVLDSTCSSTVQFLEYELNVLGTHK NKADYVETVLDSTCSSTVQFLEYELNVLGTHKIEDGTLASK |
| | | | NLLVALK |

(continued)

| Protein ID | Gene name | Protein name | Peptide sequence |
|---|---|---|---|
| | | | NLLVALKDFHSEYIVSASNFTSQLSSQVEQFLHR |
| | | | NLQDLLQFIFQLIEDNIK |
| | | | NLQNNAEWVYQGAIR |
| | | | NLTDFAEQYSIQDWAK |
| | | | NMEVSVATTTK |
| | | | NNALDFVTK |
| | | | NNALDFVTKSYNETK |
| | | | NPNGYSFSIPVK |
| | | | NQDVHSINLPFFETLQEYFER |
| | | | NRQTIIVVLENVQR |
| | | | NSEEFAAAMSR |
| | | | NSLFFSAQPFEITASTNNEGNLK |
| | | | NSLKIEIPLPFGGK |
| | | | NTASLKYENYELTLK |
| | | | NTASLKYENYELTLKSDTNGK |
| | | | NTLELSNGVIVK |
| | | | PLSTLISSSQSCQYTLDAK |
| | | | PSLMIQK |
| | | | PSVSVEFVTNMGIIIPDFAR |
| | | | PTGEIEQYSVSATYELQR |
| | | | QGFFPDSVNK |
| | | | QHIEAIDVR |
| | | | QIDDIDVR |
| | | | QSFDLSVK |
| | | | QSMTLSSEVQIPDFDVDLGTILR |
| | | | QSWSVCK |
| | | | QTEATMTFK |
| | | | QTIIVVLENVQR |
| | | | QTVNLQLQPYSLVTTLNSDLK |
| | | | QVFLYPEK |
| | | | QVFLYPEKDEPTYILNIK |
| | | | QVFLYPEKDEPTYILNIKR |
| | | | QVFPGLNYCTSGAYSNASSTDSASYYPLTGDTR |
| | | | QVLFLDTVYGNCSTHFTVK |
| | | | RGIISALLVPPETEEAK |
| | | | RHIQNIDIQHLAGK |

(continued)

| Protein ID | Gene name | Protein name | Peptide sequence |
|---|---|---|---|
| | | | RNLQNNAEWVYQGAIR |
| | | | SEILAHWSPAK |
| | | | SEYQADYESLR |
| | | | SFDYHQFVDETNDK |
| | | | SFDYHQFVDETNDKIR |
| | | | SGSSTASWIQNVDTK |
| | | | SGSSTASWIQNVDTKYQIR |
| | | | SGVQMNTNFFHESGLEAHVALK |
| | | | SISAALEHK |
| | | | SKPTVSSSMEFKYDFNSSMLYSTAK |
| | | | SLWDFLK |
| | | | SNTVASLHTEK |
| | | | SNTVASLHTEKNTLELSNGVIVK |
| | | | SPAFTDLHLR |
| | | | SPSQADINK |
| | | | SSVITLNTNAELFNQSDIVAHLLSSSSSVIDALQYK |
| | | | SVGFHLPSR |
| | | | SVMAPFTMTIDAHTNGNGK |
| | | | SVSDGIAALDLNAVANK |
| | | | SVSLPSLDPASAK |
| | | | TEHGSEMLFFGNAIEGK |
| | | | TEVIPPLIENR |
| | | | TFIEDVNK |
| | | | TFIEDVNKFLDMLIK |
| | | | TFIEDVNKFLDMLIKK |
| | | | TFQIPGYTVPVVNVEVSPFTIEMSAFGYVFPK |
| | | | TGISPLALIK |
| | | | TIDQMLNSELQWPVPDIYLR |
| | | | TIHDLHLFIENIDFNK |
| | | | TIHDLHLFIENIDFNKSGSSTASWIQNVDTK |
| | | | TILGTMPAFEVSLQALQK |
| | | | TLADLTLLDSPIK |
| | | | TLADLTLLDSPIKVPLLLSEPINIIDALEMR |
| | | | TLQGIPQMIGEVIR |

(continued)

| Protein ID | Gene name | Protein name | Peptide sequence |
|---|---|---|---|
| | | | TNPTGTQELLDIANYLMEQIQDDCTGDEDYTYLILR |
| | | | TPALHFK |
| | | | TQFNNNEYSQDLDAYNTK |
| | | | TSQCTLKEVYGFNPEGK |
| | | | TSSFALNLPTLPEVK |
| | | | TSSFALNLPTLPEVKFPEVDVLTK |
| | | | TTKQSFDLSVK |
| | | | TTLTAFGFASADLIEIGLEGK |
| | | | VAWHYDEEK |
| | | | VAWHYDEEKIEFEWNTGTNVDTK |
| | | | VEDIPLAR |
| | | | VELEVPQLCSFILK |
| | | | VHANPLLIDVVTYLVALIPEPSAQQLR |
| | | | VHNGSEILFSYFQDLVITLPFELR |
| | | | VHNGSEILFSYFQDLVITLPFELRK |
| | | | VIGNMGQTMEQLTPELK |
| | | | VKHLIDSLIDFLNFPR |
| | | | VLADKFIIPGLK |
| | | | VLLDQLGTTISFER |
| | | | VLVDHFGYTK |
| | | | VNQNLVYESGSLNFSK |
| | | | VNQNLVYESGSLNFSKLEIQSQVDSQHVGHSVLTAK |
| | | | VNWEEEAASGLLTSLK |
| | | | VNWEEEAASGLLTSLKDNVPK |
| | | | VPLLLSEPINIIDALEMR |
| | | | VPQTDMTFR |
| | | | VPSYTLILPSLELPVLHVPR |
| | | | VSALLTPAEQTGTWK |
| | | | VSSFYAK |
| | | | VSTAFVYTK |
| | | | VTQEFHMK |
| | | | WNFYYSPQSSPDK |
| | | | WNFYYSPQSSPDKK |
| | | | YDFNSSMLYSTAK |
| | | | YDKNQDVHSINLPFFETLQEYFER |
| | | | YEDGTLSLTSTSDLQSGIIK |

(continued)

| Protein ID | Gene name | Protein name | Peptide sequence |
|---|---|---|---|
| | | | YEDGTLSLTSTSDLQSGIIKNTASLK |
| | | | YEGLQEWEGK |
| | | | YENYELTLK |
| | | | YEVDQQIQVLMDK |
| | | | YEVDQQIQVLMDKLVELAHQYK |
| | | | YGMVAQVTQTLK |
| | | | YHMKADSVVDLLSYNVQGSGETTY DHK |
| | | | YHWEHTGLTLR |
| | | | YKLQDFSDQLSDYYEK |
| | | | YLRTEHGSEMLFFGNAIEGK |
| | | | YLSLVGQVYSTLVTYISDWWTLAAK |
| | | | YNALDL TNNGK |
| | | | YNALDL TNNGKLR |
| | | | YNQNFSAGNNENIMEAHVGINGEANLD FLNIPLTIPEMR |
| | | | YRITENDIQIALDDAK |
| | | | YSQPEDSLIPFFEITVPESQLTVSQFT LPK |
| | | | YTYNYEAESSSGVPGTADSR |
| | | | YYELEEK |
| | | | YYELEEKIVSLIK |
| P02654 | APOC1 | Apolipoprotein C-I | ARELISRIK |
| | | | EFGNTLEDK |
| | | | EWFSETFQK |
| | | | LKEFGNTLEDK |
| | | | MREWFSETFQK |
| P02656 | APOC3 | Apolipoprotein C-III | DALSSVQESQVAQQAR |
| | | | DKFSEFWDLDPEVR |
| | | | DKFSEFWDLDPEVRPTSAVAA |
| | | | DYWSTVK |
| | | | FSEFWDLDPEVR |
| | | | FSEFWDLDPEVRPTSAVAA |
| | | | GWVTDGFSSLK |
| | | | GWVTDGFSSLKDYWSTVK |
| | | | GWVTDGFSSLKDYWSTVKDK |
| | | | TAKDALSSVQESQVAQQAR |
| P55056 | APOC4 | Apolipoprotein C-IV | AWFLESK |
| | | | DGWQWFWSPSTFR |

(continued)

| Protein ID | Gene name | Protein name | Peptide sequence |
|---|---|---|---|
| | | | DLGPLTK |
| | | | ELLETVVNR |
| | | | GFMQTYYDDHLR |
| | | | MKELLETVVNR |
| P02649 | APOE | Apolipoprotein E | AATVGSLAGQPLQER |
| | | | ALMDETMK |
| | | | AYKSELEEQLTPVAEETR |
| | | | DRLDEVKEQVAEVR |
| | | | FWDYLR |
| | | | GEVQAMLGQSTEELRVER |
| | | | LAVYQAGAR |
| | | | LGADMEDVCGR |
| | | | LGPLVEQGR |
| | | | LQAEAFQAR |
| | | | QWAGLVEK |
| | | | SELEEQLTPVAEETR |
| | | | SWFEPLVEDMQR |
| | | | VEQAVETEPEPELR |
| | | | VQAAVGTSAAPVPSDNH |
| | | | WELALGR |
| | | | WVQTLSEQVQEELLSSQVTQELR |
| P02749 | APOH | Beta-2-glycoprotein 1 | ATFGCHDGYSLDGPEEIECTK |
| | | | ATFGCHDGYSLDGPEEIECTKLGNWSAMPSCK |
| | | | ATVVYQGER |
| | | | CFKEHSSLAFWK |
| | | | CPFPSRPDNGFVNYPAK |
| | | | CPFPSRPDNGFVNYPAKPTLYYK |
| | | | CSYTEDAQCIDGTIEVPK |
| | | | CTEEGKWSPELPVCAPIICPPPSIPTFATLR |
| | | | DKATFGCHDGYSLDGPEEIECTK |
| | | | DKA TFGCHDGYSLDGPEEIECTKLGNW |
| | | | SAMPSCK |
| | | | |
| | | | DTAVFECLPQHAMFGNDTITCTTHGNWTK DTAVFECLPQHAMFGNDTITCTTHGNWTKLPECR |

(continued)

| Protein ID | Gene name | Protein name | Peptide sequence |
|---|---|---|---|
| | | | EHSSLAFWK |
| | | | FICPLTGLWPINTLK |
| | | | KCSYTEDAQCIDGTIEVPK |
| | | | KFICPLTGLWPINTLK |
| | | | LGNWSAMPSCK |
| | | | PDDLPFSTVVPLK |
| | | | PDNGFVNYPAK |
| | | | PDNGFVNYPAKPTLYYK |
| | | | PSAGNNSLYR |
| | | | PTLYYK |
| | | | TCPKPDDLPFSTVVPLK |
| | | | TDASDVKPC |
| | | | TFYEPGEEITYSCK |
| | | | TFYEPGEEITYSCKPGYVSR |
| | | | VCPFAGILENGAVR |
| | | | VYKPSAGNNSLYR |
| | | | VYKPSAGNNSLYRDTAVFECLPQHAMFGNDTITCTTHGNWTK |
| | | | WSPELPVCAPIICPPPSIPTFATLR |
| | | | YTTFEYPNTISFSCNTGFYLNGADSAK |
| O95445 | APOM | Apolipoprotein M | AFLLTPR |
| | | | DGLCVPR |
| | | | EELATFDPVDNIVFNMAAGSAPMQLHLR |
| | | | EFPEVHLGQWYFIAGAAPTK |
| | | | EFPEVHLGQWYFIAGAAPTKEELATFDPVDNIVFNMAAGSAPMQLHLR |
| | | | FLLYNR |
| | | | KWIYHLTEGSTDLR |
| | | | MAAGSAPMQLHLR |
| | | | MFHQIWAALLYFYGIILNSIYQCPEHSQLTTLGVDGK |
| | | | NQEACELSNN |
| | | | SLTSCLDSK |
| | | | TEGRPDMKTELFSSSCPGGIMLNETGQGYQR |
| | | | TELFSSSCPGGIMLNETGQGYQR |
| | | | WIYHLTEGSTDLR |

(continued)

| Protein ID | Gene name | Protein name | Peptide sequence |
|---|---|---|---|
| | | Asialoglycoprotein | |
| P07306 | ASGR1 | receptor 1 | ETFSNFTASTEAQVK |
| | | | LEDAHLVVVTSWEEQK |
| | | | SLSCQMAALQGNGSER |
| | | Asialoglycoprotein | |
| P07307 | ASGR2 | receptor 2 | ADHDALLFHLK |
| | | | EAFSNFSSSTLTEVQAISTHGGSVGDK |
| | | | FIVQHTNPFNTWIGLTDSDGSWK |
| | | | FVACQMELLHSNGSQR |
| | | | NWAVTQPDNWHGHELGGSEDCVEVQPDGR |
| | | | TCCPVNWVEHQGSCYWFSHSGK |
| | | | WNDDFCLQVYR |
| | | | YCQLENAHLWINSWEEQK |
| O75882 | ATRN | Attractin | AATCINPLNGSVCER |
| | | | AATCINPLNGSVCERPANHSAK |
| | | | AVVNGNIMWVVGGYMFNHSDYNMVLAYDLASR |
| | | | CFSSDFMAYDIACDR |
| | | | CNPGTGQCVCPAGWVGEQCQHCGGR |
| | | | CTWLIEGQPNR |
| | | | CVWNTGSSQCISWALATDEQEEK |
| | | | DGNETVPEVVATSGYALLHFFSDAAYNLTGFNITYSFDMCPNNCSGR |
| | | | DLDMFINASK |
| | | | DNPMYYCNK |
| | | | EEYSNLKLPR |
| | | | EQYAVVGHSAHIVTLK |
| | | | |
| | | | FGHSAVLHNSTMYVFGGFNSLLLSDILVFTSEQCDAHR FNHFATECSWDHLYVYDGDSIYAPLVAAFSGLIVPER GCSCFSDWQGPGCSVPVPANQSFWTR |
| | | | |
| | | | GDECQLCEVENR |
| | | | GEACDIPHCTDNCGFPHR |
| | | | GPVKMPSQAPTGNFYPQPLLNSSMCLEDSR |

(continued)

| Protein ID | Gene name | Protein name | Peptide sequence |
|---|---|---|---|
| | | | HCETCISGFYGDPTNGGK |
| | | | IDSTGNVTNELR |
| | | | IDSTGNVTNELRVFHIHNESWVLLTPK |
| | | | INVSYWCWEDMSPFTNSLLQWMPSEPSDAGFCGILSEPSTR |
| | | | ISNSSDTVECECSENWK |
| | | | KINVSYWCWEDMSPFTNSLLQWMPSEPSDAGFCGILSEPSTR |
| | | | KVEFVLK |
| | | | LADDLYRYDVDTQMWTILK |
| | | | LADDLYRYDVDTQMWTILKDSR |
| | | | LTGSSGFVTDGPGNYK |
| | | | LTLTPWVGLR |
| | | | MPSQAPTGNFYPQPLLNSSMCLEDSR |
| | | | NFNLNITWAASFSAGTQAGEEMPVVSK |
| | | | NHNALLASLTTQK |
| | | | NHPNITFFVYVSNFTWPIK |
| | | | NHSCSEGQISIFR |
| | | | NQECIALPENICGIGWHLVGNSCLK |
| | | | NTWSILHTQGALVQGGYGHSSVYDHR |
| | | | SCALDQNCQWEPR |
| | | | SEAACLAAGPGIR |
| | | | SVNNVVVR |
| | | | TACGDCTSGSSECMWCSNMK |
| | | | VFHIHNESWVLLTPK |
| | | | VVMLVIFGHCPLYGYISNVQEYDLDK |
| | | | WSVLPRPDLHHDVNR |
| | | | YDVDTQMWTILK |
| | | | YDVDTQMWTILKDSR |
| | | | YGHSLALYK |
| | | | YLHTAVIVSGTMLVFGGNTHNDTSMSHGAK |
| | | | YNWSFIHCPACQCNGHSK |
| | | | YYTAINFVATPDEQNR |
| | | | YYTAINFVATPDEQNRDLDMFINASK |
| P20160 | AZU1 | Azurocidin | EANLTSSVTILPLPLQNATVEAGTR |
| | | | FVNVTVTPEDQCR |
| | | | FVNVTVTPEDQCRPNNVCTGVLTR |

(continued)

| Protein ID | Gene name | Protein name | Peptide sequence |
|---|---|---|---|
| Q9NY97 | B3GNT2 | N-acetyllactosaminide beta-1,3-N-acetylglucosaminyltransfe rase 2 | CRNYSLLIDQPDK |
| | | | DLFIGDVIHNAGPHR |
| | | | DTFFNLSLK |
| | | | ESWGQESNAGNQTWR |
| | | | KPQEMIDIWSQLQSAHLK |
| | | | LSNISHLNYCEPDLR |
| | | | QYNPILSMLTNQTGEAGR |
| | | | VTSVVTGFNNLPDR |
| | | | WVSTSCPDTEFVFK |
| P15291 | B4GALT1 | Beta-1,4- galactosyltransferase 1 | DYDYTCFVFSDVDLIPMNDHNAYR |
| | | | ETMLSDGLNSLTYQVLDVQR |
| | | | FGFSLPYVQYFGGVSALSK |
| | | | LLNVGFQEALK |
| | | | LPQLVGVSTPLQGGSNSAAAIGQSSGELR |
| | | | QQLDYGIYVINQAGDTIFNR |
| | | | YWLYYLHPVLQR |
| P06276 | BCHE | Cholinesterase | AILQSGSFNAPWAVTSLYEAR |
| | | | DEGTAFLVYGAPGFSK |
| | | | DNNSIITR |
| | | | DNYTKAEEILSR |
| | | | EALGDVVGDYNFICPALEFTK |
| | | | ENETEIIK |
| | | | ESILFHYTDWVDDQRPENYR |
| | | | FSEWGNNAFFYYFEHR |
| | | | FWTSFFPK |
| | | | GMNLTVFGGTVTAFLGIPYAQPPLGR |
| | | | IFFPGVSEFGK |
| | | | LPWPEWMGVMHGYEIEFVFGLPLER |
| | | | NIAAFGGNPK |
| | | | NKDPQEILLNEAFVVPYGTPLSVNFGPTVDGDFLTDMPDILLELGQFK |
| | | | NQFNDYTSK |
| | | | SVTLFGESAGAASVSLHLLSPGSHSLFTR |
| | | | TQILVGVNK |
| | | | TQILVGVNKDEGTAFLVYGAPGFSK |

(continued)

| Protein ID | Gene name | Protein name | Peptide sequence |
|---|---|---|---|
| | | | VGALGFLALPGNPEAPGNMGLFDQQL |
| | | | ALQWVQK |
| | | | VIVVSMNYR |
| | | | VLEMTGNIDEAEWEWK |
| | | | WNNYMMDWK |
| | | | WSDIWNATK |
| | | | YANSCCQNIDQSFPGFHGSEMWNPNT DLSEDCLYLNVWIPAPK |
| | | | YGNPNETQNNSTSWPVFK |
| | | | YLTLNTESTR |
| | | BPI fold-containing family | |
| Q8TDL5 | BPIFB1 | B member 1 | AAVAAVLSPEEFMVLLDSVLPESAHR |
| | | | ALGFEAAESSLTK |
| | | | ALGFEAAESSLTKDALVLTPASLWKPS SPVSQ |
| | | | DHNATSILQQLPLLSAMR |
| | | | EKPAGGIPVLGSLVNTVLK |
| | | | GDQLILNLNNISSDR |
| | | | ILTQDTPEFFIDQGHAK |
| | | | IQLMNSGIGWFQPDVLK |
| | | | LEFDLLYPAIK |
| | | | LVLSDCATSHGSLR |
| | | | NIITEIIHSILLPNQNGK |
| | | | TIVEFHMTTEAQATIR |
| | | | VPISLSIDR |
| | | | WFNNSAASLTMPTLDNIPFSLIVSQD VVK |
| P35613 | BSG | Basigin | ILLTCSLNDSATEVTGHR |
| | | | SELHIENLNMEADPGQYR |
| P43251 | BTD | Biotinidase | DVQIIVFPEDGIHGFNFTR |
| | | | FNDTEVLQR |
| | | | GDMFLVANLGTK |
| | | | HNLYFEAAFDVPLK |
| | | | HVVYPTAWMNQLPLLAAIEIQK |
| | | | ILSGDPYCEKDAQEVHCDEATK |
| | | | KHNLYFEAAFDVPLK |
| | | | LSSGLVTAALYGR |
| | | | NPVGLIGAENATGETDPSHSK |
| | | | QEALELMNQNLDIYEQQVMTAAQK |

(continued)

| Protein ID | Gene name | Protein name | Peptide sequence |
|---|---|---|---|
| | | | SHLIIAQVAK |
| | | | TSIYPFLDFMPSPQVVR |
| | | | VDLITFDTPFAGR |
| | | | WNPCLEPHR |
| | | | WNPCLEPHRFNDTEVLQR |
| | | | WNVNAPPTFHSEMMYDNFTLVPVWGK |
| | | | YQFNTNVVFSNNGTLVDR |
| P00736 | C1R | Complement C1r subcomponent | CLPVCGKPVNPVEQR |
| | | | EHEAQSNASLDVFLGHTNVEELMK |
| | | | ESEQGVYTCTAQGIWK |
| | | | FCGQLGSPLGNPPGK |
| | | | FCGQLGSPLGNPPGKK |
| | | | FLEPFDIDDHQQVHCPYDQLQIYANGK |
| | | | FVRLPVANPQACENWLR |
| | | | GFLAYYQAVDLDECASR |
| | | | GGGALLGDRWILTAAHTLYPK |
| | | | GLTLHLK |
| | | | GYGFYTK |
| | | | IQYYCHEPYYK |
| | | | LFGEVTSPLFPK |
| | | | LFGEVTSPLFPKPYPNNFETTTVITVPTGYR |
| | | | LPVANPQACENWLR |
| | | | LVFQQFDLEPSEGCFYDYVK |
| | | | MDVFSQNMFCAGHPSLK |
| | | | MGNFPWQVFTNIHGR |
| | | | MLLTFHTDFSNEENGTIMFYK |
| | | | PYPNNFETTTVITVPTGYR |
| | | | QDACQGDSGGVFAVR |
| | | | QDESYNFEGDIALLELENSVTLGPNLLPICLPDNDTFYDLGLMGYVSGFGVMEEK |
| | | | QGYQLIEGNQVLHSFTAVCQDDGTWHR |
| | | | QRPPDLDTSSNAVDLLFFTDESGDSR |
| | | | SGEEDPQPQCQHLCHNYVGGYFCSCR |
| | | | TLDEFTIIQNLQPQYQFR |
| | | | VLNYVDWIK |
| | | | VLNYVDWIKK |

(continued)

| Protein ID | Gene name | Protein name | Peptide sequence |
|---|---|---|---|
| | | | WILTAAHTLYPK |
| | | | WVATGIVSWGIGCSR |
| | | | YTTEIIK |
| | | | YTTTMGVNTYK |
| | | Complement C1s | |
| P09871 | C1S | subcomponent | CEYQIR |
| | | | CQPVDCGIPESIENGK |
| | | | CQPVDCGIPESIENGKVEDPESTLFGSVIR |
| | | | CVPVCGVPR |
| | | | DVVQITCLDGFEVVEGR |
| | | | EDFDVEAADSAGNCLDSLVFVAGDR |
| | | | EDTPNSVWEPAK |
| | | | EPTMYVGSTSVQTSR |
| | | | FYAAGLVSWGPQCGTYGLYTR |
| | | | GDSGGAFAVQDPNDK |
| | | | GFQVVVRLR |
| | | | IIGGSDADIK |
| | | | LLEVPEGR |
| | | | LPVAPLR |
| | | | LQVIFK |
| | | | MGPTVSPICLPGTSSDYNLMDGDLGLISGWGR |
| | | | MLTPEHVFIHPGWK |
| | | | NCGVNCSGDVFTALIGEIASPNYPK |
| | | | NCGVNCSGDVFTALIGEIASPNYPKPYPENSR NCGVNCSGDVFTALIGEIASPNYPKPYPENSRCEYQIR NFPWQVFFDNPWAGGALINEYWVLTAAHVVEGNR |
| | | | NYVDWIMK |
| | | | QFGPYCGHGFPGPLNIETK |
| | | | REDFDVEAADSAGNCLDSLVFVAGDR |
| | | | SNALDIIFQTDLTGQK |
| | | | SSNNPHSPIVEEFQVPYNK |
| | | | SWDIEVPEGYGIHLYFTHLDIELSENCAYDSVQIISGDTEEGR |

(continued)

| Protein ID | Gene name | Protein name | Peptide sequence |
|---|---|---|---|
| | | | TNFDNDIALVR |
| | | | VEDPESTLFGSVIR |
| | | | VEKPTADAEAYVFTPNMICAGGEK |
| | | | VGATSFYSTCQSNGK |
| | | | YHGDPMPCPKEDTPNSVWEPAK |
| | | | YTCEEPYYYMENGGGGEYHCAGNGS WVNEVLGPELPK |
| | | Complement component | |
| P13671 | C6 | C6 | AKDLHLSDVFLK |
| | | | ALNHLPLEYNSALYSR |
| | | | CLNNQQLHFLHIGSCQDGR |
| | | | CLPDGTWR |
| | | | CPINCLLGDFGPWSDCDPCIEK |
| | | | CVCLLPPQCFK |
| | | | DLHLSDVFLK |
| | | | DLTSLGHNENQQGSFSSQGGSSFSVPI FYSSK |
| | | | ECNNPAPQR |
| | | | ENPAVIDFELAPIVDLVR |
| | | | ESCGYDTCYDWEK |
| | | | EVDLPEIEADSGCPQPVPPENGFIR |
| | | | GEVLDNSFTGGICK |
| | | | GFVVAGPSR |
| | | | GGNQLYCVK |
| | | | IEEADCK |
| | | | IFDDFGTHYFTSGSLGGVYDLLYQFSSE ELK |
| | | | IFDDFGTHYFTSGSLGGVYDLLYQFSSE ELKNSGLTEEEAK |
| | | | IGESIELTCPK |
| | | | KESCGYDTCYDWEK |
| | | | KLECNGENDCGDNSDER |
| | | | KYNPIPSVQLMGNGFHFLAGEPR |
| | | | PSQFGGQPCTAPLVAFQPCIPSK |
| | | | QAIQASHK |
| | | | QLEWGLER |
| | | | QLYLVGEDVEISCLTGFETVGYQYFR |
| | | | RQEEDCTFSIMENNGQPCINDDEEMK |

(continued)

| Protein ID | Gene name | Protein name | Peptide sequence |
|---|---|---|---|
| | | | RQEEDCTFSIMENNGQPCINDDEEMKE VDLPEIEADSGCP QPVPPENGFIR |
| | | | SEYGAALAWEK |
| | | | SNAVDGQWGCWSSWSTCDATYK |
| | | | SVLRPSQFGGQPCTAPLVAFQPCIPSK |
| | | | TFSEWLESVK |
| | | | TFSEWLESVKENPAVIDFELAPIVDLVR |
| | | | TLNICEVGTIR |
| | | | VLNFTTK |
| | | | VPANLENVGFEVQTAEDDLK |
| | | | VPANLENVGFEVQTAEDDLKTDFYK |
| | | | YNPIPSVQLMGNGFHFLAGEPR |
| | | | YTCQGNSWTPPISNSLTCEK |
| | | | YYQENFCEQICSK |
| P54289 | CACNA2D1 | Voltage-dependent calcium channel subunit alpha-2/delta-1 | DAVNNITAK |
| | | | EPVTLDFLDAELENDIK |
| | | | GFSFAFEQLLNYNVSR |
| | | | HLVNISVYAFNK |
| | | | IDVNSWIENFTK |
| | | | ISDNNTEFLLNFNEFID R |
| | | | LLIQAEQTSDGPNPCDMVK |
| | | | NREEDPSLLWQVFGSATGLAR |
| | | | PDNFEESGYTFIAPR |
| | | | SFSGVLDCGNCSR |
| | | | SLDNDNYVFTAPYFNK |
| | | | SYDYQSVCEPGAAPK |
| Q6YHK3 | CD109 | CD109 antigen | EALNMLTWR |
| | | | FLVTAPGIIRPGGNVTIGVELLEHCPSQ VTVK |
| | | | GISDNYTLALITYALSSVGSPK |
| | | | HLNGTITAK |
| | | | IEFPILEDSSELQLK |
| | | | INGSANFSFNDEEMK |
| | | | INYTVPQSGTFK |
| | | | IPVQLVFK |
| | | | ISVTQPDSIVGIVAVDK |
| | | | IVTLFSDFKPYK |

(continued)

| Protein ID | Gene name | Protein name | Peptide sequence |
|---|---|---|---|
| | | | LNLYLDSVNETQFCVNIPAVR |
| | | | NYTEYWSGSNSGNQK |
| | | | PGGNVTIGVELLEHCPSQVTVK |
| | | | QHDYIIEFFDYTTVLKPSLNFTATVK |
| | | | QNSTMFSLTPENSWTPK |
| | | | SGMALMEVNLLSGFMVPSEAISLSETV KKVEYDHGK |
| | | | SNLIQQWLSQQSDLGVISK |
| | | | TASNLTVSVLEAEGVFEK |
| | | | TLTLPSLPLNSADEIYELR |
| | | | TNIQVTVTGPSSPSPVK |
| | | | TQDEILFSNSTR |
| | | | VGSPFELVVSGNK |
| | | | VQITAIGDVLGPSINGLASLIR |
| | | | YQPNIDVQESIHFLESEFSR |
| P08571 | CD14 | Monocyte differentiation antigen CD14 | AFPALTSLDLSDNPGLGER |
| | | | APQPDELPEVDNLTLDGNPFLVPGTAL PHEGSMNSGVVPACAR |
| | | | CMWSSALNSLNLSFAGLEQVPK |
| | | | CVCNFSEPQPDWSEAFQCVSAVEVEIH AGGLNLEPFLK |
| | | | ELTLEDLK |
| | | | FPAIQNLALR |
| | | | GLMAALCPHK |
| | | | ITGTMPPLPLEATGLALSSLR |
| | | | LKELTLEDLK |
| | | | LRNVSWATGR |
| | | | LTVGAAQVPAQLLVGALR |
| | | | NVSWATGR |
| | | | RLTVGAAQVPAQLLVGALR |
| | | | SWLAELQQWLK |
| | | | SWLAELQQWLKPGLK |
| | | | VLDLSCNR |
| | | | VLSIAQAHSPAFSCEQVR |
| Q86VB7 | CD163 | Scavenger receptor cysteine-rich type 1 protein M130 | AMSIPMWVDNVQCPK |
| | | | APGWANSSAGSGR |
| | | | CAGTVEVEIQR |

(continued)

| Protein ID | Gene name | Protein name | Peptide sequence |
|---|---|---|---|
| | | | CGVALSTPGGAR |
| | | | CKGNESSLWDCPAR |
| | | | EAEFGQGTGPIWLNEVK |
| | | | EDAAVNCTDISVQK |
| | | | EDAGVICSEFMSLR |
| | | | FQGEWGTICDDGWDSYDAAVACK |
| | | | GNESALWDCK |
| | | | GNESSLWDCPAR |
| | | | GPDTLWQCPSSPWEK |
| | | | HGDTWGSICDSDFSLEAASVLCR |
| | | | HKEDAGVICSEFMSLR |
| | | | HSNCTHQQDAGVTCSDGSNLEMR |
| | | | HYCNHNEDAGVTCSDGSDLELR |
| | | | IQATNTWLFLSSCNGNETSLWDCK |
| | | | IWMDHVSCRGNESALWDCK |
| | | | LASPSEETWITCDNK |
| | | | LEVFYNGAWGTVGK |
| | | | LVDGVTECSGR |
| | | | LVGGDIPCSGR |
| | | | NWQWGGLTCDHYEEAK |
| | | | QLGCGEAINATGSAHFGEGTGPIWLDEMK |
| | | | QLGCPTAVTAIGR |
| | | | SSMSETTVGVVCR |
| | | | TLGAWGSLCNSHWDIEDAHVLCQQLK |
| | | | VEIWHGGSWGTVCDDSWDLDDAQVVCQQLGCGPALK |
| | | | VEIYHEGSWGTICDDSWDLSDAHWCR |
| | | | VQEEWGTVCNNGWSMEAVSVICNQLGCPTAIK |
| | | | WGHSECGHKEDAAVNCTDISVQK |
| | | | WGTVCDDNFNIDHASVICR |
| Q8N6Q3 | CD177 | CD177 antigen | GATHCYDGYIHLSGGGLSTK |
| | | | MSIQGCVAQPSSFLLNHTR |
| | | | QEDFCNNLVNSLPLWAPQPPADPGSLR |
| P08174 | CD55 | Complement decayaccelerating factor | DSVICLKGSQWSDIEEFCNR |
| | | | EIYCPAPPQIDNGIIQGER |

(continued)

| Protein ID | Gene name | Protein name | Peptide sequence |
|---|---|---|---|
| | | | GFTMIGEHSIYCTVNNDEGEWSGPPPECR |
| | | | GSQWSDIEEFCNR |
| | | | LTCLQNLK |
| | | | QPYITQNYFPVGTVVEYECR |
| | | | TPSAAQNPMMTNASATQATLTAQK |
| P13987 | CD59 | CD59 glycoprotein | ENELTYYCCK |
| | | | LRENELTYYCCK |
| | | | TAVNCSSDFDACLITK |
| P04233 | CD74 | HLA class II histocompatibility antigen gamma chain | CQEEVSHIPAVHPGSFR |
| | | | ESLELEDPSSGLGVTK |
| | | | GHHNCSESLELEDPSSGLGVTK |
| | | | MATPLLMQALPMGALPQGPMQNATK |
| | | | YGNMTEDHVMHLLQNADPLK |
| P33151 | CDH5 | Cadherin-5 | AQVIINITDVDEPPIFQQPFYHFQLK |
| | | | DTGENLETPSSFTIK |
| | | | DWIWNQMHIDEEK |
| | | | DWIVVNQMHIDEEKNTSLPHHVGK |
| | | | ELDREVYPWYNLTVEAK |
| | | | ELDSTGTPTGKESIVQVHIEVLDENDNAPEFAKPYQPK |
| | | | ENISEYHLTAVIVDK |
| | | | ESIVQVHIEVLDEN DNAPEFAK |
| | | | ESIVQVHIEVLDEN DNAPEFAKPYQPK |
| | | | EVYPWYNLTVEAK |
| | | | FILNTENNFTLTDNHDNTANITVK |
| | | | GDYQDAFTIETNPAHNEGIIKPMKPLDYEYIQQYSFIVEATDPTIDLR |
| | | | KPLIGTVLAMDPDAAR |
| | | | LDRENISEYHLTAVIVDK |
| | | | LDRENISEYHLTAVIVDKDTGENLETPSSFTIK |
| | | | SVPEIHEQLVTYDEEGGGEMDTTSYDVSVLNSVRR |
| | | | VGTSVGSLFVEDPDEPQNR |
| | | | VHDVNDNWPVFTHR |
| | | | VHFLPVVISDNGMPSR |
| | | | YEIVVEAR |
| | | | YTFVVPEDTR |

(continued)

| Protein ID | Gene name | Protein name | Peptide sequence |
|---|---|---|---|
| Q9HBB8 | CDHR5 | Cadherin-related family member 5 | DIFEVEENTNVTEPLVDIHVPEGQEVTLGALSTPFAFR |
| | | | GNVNGTFIIHPDSGNLTVAR |
| | | | IQAQDPEFSDLNSAITYR |
| | | | IQGNQLFLNVTPDYEEK |
| | | | LDRPLDFYERPNMTFWLLVR |
| | | | SLLEAQLLCQSGGTLVTQLR |
| | | | VEEDTKVNSTVIPETQLQAEDR |
| | | | VFVSVLDVNDNAPEFPFK |
| | | | VNSTVIPETQLQAEDR |
| P13688 | CEACAM1 | Carcinoembryonic antigen-related cell adhesion molecule 1 | DAVAFTCEPETQDTTYLWWINNQSLPVSPR |
| | | | DSVNLTCSTNDTGISIR |
| | | | EDAGTYWCEVFNPISK |
| | | | ETIYPNASLLIQNVTQNDTGFYTLQVIK |
| | | | EVLLLVHNLPQQLFGYSWYK |
| | | | LSQGNTTLSINPVK |
| | | | NDTGPYECEIQNPVSANR |
| | | | NQSLPSSERMKLSQGNTTLSINPVK |
| | | | QIVGYAIGTQQATPGPANSGR |
| | | | SDLVNEEATGQFHVYPELPKPSISSNNSNPVEDK |
| | | | SDPVTLNVTYGPDTPTISPSDTYYR |
| | | | TTVTGDKDSVNLTCSTNDTGISIR |
| | | | TLTLLSVTR |
| P11597 | CETP | Cholesteryl ester transfer protein | AASILSDGDIGVDISLTGDPVITASYLESHHK |
| | | | AMMLLGQVK |
| | | | ASYPDITGEK |
| | | | DGFLLLQMDFGFPEHLLVDFLQSLS |
| | | | EINVISNIMADFVQTR |
| | | | FLFPRPDQQHSVAYTFEEDIVTTVQASYSK |
| | | | GHFIYKNVSEDLPLPTFSPTLLGDSR |
| | | | GVSLFDIINPEIITR |
| | | | GVVVNSSVMVK |
| | | | ITKPALLVLNHETAK |
| | | | LEVVFTALMNSK |
| | | | LFLSLLDFQITPK |

(continued)

| Protein ID | Gene name | Protein name | Peptide sequence |
|---|---|---|---|
| | | | LMLSLMGDEFK |
| | | | MLAATVLTLALLGNAHACSK |
| | | | MLYFWFSER |
| | | | NVSEDLPLPTFSPTLLGDSR |
| | | | QLFTNFISFTLK |
| | | | SIDVSIQNVSVVFK |
| | | | TDAPDCYLSFHK |
| | | | TVSNLTESSSESVQSFLQSMITAVGIPE VMSR |
| | | | YGLHNIQISHLSIASSQVELVEAK |
| Q9BXR6 | CFHR5 | Complement factor H-related protein 5 | AMISSPPFR |
| | | | CLDPCVVSEENMNK |
| | | | CVESTAYCGPPPSINNGDTTSFPLSVY PPGSTVTYR |
| | | | EEYGHNEVVEYDCNPNFIINGPK |
| | | | ENYLLPEAK |
| | | | EQFCPPPPQIPNAQNMTTTVNYQDGEK |
| | | | FEYPICE |
| | | | GECHVPILEANVDAQPK |
| | | | GWSTPPICSFTK |
| | | | IHHGFLYDEEDYNPFSQVPTGEVFYYS CEYNFVSPSK |
| | | | IQCVDGEWTTLPTCVEQVK |
| | | | ITCTEEGWSPTPK |
| | | | KEEYGHNEVVEYDCNPNFIINGPK |
| | | | KIQCVDGEWTTLPTCVEQVK |
| | | | LQGSVTVTCR |
| | | | MCSFPFVK |
| | | | NEYAMIGNNMITCINGIWTELPMCVATH QLK |
| | | | NGHSESSGLIHLEGDTVQIICNTGYSLQ NNEK |
| | | | NGHSESSGLIHLEGDTVQIICNTGYSLQ NNEKNISCVER |
| | | | REQFCPPPPQIPNAQNMTTTVNYQDG EK |
| | | | SCGPPPQLSNGEVK |
| | | | TCGYIPELEYGYVQPSVPPYQHGVSVE VNCR |
| | | | TGDAVEFQCK |

(continued)

| Protein ID | Gene name | Protein name | Peptide sequence |
|---|---|---|---|
| | | | VGSDSVQCYQFGWSPNFPTCK |
| | | | WNPEVDCTEK |
| P27918 | CFP | Properdin | CSAPEPSQKPPGKPCPGLAYEQR |
| | | | GLLGGGVSVEDCCLNTAFAYQK |
| | | | ITEGAQAPR |
| | | | LCTPLLPK |
| | | | NVTFWGR |
| | | | NVTFWGRPLPR |
| | | | PCPGLAYEQR |
| | | | SISCQEIPGQQSR |
| | | | YPPTVSMVEGQGEK |
| | | | YPPTVSMVEGQGEKNVTFWGR |
| | | | YPPTVSMVEGQGEKNVTFWGRPLPR |
| P36222 | CHI3L1 | Chitinase-3-like protein 1 | EAGTLAYYEICDFLR |
| | | | EGDGSCFPDALDR |
| | | | FLCTHIIYSFANISNDHIDTWEWNDVTLY GMLNTLK |
| | | | FPLTNAIK |
| | | | FSNTDYAVGYMLR |
| | | | GNQWVGYDDQESVK |
| | | | ILGQQVPYATK |
| | | | ISQHLDFISIMTYDFHGAWR |
| | | | LVCYYTSWSQYR |
| | | | LVMGIPTFGR |
| | | | QLAGAMVWALDLDDFQGSFCGQDLR |
| | | | QLLLSAALSAGK |
| | | | SFTLASSETGVGAPISGPGIPGR |
| | | | SVPPFLR |
| | | | THGFDGLDLAWLYPGR |
| | | | TLLSVGGWNFGSQR |
| | | | VTIDSSYDIAK |
| P05452 | CLEC3B | Tetranectin | CFLAFTQTK |
| | | | DQLPYICQFGIV |
| | | | EQQALQTVCLK |
| | | | GGTLGTPQTGSENDALYEYLR |
| | | | LDTLAQEVALLK |
| | | | MFEELK |
| | | | NWETEITAQPDGGK |

(continued)

| Protein ID | Gene name | Protein name | Peptide sequence |
|---|---|---|---|
| | | | QSVGNEAEIWLGLNDMAAEGTWVDMT GAR |
| | | | SRLDTLAQEVALLK |
| | | | TENCAVLSGAANGK |
| | | | TFHEASEDCISR |
| P10909 | CLU | Clusterin | ASSIIDELFQDR |
| | | | ASSIIDELFQDRFFTR |
| | | | EDALNETR |
| | | | EILSVDCSTNNPSQAK |
| | | | EIQNAVNGVK |
| | | | ELDESLQVAER |
| | | | ELPGVCNETMMALWEECK |
| | | | ELPGVCNETMMALWEECKPCLK |
| | | | EPQDTYHYLPFSLPHR |
| | | | FFTREPQDTYHYLPFSLPHR |
| | | | FMETVAEK |
| | | | HNSTGCLR |
| | | | IDSLLENDR |
| | | | KTLLSNLEEAK |
| | | | KTLLSNLEEAKK |
| | | | LANLTQGEDQYYLR |
| | | | LANLTQGEDQYYLRVTTVASHTSDSDV PSGVTEVVVK |
| | | | LFDSDPITVTVPVEVSR |
| | | | LKELPGVCNETMMALWEECKPCLK |
| | | | MLNTSSLLEQLNEQFNWVSR |
| | | | QLEEFLNQSSPFYFWMNGDR |
| | | | QLEEFLNQSSPFYFWMNGDRIDSLLEN DR |
| | | | QLEEFLNQSSPFYFWMNGDRIDSLLEN DRQQTHMLDVMQDHFSR |
| | | | QQTHMLDVMQDHFSR |
| | | | RELDESLQVAER |
| | | | SYQWKMLNTSSLLEQLNEQFNWVSR |
| | | | TLLSNLEEAK |
| | | | TLLSNLEEAKK |
| | | | VTTVASHTSDSDVPSGVTEVVVK |
| | | | VTTVASHTSDSDVPSGVTEVVVKLFDS DPITVTVPVEVSR |

(continued)

| Protein ID | Gene name | Protein name | Peptide sequence |
|---|---|---|---|
| | | | YNELLK |
| Q96KN2 | CNDP1 | Beta-Ala-His dipeptidase | AIHLDLEEYR |
| | | | AIHLDLEEYRNSSR |
| | | | ALEQDLPVNIK |
| | | | DQDFHSGTFGGILHEPMADLVALLGSL VDSSGHILVPGI YDEVVPLTEEEINTYK |
| | | | EEILMHLWR |
| | | | EWVAIESDSVQPVPR |
| | | | FFSGVDYIVISDNLWISQR |
| | | | FIIEGMEEAGSVALEELVEK |
| | | | FLFDTK |
| | | | GDGWLTDPYVLTEVDGK |
| | | | GNSYFMVEVK |
| | | | GPVLAWINAVSAFR |
| | | | GTVCFYGHLDVQPADR |
| | | | HLEDVFSK |
| | | | LFAAFFLEMAQLH |
| | | | LVPHMNVSAVEK |
| | | | MMAVAADTLQR |
| | | | MVVSMTLGLHPWIANIDDTQYLAAK |
| | | | SVVLIPLGAVDDGEHSQNEK |
| | | | TVFGTEPDMIR |
| | | | VFQYIDLHQDEFVQTLK |
| | | | WNYIEGTK |
| | | | YPSLSIHGIEGAFDEPGTK |
| Q12860 | CNTN1 | Contactin-1 | ANSTGTLVITDPTR |
| | | | AVDLIPWMEYEFR |
| | | | DVYALMGQNVTLECFALGNPVPDIR |
| | | | FIPLIPER |
| | | | FVSQTNGNLYIANVEASDKGNYSCFVS SPSITK |
| | | | GGEKNMVDSFLPVCASLPPTW |
| | | | GMVLLCDPPYHFPDDLSYR |
| | | | GNYSCFVSSPSITK |
| | | | GTEWLVNSSR |
| | | | ILIWEDGSLEINNITR |
| | | | IYVQAFPEWVEHINDTEVDIGSDLYWPC VATGK |

(continued)

| Protein ID | Gene name | Protein name | Peptide sequence |
|---|---|---|---|
| | | | STEATLSFGYLDPFPPEERPEVR |
| | | | TTKPYPADIVVQFK |
| | | | VLEPMPSTAEISTSGAVLK |
| | | | WLLNEFPVFITMDK |
| | | | YTCTAQTIVDNSSASADLVVR |
| O75976 | CPD | Carboxypeptidase D | AEATTTTTSAGAEAAEGQFDRYYHEEE LESALR |
| | | | DSITGSGLENATISVAGINHNITTGR |
| | | | FANEYPNITR |
| | | | GILNATISVAEINHPVTTYK |
| | | | GLVMNYPHITNLTNLGQSTEYR |
| | | | NMYPNEYFPHGITNGASWYNVPGGMQ DWNYLQTNCFEVTI ELGCVK |
| | | | YYHEEELESALR |
| P22792 | CPN2 | Carboxypeptidase N subunit 2 | AFGSNPNLTK |
| | | | AGGSWDLA VQER |
| | | | DHLGFQVTWPDESK |
| | | | DLEELVK |
| | | | GQVVPALNEK |
| | | | LEDLEVTGSSFLNLSTNIFSNLTSLGK |
| | | | LELLSLSK |
| | | | LFQPLTHLK |
| | | | LGSLQELFLDSNNISELPPQVFSQLFCL ER |
| | | | LLNIQTYCAGPAYLK |
| | | | LSNNALSGLPQGVFGK |
| | | | LTLNFNMLEALPEGLFQHLAALESLHLQ GNQLQALPR |
| | | | LTVSIEAR |
| | | | LYLGSNNLTALHPALFQNLSK |
| | | | LYLGSNNLTALHPALFQNLSKLELLSL SK |
| | | | NAITHLPLSIFASLGNLTFLSLQWNMLR |
| | | | NIIFVETSFTTLETR |
| | | | NQLTTLPEGIFDTNYNLFNLALHGNPW QCDCHLAYLFNWLQQYTDR |
| | | | PDAFGGLPR |
| | | | QLVCPVTR |

(continued)

| Protein ID | Gene name | Protein name | Peptide sequence |
|---|---|---|---|
| | | | SLMLSYNAITHLPAGIFR |
| | | | SLMLSYNAITHLPAGIFRDLEELVK |
| | | | SQCTYSNPEGTVVLACDQAQCR |
| | | | TLNLAQNLLAQLPEELFHPLTSLQTLK |
| | | | VLPAGLFAHTPCLVGLSLTHNQLETVAE GTFAHLSNLR |
| | | | VVFLNTQLCQFR |
| | | | VVFLNTQLCQFRPDAFGGLPR |
| | | | WLNVQLSPQQGSLGLQYNASQEWDLR |
| P20023 | CR2 | Complement receptor type 2 | CELSTSAVQCPHPQILR |
| | | | EAPYFYNDTVTFK |
| | | | EVNCSSPADMDGIQK |
| | | | HTGMMAENFLYGNEVSYECDQGFYLL GEK |
| | | | ISYYSTPIAVGTVIR |
| | | | MPVCEEIFCPSPPPILNGR |
| | | | PQHQFVRPDVNSSCGEGYK |
| | | | THSAYSHNDIVYVDCNPGFIMNGSR |
| | | | TPNGNHTGGNIAR |
| | | | YSSCPEPIVPGGYK |
| P02741 | CRP | C-reactive protein | DIGYSFTVGGSEILFEVPEVTVAPVHICT SWESASGIVE FWVDGK |
| | | | ESDTSYVSLK |
| | | | GYSIFSYATK |
| | | | GYSIFSYATKR |
| | | | QDNEILIFWSK |
| | | | RQDNEILIFWSK |
| | | | YEVQGEVFTK |
| | | | YEVQGEVFTKPQLWP |
| Q9NQ79 | CRTAC1 | Cartilage acidic protein 1 | DEASSVEVTWPDGK |
| | | | EHGDPLIEELNPGDALEPEGR |
| | | | GDGTFVDAAASAGVDDPHQHGR |
| | | | GNQGFNNNWLR |
| | | | GTGGVVTDFDGDGMLDLILSHGESMA QPLSVFR |
| | | | GVSVGPILSSSASDIFCDNENGPNFLFH NR |
| | | | IIDGGSGYLCEMEPVAHFGLGK |
| | | | LVNIAVDER |

(continued)

| Protein ID | Gene name | Protein name | Peptide sequence |
|---|---|---|---|
| | | | NVASGEMNSVLEILYPR |
| | | | QGNAIGVTACDIDGDGR |
| | | | TVITADFDNDQELEIFFNNIAYR |
| | | | VDIVYGNWNGPHR |
| | | | WEDILSDEVNVAR |
| | | | YSIYIANYAYGNVGPDALIEMDPEASDLSR |
| P01034 | CST3 | Cystatin-C | AFCSFQIYAVPWQGTMTLSK |
| | | | ALDFAVGEYNK |
| | | | LVGGPMDASVEEEGVRR |
| | | | QIVAGVNYFLDVELGR |
| | | | TQPNLDNCPFHDQPHLK |
| Q01459 | CTBS | Di-N-acetylchitobiase | DIIDPAFR |
| | | | DPAGHFHQVWYDNPQSISLK |
| | | | EIEGSQVTFDVAWSPKNIDR |
| | | | GIGMWNANCLDYSGDAVAK |
| | | | HHPDFEVFVFDVGQK |
| | | | LVMGVPWYGYDYTCLNLSEDHVCTIAK |
| | | | QINSSISGNLWDK |
| | | | QINSSISGNLWDKDQR |
| | | | SYDWSQITTVATFGK |
| | | | TQYMDGINIDIEQEVNCLSPEYDALTALVK |
| P53634 | CTSC | Dipeptidyl peptidase 1 | ILHLPTSWDWR |
| | | | ILTNNSQTPILSPQEVVSCSQYAQGCE |
| | | | GGFPYLIAGK |
| | | | SWTATTYMEYETLTLGDMIR |
| | | | VTTYCNETMTGWVHDVLGR |
| | | | YAQDFGLVEEACFPYTGTDSPCK |
| | | | YYSSEYHYVGGFYGGCNEALMK |
| Q9UBX1 | CTSF | Cathepsin F | NFVITYNR |
| | | | NLGGLETEDDYSYQGHMQSCNFSAEK |
| | | | PLRPLCSPWLIDHAVLLVGYGNR |
| | | | SAFTQGSAMISSLSQNHPDNRNETFSSVISLLNEDPLSQDLPVK |
| | | | VYINDSVELSQNEQK |
| Q9UBR2 | CTSZ | Cathepsin Z | DQECDKFNQCGTCNEFK |

(continued)

| Protein ID | Gene name | Protein name | Peptide sequence |
|---|---|---|---|
| | | | GAWPSTLLSVQNVIDCGNAGSCEGGN DLSVWDYAHQHGIP DETCNNYQAK |
| | | | LANYTGGIYAEYQDTTYINHVVSVAGW GISDGTEYWIVR |
| | | | NQHIPQYCGSCWAHASTSAMADR |
| | | | NVDGVNYASITR |
| | | | PHEYLSPADLPK |
| | | | STYPRPHEYLSPADLPK |
| | | | YNLAIEEHCTFGDPIV |
| Q14118 | DAG1 | Dystroglycan | EALPSWLHWDSQSHTLEGLPLDTDK |
| | | | EGAMSAQLGYPVVGWHIANK |
| | | | EGAMSAQLGYPVVGWHIANKK |
| | | | LFDMSAFMAGPGNAK |
| | | | LGANGSHIPQTSSVFSIEVYPEDHSELQ SVR |
| | | | LGCSLNQNSVPDIHGVEAPAR |
| | | | LVPVVNNR |
| | | | NCSTITLQNITR |
| | | | TASPDPGEVVSSACAADEPVTVLTVILD ADLTK |
| | | | VDAWVGTYFEVK |
| | | | VTIPTDLIASSGDIIK |
| | | | WENGALLSWK |
| P27487 | DPP4 | Dipeptidyl peptidase 4 | FFVVNTDSLSSVTNATSIQITAPASMLIG DHYLCDVTWAT QER |
| | | | FRPSEPHFTLDGNSFYK |
| | | | GTWEVIGIEALTSDYLYYISNEYK |
| | | | HSYTASYDIYDLNK |
| | | | IPNNTQWVTWSPVGHK |
| | | | IQNYSVMDICDYDESSGR |
| | | | ISLQWLR |
| | | | KLDFIILNETK |
| | | | LAYVWNNDIYVK |
| | | | LDFIILNETK |
| | | | LGTFEVEDQIEAAR |
| | | | LNWATYLASTENIIVASFDGR |
| | | | RIQNYSVMDICDYDESSGR |
| | | | TYTLTDYLK |

(continued)

| Protein ID | Gene name | Protein name | Peptide sequence |
|---|---|---|---|
| | | | VTCLSCELNPER |
| | | | WNCLVARQHIEMSTTGWVGR |
| | | | YMGLPTPEDNLDHYR |
| | | | YMGLPTPEDNLDHYRNSTVMSR |
| Q02487 | DSC2 | Desmocollin-2 | ANYTILK |
| | | | EQYESFEIIAFATTPDGYTPELPLPLIIK |
| | | | LSYQNDPPFGSYVVPITVR |
| | | | LTDPTGWVTIDENTGSIK |
| | | | NGIYNITVLASDQGGR |
| | | | QQMILQIGWNEAPFSR |
| | | | SFTILLSNTENQEK |
| | | | TCTGTLGIILQDVNDNSPFIPK |
| | | | TNEGVLCVVKPLNYEEK |
| | | | TSYVTSVEENTVDVEILR |
| | | | TVIICKPTMSSAEIVAVDPDEPIHGPPFD FSLESSTSEVQR |
| | | | VTVEDKDLVNTANWR |
| | | | YTYSEWHSFTQPR |
| Q03001 | DST | Dystonin | ACSTSEMMEEKPHILGDIK |
| | | | AGNDLIESSAGEEASNLQNK |
| | | | AIEIELAK |
| | | | CANGLGNDNSSNTLNTDYSFLEINNK |
| | | | DYELQTMTYRAMVDSQQKSPVK |
| | | | EVQIPELSQVFVEDVK |
| | | | ENLLNHEMVLK |
| | | | IDQILESLER |
| | | | LEMSAVADIFDR |
| | | | LESGVQFQNEAEIAGYILECENLLR |
| | | | NIQNFPSDLIENPIMKSK |
| | | | QNVDQALLNGLELLK |
| | | | SAETNIDQDINNLK |
| | | | SELNVVLQNMNQVYSMSSTYIDK |
| | | | SFSDWVSEK |
| | | | SPVQFENLEEIFDTSVSK |
| | | | STVMVRVGGGWMALDEFLVK |
| | | | SWSWHYLINEIDRIR |
| | | | VAQALCEDLSALVK |
| | | | VNNSGISLCNLISAVTTPAK |

(continued)

| Protein ID | Gene name | Protein name | Peptide sequence |
|---|---|---|---|
| | | Extracellular matrix | |
| Q16610 | ECM1 | protein 1 | ACPSHQPDISSGLELPFPPGVPTLDNIK |
| | | | APYPNYDR |
| | | | AWEDTLDK |
| | | | AWEDTLDKYCDR |
| | | | CCDLPFPEQACCAEEEK |
| | | | DILTIDIGR |
| | | | DPALCCYLSPGDEQVNCFNINYLR |
| | | | ELLALIQLER |
| | | | ELPSLQHPNEQK |
| | | | EVGPPLPQEAVPLQK |
| | | | FCEAEFSVK |
| | | | FSCFQEEAPQPHYQLR |
| | | | GQGEQGSTGGTNISSTSEPK |
| | | | HIPGLIHNMTAR |
| | | | HKHIPGLIHNMTAR |
| | | | LDGFPPGRPSPDNLNQICLPNR |
| | | | LLPAQLPAEK |
| | | | LTFINDLCGPR |
| | | | LVWEEAMSR |
| | | | NLPATDPLQR |
| | | | NVALVSGDTENAK |
| | | | PSPDNLNQICLPNR |
| | | | QGETLNFLEIGYSR |
| | | | QGNNHTCTWK |
| | | | QHVVYGPWNLPQSSYSHLTR |
| | | | SLPMDHPDSSQHGPPFEGQSGK |
| | | | SLPMDHPDSSQHGPPFEGQSQVQPPP SQEATPLQQEK |
| | | EGF-containing fibulinlike extracellular matrix | |
| Q12805 | EFEMP1 | protein 1 | ADQVCINLR |
| | | | CVNHYGGYLCLPK |
| | | | DIDECDIVPDACK |
| | | | EDEMCWNYHGGFR |
| | | | EHIVDLEMLTVSSIGTFR |
| | | | ELPQSIVYK |
| | | | FSCMCPQGYQVVR |

(continued)

| Protein ID | Gene name | Protein name | Peptide sequence |
|---|---|---|---|
| | | | GEQCVDIDECTIPPYCHQR |
| | | | GSFACQCPPGYQK |
| | | | IQCAAGYEQSEHNVCQDIDECTAGTHNCR |
| | | | LNCEDIDECR |
| | | | LTIIVGPFSF |
| | | | NPCQDPYILTPENR |
| | | | QTSPVSAMLVLVK |
| | | | RGEQCVDIDECTIPPYCHQR |
| | | | SGNENGEFYLR |
| | | | SVPSDIFQIQATTIYANTINTFR |
| | | | TAQIIVNNEQPQQETQPAEGTSGATTGVVAASSMATSGVLPGGGFVASAAAVAGPEMQTGR |
| | | | TCQDINECETTNECR |
| P98172 | EFNB1 | Ephrin-B1 | HHDYYITSTSNGSLEGLENR |
| | | | KHHDYYITSTSNGSLEGLENR |
| | | | NLEPVSWSSLNPK |
| P08246 | ELANE | Neutrophil elastase | ARPHAWPFMVSLQLR |
| | | | GGCASGLYPDAFAPVAQFVNWIDSIIQR |
| | | | GGHFCGATLIAPNFVMSAAHCVANVNVR |
| | | | GIASVLQELNVTVVTSLCR |
| | | | LGNGVQCLAMGWGLLGR |
| | | | QAGVCFGDSGSPLVCNGLIHGIASFVR |
| | | | VVLGAHNLSR |
| P00748 | F12 | Coagulation factor XII | AEEHTVVLTVTGEPCHFPFQYHR |
| | | | CFEPQLLR |
| | | | CSAPDVHGSSILPGMLCAGFLEGGTDACQGDSGGPLVCEDQAAER |
| | | | EQPPSLTR |
| | | | GGTCVNMPSGPHCLCPQHLTGNHCQK |
| | | | GRPGPQPWCATTPNFDQDQR |
| | | | LCHCPVGYTGAFCDVDTK |
| | | | LHEAFSPVSYQHDLALLR |
| | | | LHVPLMPAQPAPPK |
| | | | LQEDADGSCALLSPYVQPVCLPSGAAR |
| | | | LSWEYCDLAQCQTPTQAAPPTPVSPR |
| | | | LTLQGIISWGSGCGDR |

(continued)

| Protein ID | Gene name | Protein name | Peptide sequence |
|---|---|---|---|
| | | | NEIWYR |
| | | | NHSCEPCQTLA VR |
| | | | NKPGVYTDVAYYLAWIR |
| | | | NPDNDIRPWCFVLNR |
| | | | NWGLGGHAFCR |
| | | | PAPEDLTVVLGQER |
| | | | PGPQPWCATTPNFDQDQR |
| | | | PGVYTDVAYYLAWIR |
| | | | PSETTLCQVAGWGHQFEGAEEYASFL QEAQVPFLSLER |
| | | | RLTLQGIISWGSGCGDR |
| | | | TTLSGAPCQPWASEATYR |
| | | | TTLSGAPCQPWASEATYRNVTAEQAR |
| | | | VVGGLVALR |
| | | | WGYCLEPK |
| | | | YKAEEHTVVLTVTGEPCHFPFQYHR |
| | | Coagulation factor XIII B | |
| P05160 | F13B | chain | CFDHHFLEGSR |
| | | | CNEYYLLR |
| | | | CTKPDLSNGYISDVK |
| | | | CPPPPLPINSK |
| | | | DEEWQCLSDGWSSQPTCR |
| | | | DKVQYECATGYYTAGGK |
| | | | EAYCLDGMWTTPPLCLEPCTLSFTEM EK |
| | | | EHETCLAPELYNGNYSTTQK |
| | | | GDTYPAELYITGSILR |
| | | | GMCTSPPLIK |
| | | | HGEIVHIECELNFEIHGSAEIR |
| | | | HGVIISSTVDTYENGSSVEYR |
| | | | HPPVVMNGAVADGILASYATGSSVEYR |
| | | | IAQYYYTFK |
| | | | KEHETCLAPELYNGNYSTTQK |
| | | | KTEEVECLTYGWSLTPK |
| | | | LIENGYFHPVK |
| | | | LSFFCLAGYTTESGR |
| | | | QEEQTTCTTEGWSPEPR |
| | | | QGYDLSPLTPLSELSVQCNR |

(continued)

| Protein ID | Gene name | Protein name | Peptide sequence |
|---|---|---|---|
| | | | QSTLSYQEPLR |
| | | | QSTLSYQEPLRT |
| | | | QTYEEGDVVQFFCHENYYLSGSDLIQC YNFGWYPESPVC0 GR |
| | | | SFYFPMSIDK |
| | | | SGYLLHGSNEITCNR |
| | | | TEEVECLTYGWSLTPK |
| | | | TTGGKDEEVVQCLSDGWSSQPTCR |
| | | | VACEEPPFIENGAANLHSK |
| | | | VLHGDLIDFVCK |
| | | | VQYECATGYYTAGGK |
| | | | WDFDNRPHILHGEYIEFICR |
| | | | WSSPPVCLEPCTVNVDYMNR |
| | | | WTLPPECVENNENCK |
| P00734 | F2 | Prothrombin | ANTFLEEVR |
| | | | DKLAACLEGNCAEGLGTNYR |
| | | | DKLAACLEGNCAEGLGTNYRGHVNITR |
| | | | ELLESYIDGR |
| | | | ETAASLLQAGYK |
| | | | ETWTANVGK |
| | | | GDACEGDSGGPFVMK |
| | | | GQPSVLQVVNLPIVER |
| | | | GQPSVLQVVNLPIVERPVCK |
| | | | HQDFNSAVQLVENFCR |
| | | | ITDNMFCAGYK |
| | | | ITDNMFCAGYKPDEGK |
| | | | ITDNMFCAGYKPDEGKR |
| | | | IVEGSDAEIGMSPWQVMLFR |
| | | | KPVAFSDYIHPVCLPDR |
| | | | KPVAFSDYIHPVCLPDRETAASLLQAG YK |
| | | | KSPQELLCGASLISDR |
| | | | LAACLEGNCAEGLGTNYR |
| | | | LAACLEGNCAEGLGTNYRGHVNITR |
| | | | LAVTTHGLPCLAWASAQAK |
| | | | LKKPVAFSDYIHPVCLPDR |
| | | | NFTENDLLVR |
| | | | NPDGDEEGVWCYVAGK |

(continued)

| Protein ID | Gene name | Protein name | Peptide sequence |
|---|---|---|---|
| | | | NPDSSTTGPWCYTTDPTVR |
| | | | NPDSSTTGPWCYTTDPTVRR |
| | | | PEINSTTHPGADLQENFCR |
| | | | PGDFGYCDLNYCEEAVEEETGDGLDEDSDR |
| | | | PVAFSDYIHPVCLPDR |
| | | | PVAFSDYIHPVCLPDRETAASLLQAGYK |
| | | | QECSIPVCGQDQVTVAMTPR |
| | | | RGDACEGDSGGPFVMK |
| | | | RQECSIPVCGQDQVTVAMTPR |
| | | | SEGSSVNLSPPLEQCVPDR |
| | | | SEGSSVNLSPPLEQCVPDRGQQYQGR |
| | | | SGIECQLWR |
| | | | SPQELLCGASLISDR |
| | | | SPQELLCGASLISDRWVLTAAHCLLYPPWDKNFTENDLLVR |
| | | | SRYPHKPEINSTTHPGADLQENFCR |
| | | | TATSEYQTFFNPR |
| | | | TFGSGEADCGLR |
| | | | TFGSGEADCGLRPLFEK |
| | | | VTGWGNLK |
| | | | VTGWGNLKETWTANVGK |
| | | | WVLTAAHCLLYPPWDK |
| | | | WVLTAAHCLLYPPWDKNFTENDLLVR |
| | | | WYQMGIVSWGEGCDR |
| | | | WYQMGIVSWGEGCDRDGK |
| | | | YGFYTHVFR |
| | | | YPHKPEINSTTHPGADLQENFCR |
| | | | YTACETAR |
| P08709 | F7 | Coagulation factor VII | CHEGYSLLADGVSCTPTVEYPCGK |
| | | | DDQLICVNENGGCEQYCSDHTGTK |
| | | | FSLVSGWGQLLDR |
| | | | GATALELMVLNVPR |
| | | | KVGDSPNITEYMFCAGYSDGSK |
| | | | LHQPVVLTDHVVPLCLPER |
| | | | NCETHKDDQLICVNENGGCEQYCSDHTGTK |
| | | | NLIAVLGEHDLSEHDGDEQSR |
| | | | VAQVIIPSTYVPGTTNHDIALLR |

107

(continued)

| Protein ID | Gene name | Protein name | Peptide sequence |
|---|---|---|---|
| | | | VGDSPNITEYMFCAGYSDGSK |
| | | | VSQYIEWLQK |
| P00451 | F8 | Coagulation factor VIII | APCNIQMEDPTFK |
| | | | AWAYFSDVDLEK |
| | | | DFPILPGEIFK |
| | | | DFQITASGQYGQWAPK |
| | | | DLASGLIGPLLICYK |
| | | | DLNSGLIGALLVCR |
| | | | EAIQHESGILGPLLYGEVGDTLLIIFK |
| | | | ENGPMASDPLCLTYSYLSHVDLVK |
| | | | EWLQVDFQK |
| | | | FDDDNSPSFIQIR |
| | | | FHAINGYIMDTLPGLVMAQDQRIR |
| | | | GELNEHLGLLGPYIR |
| | | | GNSTGTLMVFFGNVDSSGIK |
| | | | HNIFNPPIIAR |
| | | | IHPQSWVHQIALR |
| | | | IQNVSSSDLLMLLR |
| | | | ISPNTSQQNFVTQR |
| | | | LHPTHYSIR |
| | | | LLESGLMNSQESSWGKNVSSTESGR |
| | | | MALYNLYPGVFETVEMLPSK |
| | | | MELMGCDLNSCSMPLGMESK |
| | | | NLFLLSTR |
| | | | NLFLTNLDNLHENNTHNQEK |
| | | | NMASHPVSLHAVGVSYWK |
| | | | PYSFYSSLISYEEDQR |
| | | | QFNATTIPENDIEK |
| | | | SFPFNTSVVYK |
| | | | SSPLTESGGPLSLSEENNDSK |
| | | | TLFVEFTDHLFNIAK |
| | | | TWVHYIAAEEEDWDYAPLVLAPDDR |
| | | | VDLLAPMIIHGIK |
| | | | VENTVLPK |
| | | | VLFQDNSSHLPAASYR |
| | | | VVFQEFTDGSFTQPLYR |
| | | | YETFSDDPSPGAIDSNNSLSEMTHFR |
| P98095 | FBLN2 | Fibulin-2 | DVDECALGTHNCSEAETCHNIQGSFR |

(continued)

| Protein ID | Gene name | Protein name | Peptide sequence |
|---|---|---|---|
| | | | EGETCGAEDNDSCGISLYK |
| | | | HYEDPYSYDQEVAEVEAATALGGEVQ AGAVQAGAGGPPAA LGGGSQPLSTIQAPPWPAVLPR |
| | | | KPQVLPHSHVEEDTDPNSVHSIPR |
| | | | PSPHNILSTSLPDAAWIPPTR |
| Q9Y6R7 | FCGBP | IgGFc-binding protein | AGCVAESTAVCR |
| | | | AIGYATAADCGR |
| | | | AISGLTIDGHAVGAK |
| | | | ALASYVAACQAAGVVIEDWR |
| | | | APGWDPLCWDECR |
| | | | ASQHGSDVVIETDFGLR |
| | | | AVGGKPAGWQVGGAQGCGECVSK |
| | | | CGPGGGSLVCTPASCGLGEVCGLLPS GQHGCQPVSTAECQ AWGDPHYVTLDGHR |
| | | | CLANGGIHYITLDGR |
| | | | CLLPGQSGPLCDALATYAAACQAAGAT VHPWR |
| | | | CPGLQNTIPWYR |
| | | | CSCSSSSGLTCQAAGCPPGR |
| | | | CSVQNGLLGCYPDR |
| | | | CVPLNNGCGCWANGTYHEAGSEFWA DGTCSQWCR |
| | | | EEFCGLLSSPTGPLSSCHK |
| | | | EGCVCDAGFVLSGDTCVPVGQCGCLH DDR |
| | | | EGCVCDAGFVLSGDTCVPVGQCGCLH DGR |
| | | | EQGGQGVCLPNYEATCWLWGDPHYH SFDGR |
| | | | EYPGQVLVDDVLQYLPFQAADGQVQV |
| | | | FR |
| | | | FAVLQENVAWGNGR |
| | | | FAVLQENVAWGNGRVSVTR |
| | | | FDFMGTCTYLLVGSCGQNAALPAFR |
| | | | FDFMGTCVYVLAQTCGTR |
| | | | FDFMGTCVYVLAQTCGTRPGLHR |
| | | | FDFQGTCEYLLSAPCHGPPLGAENFTV TVANEHR |
| | | | FGTCQGSGDPHYVSFDGR |

(continued)

| Protein ID | Gene name | Protein name | Peptide sequence |
|---|---|---|---|
| | | | FLLSQGVCIPVQDCGCTHNGR |
| | | | FNFQGTCEYLLSAPCHGPPLGAENFTV TVANEHR |
| | | | FQDQVCGLCGNYNGDPADDFLTPDGA LAPDAVEFASSWK |
| | | | GATTSPGVYELSSR |
| | | | GCGEGCGPQGCPVCLAEETAPYESNE ACGQLR |
| | | | GCVLDVCMGGGDR |
| | | | GCVLDVCMGGGDRDILCK |
| | | | GEVGFVLVDNQR |
| | | | GGGQAANALAFGNSWQEETRPGCGA TEPGDCPK |
| | | | GLCVLSVGANLTTFDGAR |
| | | | GLQAGDVVEFEVR |
| | | | GMVCQEHSCKPGQVCQPSGGILSCV TK |
| | | | HTTCNHVVEQLLPTSAWGTHYVVPTLA SQSR |
| | | | IFQHAVVIHSDYAISVQALNAK |
| | | | KVTVRPGESVMVNISAK |
| | | | LCGACGNFDGDQTNDWHDSQEK |
| | | | LCGACGNFDGDQTNDWHDSQEKPAM EK |
| | | | LCGLCGNFNGNWSDDFVLPNGSAASS VETFGAAWR |
| | | | LDDGDYLCEDGCQNNCPACTPGQAQH YEGDR |
| | | | LDGPFAVCHDTLDPR |
| | | | LDGPFAVCHDTLDPRPFLEQCVYDLCV VGGER |
| | | | LDSL VAQQLQSK |
| | | | LDSL VAQQLQSKNECGILADPK |
| | | | LEQYEGPGFCGPLAPGTGGPFTTCHA HVPPESFFK |
| | | | LLFDGDAHLLMSIPSPFR |
| | | | LLISSLSESPASVSILSQADNTSK |
| | | | LLISSLSESPASVSILSQADNTSKK |
| | | | LLVTVAGQVVSLAQGQQVTVDGEAVAL PVAVGR |
| | | | LPVSLSEGR |
| | | | LPVVLANGQIR |

110

(continued)

| Protein ID | Gene name | Protein name | Peptide sequence |
|---|---|---|---|
| | | | LRVPAAYAGSLCGLCGNYNQDPADDLK |
| | | | LTWEAVPGSEFSYAEVELGTADMIHTAEATTNLGLLTFGLAK |
| | | | LVDPQGPLK |
| | | | NEVTYDPYLVLIPDVAAYCPAYVVK |
| | | | NPNNDQVFPNGTLAPSIPIWGGSWR |
| | | | NPQGPFATCQAVLSPSEYFR |
| | | | PAGWQVGGAQGCGECVSK |
| | | | PDTAELTLLRPIQALGTEYFVLTPPGTSAR |
| | | | PFLEQCVYDLCVVGGER |
| | | | PGDEDFSIVLEK |
| | | | PGESVMVNISAK |
| | | | PGQVCQPSGGILSCVNK |
| | | | PGQVCQPSGGILSCVTK |
| | | | P IQALGTEYFVLTPPGTSAR |
| | | | PSGGSLGCVAVGSTTCQASGDPHYTTFDGHR |
| | | | PSGGSLGCVAVGSTTCQASGDPHYTTFDGR |
| | | | SLAAYTAACQAAGVAVK |
| | | | SLAAYTAACQAAGVAVKPWR |
| | | | SPANCPLSCPANSR |
| | | | SVPGCEGVALVVAQTK |
| | | | SVTLQIYNHSLTLSAR |
| | | | TCQGSCAALSGLTGCTTR |
| | | | TDFGLTVTYDWNAR |
| | | | TDSFCPLHCPAHSHYSICTR |
| | | | TEAVGQVHIFFQDGMVTLTPNK |
| | | | TPDGSLLVR |
| | | | TVLSPVEPSCEGMQCAAGQR |
| | | | VAYDLVYYVR |
| | | | VDVTLPSSYHGAVCGLCGNMDR |
| | | | VITVQVANFTLR |
| | | | VNGVLTALPVSVADGR |
| | | | VPAAYAASLCGLCGNYNQDPADDLK |
| | | | VPAAYAGSLCGLCGNYNQDPADDLK |
| | | | VPSSYAEALCGLCGNFNGDPADDLALR |

(continued)

| Protein ID | Gene name | Protein name | Peptide sequence |
|---|---|---|---|
| | | | VRVNGVLTALPVSVADGR |
| | | | VSYVGLVTVR |
| | | | VTASSPVAVLSGHSCAQK |
| | | | VTLQPYNVAQLQSSVDLSGSK |
| | | | VTVNGVDMK |
| | | | VTVNGVDMKLPVVLANGQIR |
| | | | VTVPGNYYQLMCGLCGNYNGDPK |
| | | | VTVPGNYYQQMCGLCGNYNGDPK |
| | | | VTVRPGESVMVNISAK |
| | | | VVAEVQICHGK |
| | | | VVTVAALGTNISIHK |
| | | | VVTVAALGTNISIHKDEIGK |
| | | | VVTVAALGTNISIHKDEIGKVR |
| | | | VVVCQEHSCKPGQVCQPSGGILSCVNK |
| | | | VVVCQEHSCKPGQVCQPSGGILSCVNKDPCHGVTCR |
| | | | VVVCQEHSCKPGQVCQPSGGILSCVRK |
| | | | VYDLHGSCSYVLAQVCHPK |
| | | | VYDLHGSCSYVLAQVCHPKPGDEDFSIVLEK |
| | | | YDLAFVVASQATK |
| | | | YELCGPACPTSCNGAAAPSNCSGR |
| | | | YLPVNSSLLTSDCSER |
| | | | YQKEEFCGLLSSPTGPLSSCHK |
| | | | YYPLGEVFYPGPECER |
| | | | YYPLGQTFYPGPGCDSLCR |
| P08637 | FCGR3A | Low affinity immunoglobulin gamma Fc region receptor III-A | A VVFLEPQWYR |
| | | | CQTNLSTLSDPVQLEVHIGWLLLQAPR |
| | | | YFHHNSDFYIPK |
| Q9UGM5 | FETUB | Fetuin-B | AIFYMNNPSR |
| | | | ASSQWVVGPSYFVEYLIK |
| | | | GCNDSDVLAVAGFALR |
| | | | GGLGSLFYLTLDVLETDCHVLR |
| | | | GPQEAFPVHLDLTTNPQGETLDISFLFLEPMEEK |
| | | | GSVQYLPDLDDK |
| | | | GSVQYLPDLDDKNSQEK |

(continued)

| Protein ID | Gene name | Protein name | Peptide sequence |
|---|---|---|---|
| | | | IFFESVYGQCK |
| | | | IYMTCPDCPSSIPTDSSNHQVLEAATESLAK |
| | | | LVVLPFPK |
| | | | PTNLPKVEESQQK |
| | | | SQASSCSLQSSDSVPVGLCK |
| | | | TAECPGPAQNASPLVLPP |
| | | | VLYLAAYNCTLR |
| | | | VLYLAAYNCTLRPVSK |
| P11362 | FGFR1 | Fibroblast growth factor receptor 1 | DLAARNVLVTEDNVMK |
| | | | DLSDLISEMEMMKMIGKHK |
| | | | GNYTCIVENEYGSINHTYQLDVVER |
| | | | IGPDNLPYVQILK |
| | | | LSSSGTPMLAGVSEYELPEDPR |
| | | | PILQAGLPANK |
| | | | SPHRPILQAGLPANK |
| | | | TAGVNTTDKEMEVLHLR |
| Q08830 | FGL1 | Fibrinogen-like protein 1 | DHDNYEGNCAEEDQSGWWFNR |
| | | | DYENGFGNFVQK |
| | | | IDLADFEK |
| | | | IKPLQSPAEFSVYCDMSDGGGWTVIQR |
| | | | NFYELNIGEYSGTAGDSLAGNFHPEVQWWASHQR |
| | | | NLHFLTTQEDYTLK |
| | | | QLLQENEVQFLDK |
| | | | QYADCSEIFNDGYK |
| | | | VFSFILVTTALTMGR |
| Q14314 | FGL2 | Fibroleukin | LDGSTNFTR |
| | | | LHVGNYNGTAGDALR |
| | | | LNLVNMNNIENYVDSK |
| | | | VANLTFVVNSLDGK |
| P02751 | FN1 | Fibronectin | AAVYQPQPHPQPPPYGHCVTDSGVVYSVGMQWLK |
| | | | ATGVFTTLQPGSSIPPYNTEVTETTIVITWTPAPR |
| | | | ATITGYR |
| | | | CDPVDQCQDSETGTFYQIGDSWEK |
| | | | CFDHAAGTSYVVGETWEK |

(continued)

| Protein ID | Gene name | Protein name | Peptide sequence |
|---|---|---|---|
| | | | CFDHAAGTSYVVGETWEKPYQGWMM VDCTCLGEGSGR |
| | | | CHEGGQSYK |
| | | | CNDQDTR |
| | | | DDKESVPISDTIIPAVPPPTDLR |
| | | | DDKESVPISDTIIPEVPQLTDLSFVDITD SSIGLR |
| | | | DLEVVAATPTSLLISWDAPAVTVR |
| | | | DLQFVEVTDVK |
| | | | DQCIVDDITYNVNDTFHK |
| | | | DQCIVDDITYNVNDTFHKR |
| | | | DSMIWDCTCIGAGR |
| | | | DTLTSRPAQGVVTTLENVSPPR |
| | | | EATIPGHLNSYTIK |
| | | | EDVDYHLYPHGPGLNPNASTGQEALS QTTISWAPFQDTSE YIISCHPVGTDEEPLQFR |
| | | | EESPLLIGQQSTVSDVPR |
| | | | EEVVTVGNSVNEGLNQPTDDSCFDPYT VSHYAVGDEWER |
| | | | EINLAPDSSSVVVSGLMVATK |
| | | | ESKPLTAQQTTKLDAPTNLQFVNETDS TVLVR |
| | | | ESVPISDTIIPAVPPPTDLR |
| | | | ESVPISDTIIPEVPQLTDLSFVDITDSSIG LR |
| | | | EVTSDSGSIVVSGLTPGVEYVYTIQVLR |
| | | | EYLGAICSCTCFGGQR |
| | | | FDFTTTSTSTPVTSNTVTGETTPFSPLV ATSESVTEITAS SFVVSWVSASDTVSGFR |
| | | | FGFCPMAAHEEICTTNEGVMYR |
| | | | FLATTPNSLLVSWQPPR |
| | | | FTNIGPDTMR |
| | | | FTQVTPTSLSAQWTPPNVQLTGYR |
| | | | GDSPASSKPISINYR |
| | | | GEWTCIAYSQLR |
| | | | GEWTCKPIAEK |
| | | | GFNCESKPEAEETCFDK |
| | | | GFNCESKPEAEETCFDKYTGNTYR |

114

(continued)

| Protein ID | Gene name | Protein name | Peptide sequence |
|------------|-----------|--------------|------------------|
| | | | GGNSNGALCHFPFLYNNHNYTDCTSEGR |
| | | | GGNSNGALCHFPFLYNNHNYTDCTSEGRR |
| | | | GIGEWHCQPLQTYPSSSGPVEVFITETPSQPNSHPIQWNAPQPSHISK |
| | | | GLAFTDVDVDSIK |
| | | | GLKPGVVYEGQLISIQQYGHQEVTR |
| | | | GNLLQCICTGNGR |
| | | | GNQESPK |
| | | | HEEGHMLNCTCFGQGR |
| | | | HRPRPYPPNVGEEIQIGHIPR |
| | | | HRPRPYPPNVGQEALSQTTISWAPFQDTSEYIISCHPVGTDEEPLQFR |
| | | | HTSVQTTSSGSGPFTDVR |
| | | | HYQINQQWER |
| | | | IGDQWDK |
| | | | IGDTWR |
| | | | IGDTWRRPHETGGYMLECVCLGNGK |
| | | | IGDTWSK |
| | | | ITGYIIK |
| | | | ITGYIIKYEKPGSPPR |
| | | | ITTTPTNGQQGNSLEEVVHADQSSCTFDNLSPGLEYNVSVYTVK |
| | | | ITYGETGGNSPVQEFTVPGSK |
| | | | IVYSPSVEGSSTELNLPETANSVTLSDLQPGVQYNITIYAVEENQESTPVVIQQETTGTPR |
| | | | IYLYTLNDNAR |
| | | | KTDELPQLVTLPHPNLHGPEILDVPSTVQK |
| | | | KTGQEALSQTTISWAPFQDTSEYIISCHPVGTDEEPLQFR |
| | | | LDAPTNLQFVNETDSTVLVR |
| | | | LGVRPSQGGEAPR |
| | | | LLCQCLGFGSGHFR |
| | | | NEEDVAELSISPSDNAVVLTNLLPGTEYVVSVSSVYEQHESTPLR |
| | | | NLQPASEYTVSLVAIK |

(continued)

| Protein ID | Gene name | Protein name | Peptide sequence |
|---|---|---|---|
| | | | NLQPASEYTVSLVAIKGNQESPK |
| | | | NSITLTNLTPGTEYVVSIVALNGR |
| | | | NSITLTNLTPGTEYVVSIVALNGREESPL LIGQQSTVSDVPR |
| | | | NTFAEVTGLSPGVTYYFK |
| | | | NTFAEVTGLSPGVTYYFKVFAVSHGR |
| | | | PAQGVVTTLENVSPPR |
| | | | PAQGVVTTLENVSPPRR |
| | | | PGGEPSPEGTTGQSYNQYSQR |
| | | | PGVTEATITGLEPGTEYTIYVIALK |
| | | | PGVVYEGQLISIQQYGHQEVTR |
| | | | PISINYR |
| | | | PLTAQQTTK |
| | | | PQAPITGYR |
| | | | PRPGVTEATITGLEPGTEYTIYVIALK |
| | | | PRPYPPNVGQEALSQTTISWAPFQDTS EYIISCHPVGTDEEPLQFR |
| | | | PSQMQVTDVQDNSISVK |
| | | | PYPPNVGEEIQIGHIPR |
| | | | PYPPNVGQEALSQTTISWAPFQDTSEYI ISCHPVGTDEEPLQFR |
| | | | PYQGWMMVDCTCLGEGSGR |
| | | | QAQQMVQPQSPVAVSQSK |
| | | | QAQQMVQPQSPVAVSQSKPGCYDN GK |
| | | | QDGHLWCSTTSNYEQDQK |
| | | | QKTGLDSPTGIDFSDITANSFTVHWIA PR |
| | | | QYNVGPSVSK |
| | | | RHEEGHMLNCTCFGQGR |
| | | | RPGGEPSPEGTTGQSYNQYSQR |
| | | | RPHETGGYMLECVCLGNGK |
| | | | SDTVPSPR |
| | | | SEPLIGR |
| | | | SSPVVIDASTAIDAPSNLR |
| | | | STATISGLK |
| | | | STATISGLKPGVDYTITVYAVTGR |
| | | | STTPDITGYR |
| | | | SYTITGLQPGTDYK |

(continued)

| Protein ID | Gene name | Protein name | Peptide sequence |
|---|---|---|---|
| | | | TAGPDQTEMTIEGLQPTVEYVVSVYAQ NPSGESQPLVQTA VTNIDRPK |
| | | | TAGPDQTEMTIEGLQPTVEYVVSVYAQ NPSGESQPLVQTA VTTIPAPTDLK |
| | | | TDELPQLVTLPHPNLHGPEILDVPSTV QK |
| | | | TEIDKPSQMQVTDVQDNSISVK |
| | | | TETITGFQVDAVPANGQTPIQR |
| | | | TFYSCTTEGR |
| | | | TGLDSPTGIDFSDITANSFTVHWIAPR |
| | | | TGQEALSQTTISWAPFQDTSEYIISCHP VGTDEEPLQFR |
| | | | TKTETITGFQVDAVPANGQTPIQR |
| | | | TNTNVNCPIECFM PLDVQADR |
| | | | TNTNVNCPIECFM PLDVQADREDSRE |
| | | | TPFVTHPGYDTGNGIQLPGTSGQQPSV GQQMIFEEHGFR |
| | | | TYHVGEQWQK |
| | | | TYLGNALVCTCYGGSR |
| | | | VDVIPVNLPGEHGQR |
| | | | VEYELSEEGDEPQYLDLPSTATSVNIPD LLPGR |
| | | | VEYELSEEGDEPQYLDLPSTATSVNIPD LLPGRK |
| | | | VPGTSTSATLTGLTR |
| | | | VREEVVTVGNSVNEGLNQPTDDSCFD PYTVSHYAVGDEWER |
| | | | VTDATETTITISWR |
| | | | VTIMWTPPESAVTGYR |
| | | | VTIMWTPPESAVTGYRVDVIPVNLPGE HGQR |
| | | | VTIMWTPPESAVTGYR |
| | | | VVTPLSPPTNLHLEANPDTGVLTVSW ER |
| | | | WCGTTQNYDADQK |
| | | | WKCDPVDQCQDSETGTFYQIGDSWEK |
| | | | WKEATIPGHLNSYTIK |
| | | | WLPSSSPVTGYR |
| | | | WSRPQAPITGYR |

(continued)

| Protein ID | Gene name | Protein name | Peptide sequence |
|---|---|---|---|
| | | | WTPLNSSTIIGYR |
| | | | YEVSVYALK |
| | | | YEVSVYALKDTLTSR |
| | | | YIVNVYQISEDGEQSLILSTSQTTAPDAP PDTTVDQVDDTSIVVR |
| | | | YQCYCYGR |
| | | | YSFCTDHTVLVQTR |
| | | | YSPVKNEEDVAELSISPSDNAVVLTNLL PGTEYVVSVSSV YEQHESTPLR |
| Q12841 | FSTL1 | Follistatin-related protein 1 | CALEDETYADGAETEVDCNR |
| | | | FVEQNETAINITTYPDQENNK |
| | | | GLCVDALIELSDENADWK |
| | | | GSNYSEILDK |
| | | | IIQWLEAEIIPDGWFSK |
| | | | LDSSEFLK |
| | | | LSFQEFLK |
| O95633 | FSTL3 | Follistatin-related protein 3 | AAPCPVPSSPGQELCGNNNVTYISSCH MR |
| | | | AECCASGNIDTAWSNLTHPGNK |
| | | | INLLGFLGLVHCLPCK |
| | | | PQSCVVDQTGSAHCVVCR |
| Q99988 | GDF15 | Growth/differentiation factor 15 | ANQSWEDSNTDLVPAPAVR |
| | | | ASLEDLGWADWVLSPR |
| | | | LRANQSWEDSNTDLVPAPAVR |
| 000451 | GFRA2 | GDNF family receptor alpha-2 | ILANVFCLFFFLGTGADPVVSAK |
| | | | NAIQAFGNGTDVNVSPK |
| Q9UJJ9 | GNPTG | N-acetylglucosamine-1-phosphotransferase subunit gamma | CFSLVESTYK |
| | | | DPSPVSGPVHLFR |
| | | | QWDQVEQDLADELITPQGHEK |
| | | | TLFEDAGYLK |
| | | | TPEENEPTQLEGGPDSLGFETLENCR |
| | | | VVEEPNAFGVNNPFLPQASR |
| | | | WNAYSGILGIWHEWEIANNTFTGM WMR |
| | | | YEFCPFHNVTQHEQTFR |
| Q8NBJ4 | GOLM1 | Golgi membrane protein 1 | AVLVNNITTGER |
| | | | DQLVIPDGQEEEQEAAGEGR |
| | | | DTINLLDQR |

(continued)

| Protein ID | Gene name | Protein name | Peptide sequence |
|---|---|---|---|
| | | | EETNEIQVVNEEPQR |
| | | | FSYDLSQCINQMK |
| | | | IQSSHNFQLESVNK |
| | | | LQQDVLQFQK |
| | | | LQQDVLQFQKNQTNLER |
| | | | NIDVFNVEDQK |
| | | | NIDVFNVEDQKR |
| | | | QVEKEETNEIQVVNEEPQR |
| P80108 | GPLD1 | Phosphatidylinositolglycan-specific phospholipase D | ALEFLQLHNGR |
| | | | AQYVLISPEASSR |
| | | | DLLGIYEK |
| | | | ELLLEHQDAYQAGIVFPDCFYPSICK |
| | | | ENYPLPWEK |
| | | | FGGVLHLSDLDDDGLDEIIMAAPLR |
| | | | FGSALAVLDFNVDGVPDLAVGAPSVGSEQLTYK |
| | | | FGSSLITVR |
| | | | FHDVSESTHWTPFLNASVHYIR |
| | | | GAVYVYFGSK |
| | | | GEEDFSWFGYSLHGVTVDNR |
| | | | GIVAAFYSGPSLSDK |
| | | | GIVAAFYSGPSLSDKEK |
| | | | GVFFSVNSWTPDSMSFIYK |
| | | | HVSSPLASYFLSFPYAR |
| | | | IADVTSGLIGGEDGR |
| | | | ILEGFQPSGR |
| | | | LGTSLSSGHVLMNGTLK |
| | | | LGWAMTSADLNQDGHGDLVVGAPGYSR |
| | | | LNVEAANWTVR |
| | | | LSGALHVYSLGSD |
| | | | NLTTSLTESVDR |
| | | | NQVVIAAGR |
| | | | QGGMSSSPNITISCQDIYCNLGWTLLAADVNGDSEPDLVIGSPFAPGGGK |
| | | | QVLLVGAPTYDDVSK |
| | | | SWITPCPEEK |
| | | | TLLLVGSPTWK |

(continued)

| Protein ID | Gene name | Protein name | Peptide sequence |
|---|---|---|---|
| | | | TMFIGGSQLSQK |
| | | | VAFLTVTLHQGGATR |
| | | | VITENVIVDCSHIQFLEMYGEMLA VSK |
| | | | VYLIYGNDLGLPPVDLDLDK |
| | | | VYLIYGNDLGLPPVDLDLDKEAHR |
| Q04756 | HGFAC | Hepatocyte growth factor activator | CFLGNGTGYR |
| | | | CQIAGWGHLDENVSGYSSSLR |
| | | | CSSPEVYGADISPNMLCAGYFDCK |
| | | | DSALSWEYCR |
| | | | DSVSVVLGQHFFNR |
| | | | EALVPLVADHK |
| | | | GVASTSASGLSCLAWNSDLLYQELHVD SVGAAALLGLGPHAYCR |
| | | | LEACESLTR |
| | | | NGVA YLYGIISWGDGCGR |
| | | | NPDNDERPWCYVVK |
| | | | SDACQGDSGGPLACEK |
| | | | SQFVQPICLPEPGSTFPAGHK |
| | | | TTDVTQTFGIEK |
| | | | VANYVDWINDR |
| | | | VQLSPDLLATLPEPASPGR |
| | | | YEYLEGGDR |
| | | | YIPYTLYSVFNPSDHDLVLIR |
| Q29960 | HLA-C | HLA class I histocompatibility antigen, Cw-16 alpha chain | APWVEQEGPEYWDR |
| | | | GYYNQSEAGSHTLQWMYGCDLGP DGR |
| | | | YTCHVQHEGLPEPLTLR |
| P00738 | HP | Haptoglobin | AVGDKLPECEADDGCPKPPEIAHGYVE HSVR |
| | | | DYAEVGR |
| | | | FTDHLK |
| | | | FTDHLKYVMLPVADQDQCIR |
| | | | HYEGSTVPEK |
| | | | HYEGSTVPEKK |
| | | | KQLVEIEKVVLHPNYSQVDIGLIK |
| | | | KTPKSPVGVQPILNEHTFCAGMSK |
| | | | LPECEADDGCPKPPEIAHGYVEHSVR |
| | | | LRTEGDGVYTLNNEK |

(continued)

| Protein ID | Gene name | Protein name | Peptide sequence |
|---|---|---|---|
| | | | LRTEGDGVYTLNNEKQWINK |
| | | | QLVEIEKVVLHPNYSQVDIGLIK |
| | | | SCAVAEYGVYVKVTSIQDWVQK |
| | | | SPVGVQPILNEHTFCAGMSK |
| | | | SPVGVQPILNEHTFCAGMSKYQEDTCYGDAGSAFAVHDLEEDTWYATGILSFDK |
| | | | TEGDGVYTLNNEK |
| | | | TEGDGVYTLNNEKQWINK |
| | | | TPKSPVGVQPILNEHTFCAGMSK |
| | | | VGYVSGWGR |
| | | | VGYVSGWGRNANFK |
| | | | VMPICLPSKDYAEVGR |
| | | | VTSIQDWVQK |
| | | | VTSIQDWVQKTIAEN |
| | | | VVLHPNYSQVDIGLIK |
| | | | VVLHPNYSQVDIGLIKLK |
| | | | YQEDTCYGDAGSAFAVHDLEEDTWYATGILSFDK |
| | | | YQEDTCYGDAGSAFAVHDLEEDTWYATGILSFDKSCAVAEYGVYVK |
| | | | YVMLPVADQDQCIR |
| | | | YVMLPVADQDQCIRHYEGSTVPEK |
| | | | YVMLPVADQDQCIRHYEGSTVPEKK |
| | | | AVGDKLPECEAVCGK |
| | | | AVGDKLPECEAVCGKPK |
| | | | DIAPTLTLYVGK |
| | | | DIAPTLTLYVGKK |
| | | | DIAPTLTLYVGKKQLVEIEK |
| | | | GSFPWQAK |
| | | | GSFPWQAKMVSHHNLTTGATLINEQWLLTTAK |
| | | | ILGGHLDAK |
| | | | ILGGHLDAKGSFPWQAK |
| | | | KQLVEIEK |
| | | | LPECEAVCGK |
| | | | LRTEGDGVYTLNDK |
| | | | MVSHHNLTTGATLINEQWLLTTAK |
| | | | MVSHHNLTTGATLINEQWLLTTAKNLFLNHSENATAK |

(continued)

| Protein ID | Gene name | Protein name | Peptide sequence |
|---|---|---|---|
| | | | NLFLNHSENATAK |
| | | | NLFLNHSENATAKDIAPTLTLYVGK |
| | | | NLFLNHSENATAKDIAPTLTLYVGKK |
| | | | QLVEIEK |
| | | | SCAVAEYGVYVK |
| | | | TEGDGVYTLNDK |
| | | | TEGDGVYTLNDKK |
| | | | VMPICLPSK |
| P00739 | HPR | Haptoglobin-related protein | FPKPPEIANGYVEHLFR |
| | | | MSDLGAVISLLLWGR |
| | | | QLFALYSGNDVTDISDDR |
| | | | QLFALYSGNDVTDISDDRFPKPPEIANGYVEHLFR |
| | | | SDLGAVISLLLWGR |
| | | | SPVGVQPILNEHTFCVGMSK |
| | | | VGYVSGWGQSDNFK |
| | | | VMPICLPSKNYAEVGR |
| | | | VTSIQHWVQK |
| | | | VVLHPNYHQVDIGLIK |
| | | | YQEDTCYGDAGSAFAVHDLEEDTWYAAGILSFDK |
| | | | YVMLPVADQYDCITHYEGSTCPK |
| Q9Y251 | HPSE | Heparanase | ADIFINGSQLGEDFIQLHK |
| | | | EDFLNPDVLDIFISSVQK |
| | | | EGDLTLYAINLHNVTK |
| | | | GYNISWELGNEPNSFLK |
| | | | KADIFINGSQLGEDFIQLHK |
| | | | TADLQWNSSNAQLLLDYCSSK |
| P04196 | HRG | Histidine-rich glycoprotein | ADLFYDVEALDLESPK |
| | | | ALDLINK |
| | | | DGYLFQLLR |
| | | | DHHHPHKPHEHGPPPPPDER |
| | | | DHSHGPPLPQGPPPLLPMSCSSCQHATFGTNGAQR |
| | | | DSPVLIDFFEDTER |
| | | | EENDDFASFR |
| | | | GEVLPLPEANFPSFPLPHHK |
| | | | GGEGTGYFVDFSVR |
| | | | HPLKPDNQPFPQSVSESCPGK |

(continued)

| Protein ID | Gene name | Protein name | Peptide sequence |
|---|---|---|---|
| | | | HPNVFGFCR |
| | | | HSHESQDLR |
| | | | HSHNNNSSDLHPHK |
| | | | IADAHLDRVENTTVYYLVLDVQESDCSVLSR |
| | | | IADAHLDRVENTTVYYLVLDVQESDCSVLSRK |
| | | | KGEVLPLPEANFPSFPLPHHK |
| | | | KYWNDCEPPDSR |
| | | | NLVINCEVFDPQEHENINGVPPHLGHPFHWGGHER |
| | | | PSEIVIGQCK |
| | | | Q IGSVYR |
| | | | RDGYLFQLLR |
| | | | RPSEIVIGQCK |
| | | | SGFPQVSMFFTHTFPK |
| | | | VENTTVYYLVLDVQESDCSVLSR |
| | | | VIDFNCTTSSVSSALANTK |
| | | | VIDFNCTTSSVSSALANTKDSPVLIDFFEDTER |
| | | | VIDFNCTTSSVSSALANTKDSPVLIDFFEDTERYR |
| | | | YKEENDDFASFR |
| | | | YWNDCEPPDSR |
| P14625 | HSP90B1 | Endoplasmin | EEASDYLELDTIK |
| | | | EEEAIQLDGLNASQIR |
| | | | FAFQAEVNR |
| | | | FQSSHHPTDITSLDQYVER |
| | | | GVVDSDDLPLNVSR |
| | | | GYEVIYLTEPVDEYCIQALPEFDGK |
| | | | HNNDTQHIWESDSNEFSVIADPR |
| | | | IKEDEDDKTVLDLAVVLFETATLR |
| | | | LIINSLYK |
| | | | LISLTDENALSGNEELTVK |
| | | | LTESPCALVASQYGWSGNMER |
| | | | NLLHVTDTGVGMTR |
| | | | S ILFVPTSAPR |
| | | | TDDEVVQREEEAIQLDGLNASQIR |
| | | | TETVEEPMEEEAAKEEKEESDDEAAVEEEEEK |

(continued)

| Protein ID | Gene name | Protein name | Peptide sequence |
|---|---|---|---|
| | | | VFITDDFHDMMPK |
| | | | YSQFINFPIYVWSSK |
| | | | ELISNASDALDK |
| P11021 | HSPA5 | 78 kDa glucose-regulated protein | DAGTIAGLNVMR |
| | | | DNHLLGTFDLTGIPPAPR |
| | | | ELEEIVQPIISK |
| | | | FEELNMDLFR |
| | | | GINPDEAVAYGAAVQAGVLSGDQDTG DLVLLDVCPLTLGI ETVGGVMTK |
| | | | IEIESFYEGEDFSETLTR |
| | | | IEWLESHQDADIEDFK |
| | | | IINEPTAAAIAYGLDKR |
| | | | ITPSYVAFTPEGER |
| | | | NELESYAYSLK |
| | | | NILVFDLGGGTFDVSLLTIDNGVFEVVA TNGDTHLGGEDFDQR |
| | | | NQLTSNPENTVFDAK |
| | | | SDIDEIVLVGGSTR |
| | | | SQIFSTASDNQPTVTIK |
| | | | TFAPEEISAMVLTK |
| | | | VLEDSDLKKSDIDEIVLVGGSTR |
| | | | IINEPTAAAIAYGLDK |
| Q9Y4L1 | HYOU1 | Hypoxia up-regulated protein 1 | AANSLEAFIFETQDK |
| | | | AEPPLNASASDQGEK |
| | | | DAVVYPILVEFTR |
| | | | DEPGEQVELK |
| | | | EEAEAPVEDGSQPPPPEPK |
| | | | ENGTDTVQEEEESPAEGSK |
| | | | EVQYLLNK |
| | | | FPEHELTFDPQR |
| | | | LGNTISSLFGGGTTPDAK |
| | | | LGNTISSLFGGGTTPDAKENGTDTVQE EEESPAEGSK |
| | | | LIPEMDQIFTEVEMTTLEK |
| | | | LQDLTLR |
| | | | LSAASTWLEDEGVGATTVMLK |
| | | | LSALDNLLNHSSMFLK |

(continued)

| Protein ID | Gene name | Protein name | Peptide sequence |
|---|---|---|---|
| | | | LYQPEYQEVSTEEQR |
| | | | LYQPEYQEVSTEEQREEISGK |
| | | | MVEEIGVELVVLDLPDLPEDK |
| | | | SLAEDFAEQPIK |
| | | | VAIVKPGVPMEIVLNK |
| | | | VEFEELCADLFER |
| | | | VFGSQNLTTVK |
| | | | VINETWAWK |
| | | | VINETWAWKNATLAEQAK |
| | | | VIPPAGQTEDAEPISEPEK |
| | | | VLQLINDNTATALSYGVFR |
| | | | VPGPVQQALQSAEMSLDEIEQVILVGGATR |
| P05362 | ICAM1 | Intercellular adhesion molecule 1 | ANLTVVLLR |
| | | | ASVSVTAEDEGTQR |
| | | | DCPGNWTWPENSQQTPMCQAWGNPLPELK |
| | | | DGTFPLPIGESVTVTR |
| | | | DHHGANFSCRTELDLR |
| | | | DLEGTYLCR |
| | | | EPAVGEPAEVTTTVLVR |
| | | | GGSVLVTCSTSCDQPK |
| | | | KVYELSNVQEDSQPMCYSNCPDGQSTAK |
| | | | LLGIETPLPK |
| | | | LNPTVTYGNDSFSAK |
| | | | REPAVGEPAEVTTTVLVR |
| | | | SFSCSATLEVAGQLIHK |
| | | | SFSCSATLEVAGQLIHKNQTR |
| | | | TELDLRPQGLELFENTSAPYQLQTFVLPATPPQLVSPR |
| | | | TFLTVYWTPER |
| | | | VELAPLPSWQPVGK |
| | | | VTLNGVPAQPLGPR |
| | | | VYELSNVQEDSQPMCYSNCPDGQSTAK |
| 075144 | ICOSLG | ICOS liqand | AMVGSDVELSCACPEGSR |
| | | | FDLNDVYVYWQTSESK |
| | | | GLYDVVSVLR |

(continued)

| Protein ID | Gene name | Protein name | Peptide sequence |
|---|---|---|---|
|  |  |  | LFNVTPQDEQK |
|  |  |  | PNVYWINK |
|  |  |  | TDNSLLDQALQNDTVFLNMR |
|  |  |  | TPSVNIGCCIENVLLQQNLTVGSQTGNDIGER |
|  |  |  | TVVTYHIPQNSSLENVDSR |
| P35858 | IGFALS | Insulin-like growth factorbinding protein complex acid labile subunit | AFWLDVSHNR |
|  |  |  | AGAFLGLTNVAVMNLSGNCLR |
|  |  |  | ALRDFALQNPSAVPR |
|  |  |  | ANVFVQLPR |
|  |  |  | DFALQNPSAVPR |
|  |  |  | DLHFLEELQLGHNR |
|  |  |  | DLSEAHFAPC |
|  |  |  | DNGLVGIEEQSLWGLAELLELDLTSNQLTHLPHR |
|  |  |  | ELVLAGNR |
|  |  |  | FVQAICEGDDCQPPAYTYNNITCASPPEVVGLDLR |
|  |  |  | IRPHTFTGLSGLR |
|  |  |  | LAELPADALGPLQR |
|  |  |  | LAYLQPALFSGLAELR |
|  |  |  | LEALPNSLLAPLGR |
|  |  |  | LEDGLFEGLGSLWDLNLGWNSLAVLPDAAFR |
|  |  |  | LEYLLLSR |
|  |  |  | LFQGLGK |
|  |  |  | LHSLHLEGSCLGR |
|  |  |  | LSHNAIASLR |
|  |  |  | LWLEGNPWDCGCPLK |
|  |  |  | NLIAAVAPGAFLGLK |
|  |  |  | NLPEQVFR |
|  |  |  | SFEGLGQLEVLTLDHNQLQEVK |
|  |  |  | SLALGTFAHTPALASLGLSNNR |
|  |  |  | TFTPQPPGLER |
|  |  |  | VAGLLEDTFPGLLGLR |
|  |  |  | WLDLSHNR |
| P18065 | IGFBP2 | Insulin-like growth factorbinding protein 2 | CYPHPGSELPLQALVMGEGTCEK |
|  |  |  | GPLEHLYSLHIPNCDK |

(continued)

| Protein ID | Gene name | Protein name | Peptide sequence |
|---|---|---|---|
| | | | LAACGPPPVAPPAAVAAVAGGAR |
| | | | LEGEACGVYTPR |
| | | | LIQGAPTIR |
| | | | TPCQQELDQVLER |
| P17936 | IGFBP3 | Insulin-like growth factorbinding protein 3 | ALAQCAPPPAVCAELVR |
| | | | AYLLPAPPAPGNASESEEDR |
| | | | CQPSPDEARPLQALLDGR |
| | | | EPGCGCCLTCALSEGQPCGIYTER |
| | | | FLNVLSPR |
| | | | GFCWCVDK |
| | | | GLCVNASAVSR |
| | | | PLQALLDGR |
| | | | VDYESQSTDTQNFSSESK |
| | | | YGQPLPGYTTK |
| | | | YKVDYESQSTDTQNFSSESK |
| Q16270 | IGFBP7 | Insulin-like growth factorbinding protein 7 | AGAAAGGPGVSGVCVCK |
| | | | EDAGEYECHASNSQGQASASAK |
| | | | GTCEQGPSIVTPPK |
| | | | HEVTGWVLVSPLSK |
| | | | ITWDALHEIPVK |
| | | | YPVCGSDGTTYPSGCQLR |
| P04438 | IGHV2-70 | Ig heavy chain V-II region | ALEWLAR |
| | | SESS | |
| | | | ATHTLTLTCTFSGLSVNTR |
| | | | ESGPALVK |
| | | | EVMITSNAFDIWGQGTWSPSLQ |
| | | | IDWDDDK |
| | | | QPPGKALEWLAR |
| | | | LLIYDASNLETGVPSR |
| Q14623 | IHH | Indian hedgehog protein | ELTPNYNPDIIFK |
| | | | LALTPAHLLFTADNHTEPAAR |
| | | | LLLEEGSFHPLGMSGAGS |
| | | | VLAMGEDGSPTFSDVLIFLDR |
| O95998 | IL18BP | Interleukin-18-binding protein | ALVLEQLTPALHSTNFSCVLVDPEQVV QR |
| | | | DPCPSQPPVFPAAK |
| | | | FPNFSILYWLGNGSFIEHLPGR |

(continued)

| Protein ID | Gene name | Protein name | Peptide sequence |
|---|---|---|---|
| | | | HVVLAQ LWAG LR |
| | | | QCPALEVTWPEVEVPLNGTLSLSCVAC SR |
| P27930 | IL1R2 | Interleukin-1 receptor type 2 | CVL TFAHEGQQYNITR |
| | | | EETIPVIISPLK |
| | | | GTTHLLVHDVALEDAGYYR |
| | | | LEGEPVALR |
| | | | MWAQDGALWLLPALQEDSGTYVCTTR |
| | | | QEYSENNENYIEVPLIFDPVTR |
| | | | VFLGTGTPLTTMLWWTANDTHIESAYP GGR |
| Q9NPH3 | IL1RAP | Interleukin-1 receptor accessory protein | CPLFEHFLK |
| | | | DLEEPINFR |
| | | | DVLWFR |
| | | | DVLWFRPTLLNDTGNYTCMLR |
| | | | EKDVLWFRPTLLNDTGNYTCMLR |
| | | | FNYSTAHSAGLTLIWYWTR |
| | | | IQNFNNVIPEGMNLSFLIALISNNGNYTC VVTYPENGR |
| | | | ITCPNVDGYFPSSVKPTITWYMGCYK |
| | | | KPDDITIDVTINESISHSR |
| | | | LYIEYGIQR |
| | | | NAVPPVIHSPNDHVVYEK |
| | | | NEVWWTIDGK |
| | | | PTLLNDTGNYTCMLR |
| | | | QDRDLEEPINFR |
| | | | QIQVFEDEPAR |
| | | | SSSDEQGLSYSSLK |
| | | | TQILSIK |
| | | | VAFPLEVVQK |
| Q01638 | IL1RL1 | Interleukin-1 receptor-like 1 | DEQGFSLFPVIGAPAQNEIK |
| | | | EEDLLLQYDCLALNLHGLR |
| | | | FIHNENGANYSVTATR |
| | | | FLPAAVADSGIYTCIVR |
| | | | GTQFLAAVLWQLNGTK |
| | | | IADVKEEDLLLQYDCLALNLHGLR |
| | | | IQQEEGQNQSFSNGLACLDMVLR |
| | | | IYCPTIDLYNWTAPLEWFK |

128

(continued)

| Protein ID | Gene name | Protein name | Peptide sequence |
|---|---|---|---|
| | | | NANLTCSACFGK |
| | | | PSYTVDWYYSQTNK |
| | | | QGKPSYTVDWYYSQTNK |
| | | | QSDCNVPDYLMYSTVSGSEK |
| | | | QSWGLENEALIVR |
| | | | TGYANVTIYK |
| | | | VFASGQLLK |
| P18510 | IL1RN | Interleukin-1 receptor antagonist protein | LQLEAVNITDLSENR |
| | | | LQLEAVNITDLSENRK |
| | | | NNQLVAGYLQGPNVNLEEK |
| | | | SDSGPTTSFESAACPGWFLCTAMEADQPVSLTNMPDEGVMVTK |
| P19827 | ITIH1 | Inter-alpha-trypsin inhibitor heavy chain H1 | AAISGENAGLVR |
| | | | ADVQAHGEGQEFSITCLVDEEEMK |
| | | | ADVQAHGEGQEFSITCLVDEEEMKK |
| | | | ANLSSQALQMSLDYGFVTPLTSMSIR |
| | | | DKICDLLVANNHFAHFFAPQNLTNMNK |
| | | | DPWHGAEVSCWFIHNNGAGLIDGAYTDYIVPDIF |
| | | | EDTLCFNINEEPGVILSLVQDPNTGFSVNGQLIGNK |
| | | | EERANLSSQALQMSLDYGFVTPLTSMSIR |
| | | | ELAAQTIK |
| | | | EQFTIHLTVNPQSK |
| | | | EVAFDLEIPK |
| | | | FAHYVVTSQVVNTANEAR |
| | | | FPLYNLGFGHNVDFNFLEVMSMENNGR |
| | | | GFSLDEATNLNGGLLR |
| | | | GHVLFRPTVSQQQSCPTCSTSLLNGHFK |
| | | | GIEILNQVQESLPELSNHASILIMLTDGDPTEGVTDR |
| | | | GMADQDGLKPTIDKPSEDSPPLEMLGPR |
| | | | GRFPLYNLGFGHNVDFNFLEVMSMENNGR |
| | | | GSLVQASEANLQAAQDFVR |

(continued)

| Protein ID | Gene name | Protein name | Peptide sequence |
|---|---|---|---|
| | | | GSSVHQDFLGFYVLDSHR |
| | | | ICDLLVANNHFAHFFAPQNLTNMNK |
| | | | ILGDMQPGDYFDLVLFGTR |
| | | | IYEDHDATQQLQGFYSQVAK |
| | | | IYEDHDATQQLQGFYSQVAKPLLVDVD LQYPQDAVLALTQNHHK |
| | | | KGHVLFRPTVSQQQSCPTCSTSLLNGH FK |
| | | | LDAQASFLPK |
| | | | LGIANPATDFQLEVTPQNITLNPGFGGP VFSWR |
| | | | LWAYLTIQELLAK |
| | | | LWAYLTIQELLAKR |
| | | | MDGAMGPRGLLLCMYLVSLLILQAMPA LGSATGR |
| | | | NHMQYEIVIK |
| | | | NVVFVIDISGSMR |
| | | | PLLVDVDLQYPQDAVLALTQNHHK |
| | | | PTVSQQQSCPTCSTSLLNGHFK |
| | | | QAVDTAVDGVFIR |
| | | | QLVHHFEIDVDIFEPQGISK |
| | | | QYYEGSEIVVAGR |
| | | | RNHMQYEIVIK |
| | | | RQAVDTAVDGVFIR |
| | | | TAFISDFAVTADGNAFIGDIK |
| | | | TFVLSALQPSPTHSSSNTQR |
| | | | THGLLGQFFHPIGFEVSDIHPGSDPTKP DATMVVR |
| | | | TMEQFTIHLTVNPQSK |
| | | | VTFQLTYEEVLK |
| | | | VTFQLTYEEVLKR |
| | | | VTGVDTDPHFIIHVPQK |
| | | | VTYDVSR |
| | | | VTYDVSRDKICDLLVANNHFAHFFAPQ NLTNMNK |
| P19823 | ITIH2 | Inter-a lpha-trypsin inhibitor heavy chain H2 | AEDHFSVIDFNQNIR |
| | | | AGELEVFNGYFVHFFAPDNLDPIPK |
| | | | AHVSFKPTVAQQR |

(continued)

| Protein ID | Gene name | Protein name | Peptide sequence |
|---|---|---|---|
| | | | AIFILNEANNLGLLDPNSVSLIILVSDGDP TVGELK |
| | | | ENIQDNISLFSLGMGFDVDYDFLK |
| | | | ENIQDNISLFSLGMGFDVDYDFLKR |
| | | | ETAVDGELVVLYDVK |
| | | | FDPAKLDQIESVITATSANTQLVLETLAQ MDDLQDFLSK |
| | | | FLHVPDTFEGHFDGVPVISK |
| | | | FYNQVSTPLLR |
| | | | GAFISNFSMTVDGK |
| | | | GAFISNFSMTVDGKTFR |
| | | | HADPDFTR |
| | | | HLEVDVWVIEPQGLR |
| | | | IQPSGGTNINEALLR |
| | | | IYGNQDTSSQLK |
| | | | IYLQPGR |
| | | | KFYNQVSTPLLR |
| | | | KLWAYLTINQLLAER |
| | | | LDQIESVITATSANTQLVLETLAQMDDL QDFLSK LDQIESVITATSANTQLVLETLAQMDDL QDFLSKDK |
| | | | |
| | | | LWAYLTINQLLAER |
| | | | MLADAPPQDPSCCSGALYYGSK |
| | | | NDLISATK |
| | | | NILFVIDVSGSMWGVK |
| | | | NVQFNYPHTSVTDVTQNNFHNYFGGS EIVVAGK |
| | | | SILQMSLDHHIVTPLTSLVIENEAGDER |
| | | | SILQMSLDHHIVTPLTSLVIENEAGDER MLADAPPQDPSC CSGALYYGSK |
| | | | SSALDMENFR |
| | | | SSALDMENFRTEVNVLPGAK |
| | | | TEVNVLPGAK |
| | | | TILDDLR |
| | | | TILDDLRAEDHFSVIDFNQNIR |
| | | | TWRNDLISATK |
| | | | VQFELHYQEVK |

(continued)

| Protein ID | Gene name | Protein name | Peptide sequence |
|---|---|---|---|
| | | | VVNNSPQPQNVVFDVQIPK |
| | | | YIEKIQPSGGTNINEALLR |
| Q06033 | ITIH3 | Inter-a lpha-trypsin inhibitor heavy chain H3 | ADTAKEVSFDVELPK |
| | | | ALDLSLK |
| | | | ALQERDYIFGNYIER |
| | | | AVNRADTAKEVSFDVELPK |
| | | | AVPSTFSWLDTVTVTQDGLSMMINR |
| | | | DDALCFNIDEAPGTVLR |
| | | | DFLGFYVVDSHR |
| | | | DYIFGNYIER |
| | | | EHLVQATPENLQEAR |
| | | | ESPGNVQIVNGYFVHFFAPQGLPVVPK |
| | | | EVSFDVELPK |
| | | | FPLYNLGFGNNLNYNFLENMALENHGFAR |
| | | | FTVSVNVAAGSK |
| | | | GHGATNDLTFTEEVDMK |
| | | | GHGATNDLTFTEEVDMKEMEK |
| | | | GHVSFKPSLDQQR |
| | | | GMTNINDGLLR |
| | | | HFEIEVDIFEPQGISMLDAEASFITNDLLGSALTK |
| | | | ILEDMQEEDYLNFILFSGDVSTWK |
| | | | IYEDSDADLQLQGFYEEVANPLLTGVEMEYPENAILDLTQ NTYQHFYDGSEIVVAGR |
| | | | LGIANAQMDFQVEVTTEK |
| | | | LIQDAVTGLTVNGQITGDK |
| | | | LIQDAVTGLTVNGQITGDKR |
| | | | LVDEDMNSFK |
| | | | LWAYLTIEQLLEK |
| | | | LWAYLTIEQLLEKR |
| | | | MSAQTHGLLGQFFQPFDFK |
| | | | NAHGEEKENLTAR |
| | | | NAIGGKFPLYNLGFGNNLNYNFLENMA LENHGFAR |
| | | | NMWSFGDGVTFWVLHQVWK |
| | | | NVAFVIDISGSMAGR |
| | | | PGSDPTKPDATLVVK |

(continued)

| Protein ID | Gene name | Protein name | Peptide sequence |
|---|---|---|---|
| | | | RSLPEGVANGIEVYSTK |
| | | | SCPTCTDSLLNGDFTITYDVNR |
| | | | SLPEGVANGIEVYSTK |
| | | | STSIVIMLTDGDANVGESR |
| | | | STSIVIMLTDGDANVGESRPEK |
| | | | TAFITNFTLTIDGVTYPGNVK |
| | | | VSDIRPGSDPTKPDATLVVK |
| | | | VTFELTYEELLK |
| | | | VTFELTYEELLKR |
| | | | VVCWFVHNNGEGLIDGVHTDYIVPNLF |
| | | | YHFVTPLTSMVVTKPEDNEDER |
| Q14624 | ITIH4 | Inter-a lpha-trypsin inhibitor heavy chain H4 | AEAQAQYSAAVAK |
| | | | AFITNFSMIIDGMTYPGIIK |
| | | | AFITNFSMIIDGMTYPGIIKEK |
| | | | AGFSWIEVTFK |
| | | | AGFSWIEVTFKNPLVWWHASPEHVVVTR |
| | | | AISGGSIQIENGYFVHYFAPEGLTTMPK |
| | | | ANTVQEATFQMELPK |
| | | | ANTVQEATFQMELPKK |
| | | | ANTVQEATFQMELPKKAFITNFSMIIDGMTYPGIIK |
| | | | APAVPAPIQAPSAILPLPGQSVER |
| | | | DQFNLIVFSTEATQWR |
| | | | DQFNLIVFSTEATQWRPSLVPASAENVNK |
| | | | DTDRFSSHVGGTLGQFYQEVLWGSPAASDDGRR |
| | | | EKAEAQAQYSAAVAK |
| | | | EKAGFSWIEVTFK |
| | | | ETLFSVMPGLK |
| | | | FKPTLSQQQK |
| | | | FSSHVGGTLGQFYQEVLWGSPAASDDGR |
| | | | FSSHVGGTLGQFYQEVLWGSPAASDDGRR |
| | | | GPDVLTATVSGK |
| | | | GPDVLTATVSGKLPTQNITFQTESSVAEQEAEFQSPK |

(continued)

| Protein ID | Gene name | Protein name | Peptide sequence |
|---|---|---|---|
| | | | HLQMDIHIFEPQGISFLETESTFMTNQL VDALTTWQNK |
| | | | HLQMDIHIFEPQGISFLETESTFMTNQL VDALTTWQNKTK |
| | | | HRQGPVNLLSDPEQGVEVTGQYER |
| | | | IHEDSDSALQLQDFYQEVANPLLTAVTF EYPSNAVEEVTQNNFR |
| | | | ILDDLSPR |
| | | | ILDDLSPRDQFNLIVFSTEATQWRPSLV PASAENVNK |
| | | | IPKPEASFSPR |
| | | | ITFELVYEELLK |
| | | | ITFELVYEELLKR |
| | | | KAFITNFSMIIDGMTYPGIIK |
| | | | KAFITNFSMIIDGMTYPGIIKEK |
| | | | LALDNGGLAR |
| | | | LCVDPR |
| | | | LDYQEGPPGVEISCWSVEL |
| | | | LGVYELLLK |
| | | | LLFKGSEMVVAGK |
| | | | LPEGSVSLIILL TDGDPTVGETNPR |
| | | | LPTQNITFQTESSVAEQEAEFQSPK |
| | | | LPTQNITFQTESSVAEQEAEFQSPKYIF HNFMER |
| | | | LQDRGPDVLTATVSGK |
| | | | LQDRGPDVLTATVSGKLPTQNITFQTES SVAEQEAEFQSPK |
| | | | LWAYLTIQQLLEQTVSASDADQQALR |
| | | | MNFRPGVLSSR |
| | | | NGIDIYSLTVDSR |
| | | | NMEQFQVSVSVAPNAK |
| | | | NPLVWVHASPEHVVVTR |
| | | | NQALNLSLAYSFVTPLTSMVVTK |
| | | | NQALNLSLAYSFVTPLTSMVVTKPDDQ EQSQVAEK |
| | | | NQALNLSLAYSFVTPLTSMVVTKPDDQ EQSQVAEKPMEGESR |
| | | | NVHSAGAAGSR |
| | | | NVHSGSTFFK |
| | | | NVVFVIDK |

(continued)

| Protein ID | Gene name | Protein name | Peptide sequence |
|---|---|---|---|
| | | | PSLVPASAENVNK |
| | | | QGPVNLLSDPEQGVEVTGQYER |
| | | | QGPVNLLSDPEQGVEVTGQYEREK |
| | | | QLGLPGPPDVPDHAAYHPFR |
| | | | QLGLPGPPDVPDHAAYHPFRR |
| | | | RIHEDSDSALQLQDFYQEVANPLLTAVT FEYPSNAVEEVTQNNFR |
| | | | RLDYQEGPPGVEISCWSVEL |
| | | | RLGVYELLLK |
| | | | SFAAGIQALGGTNINDAMLMAVQLLDS SNQEER |
| | | | SPEQQETVLDGNLIIR |
| | | | TGLLLLSDPDK |
| | | | TGLLLLSDPDKVTIGLLFWDGR |
| | | | TGLLLLSDPDKVTIGLLFWDGRGEGLR |
| | | | VTIGLLFWDGR |
| | | | VVNRANTVQEATFQMELPKK |
| | | | WKETLFSVMPGLK |
| | | | YIFHNFMER |
| | | | YSLFCLGFGFDVSYAFLEK |
| | | | YYLQGAK |
| Q8WWA0 | ITLN1 | Intelectin-1 | DECPSAFDGLYFLR |
| | | | DLGIWHVPNK |
| | | | EFTAGFVQFR |
| | | | E ITEAAVLLFYR |
| | | | TDTGFLQTLGHNLFGIYQK |
| Q9Y287 | ITM2B | Integral membrane protein 2B | AGTYLPQSYLIHEHMVITDR |
| | | | CYVIPLNTSIVMPPR |
| | | | IENIDHLGFFIYR |
| | | | IFEEEEVEFISVPVPEFADSDPANIVHDF NK |
| | | | LTAYLDLNLDK |
| | | | NLLELLINIK |
| P03952 | KLKB1 | Plasma kallikrein | CLLFSFLPASSINDMEK |
| | | | CQFFSYATQTFHK |
| | | | CQFFTYSLLPEDCK |
| | | | CQFFTYSLLPEDCKEEK |
| | | | DSVTGTLPK |
| | | | DTPFSQIK |

(continued)

| Protein ID | Gene name | Protein name | Peptide sequence |
|---|---|---|---|
| | | | DTPFSQIKEIIIHQNYK |
| | | | EIIIHQNYK |
| | | | EKGEIQNILQK |
| | | | FGCFLK |
| | | | FGCFLKDSVTGTLPK |
| | | | GDTSTIYTNCWVTGWGFSK |
| | | | GEIQNILQK |
| | | | GGDVASMYTPNAQYCQMR |
| | | | GVNFNVSK |
| | | | HLCGGSLIGHQWVLTAAHCFDGLPLQDVWR |
| | | | IAYGTQGSSGYSLR |
| | | | IVGGTNSSWGEWPWQVSLQVK |
| | | | IYPGVDFGGEELNVTFVK |
| | | | IYSGILNLSDITK |
| | | | IYSGILNLSDITKDTPFSQIK |
| | | | IYSGILNLSDITKDTPFSQIKEIIIHQNYK |
| | | | LCNTGDNSVCTTK |
| | | | LQAPLNYTEFQK |
| | | | LQAPLNYTEFQKPICLPSK |
| | | | LQAPLNYTEFQKPICLPSKGDTSTIYTNCWVTGWGFSK |
| | | | LSMDGSPTR |
| | | | LVGITSWGEGCAR |
| | | | TICTYHPNCLFFTFYTNVWK |
| | | | TSESGTPSSSTPQENTISGYSLLTCK |
| | | | TSESGTPSSSTPQENTISGYSLLTCKR |
| | | | VAEYMDWILEK |
| | | | VLSNVESGFSLKPCALSEIGCHMNIFQHLAFSDVDVAR |
| | | | VLTPDAFVCR |
| | | | VNIPLVTNEECQK |
| | | | VSEGNHDIALIK |
| | | | YSPGGTPTAIK |
| | | | GDSGGPLVCK |
| P01042 | KNG1 | Kininogen-1 | AATGECTATVGK |
| | | | DFVQPPTK |
| | | | DIPTNSPELEETLTHTITK |

(continued)

| Protein ID | Gene name | Protein name | Peptide sequence |
|---|---|---|---|
| | | | DIPTNSPELEETLTHTITKLNAENNATFYFK |
| | | | ENFLFLTPDCK |
| | | | ESNEELTESCETK |
| | | | ESYYFDLTDGLS |
| | | | FKLDDDLEHQGGHVLDHGHK |
| | | | FSVATQTCQITPAEGPVVTAQYDCLGCVHPISTQSPDLEPILR |
| | | | GHGLGHGHEQQHGLGHGHK |
| | | | HGIQYFNNNTQHSSLFMLNEVK |
| | | | HGIQYFNNNTQHSSLFMLNEVKR |
| | | | IASFSQNCDIYPGK |
| | | | IASFSQNCDIYPGKDFVQPPTK |
| | | | ICVGCPR |
| | | | IGEIKEETTSHLR |
| | | | ITYSIVQTNCSK |
| | | | ITYSIVQTNCSKENFLFLTPDCK |
| | | | IYPTVNCQPLGMISLMK |
| | | | IYPTVNCQPLGMISLMKRPPGFSPFR |
| | | | KIYPTVNCQPLGMISLMK |
| | | | KLGQSLDCNAEVYVVPWEK |
| | | | KLGQSLDCNAEVYVVPWEKK |
| | | | KYFIDFVAR |
| | | | KYNSQNQSNNQFVLYR |
| | | | LDDDLEHQGGHVLDHGHK |
| | | | LGQSLDCNAEVYVVPWEK |
| | | | LGQSLDCNAEVYVVPWEKK |
| | | | LNAENNATFYFK |
| | | | LNAENNATFYFKIDNVK |
| | | | LNAENNATFYFKIDNVKK |
| | | | QVVAGLNFR |
| | | | RPPGFSPFR |
| | | | SLWNGDTGECTDNAYIDIQLR |
| | | | SSTKFSVATQTCQITPAEGPVVTAQYDCLGCVHPISTQSPDLEPILR |
| | | | SVSE IN PTTQM K |
| | | | TVGSDTFYSFK |
| | | | TVGSDTFYSFKYEIK |
| | | | TWQDCEYK |

(continued)

| Protein ID | Gene name | Protein name | Peptide sequence |
|---|---|---|---|
| | | | TWQDCEYKDAAK |
| | | | YEIKEGDCPVQSGK |
| | | | YFIDFVAR |
| | | | YNSQNQSNNQFVLYR |
| P24043 | LAMA2 | Laminin subunit alpha-2 | AEQTILPLVDEALQHTTTK |
| | | | EDFLDILYDIHYILIK |
| | | | EDLHLEPFYWK |
| | | | GLFPAVLNLASNALITTNATCGEK |
| | | | GTSEDCQPCACPLNIPSNNFSPTCHLDR |
| | | | LADEINSIIDYVEDIQTK |
| | | | LEQMVMSINLTGPLPAPYK |
| | | | LIQLAEGNLNTLVTEMNELLTR |
| | | | MLYGLENMTQELK |
| | | | MDGMGIEMIDEK |
| | | | QANISIVDIDTNQEENIATSSSGNNFGLDLK |
| | | | QEGILYVDGASNR |
| | | | RVNGTIFGGICEPCQCFGHAESCDDVTGECLNCK |
| | | | SLGLICDGCPVGYTGPR |
| | | | TPYNILSSPDYVGVTK |
| | | | VSQAESHAAQLNDSSA VLDGILDEAK |
| | | | VSQAESHAAQLNDSSAVLDGILDEAKNISFNATAAFK |
| | | | WYPNISTVMFK |
| | | | YIGGGVCINCTQNTAGINCETCTDGFFRPK |
| | | | YMQNLTVEQPIEVK |
| P07942 | LAMB1 | Laminin subunit beta-1 | AMDLDQDVLSALAEVEQLSK |
| | | | CGGPGCGGLVTVAHNAWQK |
| | | | CLAGYYGDPIIGSGDHCRPCPCPDGPDSGR |
| | | | CVCNYLGTVQEHCNGSDCQCDK |
| | | | DILAQSPAAEPLK |
| | | | EGFYDLSSEDPFGCK |
| | | | ELAEQLEFIK |
| | | | GIETPQCDQSTGQCVCVEGVEGPR |
| | | | GLNCELCMDFYHDLPWRPAEGR |
| | | | IPSWTGAGFVR |

(continued)

| Protein ID | Gene name | Protein name | Peptide sequence |
|---|---|---|---|
| | | | KCGGPGCGGLVTVAHNAWQK |
| | | | MEMPSTPQQLQNLTEDIR |
| | | | NFLTQDSADLDSIEAVANEVLK |
| | | | QADEDIQGTQNLLTSIESETAASEETLFNASQR |
| | | | SCYQDPVTLQLACVCDPGYIGSR |
| | | | SLDIFTVGGSGDGWTNSAWETFQR |
| | | | VESLSQVEVILQHSAADIAR |
| | | | VNASTTEPNSTVEQSALMR |
| | | | YFAYDCEASFPGISTGPMK |
| | | | YSDIEPSTEGEVIFR |
| P11047 | LAMC1 | Laminin subunit gamma-1 | AFDITYVR |
| | | | A VEIY ASVAQLSPLDSETLENEANNIK |
| | | | CDQCEENYFYNR |
| | | | IPAINQTITEANEK |
| | | | KIPAINQTITEANEK |
| | | | KQDDADQDMMMAGMASQAAQEAEINAR |
| | | | LGNNEACSSCHCSPVGSLSTQCDSYGR |
| | | | LLNNLTSIK |
| | | | LNTFGDEVFNDPK |
| | | | LQELESLIANLGTGDEMVTDQAFEDR |
| | | | LSAEDLVLEGAGLR |
| | | | PSAYNFDNSPVLQEWVTA TDIR |
| | | | QDIAVISDSYFPR |
| | | | QVLSYGQNLSFSFR |
| | | | SYYYAISDFAVGGR |
| | | | TANDTSTEAYNLLLR |
| | | | TGGDEQQALCTDEFSDISPLTGGNVAFSTLEGR |
| | | | TLAGENQTAFEIEELNR |
| | | | VAAANVSVTQPESTGDPNNMTLLAEEAR |
| | | | VNNTLSSQISR |
| P18428 | LBP | Lipopolysaccharidebinding protein | ATAQMLEVMFK |
| | | | ATAQMLEVMFKGEIFHR |
| | | | DFLFLGANVQYMR |
| | | | FNDKLAEGFPLPLLK |

(continued)

| Protein ID | Gene name | Protein name | Peptide sequence |
|---|---|---|---|
| | | | GISISVNLLLGSESSGR |
| | | | GLQYAAQEGLLALQSELLR |
| | | | GRYEFHSLNIHSCELLHSALRPVPGQG LSLSISDSSIR |
| | | | ICEMIQK |
| | | | ITDKGLQYAAQEGLLALQSELLR |
| | | | ITLPDFTGDLR |
| | | | ITLPDFTGDLRIPHVGR |
| | | | LAEGFPLPLLK |
| | | | LAEGFPLPLLKR |
| | | | LQGSFDVSVK |
| | | | LSVATNVSATLTFNTSK |
| | | | LYPNMNLELQGSVPSAPLLNFSPGNLS VDPYMEIDAFVLLPSSSK |
| | | | LYPNMNLELQGSVPSAPLLNFSPGNLS VDPYMEIDAFVLLPSSSKEPVFR |
| | | | MVYFAISDYVFNTASLVYHEEGYLNFSI TDDMIPPDSNIR |
| | | | NHRSPVTLLAAVMSLPEEHNK |
| | | | PTVTASSCSSDIADVEVDMSGDLGWLL NLFHNQIESK |
| | | | PVPGQGLSLSISDSSIR |
| | | | RVQLYDLGLQIHK |
| | | | SPVTLLAAVMSLPEEHNK |
| | | | SVSSDLQPYLQTLPVTTEIDSFADIDYSL VEAPR |
| | | | VGLFNAELLEALLNYYILNTFYPK |
| | | | VQLYDLGLQIHK |
| | | | YEFHSLNIHSCELLHSALR |
| | | | YEFHSLNIHSCELLHSALRPVPGQGLSL SISDSSIR |
| P04180 | LCAT | Phosphatidylcholinesterol acyltransferase | AELSNHTRPVILVPGCLGNQLEAK |
| | | | DLLAGLPAPGVEVYCLYGVGLPTPR |
| | | | FFADLHFEEGWYMWLQSR |
| | | | FIDGFISLGAPWGGSIK |
| | | | FIDGFISLGAPWGGSIKPMLVLASGDNQ GIPIMSSIK |
| | | | ITTTSPWMFPSR |
| | | | KTEDFFTIWLDLNMFLPLGVDCWIDNTR |
| | | | LAGLVEEMHAAYGK |

(continued)

| Protein ID | Gene name | Protein name | Peptide sequence |
|---|---|---|---|
| | | | LAGYLHTLVQNLVNNGYVR |
| | | | LDKPDVVNWMCYR |
| | | | LEPGQQEEYYR |
| | | | MAWPEDHVFISTPSFNYTGR |
| | | | PVILVPGCLGNQLEAK |
| | | | SSGLVSNAPGVQIR |
| | | | STELCGLWQGR |
| | | | TEDFFTIWLDLNMFLPLGVDCWIDNTR |
| | | | TYIYDHGFPYTDPVGVLYEDGDDTVATR |
| | | | TYSVEYLDSSK |
| P80188 | LCN2 | Neutrophil gelatinaseassociated lipocalin | CDYWIR |
| | | | EDKDPQKMYATIYELK |
| | | | EDKSYNVTSVLFR |
| | | | ELTSELK |
| | | | ELTSELKENFIR |
| | | | MYATIYELK |
| | | | MYATIYELKEDK |
| | | | MYAT IYELKEDKSYNVTSVLFR |
| | | | SLGLPENHIVFPVPIDQCIDG |
| | | | SYNVTSVLFR |
| | | | SYPGLTSYLVR |
| | | | TFVPGCQPGEFTLGNIK |
| | | | TKELTSELKENFIR |
| | | | VPLQQNFQDNQFQGK |
| | | | VVSTNYNQHAMVFFK |
| | | | VVYVVGLAGNAILR |
| P13796 | LCP1 | Plastin-2 | AYYHLLEQVAPK |
| | | | EGICAIGGTSEQSSVGTQHSYSEEEK |
| | | | FSLVGIGGQDLNEGNR |
| | | | HVIPMNPNTNDLFNAVGDGIVLCK |
| | | | ISFDEFIK |
| | | | ISTSLPVLDLIDAIQPGSINYDLLK |
| | | | LNLAFIANLFNR |
| | | | LSPEELLLR |
| | | | MINLSVPDTIDER |
| | | | VNDDIIVNWVNETLR |

(continued)

| Protein ID | Gene name | Protein name | Peptide sequence |
|---|---|---|---|
| | | | VNHLYSDLSDALVIFQLYEK |
| | | | VYALPEDLVEVNPK |
| | | | YAFVNWINK |
| P48357 | LEPR | Leptin receptor | FNSSGTHFSNLSK |
| | | | GSFQMVHCNCSVHECCECLVPVPTAK |
| | | | ILTSVGSNVSFHCIYK |
| | | | INHSLGSLDSPPTCVLPDSVVKPLPPSSVK |
| | | | IPQSQYDVVSDHVSK |
| | | | ISWSSPPLVPFPLQYQVK |
| | | | IVSATSLLVDSILPGSSYEVQVR |
| | | | LDGLGYWSNWSNPAYTVVMDIK |
| | | | LNDTLLMCLK |
| | | | LSCMPPNSTYDYFLLPAGLSK |
| | | | SEQDRNCSLCADNIEGK |
| | | | SSLYCSDIPSIHPISEPK |
| | | | VHLLYVLPEVLEDSPLVPQK |
| | | | VTFFNLNETKPR |
| | | | YSENSTTVIR |
| | | | YVINHHTSCNGTWSEDVGNHTK |
| | | | YYIHDHFIPIEK |
| Q08380 | LGALS3BP | Galectin-3-binding protein | AAFGQGSGPIMLDEVQCTGTEASLADCK |
| | | | AAIPSALDTNSSK |
| | | | ALGFENATQALGR |
| | | | ALMLCEGLFVADVTDFEGWK |
| | | | ASHEEVEGLVEK |
| | | | A VDTWSWGER |
| | | | DAGVVCTNETR |
| | | | DAGVVCTNETRSTHTLDLSR |
| | | | ELSEALGQIFDSQR |
| | | | EPGSNVTMSVDAECVPMVR |
| | | | FPMMLPEELFELQFNLSLYWSHEALFQK |
| | | | GCDLSISVNVQGEDALGFCGHTVILTANLEAQALWK |
| | | | GLNLTEDTYK |
| | | | GLNLTEDTYKPR |
| | | | GQWGTVCDNLWDLTDASVVCR |

(continued)

| Protein ID | Gene name | Protein name | Peptide sequence |
|---|---|---|---|
| | | | IDITLSSVK |
| | | | IYTSPTWSAFVTDSSWSAR |
| | | | KTLQALEFHTVPFQLLAR |
| | | | LCLQFLAWNFEALTQAEAWPSVPTDLL QLLLPR |
| | | | PFYLTNSSGVD |
| | | | QLQGYCASLFAILLPQDPSFQMPLDLY AYAVATGDALLEK |
| | | | RIDITLSSVK |
| | | | SDLA VPSELALLK |
| | | | SQLVYQSR |
| | | | STSSFPCPAGHFNGFR |
| | | | TLQALEFHTVPFQLLAR |
| | | | TVIRPFYLTNSSGVD |
| | | | VEIFYR |
| | | | VSWSLVYLPTIQSCWNYGFSCSSDELP VLGLTK |
| | | | YKGLNLTEDTYKPR |
| | | | YSSDYFQAPSDYR |
| | | | YYPYQSFQTPQHPSFLFQDK |
| | | | YYPYQSFQTPQHPSFLFQDKR |
| Q8N6C8 | LILRA3 | Leukocyte immunoglobulin-like receptor subfamily A member 3 | APYVWSLPSDLLGLLVPGVSK |
| | | | ARPFLSVRPGPTVASGENVTLLCQSQG GMHTFLLTK |
| | | | CQGSLETQEYHLYR |
| | | | CYGSLSSNPYLLTHPSDPLELVVSGAA ETLSPPQNK |
| | | | EGAADSPLR |
| | | | GQFPILSITWEHAGR |
| | | | KPSLSVQPGPVVAPGEK |
| | | | LTFQCGSDAGYDR |
| | | | PFLSVRPGPTVASGENVTLLCQSQGG MHTFLLTK |
| | | | PGPTVASGENVTLLCQSQGGMHTFLLT K |
| | | | PTLSALPSPVVTSGGNVTIQCDSQVAF DGFILCK |
| | | | PTLWAEPGSVITQGSPVTLR |
| | | | SYGGQYTCSGAYNLSSEWSAPSDPLDI LITGQIR |

143

(continued)

| Protein ID | Gene name | Protein name | Peptide sequence |
|---|---|---|---|
| | | | AIFSVGPVSPSR |
| | | | YQAEFPMSPVTSAHAGTYR |
| | | | PTLWAEPGSVITQGSPVTLR |
| | | | QPQAGLSQANFTLGPVSR |
| P08519 | LPA | Apolipoprotein(a) | ATTVTGTPCQEWAAQEPHR |
| | | | CPGSIVGGCVAHPHSWPWQVSLR |
| | | | EAQLLVIENEVCNHYK |
| | | | FVTWIEGMMR |
| | | | GISSTTVTGRTCQSWSSMIPHWHQR |
| | | | GSFSTTVTGR |
| | | | GTDSCQGDSGGPLVCFEK |
| | | | HFCGGTLISPEWVLTAAHCLK |
| | | | HSTFIPGTNKWAGLEK |
| | | | IPLYYPNAGLTR |
| | | | KLFDYCDIPLCASSSFDCGKPQVEPK |
| | | | LFDYCDIPLCASSSFDCGK |
| | | | LFDYCDIPLCASSSFDCGKPQVEPK |
| | | | LFLEPTQADIALLK |
| | | | NPDADTGPWCFTMDPSIR |
| | | | NPDAEIRPWCYTMDPSVR |
| | | | NPDAEISPWCYTMDPNVR |
| | | | NPDAVAAPYCYTR |
| | | | NPDGDINGPWCYTMNPR |
| | | | NPDPVAAPWCYTTDPSVR |
| | | | NPDPVAAPYCYTR |
| | | | NPDSGKQPWCYTTDPCVR |
| | | | PWCYTMDPSVR |
| | | | QPWCYTTDPCVR |
| | | | RIPLYYPNAGLTR |
| | | | TCQAWSSMTPHQHNR |
| | | | TCQAWSSMTPHSHS |
| | | | TECYITGWGETQGTFGTGLLK |
| | | | TPAYYPNAGLIK |
| | | | TPENYPNAGLTENYCR |
| | | | TPENYPNAGLTR |
| | | | TPENYPNDGLTMNYCR |
| | | | TPEYYPNAGLIMNYCR |
| | | | TTENYPNAGLIMNYCR |

(continued)

| Protein ID | Gene name | Protein name | Peptide sequence |
|---|---|---|---|
| | | | TTEYYPNGGLTR |
| | | | VILGAHQEVNLESHVQEIEVSR |
| | | | VMPACLPSPDYMVTAR |
| | | | YICAEHLAR |
| | | | WEYCNLTR |
| P02750 | LRG1 | Leucine-rich alpha-2-glycoprotein | ALGHLDLSGNR |
| | | | CAGPEAVK |
| | | | CAGPEAVKGQTLLAVAK |
| | | | DCQVFR |
| | | | DGFDISGNPWICDQNLSDLYR |
| | | | DKMFSQNDTR |
| | | | DLLLPQPDLR |
| | | | ENQLEVLEVSWLHGLK |
| | | | GPLQLER |
| | | | GQTLLA VAK |
| | | | KLPPGLLANFTLLR |
| | | | LHLEGNKLQVLGK |
| | | | LPPGLLANFTLLR |
| | | | LQELHLSSNGLESLSPEFLR |
| | | | LQELHLSSNGLESLSPEFLRPVPQLR |
| | | | LQVLGKDLLLPQPDLR |
| | | | MFSQNDTR |
| | | | NALTGLPPGLFQASATLDTLVLK |
| | | | QLDMLDLSNNSLASVPEGLWASLGQP |
| | | | NWDMR |
| | | | QLDMLDLSNNSLASVPEGLWASLGQP NWDMRDGFDISGNP WICDQNLSDLYR |
| | | | SDHGSSISCQPPAEIPGYLPADTVHLAV EFFNLTHLPANLLQGASK |
| | | | TLDLGENQLETLPPDLLR |
| | | | VAAGAFQGLR |
| | | | WLQAQK |
| | | | YLFLNGNK |
| | | | YLFLNGNKLAR |
| Q14767 | LTBP2 | Latent-transforming growth factor betabinding protein 2 | CEEVIPDEEFDPQNSR |
| | | | DGTQQAVPLEHPSSPWGLNLTEK |
| | | | LNLTHCQDINECLTLGLCK |

(continued)

| Protein ID | Gene name | Protein name | Peptide sequence |
|---|---|---|---|
| | | | NVCGGQCCPGWTTANSTNHCIKPVCEPPCQNR |
| | | | VHIHHPPEASVQIHQVAQVR |
| P02788 | LTF | Lactotransferrin | ADAVTLDGGFIYEAGLAPYK |
| | | | CAFSSQEPYFSYSGAFK |
| | | | CGLVPVLAENYK |
| | | | CLAENAGDVAFVK |
| | | | CNQWSGLSEGSVTCSSASTTEDCIALVLK |
| | | | CSTSPLLEACEFLR |
| | | | DSPIQCIQAIAENR |
| | | | DVTVLQNTDGNNNEAWAK |
| | | | EDAIWNLLR |
| | | | ESTVFEDLSDEAER |
| | | | ESTVFEDLSDEAERDEYELLCPDNTR |
| | | | FDEYFSQSCAPGSDPR |
| | | | FQLFGSPSGQK |
| | | | FQLFGSPSGQKDLLFK |
| | | | FQLFGSPSGQKDLLFKDSAIGFSR |
| | | | GEADAMSLDGGYVYTAGK |
| | | | GGSFQLNELQGLK |
| | | | IDSGLYLGSGYFTAIQNLR |
| | | | KGGSFQLNELQGLK |
| | | | LADFALLCLDGK |
| | | | LRPVAAEVYGTER |
| | | | NLLFNDNTECLAR |
| | | | PFLNWTGPPEPIEAAVAR |
| | | | SNLCALCIGDEQGENK |
| | | | SQQSSDPDPNCVDRPVEGYLAVAVVR |
| | | | SVNGKEDAIWNLLR |
| | | | SVQWCAVSQPEATK |
| | | | TAGWNIPMGLLFNQTGSCK |
| | | | TAGWNVPIGTLR |
| | | | TAGWNVPIGTLRPFLNWTGPPEPIEAAVAR |
| | | | VVWCAVGEQELR |
| | | | YLGPQYVAGITNLK |
| | | | YLGPQYVAGITNLKK |
| | | | YYGYTGAFR |

(continued)

| Protein ID | Gene name | Protein name | Peptide sequence |
|---|---|---|---|
| Q9Y5Y7 | LYVE1 | Lymphatic vessel endothelial hyaluronic acid receptor 1 | AEELSIQVSCR |
| | | | ANQQLNFTEAK |
| | | | ASFETCSYGWVGDGFVVISR |
| | | | DQVETALK |
| | | | IMGITLVSK |
| | | | KANQQLNFTEAK |
| | | | LLGLSLAGK |
| | | | LLGLSLAGKDQVETALK |
| | | | NGVGVLIWK |
| Q7Z7M0 | MEGF8 | Multiple epidermal growth factor-like domains protein 8 | ACDLHLWENQGAGWWHNVSAR |
| | | | ALL TNVSSVALGSR |
| | | | CLQGDFSGPLGGGNCSLWVGEGLGLP VALPAR |
| | | | DFWVLNLTTLQWR |
| | | | EAPGFVTDGAGNYSVNGNCEWLIEAPS PQHR |
| | | | EVFWAGNCSEAACGAADCEQCTR |
| | | | GFIYPMLPGGPGGPGAEDVAVWTR |
| | | | GPGTLGWCVHNESCLPR |
| | | | GPGTLGWCVHNESCLPRPEQAR |
| | | | ILLDFLFLDTECTYDYLFVYDGDSPR |
| | | | LLSSPEACNQSGACTWCHGACLSGDQ AHR |
| | | | MLLHLFSDANYNLLGFNASFR |
| | | | TGYTMDNMTGLCR |
| | | | TPHDLFSSGLFR |
| | | | VNSTELFHVDR |
| P22894 | MMP8 | Matrix metalloproteinase 8 | DAFELWSVASPLIFTR |
| | | | DISNYGFPSSVQAIDAAVFYR |
| | | | FFGLNVTGK |
| | | | FFGLNVTGKPNEETLDMMK |
| | | | FYQLPSNQYQSTR |
| | | | GNQYWALSGYDILQGYPK |
| | | | NYTPQLSEAEVER |
| | | | SISGAFPGIESK |

(continued)

| Protein ID | Gene name | Protein name | Peptide sequence |
|---|---|---|---|
| | | | YYAFDLIAQR |
| | | Matrix metalloproteinase-9 | |
| P14780 | MMP9 | | AFALWSAVTPLTFTR |
| | | | DGLLAHAFPPGPGIQGDAHFDDDELWSLGK |
| | | | FGNADGAACHFPFIFEGR |
| | | | FQTFEGDLK |
| | | | LFFFSGR |
| | | | LFGFCPTR |
| | | | LGLGADVAQVTGALR |
| | | | LWCATTSNFDSDK |
| | | | LYTQDGNADGK |
| | | | MFPGVPLDTHDVFQYR |
| | | | MLLFSGR |
| | | | QLAEEYLYR |
| | | | QLSLPETGELDSATLK |
| | | | QSTLVLFPGDLR |
| | | | QVWVYTGASVLGPR |
| | | | SDGLPWCSTTANYDTDDR |
| | | | SLGPALLLLQK |
| | | | WCATTANYDR |
| | | | WHHHNITYWIQNYSEDLPR |
| Q13201 | MMRN1 | Multimerin-1 | AQEQQSLIHTNQAESHTAVGR |
| | | | DTEENLHVLNQTLAEVLFPMDNK |
| | | | EVHEQLLSTEQVSDQK |
| | | | FNPGAESVVLSNSTLK |
| | | | FVLVQENRPTLTDIVELR |
| | | | GLTEFVEPIIQIK |
| | | | HPFTGDNCTIK |
| | | | HSWTIPEDGNSQK |
| | | | IDNISLTVNDVR |
| | | | IENLTSAVNSLNFIIK |
| | | | IFQNDMQETVAQLFK |
| | | | INEYALEMEDGLNK |
| | | | INNLTVSLEMEK |
| | | | KIDNISLTVNDVR |
| | | | KPTVNLTTVLIGR |

(continued)

| Protein ID | Gene name | Protein name | Peptide sequence |
|---|---|---|---|
| | | | KSNEQATSLNTVGGTGGIGGVGGTGGVGNR |
| | | | LNDSIQTLVNDNQR |
| | | | LQNLTLPTNASIK |
| | | | LSPTVILDNQVTYVPGGK |
| | | | LVEENALAPDFSK |
| | | | MSEQLNDLTYDMEILQPLLEQGASLR |
| | | | MYQMFNETTSQVR |
| | | | NAPAAESVSNNVTEYMSTLHENIK |
| | | | NTDNIIYPEEYSSCSR |
| | | | SILYYESLNK |
| | | | SNEQATSLNTVGGTGGIGGVGGTGGVGNR |
| | | | TLHEVLTMCHNASTSVSELNATIPK |
| | | | TMTIINNAIDFIQDNYALK |
| | | | TQAALSNLTCCIDR |
| | | | TVSSLSEDLESTR |
| | | | VLTGDALLELNYGQEVWLR |
| | | | VMSAEIATTPEK |
| | | | VNESVVSIAAQQK |
| P05164 | MPO | Myeloperoxidase | AADYLHVALDLLER |
| | | | DFVNCSTLPALNLASWR |
| | | | DYLPLVLGPTAMR |
| | | | FCGLPQPETVGQLGTVLR |
| | | | FPTDQLTPDQER |
| | | | IANVFTNAFR |
| | | | FWWENEGVFSMQQR |
| | | | IGLDLPALNMQR |
| | | | IICDNTGITTVSK |
| | | | NLRNMSNQLGLLAVNQR |
| | | | NMSNQLGLLA VNQR |
| | | | NQADCIPFFR |
| | | | NQINALTSFVDASMVYGSEEPLAR |
| | | | QNQIAVDEIR |
| | | | QALAQISLPR |
| | | | RPFNVTDVLTPAQLNVLSK |
| | | | SCPACPGSNITIR |
| | | | SLMFMQWGQLLDHDLDFTPEPAAR |

149

(continued)

| Protein ID | Gene name | Protein name | Peptide sequence |
|---|---|---|---|
| | | | SSEMPELTSMHTLLLR |
| | | | VFFASWR |
| | | | VGPLLACIIGTQFR |
| | | | VVLEGGIDPILR |
| | | | WLPAEYEDGFSLPYGWTPGVK |
| | | | ALLPFDNLHDDPCLLTNR |
| P22897 | MRC1 | Macrophage mannose receptor 1 | ACIGFGGNLVSIQNEK |
| | | | ALGGDLASINNK |
| | | | CVDAVSPSAVQTAACNQDAESQK |
| | | | DSTFSAWTGLNDVNSEHTFLWTDGR |
| | | | EGGDLTSIHTIEELDFIISQLGYEPNDEL WIGLNDIK |
| | | | EGWNFYSNK |
| | | | EQAFLTYHMKDSTFSAWTGLNDVNSE HTFLWTDGRGVHYTNWGK |
| | | | FEGSESLWNK |
| | | | FEGSESLWNKDPLTSVSYQINSK |
| | | | FQWHEAETYCK |
| | | | FTHWNSDMPGR |
| | | | GDPTMSWNDINCEHLNNWICQIQK |
| | | | GEDLFFNYGNR |
| | | | GNTTLNSFVIPSESDVPTHCPSQWWPY AGHCYK |
| | | | GTFQWTIEEEVR |
| | | | GYEAMYTLLGNANGATCAFPFK |
| | | | HHFYCYMIGHTLSTFAEANQTCNNENA YLTTIEDR |
| | | | IFGFMEEER |
| | | | IQMYFEWSDGTPVTFTK |
| | | | IYGTTDNLCSR |
| | | | KGNTTLNSFVIPSESDVPTHCPSQWWP YAGHCYK |
| | | | LCLGVPSK |
| | | | LFGYCPLK |
| | | | LFWLGLTYGSPSEGFTWSDGSPVSYE NWAYGEPNNYQNVEYCGELK |
| | | | LHNSLIASILDPYSNAFAWLQMETSNER |
| | | | MGSSLVSIESAAESSFLSYR |
| | | | NDAQSAYFIGLLISLDK |

(continued)

| Protein ID | Gene name | Protein name | Peptide sequence |
|---|---|---|---|
| | | | NDAQSAYFIGLLISLDKK |
| | | | NDCVALHASSGFWSNIHCSSYK |
| | | | NDTLLGIK |
| | | | NFGDLVSIQSESEK |
| | | | NVEGIWLWINNSPVSFVNWNTGDPSGER |
| | | | NWGQASLECLR |
| | | | PEPTPAPQDNPPVTEDGWVIYK |
| | | | SCVSLNPGK |
| | | | SDGWLWCGTTTDYDTDK |
| | | | SDGWLWCGTTTDYDTDKLFGYCPLK |
| | | | TAHCNESFYFLCK |
| | | | TGIAGGLWDVLK |
| | | | TNFWIGLFR |
| | | | VWIALNSNLTDNQYTWTDK |
| | | | WECKNDTLLGIK |
| | | | WENLECVQK |
| | | | WVSESQIMSVAFK |
| | | | YFWTGLSDIQTK |
| | | | YLNWLPGSPSAEPGK |
| | | | YTNWAADEPK |
| P13591 | NCAM1 | Neural cell adhesion molecule 1 | AVGEEVWHSK |
| | | | CVVTGEDGSESEATVNVK |
| | | | DGEQIEQEEDDEKYIFSDDSSQLTIK |
| | | | DGQLLPSSNYSNIK |
| | | | EASMEGIVTIVGLKPETTYAVR |
| | | | EGEDAVIVCDWSSLPPTIIWK |
| | | | FFLCQVAGDAK |
| | | | FIVLSNNYLQIR |
| | | | GLGEISAASEFK |
| | | | IGQESLEFILVQADTPSSPSIDQVEPYSSTAQVQFDEPEATGGVPILK |
| | | | ISVVWNDDSSSTLTIYNANIDDAGIYK |
| | | | ITYVENQTAMELEEQVTLTCEASGDPIPSITWR |
| | | | IYNTPSASYLEVTPDSENDFGNYNCTAVNR |
| | | | LQGPVAVYTWEGNQVNITCEVFAYPSATISWFR |

(continued)

| Protein ID | Gene name | Protein name | Peptide sequence |
|---|---|---|---|
| | | | NDEAEYICIAENK |
| | | | SIQYTDAGEYICTASNTIGQDSQSMYLEVQYAPK |
| Q7Z3B1 | NEGR1 | Neuronal growth regulator 1 | IYDISNDMTVNEGTNVTLTCLATGKPEPSISWR |
| | | | LFNGQQGIIIQNFSTR |
| | | | SILTVTNVTQEHFGNYTCVAANK |
| P61916 | NPC2 | Epididymal secretory protein E1 | A VVHGILMGVPVPFPIPEPDGCK |
| | | | EVNVSPCPTQPCQLSK |
| | | | GQSYSVNVTFTSNIQSK |
| | | | LVVEWQLQDDKNQSLFCWEIPVQIVSHL |
| 014786 | NRP1 | Neuropilin-1 | CEWLIQAPDPYQR |
| | | | EGFSANYSVLQSSVSEDFK |
| | | | EGNKPVLFQGNTNPTDVVVAVFPK |
| | | | EWIQVDLGLLR |
| | | | FEVYGCK |
| | | | FVSDYETHGAGFSIR |
| | | | FVTAVGTQGAISK |
| | | | GPECSQNYTTPSGVIK |
| | | | IAPPPVVSSGPFLFIK |
| | | | IESPGYLTSPGYPHSYHPSEK |
| | | | IGYSNNGSDWK |
| | | | IKPATWETGISMR |
| | | | IMINFNPHFDLEDR |
| | | | LEIWDGFPDVGPHIGR |
| | | | LNYPENGWTPGEDSYR |
| | | | MSEIILEFESFDLEPDSNPPGGMFCR |
| | | | NLSALENYNFELVDGVK |
| | | | RGPECSQNYTTPSGVIK |
| | | | SFEGNNNYDTPELR |
| | | | SSSGILSMVFYTDSAIAK |
| | | | TGPIQDHTGDGNFIYSQADENQK |
| | | | YDYVEVFDGENENGHFR |
| Q02818 | NUCB1 | Nucleobindin-1 | DLELLIQTATR |
| | | | EFGDTGEGWETVEMHPAYTEEELR |
| | | | EVWEELDGLDPNR |
| | | | FHPDTDDVPVPAPAGDQK |
| | | | LPEVEVPQHL |

(continued)

| Protein ID | Gene name | Protein name | Peptide sequence |
|---|---|---|---|
| | | | LSQETEALGR |
| | | | LVTLEEFLASTQR |
| | | | MDAEQDPNVQVDHLNLLK |
| | | | QFEHLDPQNQHTFEAR |
| | | | TFFILHDINSDGVLDEQELEALFTK |
| | | | VNVPGSQAQLK |
| | | | YLQEVIDVLETDGHFR |
| Q86UD1 | OAF | Out at first protein homolog | ALILGELEK |
| | | | FWLEQGVDSSVFEALPK |
| | | | GLEHLHMDVAVNFSQGALLSPHLHNVCAEAVDAIYTR |
| | | | GQSQFQALCFVTQLQHNEIIPSEAMAK |
| | | | LPDGQVTEESLQADSDADSISLELR |
| | | | QLCLWDEDPYPG |
| | | | SYSFDFYVPQR |
| | | | YGLSLAWYPCMLK |
| Q8WWZ8 | OIT3 | Oncoprotein-induced transcript 3 protein | DSLYFGIEPVVHVSGLESLVESCFATPTSK |
| | | | ELVGGLELFLTNTSCR |
| | | | GAGGEDSAGLQGQTLTGGPIR |
| | | | GLVLSEDNHTCQVPVLCK |
| | | | GVSNGTHVNILFSLK |
| | | | HFQVPVFK |
| | | | IVASNLVTGLPK |
| | | | LLIPVTCEFPR |
| | | | LYTISEGYVPNLR |
| | | | QACASFNGNCCLWNTTVEVK |
| | | | QTPGSSGDFIIR |
| | | | TCGTVVDWNDK |
| | | | VLVCGVLDER |
| Q6UX06 | OLFM4 | Olfactomedin-4 | DQNTPVVPPPTPGSCGHGGWNISK |
| | | | DQNTPVVHPPPTPGSCGHGGVVNISKPSVVQLNWR |
| | | | DTISYTELDFELIK |
| | | | GFSYLYGAWGR |
| | | | GLYWVAPLNTDGR |
| | | | LLNLTVR |
| | | | LNDTTLQVLNTWYTK |
| | | | LYNTLDDLLLYINAR |

(continued)

| Protein ID | Gene name | Protein name | Peptide sequence |
|---|---|---|---|
| | | | LYVYNDGYLLNYDLSVLQK |
| | | | PSWQLNWR |
| | | | SLGSGGSVSQLFSNFTGSVDDR |
| | | | TEEIFYYYDTNTGK |
| | | | VNLTTNTIAVTQTLPNAAYNNR |
| P02763 | ORM1 | Alpha-1-acid glycoprotein 1 | DTKTYMLAFDVNDEK |
| | | | ENGTISRYVGGQEHFAHLLILR |
| | | | EQLGEFYEALDCLR |
| | | | EQLGEFYEALDCLRIPK |
| | | | EYQTRQDQCIYNTTYLNVQR |
| | | | EYQTRQDQCIYNTTYLNVQRENGTISR |
| | | | IPKSDWYTDWK |
| | | | NWGLSVYADK |
| | | | NWGLSVYADKPETTK |
| | | | NWGLSVYADKPETTKEQLGEFYEALDCLR |
| | | | QDQCIYNTTYLNVQR |
| | | | QDQCIYNTTYLNVQRENGTISR |
| | | | SDWYTDWK |
| | | | SDWYTDWKK |
| | | | TYMLAFDVNDEK |
| | | | TYMLAFDVNDEKNWGLSVYADKPETTK |
| | | | YVGGQEHFAHLLILR |
| | | | YVGGQEHFAHLLILRDTK |
| | | | *CEPLEK* |
| | | | *DKCEPLEK* |
| | | | *KQEEGES* |
| | | | *NEEYNKSVQEIQATFFYFTPNK* |
| | | | *NEEYNKSVQEIQATFFYFTPNKTEDTIFLR* |
| | | | *SVQEIQATFFYFTPNK* |
| | | | *SVQEIQATFFYFTPNKTEDTIFLR* |
| | | | *TEDTIFLR* |
| | | | *TEDTIFLREYQTR* |
| | | | *WFYIASAFR* |
| | | | *WFYIASAFRNEEYNK* |
| P19652 | ORM2 | Alpha-1-acid glycoprotein 2 | EHVAHLLFLR |
| | | | EHVAHLLFLRDTK |

(continued)

| Protein ID | Gene name | Protein name | Peptide sequence |
|---|---|---|---|
| | | | EQLGEFYEALDCLCIPR |
| | | | EYQTRQNQCFYNSSYLNVQR |
| | | | ITGKWFYIASAFR |
| | | | NWGLSFYADK |
| | | | NWGLSFYADKPETTK |
| | | | NWGLSFYADKPETTKEQLGEFYEALDC LCIPR |
| | | | QNQCFYNSSYLNVQR |
| | | | QNQCFYNSSYLNVQRENGTVSR |
| | | | SDVMYTDWK |
| | | | SDVMYTDWKK |
| | | | TLMFGSYLDDEK |
| | | | TLMFGSYLDDEKNWGLSFYADKPETTK |
| | | | YEGGREHVAHLLFLR |
| | | | YEGGREHVAHLLFLRDTK |
| Q99650 | OSMR | Oncostatin-M-specific receptor subunit beta | DKLVEEGTNVTICYVSR |
| | | | GTNIYCEASQGNVSEGMK |
| | | | IETSNVIWVGNYSTTVK |
| | | | ILFYNVVVENLDKPSSSELHSIPAPANS TK |
| | | | LPLTPVSLK |
| | | | LVEEGTNVTICYVSR |
| | | | MMQYNVSIK |
| | | | NIQNNVSCYLEGK |
| | | | NNFTYLCQIELHGEGK |
| | | | NVGPNTTSTVISTDAFR |
| | | | NVGPNTTSTVISTDAFRPGVR |
| | | | NWCNWQITQDSQETYNFTLIAENYLR |
| | | | SVNILFNLTHR |
| | | | VTTPDEHSSMLIHILLPMVFCVLLIMVMC YLK |
| | | | VYLMNPFSVNFENVNATNAIMTWKVHS IR |
| | | | WNQVLHWSWESELPLECATHFVR |
| | | | WSEWSGQNFTTLEAAPSEAPDVWR |
| Q15113 | PCOLCE | Procollagen C-endopeptidase enhancer 1 | ECIWTITVPEGQTVSLSFR |
| | | | FCGDAVPGSISSEGNELLVQFVSDLSV TADGFSASYK |

(continued)

| Protein ID | Gene name | Protein name | Peptide sequence |
|---|---|---|---|
| | | | FDLEPDTYCR |
| | | | GESGYVASEGFPNLYPPNK |
| | | | GFLLWYSGR |
| | | | GPVLPPESFVVLHRPNQDQILTNLSK |
| | | | GVSYLLMGQVEENR |
| | | | PAPLVAPGNQVTLR |
| | | | TEESPSAPDAPTCPK |
| | | | TGGLDLPSPPTGASLK |
| | | | TGTLQSNFCASSLWTATVK |
| | | | VFDLELHPACR |
| | | | YDALEVFAGSGTSGQR |
| | | | YDSVSVFNGAVSDDSR |
| Q8NBP7 | PCSK9 | Proprotein convertase subtilisin/kexin type 9 | ADEYQPPDGGSLVEVYLLDTSIQSDHR |
| | | | AGVVLTAAGNFR |
| | | | AHNAFGGEGVYAIAR |
| | | | CCLLPQANCSVHTAPPAEASMGTR |
| | | | DDACLYSPASAPEVITVGATNAQDQPV TLGTLGTNFGR |
| | | | DVINEAWFPEDQR |
| | | | EHGIPAPQEQVTVACEEGWTLTGCSAL PGTSHVLGAYAVDNTCVVR |
| | | | GTVSGTLIGLEFIR |
| | | | SQLVQPVGPLVVLLPLAGGYSR |
| | | | VMVTDFENVPEEDGTR |
| Q9UHG3 | PCYOX1 | Prenylcysteine oxidase 1 | EKEDPEPSTDGTYVWK |
| | | | FGLNTVLTTDNSDLFINSIGIVPSVR |
| | | | FLNEMIAPVMR |
| | | | GELNTSIFSSR |
| | | | GELNTSIFSSRPIDK |
| | | | IAIIGAGIGGTSAAYYLR |
| | | | IDLFER |
| | | | KMSNITFLNFDPPIEEFHQYYQHIVTTLV K |
| | | | LATMMVQGQEYEAGGSVIHPLNLHMK |
| | | | LFLSYDYAVK |
| | | | LLHALGGDDFLGMLNR |
| | | | MHMWVEDVLDK |
| | | | MMVQGQEYEAGGSVIHPLNLHMK |

(continued)

| Protein ID | Gene name | Protein name | Peptide sequence |
|---|---|---|---|
| | | | MSNITFLNFDPPIEEFHQYYQHIVTTLVK |
| | | | MYEVVYQIGTETR |
| | | | SDFYDIVLVATPLNR |
| | | | SNLISGSVMYIEEK |
| | | | TLLETLQK |
| | | | VNYGQSTDINAFVGAVSLSCSDSGLWAVEGGNK |
| | | | WNGHTDMIDQDGLYEK |
| | | | YQSHDYAFSSVEK |
| O75594 | PGLYRP1 | Peptidoglycan recognition protein 1 | AAQGLLACGVAQGALR |
| | | | ALASECAQHLSLPLR |
| | | | TLGWCDVGYNFLIGEDGLVYEGR |
| | | | TLSPGNQLYHLIQNWPHYR |
| | | | YVVVSHTAGSSCNTPASCQQQAR |
| Q96PD5 | PGLYRP2 | N-acetylmuramoyl-L-alanine amidase | AGLLRPDYALLGHR |
| | | | ASLLTMAFLNGALDGVILGDYLSR |
| | | | DGSPDVTTADIGANTPDATK |
| | | | DTLPSCAVR |
| | | | EFTEAFLGCPAIHPR |
| | | | EGKEYGVVLAPDGSTVAVEPLLAGLEAGLQGR |
| | | | EYGVVLAPDGSTVAVEPLLAGLEAGLQGR |
| | | | GCPDVQASLPDAK |
| | | | GFGVAIVGNYTAALPTEAALR |
| | | | GSQTQSHPDLGTEGCWDQLSAPR |
| | | | HTASAWLMSAPNSGPHNR |
| | | | LEPVHLQLQCMSQEQLAQVAANATK |
| | | | LEPVHLQLQCMSQEQLAQVAANATKEFTEAFLGCPAIHPR |
| | | | LLQLPLGFLYVHHTYVPAPPCTDFTR |
| | | | LYHFLLGAWSLNATELDPCPLSPELLGLTK |
| | | | PSLSHLLSQYYGAGVAR |
| | | | QNGAALTSASILAQQVWGTLVLLQR |
| | | | RVINLPLDSMAAPWETGDTFPDVVAIAPDVR |
| | | | SPPTMVDSLLAVTLAGNLGLTFLR |
| | | | TDCPGDALFDLLR |
| | | | TFTLLDPK |

(continued)

| Protein ID | Gene name | Protein name | Peptide sequence |
|---|---|---|---|
| | | | TPEPRPSLSHLLSQYYGAGVAR |
| | | | TWPHFTATVK |
| | | | VINLPLDSMAAPWETGDTFPDVVAIAPDVR |
| | | | WGAAPYR |
| | | | YHQDTQGWGDIGYSFVVGSDGYVYEGR |
| P01833 | PIGR | Polymeric immunoglobulin receptor | ADEGWYWCGVK |
| | | | AFVNCDENSR |
| | | | AIQDPRLFAEEKAVADTR |
| | | | ANLTNFPENGTFVVNIAQLSQDDSGR |
| | | | ANLTNFPENGTFVVNIAQLSQDDSGRYK |
| | | | CGLGINSR |
| | | | CPLLVDSEGWVK |
| | | | DAGFYWCLTNGDTLWR |
| | | | DGSFSVVITGLR |
| | | | GGCITLISSEGYVSSK |
| | | | GLSFDVSLEVSQGPGLLNDTK |
| | | | GSVTFHCALGPEVANVAK |
| | | | GVAGGSVAVLCPYNR |
| | | | IIEGEPNLK |
| | | | IIEGEPNLKVPGNVTAVLGETLK |
| | | | ILLNPQDK |
| | | | LDIQGTGQLLFSVVINQLR |
| | | | LSDAGQYLCQAGDDSNSNK |
| | | | LSDAGQYLCQAGDDSNSNKK |
| | | | LSLLEEPGNGTFTVILNQLTSR |
| | | | LVSLTLNLVTR |
| | | | NADLQVLKPEPELVYEDLR |
| | | | QGHFYGETAAVYVAVEER |
| | | | QIGLYPVLVIDSSGYVN PNYTGR |
| | | | QSSGENCDVVVNTLGK |
| | | | SPIFGPEEVNSVEGNSVSITCYYPPTSVNR |
| | | | TVTINCPFK |
| | | | VPGNVTAVLGETLK |
| | | | VPGNVTAVLGETLKVPCHFPCK |
| | | | VYTVDLGR |

(continued)

| Protein ID | Gene name | Protein name | Peptide sequence |
|---|---|---|---|
| | | | WNNTGCQALPSQDEGPSK |
| | | | YLCGAHSDGQLQEGSPIQAWQLFVNEESTIPR |
| | | | YWCLWEGAQNGR |
| Q9UKJ1 | PILRA | Paired immunoglobulin-like type 2 receptor alpha | HLSASMGGSVEIPFSFYYPWELATAPDVR |
| | | | LFLNWTEGQK |
| | | | SPQNETLYSVLK |
| P00747 | PLG | Plasminogen | APWCHTTNSQVR |
| | | | ATTVTGTPCQDWAAQEPHR |
| | | | CEEDEEFTCR |
| | | | CSGTEASVVAPPPVVLLPDVETPSEEDCMFGNGK |
| | | | CTTPPPSSGPTYQCLK |
| | | | EAQLPVIENK |
| | | | ELRPWCFTTDPNK |
| | | | ELRPWCFTTDPNKR |
| | | | FGMHFCGGTLISPEWVLTAAHCLEK |
| | | | FSPATHPSEGLEENYCR |
| | | | FSPATHPSEGLEENYCRNPDNDPQGPWCYTTDPEKR |
| | | | FVTWIEGVMR |
| | | | GNVAVTVSGHTCQHWSAQTPHTHNR |
| | | | GNVAVTVSGHTCQHWSAQTPHTHNRTPENFPCK |
| | | | GPWCFTTDPSVR |
| | | | GTSSTTTGK |
| | | | HSIFTPETNPR |
| | | | ISKTMSGLECQAWDSQSPHAHGYIPSK |
| | | | KCSGTEASVVAPPPVVLLPDVETPSEEDCMFGNGK |
| | | | KLYDYCDVPQCAAPSFDCGK |
| | | | KLYDYCDVPQCAAPSFDCGKPQVEPK |
| | | | KQLGAGSIEECAAK |
| | | | LFLEPTR |
| | | | LSSPAVITDK |
| | | | LYDYCDVPQCAAPSFDCGK |
| | | | LYDYCDVPQCAAPSFDCGKPQVEPK |
| | | | NLDENYCR |
| | | | NPDADKGPWCFTTDPSVR |

(continued)

| Protein ID | Gene name | Protein name | Peptide sequence |
|---|---|---|---|
| | | | NPDGDVGGPWCYTTNPR |
| | | | NPDNDPQGPWCYTTDPEK |
| | | | NPDNDPQGPWCYTTDPEKR |
| | | | QLGAGSIEECAAK |
| | | | QLGAGSIEECAAKCEEDEEFTCR |
| | | | RATTVTGTPCQDWAAQEPHR |
| | | | RWELCDIPR |
| | | | TECFITGWGETQGTFGAGLLK |
| | | | TMSGLECQAWDSQSPHAHGYIPSK |
| | | | TPENFPCK |
| | | | TPENYPNAGLTMNYCR |
| | | | VILGAHQEVNLEPHVQEIEVSR |
| | | | VIPACLPSPNYVVADR |
| | | | VQSTELCAGHLAGGTDSCQGDSGGPLVCFEK |
| | | | WGGCVAHPHSWPWQVSLR |
| | | | VYLSECK |
| | | | WELCDIPR |
| | | | WEYCNLK |
| | | | YDYCDILECEEECMHCSGENYDGK |
| | | | YEFLNGR |
| | | | DKYILQGVTSWGLGCAR |
| | | | YILQGVTSWGLGCAR |
| | | | DVVLFEK |
| | | | EQQCVIMAENR |
| P55058 | PLTP | Phospholipid transfer protein | AGALQLLLVGDK |
| | | | AGALQLLLVGDKVPHDLDMLLR |
| | | | ALELVKQEGLR |
| | | | ATYFGSIVLLSPAVIDSPLK |
| | | | AVEPQLQEEER |
| | | | DPVASTSNLDMDFR |
| | | | EGHFYYN ISEVK |
| | | | FLEQELETITIPDLR |
| | | | FLLNQQICPVLYHAGTVLLNSLLDTVPVR |
| | | | FRIYSNHSALESLALIPLQAPLK |
| | | | GAFFPLTER |
| | | | GAFFPLTERNWSLPNR |

(continued)

| Protein ID | Gene name | Protein name | Peptide sequence |
|---|---|---|---|
| | | | GKEGHFYYNISEVK |
| | | | GVQIPLPEGINFVHEVVTNHAGFLTIGADLHFAK |
| | | | IYSNHSALESLALIPLQAPLK |
| | | | KVYDFLSTFITSGMR |
| | | | LQITNASLGLR |
| | | | MHAAFGGTFK |
| | | | MKVSNVSCQASVSR |
| | | | MLQITNASLGLR |
| | | | MVYVAFSEFFFDSAMESYFR |
| | | | QLLYWFFYDGGYINASAEGVSIR |
| | | | SSVDELVGIDYSLMK |
| | | | SSVDELVGIDYSLMKDPVASTSNLDMDFR |
| | | | TGLELSR |
| | | | TMLQIGVMPMLNER |
| | | | VPHDLDMLLR |
| | | | VSNVSCQASVSR |
| | | | VTELQLTSSELDFQPQQELMLQITNASLGLR |
| | | | VYDFLSTFITSGMR |
| P27169 | PON1 | Serum paraoxonase/arylesterase 1 | AKLIALTLLGMGLALFR |
| | | | EVQPVELPNCNLVK |
| | | | FDVSSFNPHGISTFTDEDNAMYLLVVNHPDAK |
| | | | GIETGSEDLEILPNGLAFISSGLKYPGIK |
| | | | HANWTLTPLK |
| | | | IFFYDSENPPASEVLR |
| | | | ILLMDLNEEDPTVLELGITGSK |
| | | | IQNILTEEPK |
| | | | IQNILTEEPKVTQVYAENGTVLQGSTVASVYK |
| | | | IQNILTEEPKVTQVYAENGTVLQGSTVASVYKGK |
| | | | LIALTLLGMGLALFR |
| | | | LLIGTVFHK |
| | | | LLPNLNDIVAVGPEHFYGTNDHYFLDPYLQSWEMYLGLAWSYVVYYSPSEVR |
| | | | NHQSSYQTR |

(continued)

| Protein ID | Gene name | Protein name | Peptide sequence |
|---|---|---|---|
| | | | SFNPNSPGK |
| | | | SLDFNTLVDNISVDPETGDLWVGCHPNGMK |
| | | | STVELFK |
| | | | STVELFKFQEEEKSLLHLK |
| | | | STVELFKFQEEEKSLLHLK |
| | | | VTQVYAENGTVLQGSTVASVYK |
| | | | VTQVYAENGTVLQGSTVASVYKGK |
| | | | VVAEGFDFANGINISPDGK |
| | | | VVAEGFDFANGINISPDGKYVYIAELLAHK |
| | | | YVYIAELLAHK |
| | | | YVYIAELLAHKIHVYEK |
| Q15166 | PON3 | Serum paraoxonase/lactonase 3 | DHYFTNSLLSFFEMILDLR |
| | | | ELFNPHGISIFIDK |
| | | | EVEPVEPENCHLIEELESGSEDIDILPSGLAFISSGLK |
| | | | HDNWDLTQLK |
| | | | IFLMDLNEQNPR |
| | | | LLNYNPEDPPGSEVLR |
| | | | SVNDIVVLGPEQFYATR |
| | | | VIQLGTLVDNLTVDPATGDILAGCHPNPMK |
| | | | VSTVYANNGSVLQGTSVASVYHGK |
| | | | YPGMPNFAPDEPGK |
| | | | YVYVADVAAK |
| P04070 | PROC | Vitamin K-dependent protein C | DTEDQEDQVDPR |
| | | | EIFQNVDDTLAFWSK |
| | | | ELNQAGQETLVTGWGYHSSR |
| | | | EVFVHPNYSK |
| | | | EVSFLNCSLDNGGCTHYCLEEVGWR |
| | | | GDSPWQVVLLDSK |
| | | | HVDGDQCLVLPLEHPCASLCCGHGTCIDGIGSFSCDCR |
| | | | IPVVPHNECSEVMSNMVSENMLCAGILGDR |
| | | | LACGAVLIHPSWVLTAAHCMDESK |
| | | | LACGAVLIHPSWVLTAAHCMDESKK |
| | | | LGDDLLQCHPAVK |

(continued)

| Protein ID | Gene name | Protein name | Peptide sequence |
|---|---|---|---|
| | | | RGDSPWQVVLLDSK |
| | | | STTDNDIALLHLAQPATLSQTIVPICLPDSGLAER |
| | | | TFVLNFIK |
| | | | WELDLDIK |
| | | | YLDWIHGHIR |
| Q9UNN8 | PROCR | Endothelial protein C receptor | ALWQADTQVTSGVVTFTLQQLNAYNR |
| | | | CFLGCELPPEGSR |
| | | | DPYHVWYQGNASLGGHLTHVLEGPDTNTTIIQLQPLQEPESWAR |
| | | | EFLEDTCVQYVQK |
| | | | LHMLQISYFR |
| | | | TLAFPLTIR |
| | | | TQSGLQSYLLQFHGLVR |
| P24158 | PRTN3 | Myeloblastin | AGICFGDSGGPLICDGIIQGIDSFVIWGCATR |
| | | | GNPGSHFCGGTLIHPSFVLTAAHCLR |
| | | | LFPDFFTR |
| | | | LNDVLLIQLSSPANLSASVATVQLPQQDQPVPHGTQCLAMGWGR |
| | | | LVNVVLGAHNVR |
| | | | TQEPTQQHFSVAQVFLNNYDAENK |
| | | | VALYVDWIR |
| | | | VGAHDPPAQVLQELNVTVVTFFCR |
| | | | VGAHDPPAQVLQELNVTVVTFFCRPHNICTFVPR |
| P23467 | PTPRB | Receptor-type tyrosine-protein phosphatase beta | HANETSLSIMWQTPVAEWEK |
| | | | HATSYAFHGLTPGYLYNLTVMTEAAGLQNYR |
| | | | IDNTTYGCNLQDLQAGTIYNFR |
| | | | LSNVDDDPCSDYINASYIPGNNFR |
| | | | LVNESLCLQK |
| | | | NRSTEDLHVTWSGANGDVDQYEIQLLFNDMK |
| | | | SALCLAISNSSR |
| | | | SFSVYTNGSTVPSPVK |
| | | | TIQNQQWMWTEDEK |
| | | | VTSYEVQLFDENNQK |
| | | | WQRPPGNVDSYNITLSHK |

(continued)

| Protein ID | Gene name | Protein name | Peptide sequence |
|---|---|---|---|
| P15151 | PVR | Poliovirus receptor | HGESGSMAVFHQTQGPSYSESK |
| | | | LEFVAAR |
| | | | PINTTLICNVTNALGAR |
| | | | PVDKPINTTLICNVTNALGAR |
| | | | QAELTVQVK |
| | | | SNPEPTGYNWSTTMGPLPPFAVAQGAQLLIR |
| | | | VEDEGNYTCLFVTFPQGSR |
| | | | VEHESFEKPQLLTVNLTVYYPPEVSISGYDNNWYLGQNEATLTCDAR |
| Q92626 | PXDN | Peroxidasin homolog | ANEQLGLTSMHTLWFR |
| | | | DGQVTCFVEACPPATCAVPVNIPGACCPVCLQK |
| | | | FHISPEGFLTINDVGPADAGR |
| | | | ILCDNADNITR |
| | | | ISGVALHDQGQYECQAVNIIGSQK |
| | | | LDSNTLHCDCEILWLADLLK |
| | | | LSTTECVDAGGESHANNTK |
| | | | LWYENPGVFSPAQLTQIK |
| | | | LYGSTLNIDLFPALVVEDLVPGSR |
| | | | QGEHLSNSTSAFSTR |
| | | | TLQLIQEHVQHGLMVDLNGTSYHYNDLVSPQYLNLIANLSGCTAHRR |
| | | | VAEFPHGYGSCDEIPR |
| | | | VYCNLSAAHTFEDLK |
| 000391 | QSOX1 | Sulfhydryl oxidase 1 | AHFSPSNIILDFPAAGSAAR |
| | | | AWRPALYLAALDCAEETNSAVCR |
| | | | DCASHFEQMAAASMHR |
| | | | DFNIPGFPTVR |
| | | | DVQNVAAAPELAMGALELESR |
| | | | EVLPAIR |
| | | | FGVTDFPSCYLLFR |
| | | | FPVLEGQR |
| | | | GYVHYFFGCR |
| | | | IYMADLESALHYILR |
| | | | KFGVTDFPSCYLLFR |
| | | | LAGAPSEDPQFPK |
| | | | LDVPVWDVEATLNFLK |
| | | | LEEIDGFFAR |

(continued)

| Protein ID | Gene name | Protein name | Peptide sequence |
|---|---|---|---|
| | | | LIDALESHHDTWPPACPPLEPAK |
| | | | LIDALESHHDTWPPACPPLEPAKLEEID |
| | | | GFFAR |
| | | | NGSGAVFPVAGADVQTLR |
| | | | NNEEYLALIFEK |
| | | | PALYLAALDCAEETNSAVCR |
| | | | PEMMKSPTNTTPHVPAEGPEASR |
| | | | PLVQNFLHSVNEWLK |
| | | | RDVQNVAAAPELAMGALELESR |
| | | | SALYSPSDPLTLLQADTVR |
| | | | SFYTAYLQR |
| | | | VGSPNAAVLWLWSSHNR |
| | | | VLNTEANVVR |
| | | | VNWIGCQGSEPHFR |
| | | | YFPGRPLVQNFLHSVNEWLK |
| P05451 | REG1A | Lithostathine-1-alpha | DVPCEDKFSFVCK |
| | | | ESGTDDFNVWIGLHDPK |
| | | | ETWVDADLYCQNMNSGNLVSVLTQAEGAFVASLIK |
| | | | SWGIGAPSSVNPGYCVSLTSSTGFQK |
| | | | SYCYYFNEDR |
| | | | ISCPEGTNAYR |
| | | | WHWSSGSLVSYK |
| P07998 | RNASE1 | Ribonuclease pancreatic | CKPVNTFVHEPLVDVQNVCFQEK |
| | | | HIIVACEGSPYVPVHFDASVEDST |
| | | | PVNTFVHEPLVDVQNVCFQEK |
| P10153 | RNASE2 | Non-secretory ribonuclease | SNSSMHITDCR |
| | | | DPPQYPVVPVHLDR |
| | | | NCHHSGSQVPLIHCNLTTPSPQNISNCR |
| | | | NQNTFLLTTFANVVNVCGNPNMTCPSNK |
| | | | RDPPQYPVVPVHLDR |
| | | | YAQTPANMFYIVACDNR |
| Q8WZ75 | ROBO4 | Roundabout homolog 4 | ARGPDSNVLLLR |
| | | | DMVAVVGEQFTLECGPPWGHPEPTVSWWK |
| | | | GHAHDGQALSTDLGVYTCEASNR |
| | | | GPDSNVLLLR |

(continued)

| Protein ID | Gene name | Protein name | Peptide sequence |
|---|---|---|---|
| | | | IQLENVTLLNPDPAEGPK |
| | | | IQLENVTLLNPDPAEGPKPR |
| | | | LSVAVLR |
| | | | PAVWLSWK |
| | | | PDWLEDMEVSHTQR |
| | | | TQTAPGGQGAPWAEELLAGWQSAELGGLHWGQDYEFK |
| | | | VPSAPPQEVTLKPGNGTVFVSWVPPPAENHNGIIR |
| | | | VSGPAAPAQSYTALFR |
| | | | VSIQEPQDYTEPVELLAVR |
| P0DJI8 | SAA1 | Serum amyloid A-1 protein | FFGHGAEDSLADQAANEWGR |
| | | | GPGGVWAAEAISDAR |
| | | | RGPGGVWAAEAISDAR |
| | | | RGPGGVWAAEAISDARENIQR |
| P0DJI9 | SAA2 | Serum amyloid A-2 protein | GAEDSLADQAANK |
| | | | GPGGAWAAEVISNAR |
| | | | GPGGAWAAEVISNARENIQR |
| | | | RGPGGAWAAEVISNAR |
| | | | RGPGGAWAAEVISNARENIQR |
| | | | A YSDMREANYIGSDKYFHAR |
| | | | DPNHFRPAGLPEK |
| | | | EANYIGSDK |
| | | | EANYIGSDKYFHAR |
| | | | PAGLPEKY |
| | | | RGPGGAWAAEVISNAR |
| | | | RGPGGAWAAEVISNARENIQR |
| | | | SFFSFLGEAFDGAR |
| P16581 | SELE | E-selectin | CEQIVNCTALESPEHGSLVCSHPLGNFSYNSSCSISCDR |
| | | | EEIEYLNSILSYSPSYYWIGIR |
| | | | FACPEGWTLNGSAAR |
| | | | GYMNCLPSASGSFR |
| | | | INMSCSGEPVFGTVCK |
| | | | KFVPASSCQSLESDGSYQK |
| | | | LALCYTAACTNTSCSGHGECVETINNYTCK |

(continued)

| Protein ID | Gene name | Protein name | Peptide sequence |
|---|---|---|---|
| | | | LQCGPTGEWDNEKPTCEAVR |
| | | | SSCNFTCEEGFMLQGPAQVECTTQGQ WTQQIPVCEAFQCT ALSNPER |
| | | | VNNVWVWVGTQKPLTEEAK |
| | | | YGSSCEFSCEQGFVLK |
| P49908 | SEPP1 | Selenoprotein P | CGNCSLTTLKDEDFCK |
| | | | CINQLLCK |
| | | | CGNCSLTTLK |
| | | | CGNCSLTTLKDEDFCKR |
| | | | DDFLIYDR |
| | | | DMPASEDLQDLQK |
| | | | EGYSNISYIWNHQGISSR |
| | | | KCGNCSLTTLKDEDFCK |
| | | | KEGYSNISYIWNHQGISSR |
| | | | LPTDSELAPR |
| | | | LVYHLGLPFSFLTFPYVEEAIK |
| | | | QPPAWSIR |
| | | | VSEHIPVYQQEENQTDVWTLLNGSK |
| | | | VSEHIPVYQQEENQTDVWTLLNGSKDD FLIYDR |
| P01009 | SERPINA1 | Alpha-1-antitrypsin | ADTHDEILEGLNFNLTEIPEAQIHEGFQE LLR |
| | | | ADTHDEILEGLNFNLTEIPEAQIHEGFQE LLRTLNQPDSQ LQLTTGNGLFLSEGLK |
| | | | AVHKAVLTIDEKGTEAAGAMFLEAIPMS IPPEVK |
| | | | AVLTIDEK |
| | | | AVLTIDEKGTEAAGAMFLEAIPMSIPP EVK |
| | | | DTEEEDFHVDQVTTVK |
| | | | DTEEEDFHVDQVTTVKVPMMK |
| | | | DTVFALVNYIFFK |
| | | | DTVFALVNYIFFKGK |
| | | | ELDRDTVFALVNYIFFK |
| | | | ELDRDTVFAL VNYIFFKGK |
| | | | FLEDVK |
| | | | FLEDVKK |
| | | | FLENEDR |

(continued)

| Protein ID | Gene name | Protein name | Peptide sequence |
|---|---|---|---|
| | | | FLENEDRR |
| | | | FNKPFVFLMIEQNTK |
| | | | FNKPFVFLMIEQNTKSPLFMGK |
| | | | GKWERPFEVK |
| | | | GTEAAGAMFLEAIPMSIPPEVK |
| | | | ITPNLAEFAFSLYR |
| | | | ITPNLAEFAFSLYRQLAHQSNSTNIFFSP VSIATAFAMLSLGTK |
| | | | IVDLVKELDR |
| | | | IVDLVKELDRDTVFALVNYIFFK |
| | | | KLSSWVLLMK |
| | | | KLYHSEAFTVNFGDTEEAK |
| | | | KLYHSEAFTVNFGDTEEAKK |
| | | | KQINDYVEK |
| | | | LGMFNIQHCK |
| | | | LQHLENEL THDIITK |
| | | | LQHLENEL THDIITKFLENEDR |
| | | | LQHLENEL THDIITKFLENEDRR |
| | | | LSITGTYDLK |
| | | | LSITGTYDLKSVLGQLGITK |
| | | | LSSWVLLMK |
| | | | LSSWVLLMKYLGNATAIFFLPDEGK |
| | | | LVDKFLEDVK |
| | | | LVDKFLEDVKK |
| | | | LYHSEAFTVNFGDTEEAK |
| | | | LYHSEAFTVNFGDTEEAKK |
| | | | LYHSEAFTVNFGDTEEAKKQINDYVEK |
| | | | PFEVKDTEEEDFHVDQVTTVK |
| | | | PFVFLMIEQNTK |
| | | | QINDYVEK |
| | | | QLAHQSNSTNIFFSPVSIATAFAMLSLG TK |
| | | | QLAHQSNSTNIFFSPVSIATAFAMLSLG TKADTHDEILEG LNFNLTEIPEAQIHEGFQELLR |
| | | | RLGMFNIQHCK |
| | | | SASLHLPK |
| | | | SASLHLPKLSITGTYDLK |
| | | | SPLFMGK |

(continued)

| Protein ID | Gene name | Protein name | Peptide sequence |
|---|---|---|---|
| | | | SVLGQLGITK |
| | | | SVLGQLGITKVFSNGADLSGVTEEAPLK |
| | | | SVLGQLGITKVFSNGADLSGVTEEAPLK LSK |
| | | | TDTSHHDQDHPTFNK |
| | | | TDTSHHDQDHPTFNKITPNLAEFAFS LYR |
| | | | TLNQPDSQLQLTTGNGLFLSEGLK |
| | | | TLNQPDSQLQLTTGNGLFLSEGLKL VDK |
| | | | TLNQPDSQLQLTTGNGLFLSEGLKLVD KFLEDVK |
| | | | VFSNGADLSGVTEEAPLK |
| | | | VFSNGADLSGVTEEAPLKLSK |
| | | | VFSNGADLSGVTEEAPLKLSKAVHK |
| | | | WERPFEVKDTEEEDFHVDQVTTVK |
| | | | YLGNATAIFFLPDEGK |
| | | | YLGNATAIFFLPDEGKLQHLENELTHDII TK |
| | | | VVNPTQK |
| Q9UK55 | SERPINA10 | Protein Z-dependent protease inhibitor | ETFFNLSK |
| | | | ETSNFGFSLLR |
| | | | FASTFDK |
| | | | GLHLQALKPTKPGLLPSLFK |
| | | | GTEAVAGILSEITAYSMPPVIK |
| | | | HDGNMVFSPFGMSLAMTGLMLGATGP TETQIK |
| | | | IFSPFADLSELSATGR |
| | | | LFDEINPETK |
| | | | LILVDYILFK |
| | | | LPYQGNATMLVVLMEK |
| | | | MGDHLALEDYLTTDLVETWLR |
| | | | NLELGL TQGSFAFIHK |
| | | | NLELGL TQGSFAFIHKDFDVK |
| | | | NMEVFFPK |
| | | | PGLLPSLFK |
| | | | PTKPGLLPSLFK |
| | | | TVIEVDER |
| | | | VDRPFHFMIYEETSGMLLFLGR |

(continued)

| Protein ID | Gene name | Protein name | Peptide sequence |
|---|---|---|---|
| | | | VVNPTLL |
| | | | WLTPFDPVFTEVDTFHLDK |
| | | | WLTPFDPVFTEVDTFHLDKYK |
| | | | YEMHELLR |
| | | | YFDTECVPMNFR |
| P01011 | SERPINA3 | Alpha-1-antichymotrypsin | ADLSGITGAR |
| | | | ADLSGITGARNLAVSQVVHK |
| | | | AKWEMPFDPQDTHQSR |
| | | | APDKNVIFSPLSISTALAFLSLGAHNTTLTEILK |
| | | | AVLDVFEEGTEASAATAVK |
| | | | DEELSCTVVELK |
| | | | DEELSCTVVELKYTGNASALFILPDQDK |
| | | | DEELSCTVVELKYTGNASALFILPDQDKMEEVEAMLLPETLK |
| | | | DLDSQTMMVLVNYIFFK |
| | | | DLDSQTMMVLVNYIFFKAK |
| | | | DSLEFR |
| | | | DSLEFREIGELYLPK |
| | | | DYNLNDILLQLGIEEAFTSK |
| | | | DYNLNDILLQLGIEEAFTSKADLSGITGAR |
| | | | EIGELYLPK |
| | | | EIGELYLPKFSISR |
| | | | EQLSLLDR |
| | | | EQLSLLDRFTEDAK |
| | | | EQLSLLDRFTEDAKR |
| | | | FNLTETSEAEIHQSFQHLLR |
| | | | FNRPFLMIIVPTDTQNIFFMSK |
| | | | FSISRDYNLNDILLQLGIEEAFTSK |
| | | | GKITDLIKDLDSQTMMVLVNYIFFK |
| | | | GLKFNLTETSEAEIHQSFQHLLR |
| | | | GLKFNLTETSEAEIHQSFQHLLRTLNQSSDELQLSMGNAMFVK |
| | | | GTHVDLGLASANVDFAFSLYK |
| | | | ITDLIKDLDSQTMMVLVNYIFFK |
| | | | ITLLSALVETR |
| | | | KWVMVPMMSLHHLTIPYFR |

170

(continued)

| Protein ID | Gene name | Protein name | Peptide sequence |
|---|---|---|---|
| | | | KWVMVPMMSLHHLTIPYFRDEELSCTVVELK |
| | | | LINDYVK |
| | | | LYGSEAFATDFQDSAAAK |
| | | | LYGSEAFATDFQDSAAAKK |
| | | | MEEVEAMLLPETLK |
| | | | MEEVEAMLLPETLKR |
| | | | NLAVSQVVHK |
| | | | NLAVSQVVHKAVLDVFEEGTEASAATAVK |
| | | | NVIFSPLSISTALAFLSLGAHNTTLTEILK |
| | | | PFLMIIVPTDTQNIFFMSK |
| | | | RLYGSEAFATDFQDSAAAK |
| | | | RLYGSEAFATDFQDSAAAKK |
| | | | TLNQSSDELQLSMGNAMFVK |
| | | | TLNQSSDELQLSMGNAMFVKEQLSLLDR |
| | | | TLNQSSDELQLSMGNAMFVKEQLSLLDRFTEDAK |
| | | | WEMPFDPQDTHQSR |
| | | | WRDSLEFR |
| | | | WRDSLEFREIGELYLPK |
| | | | WVMVPMMSLHHLTIPYFR |
| | | | WVMVPMMSLHHLTIPYFRDEELSCTVVELK |
| | | | YTGNASALFILPDQDK |
| | | | YTGNASALFILPDQDKMEEVEAMLLPETLK |
| | | | YTGNASALFILPDQDKMEEVEAMLLPETLKR |
| P29622 | SERPINA4 | Kallistatin | ALWEKPFISSR |
| | | | ATLDVDEAGTEAAAATSFAIK |
| | | | DFYVDENTTVR |
| | | | DVLMVLVNYIYFK |
| | | | EIEEVLTPEMLMR |
| | | | FFSAQTNR |
| | | | FLNDTMAVYEAK |
| | | | FSISGSYVLDQILPR |
| | | | FYYLIASETPGK |
| | | | GDATVFFILPNQGK |

(continued)

| Protein ID | Gene name | Protein name | Peptide sequence |
|---|---|---|---|
| | | | GFQHLLHTLNLPGHGLETR |
| | | | IAPANADFAFR |
| | | | IVDLVSELK |
| | | | IVDLVSELKK |
| | | | KDVLMVLVNYIYFK |
| | | | LFHTNFYDTVGTIQLINDHVK |
| | | | LFHTNFYDTVGTIQLINDHVKK |
| | | | LGFTDLFSK |
| | | | MDYKGDATVFFILPNQGK |
| | | | MREIEEVLTPEMLMR |
| | | | NIFFSPLSISAAYAMLSLGACSHSR |
| | | | SQILEGLGFNLTELSESDVHR |
| | | | TTPKDFYVDENTTVR |
| | | | VGSALFLSHNLK |
| | | | VPMMLQDQEHHWYLHDR |
| | | | WADLSGITK |
| | | | WNNLLR |
| | | | YLPCSVLR |
| P05154 | SERPINA5 | Plasma serine protease inhibitor | AAAATGTIFTFR |
| | | | ALASAAPSQSIFFSPVSISMSLAMLSLGAGSSTK |
| | | | AVVEVDESGTR |
| | | | DFTFDLYR |
| | | | DGFQLSLGNALFTDLVVDLQDTFVS AMK |
| | | | EDQYHYLLDR |
| | | | EDQYHYLLDRNLSCR |
| | | | FSIEGSYQLEK |
| | | | GFQQLLQELNQPR |
| | | | GTQEQDFYVRSETVVR |
| | | | LVFNRPFLMFIVDNNILFLGK |
| | | | MQILEGLGLNLQK |
| | | | NLDSNAVVIMVNYIFFK |
| | | | QLELYLPK |
| | | | RDFTFDLYR |
| | | | TLYLADTFPTNFR |
| | | | VLPSLGISNVFTSHADLSGISNHSNIQVS EMVHK |

(continued)

| Protein ID | Gene name | Protein name | Peptide sequence |
|---|---|---|---|
| | | | VVGVPYQGNATALFILPSEGK |
| P01008 | SERPINC1 | Antithrombin-III | ADGESCSASMMYQEGK |
| | | | AFLEVNEEGSEAAASTAWIAGR |
| | | | ANRPFLVFIR |
| | | | ATEDEGSEQK |
| | | | ATEDEGSEQKIPEATNR |
| | | | DDLYVSDAFHK |
| | | | DIPMNPMCIYR |
| | | | ELFYKADGESCSASMMYQEGK |
| | | | ELTPEVLQEWLDELEEMMLVVHMPR |
| | | | ENAEQSRAAINK |
| | | | EQLQDMGLVDLFSPEK |
| | | | EVPLNTIIFMGR |
| | | | FATTFYQHLADSK |
| | | | FATTFYQHLADSKNDNDNIFLSPLSISTAFAMTK |
| | | | FDTISEK |
| | | | FDTISEKTSDQIHFFFAK |
| | | | FRIEDGFSLK |
| | | | FRIEDGFSLKEQLQDMGLVDLFSPEK |
| | | | GDDITMVLILPK |
| | | | GDDITMVLILPKPEK |
| | | | IEDGFSLK |
| | | | IEDGFSLKEQLQDMGLVDLFSPEK |
| | | | IPEATNR |
| | | | ITDVIPSEAINELTVLVLVNTIYFK |
| | | | KATEDEGSEQK |
| | | | KELFYKADGESCSASMMYQEGK |
| | | | LFGDKSLTFNETYQDISELVYGAK |
| | | | LGACNDTLQQLMEVFK |
| | | | LGACNDTLQQLMEVFKFDTISEK |
| | | | LGACNDTLQQLMEVFKFDTISEKTSDQIHFFFAK |
| | | | LPGIVAEGR |
| | | | LPGIVAEGRDDLYVSDAFHK |
| | | | LQPLDFK |
| | | | LQPLDFKENAEQSR |
| | | | LVSANRLFGDKSLTFNETYQDISELVYGAK |

(continued)

| Protein ID | Gene name | Protein name | Peptide sequence |
|---|---|---|---|
| | | | NDNDNIFLSPLSISTAFAMTK |
| | | | NDNDNIFLSPLSISTAFAMTKLGACNDT LQQLMEVFK |
| | | | PFLVFIR |
| | | | RVAEGTQVLELPFK |
| | | | SKLPGIVAEGR |
| | | | SKLPGIVAEGRDDLYVSDAFHK |
| | | | SLAKVEKELTPEVLQEWLDELEEMMLV VHMPR |
| | | | SLTFNETYQDISELVYGAK |
| | | | TSDQIHFFFAK |
| | | | VAEGTQVLELPFK |
| | | | VAEGTQVLELPFKGDDITMVLILPK |
| | | | VAEGTQVLELPFKGDDITMVLILPKPEK |
| | | | VEKELTPEVLQEWLDELEEMMLVVHM PR |
| | | | VWELSK |
| P05546 | SERPIND1 | Heparin cofactor 2 | DALENIDPATQMMILNCIYFK |
| | | | DFVNASSK |
| | | | DFVNASSKYEITTIHNLFR |
| | | | DFVNASSKYEITTIHNLFRK |
| | | | DQVNTFDNIFIAPVGISTAMGMISLGLK |
| | | | ENTVTNDWIPEGEEDDDYLDLEK |
| | | | EYYFAEAQIADFSDPAFISK |
| | | | FAFNLYR |
| | | | FPVEMTHNHNFR |
| | | | FTVDRPFLFLIYEHR |
| | | | GETHEQVHSILHFK |
| | | | GETHEQVHSILHFKDFVNASSK |
| | | | GETHEQVHSILHFKDFVNASSKYEITTIH NLFR |
| | | | GGETAQSADPQWEQLNNK |
| | | | |
| | | | GGETAQSADPQWEQLNNKNLSMPLLP ADFHK GNFLAANDQELDCDILQLEYVGGISMLI VVPHK GPLDQLEK HQGTITVNEEGTQATTVTTVGFMPLST QVR |
| | | | |

(continued)

| Protein ID | Gene name | Protein name | Peptide sequence |
|---|---|---|---|
| | | | |
| | | | IAIDLFK |
| | | | IFSEDDDYIDIVDSLSVSPTDSDVSAGNI LQLFHGK |
| | | | LNILNAK |
| | | | NFGYTLR |
| | | | NGNMAGISDQR |
| | | | NLSMPLLPADFHK |
| | | | NYNLVESLK |
| | | | PFLFLIYEHR |
| | | | QFPILLDFK |
| | | | SVNDLYIQK |
| | | | TLEAQLTPR |
| | | | TSCLLFMGR |
| | | | VLKDQVNTFDNIFIAPVGISTAMGMISLG LK |
| | | | VREYYFAEAQIADFSDPAFISK |
| | | | YEITTIHNLFR |
| P05155 | SERPING1 | Plasma protease C1 inhibitor | AKVGQLQLSHNLSLVILVPQNLK |
| | | | ASSNPNATSSSSQDPESLQDR |
| | | | DFTCVHQALK |
| | | | DFTCVHQALKGFTTK |
| | | | DTFVNASR |
| | | | DTFVNASRTLYSSSPR |
| | | | FPVFMGR |
| | | | FQPTLLTLPR |
| | | | GVTSVSQIFHSPDLAIR |
| | | | GVTSVSQIF HSPDLAIRDTFVNASR |
| | | | HRLEDMEQALSPSVFK |
| | | | IKVTTSQDMLSIMEK |
| | | | KVETNMAFSPFSIASLLTQVLLGAGE NTK |
| | | | KYPVAHFIDQTLK |
| | | | LEDMEQALSPSVFK |
| | | | LEFFDFSYDLNLCGLTEDPDLQVSAMQ HQTVLELTETGVEAAAASAISVAR |
| | | | LEMSKFQPTLLTLPR |
| | | | LLDSLPSDTR |
| | | | LLDSLPSDTRLVLLNAIYLSAK |

(continued)

| Protein ID | Gene name | Protein name | Peptide sequence |
|---|---|---|---|
| | | | LVLLNAIYLSAK |
| | | | LYHAFSAMK |
| | | | MEPFHFK |
| | | | MLFVEPILEVSSLPTTNSTTNSATK |
| | | | NSVIKVPMMNSK |
| | | | TLLVFEVQQPFLFVLWDQQHK |
| | | | TLLVFEVQQPFLFVLWDQQHKFPVF MGR |
| | | | TLYSSSPR |
| | | | TLYSSSPRVLSNNSDANLELINTWVAK |
| | | | TNLESILSYPK |
| | | | TNLESILSYPKDFTCVHQALK |
| | | | VETNMAFSPFSIASLLTQVLLGAGENTK |
| | | | VGQLQLSHNLSLVILVPQNLK |
| | | | VLSNNSDANLELINTWVAK |
| | | | VLSNNSDANLELINTWVAKNTNNK |
| | | | VTTSQDMLSIMEK |
| | | | YPVAHFIDQTLK |
| Q08ET2 | SIGLEC14 | Sialic acid-binding Ig-like lectin 14 | ALNPSQTSMSGTLELPNIGAR |
| | | | DGEIPYYAEVVATNNPDR |
| | | | LNLEVTALIEKPDIHFLEPLESGR |
| | | | LSCSLPGSCEAGPPLTFSWTGNALSPL DPETTR |
| | | | MEDTGSYFFR |
| | | | NCSLSIGDAR |
| | | | SSELTLTPRPEDHGTNLTCQVK |
| | | | SWYSSPPLYVYWFR |
| P78324 | SIRPA | Tyrosine-protein phosphatase non-receptor type substrate 1 | CTATSLIPVGPIQWFR |
| | | | IGN ITPADAGTYYCVK |
| | | | AENQVNVTCQVR |
| | | | DGTYNWMSWLLVNVSAHR |
| | | | GTANLSETIR |
| | | | LQLTWLENGNVSR |
| P10451 | SPP1 | Osteopontin | AIPVAQDLNAPSDWDSR |
| | | | EFHSHEFHSHEDMLVVDPK |
| | | | GDSVVYGLR |
| | | | ISHELDSASSEVN |
| | | | QNLLAPQNAVSSEETNDFK |

(continued)

| Protein ID | Gene name | Protein name | Peptide sequence |
|---|---|---|---|
| | | | QNLLAPQNAVSSEETNDFKQETLPSK |
| | | | RPDIQYPDATDEDITSHMESEELNG AYK |
| | | | YPDAVATWLNPDPSQK |
| Q4LDE5 | SVEP1 | Sushi, von Willebrand factor type A, EGF and pentraxin domain-containing protein 1 | ALHEDLPSGSFIQDDMVHCSYLCDEGK |
| | | | APPACHLVFCGEPPAIK |
| | | | CALLLQEIPAISYR |
| | | | CLEGYTMDTDTDTFTCQK |
| | | | CLPSQQWNDSFPVCK |
| | | | CLSNGSWSGSSPSCLPCR |
| | | | CMEGFVLNTSAK |
| | | | CPLPENITHILVHGDDFSVNR |
| | | | CSTPVIEYGTVNGTDFDCGK |
| | | | CVAPYQCDCPPGWTGSR |
| | | | DAVITGNNFTFR |
| | | | EEHCYLLHSFEEFEALAR |
| | | | EFYVDQNVSIK |
| | | | EGFLLQGHGIITCNPDETWTQTSAK |
| | | | EIEYTCNEGFLLEGAR |
| | | | ESSCLANSSWSHSPPVCEPVK |
| | | | FAAGSVVSFK |
| | | | FSEAFETTLGK |
| | | | GAFQQAAQILLHAR |
| | | | GAVNISACGVPCPEGK |
| | | | GEGFSPAESFVGSISQLNLWDYVLSPQ QVK |
| | | | GNVLAWPDFLSGIVGK |
| | | | GSNYTYLSTLYYECDPGYVLNGTER |
| | | | GSPCEIPFTPVNGDFICTPDNTGVNCTL TCLEGYDFTEGSTDK |
| | | | GVWSQPYPVCEPLSCGSPPSVANAVA TGEAHTYESEVK |
| | | | GYTLAGDKESSCLANSSWSHSPPVCE PVK |
| | | | IGSYQDEEGQLECK |
| | | | ISCGPPAHVENAIAR |
| | | | KCPLPENITHILVHGDDFSVNR |
| | | | LIFNITASVPLPDER |

(continued)

| Protein ID | Gene name | Protein name | Peptide sequence |
|---|---|---|---|
| | | | LIFNITASVPLPDERNDTLEWENQQR |
| | | | LLQTLETITNK |
| | | | LLSDFPVVPTATR |
| | | | MCVNCPLGTYYNLEHFTCESCR |
| | | | MVPSFCSDAEDIDCR |
| | | | NWDEDEPICIPVDCSSPPVSANGQVR |
| | | | PGFELVGNTTTLCGENGHWLGGK |
| | | | SDQQCLA VSCDEPPIVDHASPETAHR |
| | | | SGYVIQGSSDLICTEK |
| | | | SHTQGDLFPQGETIVQYTATDPSGNNR |
| | | | SVCLENGTWTSPPICR |
| | | | TLEQQDSANVTWQIPTAK |
| | | | VCLANGSWSGATPDCVPVR |
| | | | VIDAEPPVIDWCR |
| | | | YYCAYEDGVWKPTYTTEWPDCAK |
| P02787 | TF | Serotransferrin | ADRDQYELLCLDNTR |
| | | | ADRDQYELLCLDNTRK |
| | | | AIAANEADAVTLDAGLVYDAYLAPNNLK |
| | | | AIAANEADAVTLDAGLVYDAYLAPNNLK PVVAEFYGSK |
| | | | AIAANEADAVTLDAGLVYDAYLAPNNLK PVVAEFYGSKED PQTFYYAVAVVK |
| | | | APNHAVVTR |
| | | | ASYLDCIR |
| | | | AVANFFSGSCAPCADGTDFPQLCQLCP |
| | | | GCGCSTLNQYFGYSGAFK |
| | | | AVGNLRKCSTSSLLEACTFR |
| | | | CDEWSVNSVGK |
| | | | CDEWSVNSVGKIECVSAETTEDCIAK |
| | | | CGLVPVLAENYNK |
| | | | CLKDGAGDVAFVK |
| | | | CLVEKGDVAFVK |
| | | | CSTSSLLEACTFR |
| | | | DCHLAQVPSHTVVAR |
| | | | DDTVCLAK |
| | | | DGAGDVAFVK |
| | | | DGAGDVAFVKHSTIFENLANK |
| | | | DHMKSVIPSDGPSVACVK |

(continued)

| Protein ID | Gene name | Protein name | Peptide sequence |
|---|---|---|---|
| | | | DLLFKDSAHGFLK |
| | | | DLLFRDDTVCLAK |
| | | | DQYELLCLDNTR |
| | | | DSAHGFLK |
| | | | DSGFQMNQLR |
| | | | DYELLCLDGTR |
| | | | DYELLCLDGTRK |
| | | | DYELLCLDGTRKPVEEYANCHLAR |
| | | | EDLIWELLNQAQEHFGK |
| | | | EDLIWELLNQAQEHFGKDK |
| | | | EDPQTFYYAVAVVK |
| | | | EDPQTFYYAVAVVKK |
| | | | EFQLFSSPHGK |
| | | | EFQLFSSPHGKDLLFK |
| | | | EFQLFSSPHGKDLLFKDSAHGFLK |
| | | | EGTCPEAPTDECK |
| | | | EGTCPEAPTDECKPVK |
| | | | EGTCPEAPTDECKPVKWCALSHHER |
| | | | EGYYGYTGAFR |
| | | | FDEFFSEGCAPGSK |
| | | | FDEFFSEGCAPGSKK |
| | | | FDEFFSEGCAPGSKKDSSLCK |
| | | | GDVAFVK |
| | | | HQTVPQNTGGK |
| | | | HSTIFENLANK |
| | | | HSTIFENLANKADR |
| | | | HSTIFENLANKADRDQYELLCLDNTR |
| | | | IECVSAETTEDCIAK |
| | | | ILRQQQHLFGSNVTDCSGNFCLFR |
| | | | IMNGEADAMSLDGGFVYIAGK |
| | | | IMNGEADAMSLDGGFVYIAGKCGLVPVLAENYNK |
| | | | INHCRFDEFFSEGCAPGSK |
| | | | INHCRFDEFFSEGCAPGSKK |
| | | | KASYLDCIR |
| | | | KCSTSSLLEACTFR |
| | | | KDSGFQMNQLR |
| | | | KPVDEYKDCHLAQVPSHTVVAR |

(continued)

| Protein ID | Gene name | Protein name | Peptide sequence |
|---|---|---|---|
| | | | KPVEEYANCHLAR |
| | | | KSASDLTWDNLK |
| | | | LCMGSGLNLCEPNNK |
| | | | LCMGSGLNLCEPNNKEGYYGYTGAFR |
| | | | LHDRNTYEKYLGEEYVK |
| | | | LKCDEWSVNSVGK |
| | | | LKCDEWSVNSVGKIECVSAETTEDCIAK |
| | | | MYLGYEYVTAIR |
| | | | MYLGYEYVTAIRNLR |
| | | | NLNEKDYELLCLDGTR |
| | | | NLNEKDYELLCLDGTRK |
| | | | NPDPWAK |
| | | | NPDPWAKNLNEK |
| | | | NTYEKYLGEEYVK |
| | | | PVDEYKDCHLAQVPSHTVVAR |
| | | | PVEEYANCHLAR |
| | | | PVVAEFYGSK |
| | | | QQQHLFGSNVTDCSGNFCLFR |
| | | | QQQHLFGSNVTDCSGNFCLFRSETK |
| | | | SAGWNIPIGLLYCDLPEPR |
| | | | SAGWNIPIGLLYCDLPEPRK |
| | | | SASDLTWDNLK |
| | | | SCHTAVGR |
| | | | SCHTGLGRSAGWNIPIGLLYCDLPEPR |
| | | | SDNCEDTPEAGYFAIAVVK |
| | | | SETKDLLFRDDTVCLAK |
| | | | SKEFQLFSSPHGK |
| | | | SKEFQLFSSPHGKDLLFK |
| | | | SMGGKEDLIWELLNQAQEHFGK |
| | | | SMGGKEDLIWELLNQAQEHFGKDK |
| | | | SVIPSDGPSVACVK |
| | | | SVIPSDGPSVACVKK |
| | | | TAGWNIPMGLLYNK |
| | | | TAGWNIPMGLLYNKINHCR |
| | | | WCALSHHER |
| | | | WCAVSEHEATK |
| | | | WCAVSEHEATKCQSFR |
| | | | YLGEEYVK |

(continued)

| Protein ID | Gene name | Protein name | Peptide sequence |
|---|---|---|---|
| P01033 | TIMP1 | Metalloproteinase inhibitor 1 | AKFVGTPEVNQTTLYQR |
| | | | EPGLCTWQSLR |
| | | | FVGTPEVNQTTLYQR |
| | | | FVYTPAMESVCGYFHR |
| | | | FVYTPAMESVCGYFHRSHNRSEEFLIAGK |
| | | | GFQALGDAADIR |
| | | | LQDGLLHITTCSFVAPWNSLSLAQR |
| | | | LQSGTHCLWTDQLLQGSEK |
| | | | SEEFLIAGK |
| | | | SHNRSEEFLIAGK |
| | | | TYTVGCEECTVFPCLSIPCK |
| P24821 | TNC | Tenascin | AGTPYTVTLHGEVR |
| | | | ASTAKEPEIGNLNVSDITPESFNLSWMATDGIFETFTIEIIDSNRLLETVEYNISGAER |
| | | | ASTEQAPELENLTVTEVGWDGLR |
| | | | AVDIPGLEAATPYR |
| | | | CEEGQCVCDEGFAGVDCSEK |
| | | | CICNEGYSGEDCSEVSPPK |
| | | | CINGTCYCEEGFTGEDCGK |
| | | | CVENECVCDEGFTGEDCSELICPNDCFDR |
| | | | CVNGQCVCDEGYTGEDCSQLR |
| | | | DHGETAFAVYDK |
| | | | DLAPPSEPSESFQEHTVDGENQIVFTHR |
| | | | DVTDTTALITWFK |
| | | | DVTDTTALITWFKPLAEIDGIELTYGIK |
| | | | EATEYEIELYGISK |
| | | | EDKESNPATINAATELDTPK |
| | | | EEFWLGLDNLNK |
| | | | EPEIGNLNVSDITPESFNLSWMATDGIFETFTIEIIDSNR |
| | | | ESNPATINAATELDTPK |
| | | | ETFTTGLDAPR |
| | | | ETSVEVEWDPLDIAFETWEIIFR |
| | | | EVIVGPDTTSYSLADLSPSTHYTAK |

(continued)

| Protein ID | Gene name | Protein name | Peptide sequence |
|---|---|---|---|
| | | | GFEESEPVSGSFTTALDGPSGLVTANITDSEALAR GHSTRPLAVEVVTEDLPQLGDLAVSEVGWDGLR |
| | | | |
| | | | GLEPGQEYNVLLTAEK |
| | | | GNFSTEGCGCVCEPGWK |
| | | | GVTQDFSTTPLSVEVLTEEVPDMGNLTVTEVSWDALR |
| | | | IT AQGQYELR |
| | | | ITYVPITGGTPSMVTVDGTK |
| | | | KQSEPLEITLLAPER |
| | | | LDAPSQIEVK |
| | | | LEELENLVSSLR |
| | | | LIPGVEYLVSIIAMK |
| | | | LLDPQEFTLSGTQR |
| | | | LLETVEYNISGAER |
| | | | LNWTAADNAYEHFVIQVQEVNK |
| | | | LNWTAADQAYEHFIIQVQEANK |
| | | | LNYSLPTGQWVGVQLPR |
| | | | LPVGSQCSVDLESASGEK |
| | | | LSWTADEGVFDNFVLK |
| | | | NLTVPGSLR |
| | | | PLAEIDGIELTYGIK |
| | | | PLAVEVVTEDLPQLGDLAVSEVGWDGLR |
| | | | QSGVNATLPEENQPVVFNHVYNIK |
| | | | QTGLAPGQEYEISLHIVK |
| | | | SFSTFDKDTDSAITNCALSYK |
| | | | SNMIQTIFTTIGLLYPFPK |
| | | | SQTVSAIATTAMGSPK |
| | | | TAHISGLPPSTDFIVYLSGLAPSIR |
| | | | TISATATTEAEPEVDNLLVSDATPDGFR |
| | | | TPVLSAEASTGETPNLGEVVVAEVGWDALK |
| | | | TTIDLTEDENQYSIGNLKPDTEYEVSLISR |
| | | | TTLTGLRPGTEYGIGVSAVK |
| | | | TVSGNTVEYALTDLEPATEYTLR |
| | | | VATYLPAPEGLK |

(continued)

| Protein ID | Gene name | Protein name | Peptide sequence |
|---|---|---|---|
| | | | VEAAQNLTLPGSLR |
| | | | VPGDQTSTIIQELEPGVEYFIR |
| | | | VSQTDNSITLEWR |
| | | | VTEYLVVYTPTHEGGLEMQFR |
| | | | WQPAIATVDSYVISYTGEK |
| | | | YAPISGGDHAEVDVPK |
| | | | YGDNNHSQGVNWFHWK |
| P20333 | TNFRSF1B | Tumor necrosis factor receptor superfamily member 1B | GTQGPEQQHLLITAPSSSSSSLESSAS ALDR |
| | | | PGTETSDVVCKPCAPGTFSNTTSSTDICR |
| | | | PGTETSDVVCKPCAPGTFSNTTSSTDI CRPHQICNVVAIP GNASMDAVCTSTSPTR |
| P02766 | TTR | Transthyretin | AADDTWEPFASGK |
| | | | AADDTWEPFASGKTSESGELHGLTTEE EFVEGIYK |
| | | | ALGISPFHEHAEVVFTANDSGPR |
| | | | ALGISPFHEHAEVVFTANDSGPRR |
| | | | GSPAINVAVHVFR |
| | | | KAADDTWEPFASGK |
| | | | RYTIAALLSPYSYSTTAVVTNPK |
| | | | RYTIAALLSPYSYSTTAVVTNPKE |
| | | | TSESGELHGL TTEEEFVEGIYK |
| | | | TSESGELHGL TTEEEFVEGIYKVEIDTK |
| | | | YTIAALLSPYSYSTTAVVTNPK |
| | | | YTIAALLSPYSYSTTAWTNPKE |
| P19320 | VCAM1 | Vascular cell adhesion protein 1 | CSVADVYPFDR |
| | | | DPEIEMSGGLVNGSSVTVSCK |
| | | | DPEIHLSGPLEAGK |
| | | | DPEIHLSGPLEAGKPITVK |
| | | | DTTVLVSPSSILEEGSSVNMTCLSQGFP APK |
| | | | EGDTVIISCTCGNVPETWIILK |
| | | | ELQVYISPK |
| | | | EVELIIQVTPK |
| | | | EVELIVQEKPFTVEISPGPR |
| | | | EVELIVQEK |
| | | | EVELIVQEKPFTVEISPGPR |
| | | | GETILENIEFLEDTDMK |

(continued)

| Protein ID | Gene name | Protein name | Peptide sequence |
|---|---|---|---|
| | | | GIQVEIYSFPK |
| | | | GIQVELYSFPR |
| | | | GIQVELYSFPRDPEIEMSGGLVNGSSVTVSCK |
| | | | KLDNGNLQHLSGNATLTLIAMR |
| | | | LDNGNLQHLSGNATLTLIAMR |
| | | | LEIDLLK |
| | | | LEIELLK |
| | | | LHIDDMEFEPK |
| | | | LHIDEMDSVPTVR |
| | | | LQEGGSVTMTCSSEGLPAPEIFWSK |
| | | | MEDSGIYVCEGVNLIGK |
| | | | MEDSGVYLCEGINQAGR |
| | | | NTVISVNPSTK |
| | | | PFTVEISPGPR |
| | | | QLPNGELQPLSENATLTLISTK |
| | | | QSTQTLYVNVAPR |
| | | | SEGTNSTLTLSPVSFENEHSYLCTVTCGHK |
| | | | SLEMTFIPTIEDTGK |
| | | | SLEVTFTPVIEDIGK |
| | | | SLTLDVQGR |
| | | | SQEFLEDADR |
| | | | VPSVYPLDR |
| | | | VRSEGTNSTLTLSPVSFENEHSYLCTVTCGHK |
| | | | VTNEGTTSTLTMNPVSFGNEHSYLCTATCESR |
| | | | YLAQIGDSVSLTCSTTGCESPFFSWR |
| Q9Y279 | VSIG4 | V-set and immunoglobulin domain-containing protein 4 | GDVNLPCTYDPLQGYTQVLVK |
| | | | GSDPVTIFLR |
| | | | GSPPISYIWYK |
| | | | ISLQCQAR |
| | | | LSVSKPTVTTGSYGFTVPQGMR |
| | | | PAVIADSGSYFCTAK |
| | | | PTVTTGSYGFTVPQGMR |
| | | | SHYTCEVTWQTPDGNQWR |
| | | | VATLSTLLFK |

184

(continued)

| Protein ID | Gene name | Protein name | Peptide sequence |
|---|---|---|---|
|  |  |  | VPGDVSLQLSTLEMDDR |
| P04275 | VWF | von Willebrand factor | AFVLSSVDELEQQR |
|  |  |  | AFVVDMMER |
|  |  |  | AHLLSLVDVMQR |
|  |  |  | ALSVVWDR |
|  |  |  | AMYSIDINDVQDQCSCCSPTR |
|  |  |  | APTCGLCEVAR |
|  |  |  | AVSPLPYLR |
|  |  |  | AVVILVTDVSVDSVDAAADAAR |
|  |  |  | CHPLVDPEPFVALCEK |
|  |  |  | CLPSACEVVTGSPR |
|  |  |  | CLPTACTIQLR |
|  |  |  | CMVQVGVISGFK |
|  |  |  | CVDGCSCPEGQLLDEGLCVESTECPCVHSGK |
|  |  |  | DCQDHSFSIVIETVQCADDR |
|  |  |  | DCQDHSFSIVIETVQCADDRDAVCTR |
|  |  |  | DETHFEVVESGR |
|  |  |  | DETLQDGCDTHFCK |
|  |  |  | DGTVTTDWK |
|  |  |  | EAPDLVLQR |
|  |  |  | ECLCGALASYAAACAGR |
|  |  |  | EEVFIQQR |
|  |  |  | EFMEEVIQR |
|  |  |  | EGGPSQIGDALGFAVR |
|  |  |  | ENGYECEWR |
|  |  |  | EQAPNLVYMVTGNPASDEIK |
|  |  |  | EQAPNLVYMVTGNPASDEIKR |
|  |  |  | EQDLEVILHNGACSPGAR |
|  |  |  | FNHLGHIFTFTPQNNEFQLQLSPK |
|  |  |  | FSEEACAVLTSPTFEACHR |
|  |  |  | GEYFWEK |
|  |  |  | GLQPTLTNPGECR |
|  |  |  | GLQPTLTNPGECRPNFTCACR |
|  |  |  | GLWEQCQLLK |
|  |  |  | GLYLETEAGYYK |
|  |  |  | GQVYLQCGTPCNLTCR |
|  |  |  | HCDGNVSSCGDHPSEGCFCPPDK |

(continued)

| Protein ID | Gene name | Protein name | Peptide sequence |
|---|---|---|---|
| | | | HGAGVAMDGQDVQLPLLK |
| | | | HIVTFDGQNFK |
| | | | HLSISVVLK |
| | | | HSALSVELHSDMEVTVNGR |
| | | | IDGSGNFQVLLSDR |
| | | | IEDLPTMVTLGNSFLHK |
| | | | IGWPNAPILIQDFETLPR |
| | | | ILAGPAGDSNVVK |
| | | | ILDELLQTCVDPEDCPVCEVAGR |
| | | | ILTSDVFQDCNK |
| | | | IPGTCCDTCEEPECNDITAR |
| | | | ITLLLMASQEPQR |
| | | | KVIVIPVGIGPHANLK |
| | | | KWNCTDHVCDATCSTIGMAHYLTFDGLK |
| | | | LLDLVFLLDGSSR |
| | | | LPGDIQVVPIGVGPNANVQELER |
| | | | LSEAEFEVLK |
| | | | LSGEAYGFVAR |
| | | | LSYGEDLQMDWDGR |
| | | | LTGSCSYVLFQNK |
| | | | LTQVSVLQYGSITTIDVPWNVVPEK |
| | | | LVCPADNLR |
| | | | LVSVPYVGGNMEVNVYGAIMHEVR |
| | | | MEACMLNGTVIGPGK |
| | | | NSMVLDVAFVLEGSDK |
| | | | NSQWICSNEECPGECLVTGQSHFK |
| | | | NVSCPQLEVPVCPSGFQLSCK |
| | | | PGQTCQPILEEQCLVPDSSHCQVLLLPLFAECHK |
| | | | QNADQCCPEYECVCDPVSCDLPPVPHCER |
| | | | RDETLQDGCDTHFCK |
| | | | RLPGDIQVVPIGVGPNANVQELER |
| | | | RNSMVLDVAFVLEGSDK |
| | | | RPGDVWTLPDQCHTVTCQPDGQTLLK |
| | | | RPMKDETHFEVVESGR |
| | | | SEVEVDIHYCQGK |
| | | | SFSIIGDFQNGK |

(continued)

| Protein ID | Gene name | Protein name | Peptide sequence |
|---|---|---|---|
| | | | SFSIIGDFQNGKR |
| | | | SGFTYVLHEGECCGR |
| | | | SLSYPDEECNEACLEGCFCPPGLYMDER |
| | | | STIYPVGQFWEEGCDVCTCTDMEDAVMGLR |
| | | | SVGSQWASPENPCLINECVR |
| | | | TATLCPQSCEER |
| | | | TCAQEGMVLYGWTDHSACSPVCPAGMEYR |
| | | | TCGLCGNYNGNQGDDFLTPSGLAEPR |
| | | | TCQNYDLECMSMGCVSGCLCPPGMVR |
| | | | TCQSLHINEMCQER |
| | | | TEPMQVALHCTNGSVVYHEVLNAMECK |
| | | | TLCECAGGLECACPALLEYAR |
| | | | TLVQEWTVQR |
| | | | TLVQEWTVQRPGQTCQPILEEQCLVPDSSHCQVLLLPLFA ECHK |
| | | | TNGVCVDWR |
| | | | TNTGLALR |
| | | | TPDFCAMSCPPSLVYNHCEHGCPR |
| | | | TTCNPCPLGYK |
| | | | TVMIDVCTTCR |
| | | | TYGLCGICDENGANDFMLR |
| | | | VAVVEYHDGSHAYIGLK |
| | | | VCGLCGNFDGIQNNDLTSSNLQVEEDPVDFGNSWK |
| | | | VEDFGNAWK |
| | | | VIVIPVGIGPHANLK |
| | | | VKEEVFIQQR |
| | | | VLAPATFYAICQQDSCHQEQVCEVIASYAHLCR |
| | | | VMLEGSCVPEEACTQCIGEDGVQHQFLEAWVPDHQPCQICTCLSGR |
| | | | VPLDSSPATCHNNIMK |
| | | | VTGCPPFDEHK |
| | | | VTILVEGGEIELFDGEVNVK |
| | | | VTVFPIGIGDR |

(continued)

| Protein ID | Gene name | Protein name | Peptide sequence |
|---|---|---|---|
| | | | WNCTDHVCDATCSTIGMAHYLTFDGLK |
| | | | WTCPCVCTGSSTR |
| | | | YAGSQVASTSEVLK |
| | | | YFTFSGICQYLLAR |
| | | | YIILLLGK |
| | | | YLFPGECQYVLVQDYCGSNPGTFR |
| | | | YLSDHSFLVSQGDR |
| | | | YLSDHSFLVSQGDREQAPNLVYMVTGNPASDEIK |
| | | | YNSCAPACQVTCQHPEPLACPVQCVEGCHAHCPPGK |
| | | | YTLFQIFSK |
| Q14508 | WFDC2 | WAP four-disulfide core domain protein 2 | CCSAGCATFCSLPNDK |
| | | | DQCQVDSQCPGQMK |
| | | | EGSCPQVNINFPQLGLCR |
| | | | TGVCPELQADQNCTQECVSDSECADNLK |

**Claims**

1. A method of *in vitro* diagnosing a systemic inflammation or prognosing a risk of mortality of a subject with a systemic inflammation, wherein the method comprises

   a) determining the level of soluble V-set and immunoglobulin domain-containing protein 4 (sVSIG4) in a biological sample, and
   b) drawing a conclusion as to the diagnosis of a systemic inflammation or the prognosis of a risk of mortality of a subject with a systemic inflammation from the presence and/or level of sVSIG4.

2. The method according to claim 1, wherein the systemic inflammation is caused by an infectious agent.

3. The method according to claim 2, wherein the systemic inflammation caused by an infectious agent is a sepsis, a systemic infection, or a bloodstream infection, preferably a sepsis.

4. The method according to claim 2 or 3, wherein the infectious agent is a bacterium, a fungus or a virus.

5. The method according to claim 1, wherein the systemic inflammation is not caused by an infectious agent.

6. The method according to claim 5, wherein the systemic inflammation not caused by an infectious agent is systemic inflammatory reaction syndrome (SIRS).

7. The method according to one or more of claims 1 to 6, wherein sVSIG4 comprises the extracellular domain or a fragment of the extracellular domain of VSIG4.

8. The method according to claim 7, wherein the extracellular domain comprises Ig-like domain 1 (SEQ ID NO: 4), or Ig-like domain 1 (SEQ ID NO: 4) and Ig-like domain-2 (SEQ ID NO: 5), or the extracellular domain as defined in SEQ ID NO: 6.

9. The method according to one or more of claims 1 to 8, wherein the method further comprises:

   - determining the level of one or more additional biomarkers in the biological sample; and
   - in step b) drawing a conclusion as to the diagnosis of a systemic inflammation or the prognosis of a risk of mortality of a subject with a systemic inflammation from the presence and/or level of sVSIG4 in combination with the presence and/or level of the one or more additional biomarkers.

10. The method according to claim 9, wherein the one or more additional biomarkers in the biological sample are selected from one or more of the following:

   (i) Inter-alpha-trypsin inhibitor heavy chain H2 (ITIH2), Inter-alpha-trypsin inhibitor heavy chain H1 (ITIH1), Phosphatidylinositol-glycan-specific phospholipase D (PHLD), N-acetylmuramoyl-L-alanine amidase (PGRP2), Kallistatin (KAIN), Alpha-2-HS-glycoprotein (FETUA), Inter-alpha-trypsin inhibitor heavy chain H3 (ITIH3), Afamin (AFAM), Macrophage mannose receptor 1 (MRC1), Cholinesterase (CHLE), Insulin-like growth factor-binding protein complex acid labile subunit (ALS), Serotransferrin (TRFE), Phosphatidylcholine-sterol acyltransferase (LCAT), Beta-Ala-His dipeptidase (CNDP1), Plasma kallikrein (KLKB1), Alpha-1-antichymotrypsin (AACT), Monocyte differentiation antigen CD14 (CD14), Neutrophil gelatinase-associated lipocalin (NGAL), Hepatocyte growth factor activator (HGFA), Scavenger receptor cysteine-rich type 1 protein M130 (C163A), Fibronectin (FINC), Histidine-rich glycoprotein (HRG), and Alpha-1-antitrypsin (A1AT);
   (ii) Phosphatidylinositol-glycan-specific phospholipase D (PHLD), Kallistatin (KAIN), Alpha-2-HS-glycoprotein (FETUA), Afamin (AFAM), Macrophage mannose receptor 1 (MRC1), Cholinesterase (CHLE), Insulin-like growth factor-binding protein complex acid labile subunit (ALS), Beta-Ala-His dipeptidase (CNDP1), Neutrophil gelatinase-associated lipocalin (NGAL), Scavenger receptor cysteine-rich type 1 protein M130 (C163A), Plasma serine protease inhibitor (IPSP), Leucine-rich alpha-2-glycoprotein (A2GL), Lithostathine-1-alpha (REG1A), Lipopolysaccharide-binding protein (LBP), Lymphatic vessel endothelial hyaluronic acid receptor 1 (LYVE1), Insulin-like growth factor-binding protein 3 (IBP3), Complement receptor type 2 (CR2), Fibronectin (FINC), Asialoglycoprotein receptor 2 (ASGR2), Alkaline phosphatase, tissue-nonspecific isozyme (PPBT), Serum amyloid A-2 protein (SAA2), and Dipeptidylpeptidase 4 (DPP4);
   (iii) Inter-alpha-trypsin inhibitor heavy chain H2 (ITIH2), N-acetylmuramoyl-L-alanine amidase (PGRP2), and Monocyte differentiation antigen CD14 (CD14);
   (iv) Inter-alpha-trypsin inhibitor heavy chain H2 (ITIH2);
   (v) Phosphatidylinositol-glycan-specific phospholipase D (PHLD), Leucine-rich alpha-2-glycoprotein (A2GL), and Insulin-like growth factor-binding protein 3 (IBP3);
   (vi) Phosphatidylinositol-glycan-specific phospholipase D (PHLD);
   (vii) C-reactive protein (CRP);
   (viii) Procalcitonin (PCT);
   (ix) Lithostathine-1-alpha **(REG1A);**
   (x) Myeloblastin (PRTN3);
   (xi) CRP and PCT,

   wherein in step b) a conclusion is drawn as to the diagnosis of a systemic inflammation.

**11.** The method according to claim 9, wherein the one or more additional biomarkers in the biological sample are selected from one or more of the following:

(i) Kininogen-1 (KNG1) and Tenascin (TENA);
(ii) Versican core protein (CSPG2), Cadherin-related family member 2 (CDHR2), EMILIN-2 (EMIL2), Osteopontin (OSTP), Tyrosine-protein phosphatase nonreceptor type substrate 1 (SHPS1), Lithostathine-1-beta (REG1B), Carcinoembryonic antigen-related cell adhesion molecule 6 (CEAM6), Paired immunoglobulin-like type 2 receptor alpha (PILRA), HLA class II histocompatibility antigen gamma chain (HG2A), Scavenger receptor cysteine-rich type 1 protein M130 (C163A), Fibroleukin (FGL2), Follistatin-related protein 3 (FSTL3), Fibromodulin (FMOD), Beta-galactoside alpha-2,6-sialyltransferase 1 (SIAT1), Myeloblastin (PRTN3), Leukocyte immunoglobulin-like receptor subfamily B member 5 (LIRB5), N-acetylmuramoyl-L-alanine amidase (PGRP2), Interleukin-1 receptor-like 1 (ILRL1), Neutrophil gelatinase-associated lipocalin (NGAL), Latent-transforming growth factor beta-binding protein 2 (LTBP2), Interleukin-1 receptor antagonist protein (IL1RA), Chromogranin-A (CMGA), Phosphoinositide-3-kinase-interacting protein 1 (P3IP1), Ribonuclease pancreatic (RNAS1), Ganglioside GM2 activator (SAP3), Neutrophil elastase (ELNE), Adseverin (ADSV), Disintegrin and metalloproteinase domain-containing protein 9 (ADAM9), Lithostathine-1-alpha (REG1A), Nucleobindin-1 (NUCB1), and WAP four-disulfide core domain protein 2 (WFDC2);
(iii) Scavenger receptor cysteine-rich type 1 protein M130 (C163A), Kallistatin (KAIN), Macrophage mannose receptor 1 (MRC1), Lymphatic vessel endothelial hyaluronic acid receptor 1 (LYVE1), Cholinesterase (CHLE), Ribonuclease pancreatic (RNAS1), Phospahtidylinositol-glycan-specific phospholipase D (PHLD), Afamin (AFAM), Insulin-like growth factor-binding protein complex acid labile subunit (ALS), Neutrophil gelatinase-associated lipocalin (NGAL), Phosphoinositide-3-kinase-interacting protein 1 (P3IP1), Alpha-2-HS-glycoprotein (FETUA), Ganglioside GM2 activator (SAP3), Beta-Ala-His-dipeptidase (CNDP1), Paired immunoglobulin-like type 2 receptor alpha (PILRA), CD177 antigen (CD177), V-type proton ATPase subunit S1 (VAS1), Plasma serine protease inhibitor (IPSP), Follistatin-related protein 3 (FSTL3), Pulmonary surfactant-associated protein B (PSPB), Tumor necrosis factor receptor superfamily member 1B (TNR1B), WAP four-disulfide core domain protein 2 (WFDC2), Alkaline phosphatase, tissue-nonspecific isozyme (PPBT), and Metalloproteinase inhibitor 1 (TIMP1);
(iv) C-reactive protein (CRP);
(v) Procalcitonin (PCT);
(vi) Lithostathine-1-alpha;
(vii) CRP and PCT,

wherein in step b) a conclusion is drawn as to the prognosis of a risk of mortality of a subject with a systemic inflammation.

**12.** A method of monitoring a systemic inflammation of a subject, wherein the method comprises:

i) performing the method of *in vitro* diagnosing a systemic inflammation or prognosing a risk of mortality of a subject with a systemic inflammation according to one or more of claims 1 to 11; and
ii) repeating step i) at least one time.

**13.** The method according to claim 12, wherein the method comprises repeating step ii) until diagnosing the absence of the systemic inflammation, or for monitoring the therapeutic success or therapeutic failure.

**14.** An antibiotic agent for use in a method of treating an infection in a subject or treating a subject with a suspected infection, wherein the infection is part of a bloodstream infection, systemic infection or sepsis and wherein the bloodstream infection, systemic infection or sepsis is diagnosed or monitored by the level of sVSIG4 in a biological sample.

**15.** A kit comprising a binding molecule to sVSIG4 and a binding molecule to at least one further biomarker for the quantitative detection of sVSIG4 and the at least one further biomarker.

**Figure 1**

# Figure 2A

Figure 2B

**Figure 2C**

**Figure 2D**

**Figure 2E**

**Figure 3A**

Figure 3B

# Figure 4

# Figure 5

## Figure 6A

## Figure 6B

**Figure 7**

Figure 8

# Figure 9A

## Figure 9B

## Figure 9C

## Figure 9D

Sepsis

MiBi pos

39
33,1%

79
66.9
%

# Figure 10A

## Figure 10B

# Figure 11A

# Figure 11B

**Figure 12A**

Figure 12B

**Figure 12C**

Figure 13A

**Figure 13B**

**Figure 13C**

Figure 14A

Figure 14B

**Figure 14C**

**Figure 14D**

Figure 14E

Figure 14F

## Figure 15A

CRP:VSIG all patients

## Figure 15B

SIRS patients

Figure 15C

Sepsis patients

Figure 15D

All patients

## Figure 15E

### SIRS patients

### Figure 15F

### Sepsis patients

**Figure 16A**

**Figure 16B**

**Figure 16C**

LFQ(GPLD1)

**Figure 16D**

crp.day.sample

**Figure 16E**

**Figure 16F**

## Figure 16G

Figure 17A

Figure 17B

CRP

PCT

**Figure 17C**

**Figure 17D**

SOFA

sVSIG4

Europäisches
Patentamt

European
Patent Office

Office européen
des brevets

# EUROPEAN SEARCH REPORT

**Application Number**

EP 21 16 8225

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/200755 A1 (VANDERBILT UNIVERSITY [US]) 1 November 2018 (2018-11-01) * abstract * * claim 38 * | 15 | INV. G01N33/68 |
| X | US 2015/024969 A1 (LANGLEY R. [US] ET AL.) 22 January 2015 (2015-01-22) * abstract * * paragraphs [0003], [0012], [0111] * * Table 13 (p. 36, 40, 43, 47, 50) * * example 4 * | 1-14 | |
| X | EISFELD A.J. ET AL.: "Multi-platform 'omics analysis of human Ebola virus disease pathogenesis", CELL HOST & MICROBE, vol. 22, no. 6, 13 December 2017 (2017-12-13), pages 817-829, XP055845926, * abstract * * page 817, column 2 - page 818, column 1, paragraph 1 * * figure 4 * * page 822, column 1, line 1 * | 1-4, 12-14 | |
| X | SMALL A.G. ET AL.: "Complement receptor immunoglobulin: a control point in infection and immunity, inflammation and cancer", SWISS MED. WKLY, vol. 146, W14301, 5 April 2016 (2016-04-05), pages 1-9, XP055845606, * abstract * * page 3, column 2 * | 1-4, 12-14 | TECHNICAL FIELDS SEARCHED (IPC) G01N |
| A | US 2009/004755 A1 (LEE S.-W. [US] ET AL.) 1 January 2009 (2009-01-01) * the whole document * | 1-15 | |

The present search report has been drawn up for all claims

| Place of search | Date of completion of the search | Examiner |
|---|---|---|
| Munich | 30 September 2021 | Giry, Murielle |

EPO FORM 1503 03.82 (P04C01)

## ANNEX TO THE EUROPEAN SEARCH REPORT
## ON EUROPEAN PATENT APPLICATION NO.

EP 21 16 8225

This annex lists the patent family members relating to the patent documents cited in the above-mentioned European search report.
The members are as contained in the European Patent Office EDP file on
The European Patent Office is in no way liable for these particulars which are merely given for the purpose of information.

30-09-2021

| Patent document cited in search report | Publication date | Patent family member(s) | Publication date |
|---|---|---|---|
| WO 2018200755 A1 | 01-11-2018 | NONE | |
| US 2015024969 A1 | 22-01-2015 | US 2015024969 A1<br>WO 2013040099 A2 | 22-01-2015<br>21-03-2013 |
| US 2009004755 A1 | 01-01-2009 | EP 2167962 A2<br>US 2009004755 A1<br>WO 2009006347 A2 | 31-03-2010<br>01-01-2009<br>08-01-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Clin. Microbiol. Infect.,* 2020, vol. 26, 142-150
- *Crit. Care,* 2020, vol. 24 (1), 287
- *Anaesthesiol. Intensive Ther.,* 2019, vol. 51, 299-305
- *Int. Arch. Allergy Appl. Immunol.,* 1984, vol. 73, 97-103
- sepsis-3 definition. *JAMA,* 2016, vol. 315, 801-10
- *JAMA,* 2016, vol. 315, 775-87
- *Intensive Care Med.,* 1996, vol. 22, 707-10
- *Jama,* 2016, vol. 315, 762-74